(19) Europäisches Patentamt
European Patent Office
Office européen des brevets

(11) Publication number: 0 333 304 A2

# (12) EUROPEAN PATENT APPLICATION

(21) Application number: 89200910.1

(22) Date of filing: 20.02.85

(51) Int. Cl.[4]: C07D 403/12 , C07D 405/12 , C07D 409/12 , C07D 239/42 , C07D 239/52 , C07D 251/46 , C07D 251/16 , A01N 47/36

Claims for the following Contracting State: ES.

(30) Priority: 01.03.84 US 585170
26.12.84 US 686796
21.02.84 US 581817
11.12.84 US 679145

(43) Date of publication of application:
20.09.89 Bulletin 89/38

(60) Publication number of the earlier application in accordance with Art.76 EPC: 0 155 767

(84) Designated Contracting States:
AT BE CH DE FR GB IT LI LU NL SE

(71) Applicant: E.I. DU PONT DE NEMOURS AND COMPANY
1007 Market Street
Wilmington Delaware 19898(US)

(72) Inventor: Pasteris, Robert James
305 Plymouth Road Fairfax
Wilmington Delaware 19803(US)
Inventor: Thompson, Mark Ewell
34 Austin Circle
Wilmington Delaware 19810(US)
Inventor: Wexler, Barry Arthur
2205 Patwynn Road
Wilmington Delaware 19810(US)

(74) Representative: Hildyard, Edward Martin et al
Frank B. Dehn & Co. European Patent Attorneys Imperial House 15-19 Kingsway
London WC2B 6UZ(GB)

## (54) Herbicidal sulfonamides.

(57) Arylmethanesulfonylureas and arylsulfonylureas of the formula

$$JSO_2NH\overset{O}{\overset{\|}{C}}\underset{R}{N}A \qquad (I)$$

wherein J is an arylmethyl or aryloxy group;
R is H or $CH_3$; and
A is a mono- or bicylic heterocyclic group, e.g. pyrimidyl or triazinyl;
and their agriculturally suitable salts, exhibit herbicidal activity. Some of these compounds may also be used as plant growth regulants.

The compounds may be formulated for use in conventional manner. They may be synthesised by a variety of routes, e.g. by reacting a sulfonyl isocyanate $JSO_2NCO$ with an appropriate heterocyclic amine $H\underset{R}{N}A$.

EP 0 333 304 A2

## HERBICIDAL SULFONAMIDES

### Background of the Invention

This invention relates to novel arylmethane- and arylsulfamoyl- sulfonylureas which are useful as pre- and post-emergence herbicides. The compounds of this invention and their agriculturally suitable salts, are useful as agricultural chemicals such as herbicides.

U.S. Patent Nos. 4,169,719 and 4,127,405 disclose herbicidal benzene and thiophene sulfonamides.

U.S. Patent No. 4,435,206 discloses herbicidal pyridine sulfonamides

U.S. Patent 4,420,325 discloses benzyl sulfonylureas such as:

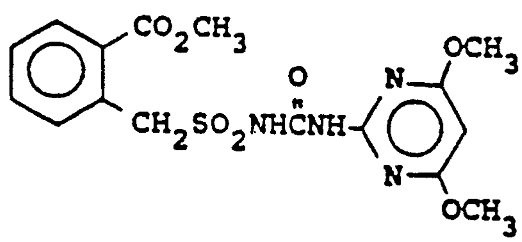

EP-A-87,780 (published September 7, 1983), filed by Nissan Chemical Industries, discloses pyrazole sulfonamides of the general formula shown below.

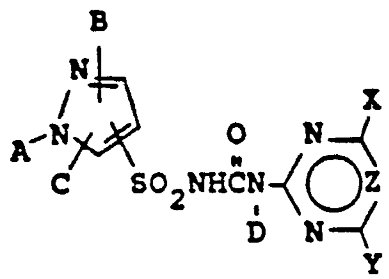

wherein

A is H, $C_1$-$C_8$ alkyl or optionally substituted phenyl;

B and C are independently H, halogen, $NO_2$, $C_1$-$C_8$ alkyl, $CO_2R$, etc.;

D is H or $C_1$-$C_8$ alkyl;

Z is CH or N; and

X and Y are methyl, methoxy, etc.

European Patent Application 71,441, published February 9, 1983, discloses compounds of the formula

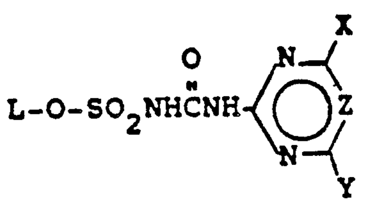

where Z is CH or N; X is $CH_3$ or $OCH_3$; Y is Cl, $CH_3$, $OCH_3$, $OC_2H_5$, $CH_2OCH_3$, $NH_2$, $NHCH_3$, $N(CH_3)_2$, $CH(OCH_3)_2$,

or 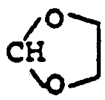 ; and L is 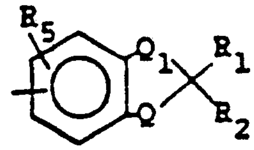 or 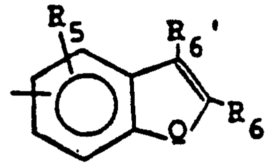

where $R_1$ is H or $C_1$-$C_3$ alkyl; $R_2$ is H or $CH_3$, $R_5$ is H, $CH_3$, $OCH_3$, Cl, Br, $NO_2$, $CO_2R_7$, $SO_2R_8$, $OSO_2R_9$, $SO_2NR_{10}R_{11}$; $R_6$ is H or $C_1$-$C_3$ alkyl; $R_6'$ is H or $CH_3$; Q is O, S or $SO_2$; and $Q_1$ is $CR_3R_4$ or O. Utility as a pre- or post-emergent herbicide and as a plant growth regulant is disclosed.

European Patent Application 79,683, published May 25, 1983, discloses compounds, useful as herbicides or plant growth regulants, of the formula

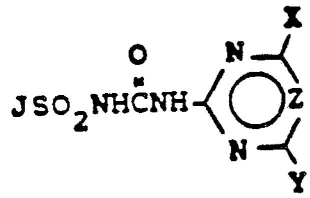

where J is

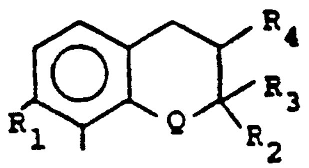 ,

$J_1$

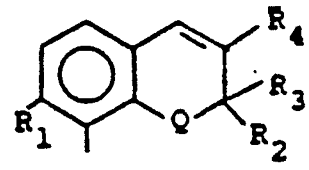 ,

$J_2$

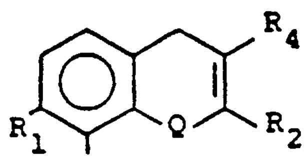 ,

$J_3$

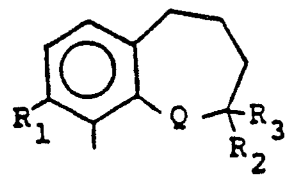

$J_4$

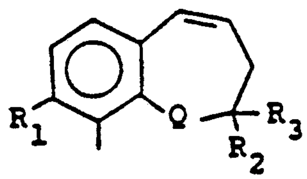 ,

$J_5$

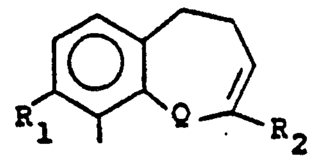

$J_6$

or 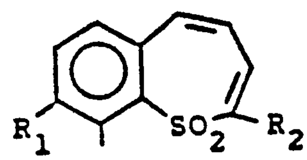 ;

$J_7$

Q is O, S or $SO_2$;
R is H or $CH_3$;
$R_1$ is H, $CH_3$, $OCH_3$, Cl, Br, $CO_2R_5$, $SO_2R_6$, $OSO_2R_7$ or $SO_2NR_8R_9$;
$R_2$ and $R_3$ are independently H or $C_1$-$C_3$ alkyl;
$R_4$ is H or $CH_3$;
$R_5$ is $C_1$-$C_3$ alkyl, $CH_2CH=CH_2$, $CH_2CH_2OCH_3$ or $CH_2CH_2Cl$;
$R_6$ is $C_1$-$C_3$ alkyl;
$R_7$ is $C_1$-$C_3$ alkyl or $CF_3$; and
$R_8$ and $R_9$ are independently $C_1$-$C_2$ alkyl.

Further herbicidal sulfonylureas are disclosed in EP-A-95,925; EP-A-84,224; EP-A-99,339 and EP-A-51,465.

The presence of undesired vegetation causes substantial damage to useful crops, especially agricultural products that satisfy mans' basic food and fiber needs, such as cotton, rice, corn, wheat and the like. The current population explosion and concomitant world food and fiber shortage demand improvements in the efficiency of producing these crops. Preventing or minimizing loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides.

## Summary of the Invention

This invention relates to novel compounds of Formula I, agriculturally suitable compositions containing them and their method-of-use as preemergent and/or postemergent herbicides or as plant growth regulants.

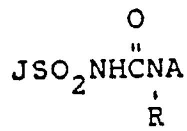

I

wherein

J is

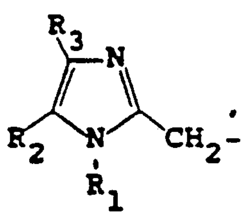,

J-1

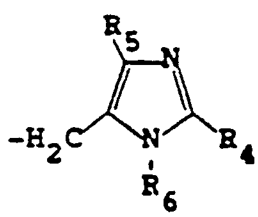,

J-2

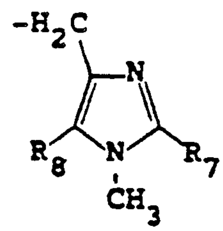,

J-3

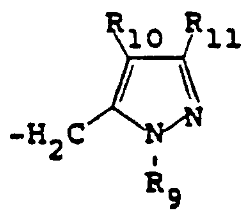,

J-4

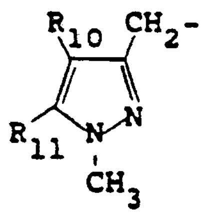,

J-5

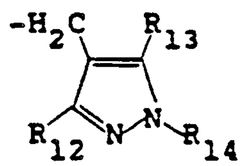,

J-6

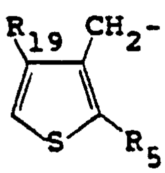,

J-7

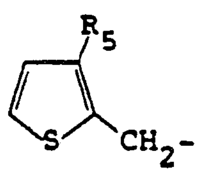,

J-8

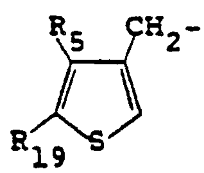,

J-9

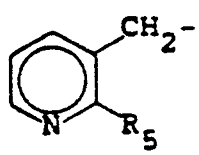 , 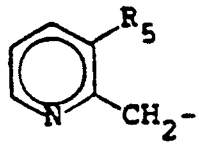 ,

J-10 J-11

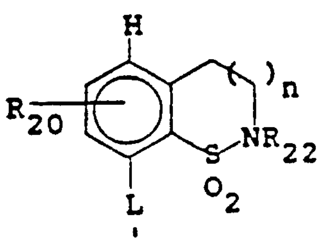 , 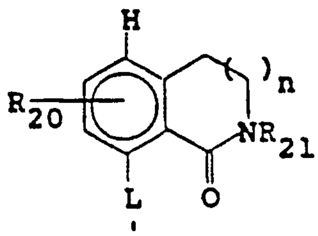 , 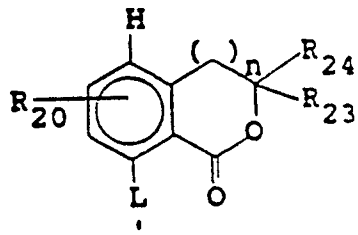 ,

$J_{12}$ $J_{13}$ $J_{14}$

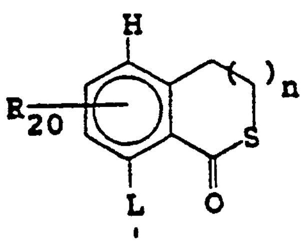 , 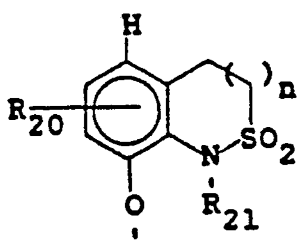 , 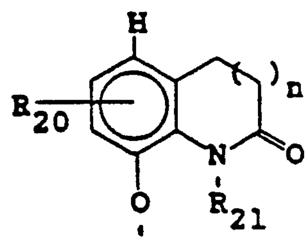 .

$J_{15}$ $J_{16}$ $J_{17}$

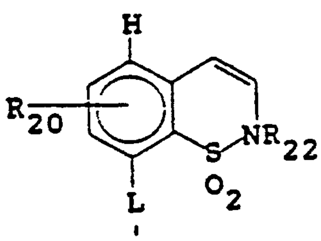 , 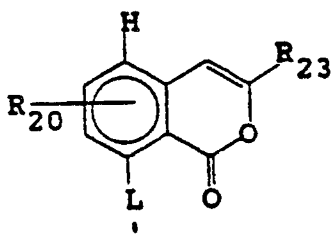 , 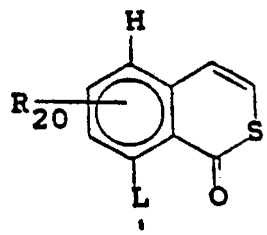 .

$J_{18}$ $J_{19}$ $J_{20}$

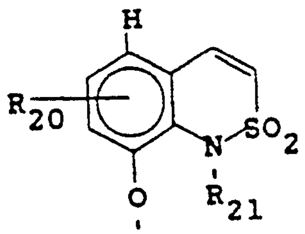

$J_{21}$ ,

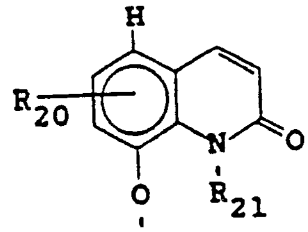

$J_{22}$ ,

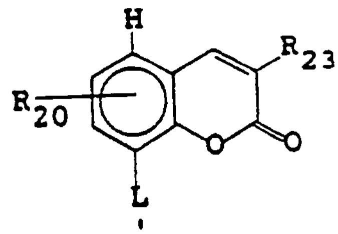

$J_{23}$ ,

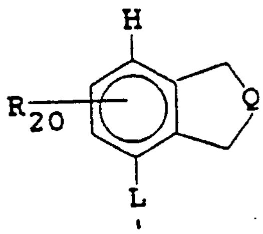

$J_{24}$ ,

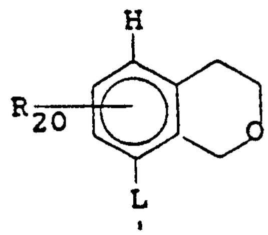

$J_{25}$ ,

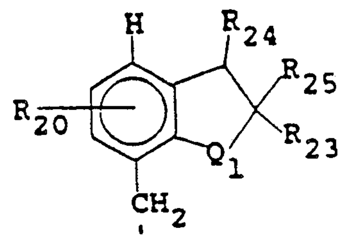

$J_{26}$ ,

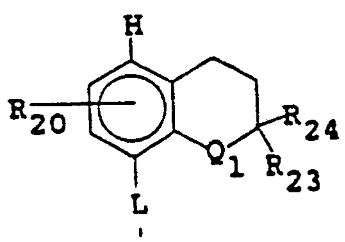

$J_{27}$ ,

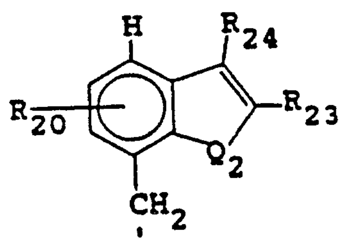

$J_{28}$ ,

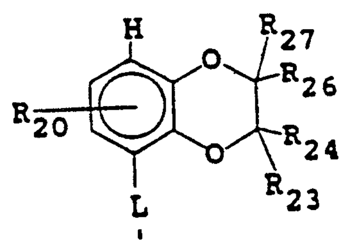

$J_{29}$ ,

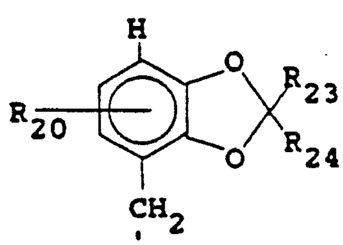

$J_{30}$ ,

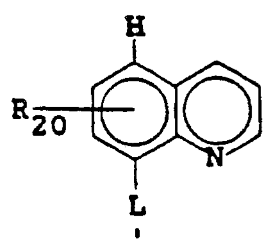

$J_{31}$ ,

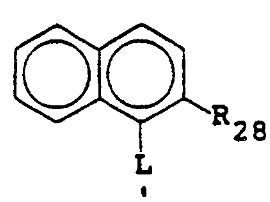

$J_{32}$ ,

n is 0 or 1;
L is $CH_2$ or O;
Q is $CH_2$ or O;
$Q_1$ is O, S or $SO_2$;
$Q_2$ is O or S;
R is H or $CH_3$;

$R_1$ is H, $C_1$-$C_3$ alkyl, $CO_2R_{15}$, $SO_2NR_{16}R_{17}$ or $SO_2R_{18}$;
$R_2$ is H or $CH_3$;
$R_3$ is H or $CH_3$;
$R_4$ is H or $CH_3$;
$R_5$ is H, $CH_3$, Cl, Br, $CO_2R_{15}$, $SO_2NR_{16}R_{17}$, $SO_2N(OCH_3)CH_3$, $SO_2R_{18}$ or $NO_2$;
$R_6$ is H, $C_1$-$C_3$ alkyl, $CO_2R_{15}$, $So_2NR_{16}R_{17}$ or $SO_2R_{18}$;
$R_7$ is H or $CH_3$;
$R_8$ is $CO_2R_{15}$, $SO_2NR_{16}R_{17}$, $SO_2N(OCH_3)CH_3$, $SO_2R_{18}$ or $NO_2$;
$R_9$ is H, $C_1$-$C_3$ alkyl or phenyl;
$R_{10}$ is $CO_2R_{15}$, $SO_2NR_{16}R_{17}$, $SO_2N(OCH_3)CH_3$, $SO_2R_{18}$ or $NO_2$;
$R_{11}$ is H or $CH_3$;
$R_{12}$ is H, $CO_2R_{15}$, $SO_2NR_{16}R_{17}$, $SO_2N(OCH_3)CH_3$, $SO_2R_{18}$ or $NO_2$;
$R_{13}$ is H, $CO_2R_{15}$, $SO_2NR_{16}R_{17}$, $SO_2N(OCH_3)CH_3$, $SO_2R_{18}$ or $NO_2$;
$R_{14}$ is H or $CH_3$;
$R_{15}$ is $C_1$-$C_2$ alkyl;
$R_{16}$ is $C_1$-$C_2$ alkyl;
$R_{17}$ is $C_1$-$C_2$ alkyl;
$R_{18}$ is $C_1$-$C_2$ alkyl;
$R_{19}$ is $CH_3$, Cl, Br, $NO_2$, $C_1$-$C_2$ alkylthio or $C_1$-$C_2$ alkylsulfonyl;
$R_{20}$ is H, F, Cl, Br, $CH_3$ or $OCH_3$;
$R_{21}$ is H or $C_1$-$C_3$ alkyl;
$R_{22}$ is H, OH, $C_1$-$C_6$ alkyl, $C_1$-$C_6$ haloalkyl, $C_1$-$C_6$ alkoxylalkyl, $C_3$-$C_6$ alkenyl, $C_3$-$C_6$ alkynyl, $C_1$-$C_6$ alkoxy, phenyl, $C_3$-$C_6$ cycloalkyl, $C_4$-$C_7$ cycloalkylalkyl, $C_1$-$C_6$ alkylcarbonyl, $C_1$-$C_6$ alkoxycarbonyl, benzyl, $C_3$-$C_4$ haloalkenyl, $C_3$-$C_6$ haloalkynyl, $C_3$-$C_6$ alkylcarbonylalkyl, $C_3$-$C_6$ alkoxycarbonylalkyl or $C_1$-$C_4$ cyanoalkyl;
$R_{23}$ is H or $CH_3$;
$R_{24}$ is H or $CH_3$;
$R_{25}$ is H, $CH_3$ or $C_2H_5$;
$R_{26}$ is H or $CH_3$;
$R_{27}$ is H or $CH_3$;
$R_{28}$ is F, Cl, Br, $OCH_3$, $SCH_3$, $SO_2CH_3$, $CO_2CH_3$, $SO_2N(CH_3)_2$ or $OSO_2CH_3$;

A is 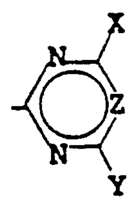 , 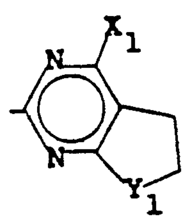 , 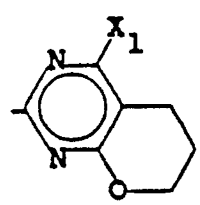 , 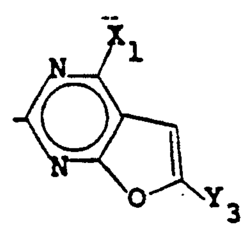 ,

A-1 A-2 A-3 A-4

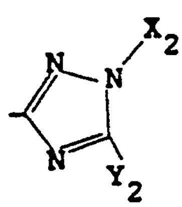 , 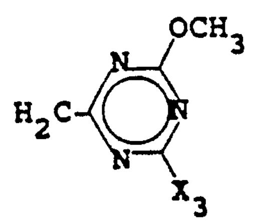 ;

A-5 A-6

X is $CH_3$, $OCH_3$, Cl, Br, $OCH_2CF_3$ or $OCHF_2$;
Y is $C_1$-$C_3$ alkyl, $CH_2F$, cyclopropyl, C≡CH, $OCH_3$, $OC_2H_5$, $NH_2$, $NHCH_3$, $N(CH_3)_2$, $OCH_2CH_2F$, $CF_3$, $SCH_3$, $OCH_2CH=CH_2$, $OCH_2C$≡CH, $OCH_2CH_2OCH_3$, or $OCF_2H$;
Z is CH or N;
$X_1$ is $CH_3$, $OCH_3$, $OC_2H_5$ or $OCF_2H$;
$Y_1$ is O or $CH_2$;

$X_2$ is $CH_3$, $C_2H_5$ or $CH_2CF_3$;
$Y_2$ is $OCH_3$, $OC_2H_5$, $SCH_3$, $SC_2H_5$, $OCF_2H$, $SCF_2H$, $CH_3$ or $CH_2CH_3$;
$X_3$ is $CH_3$ or $OCH_3$;
$Y_3$ is H or $CH_3$; and agriculturally suitable salts thereof; provided that

1) when X is Cl or Br, then Z is CH and Y is $OCH_3$, $OC_2H_5$, $NHCH_3$, $N(CH_3)_2$ or $OCF_2H$;
2) when X or Y is $OCF_2H$, then Z is CH;
3) $R_5$ and $R_6$ are not simultaneously H;
4) when $R_6$ is other than H or $C_1$-$C_3$ alkyl, then $R_5$ must be H;
5) $R_{12}$ and $R_{13}$ are not simultaneously H;
6) when $R_{12}$ is other than H, then $R_{13}$ must be H;
7) $R_5$ and $R_{19}$ are not simultaneously H; and
8) when J is J-8 or J-11 then $R_5$ is other than H.

Preferred Compounds

Preferred for reasons of their higher herbicidal activity, greater plant growth regulant activity or more favorable ease of synthesis are:

1) Compounds of Formula I where R is H and A is A-1.
2) Compounds of Preferred 1 where Y is $CH_3$, $C_2H_5$, $OCH_3$, $OC_2H_5$, $CH_2F$ or $CF_3$; and L is $CH_2$.
3) Compounds of Preferred 2 where J is $J_{12}$, $J_{14}$, $J_{18}$, $J_{19}$, $J_{26}$ or $J_{27}$.
4) Compounds of Preferred 3 where $R_{20}$ is H and X is $CH_3$ or $OCH_3$.
5) Compounds of Preferred 1 where J is J-1, J-4, J-5 or J-6; X is $CH_3$ or $OCH_3$; and Y is $C_1$-$C_2$ alkoxy or $C_1$-$C_2$ alkyl.

Specifically Preferred for reasons of their highest herbicidal activity, greatest plant growth regulant activity and/or most favourable ease of synthesis are:

- 1,3-dihydro-N-[(4,6-dimethoxypyrimidin-2-yl)aminocarbonyl]-1-oxo-benzo[C]furan-7-methanesulfonamide, m.p. 181-184° C(d);
- 1,3-dihydro-N-[(4-methoxy-6-methyl-1,3,5-triazin-2-yl)aminocarbonyl]-1-oxo-benzo[C]furan-7-methanesulfonamide, m.p. 175-183° C(d);
- N-[(4,6-dimethoxypyrimidin-2-yl)aminocarbonyl]-2,3-dihydro-2-methylbenzo[B]thiophene-7-methanesulfonamide-1,1-dioxide, m.p. 222-222.5° C;
- N-[(4-methoxy-6-methyl-1,3,5-triazin-2-yl)aminocarbonyl]-2-methylbenzo[B]thiophene-7-methanesulfonamide-1,1- dioxide, m.p. 186-187° C;
- 3-[[(4,6-dimethoxypyrimidin-2-yl)aminocarbonyl]-aminosulfonylmethyl]-1,5-dimethyl-1H-pyrazole-4-carboxylic acid, ethyl ester, m.p. 180-183° C; and
- 5-[[(4,6-dimethoxypyrimidin-2-yl)aminocarbonyl]-aminosulfonylmethyl]-1,3-dimethyl-1H-pyrazole-4-carboxylic acid, ethyl ester, m.p. 190° C.

## DETAILED DESCRIPTION OF THE INVENTION

### Synthesis

The compounds of Formula I can be prepared by one or more of the methods described below in Equations 1, 2, 3 and 4. Preferred conditions and reagents are given below for each reaction, but these may be varied within the scope conventional in the art.

As shown in Equation 1, compounds of Formula I can be prepared by reacting a sulfonyl isocyanate of Formula II with an appropriate heterocyclic amine of Formula III. J, R and A are as previously defined, $R_{21}$ is $C_1$-$C_3$ alkyl and $R_{22}$ is other than H or OH.

Equation 1

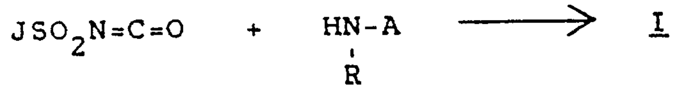

II III

The reaction is preferably carried out at 25° to 100°C in an inert, aprotic solvent such as methylene chloride or xylene for 0.5 to 24 hours as taught in U.S. Patent 4,127,405.

Compounds of Formula I, where J is other than $J_{14}$, $J_{15}$, $J_{16}$, $J_{17}$, $J_{19}$, $J_{20}$, $J_{21}$, $J_{22}$ and $J_{23}$, and where L is $-CH_2-$, and where $R_1$, $R_5$, $R_6$, $R_8$, $R_{10}$, $R_{12}$ and $R_{13}$ of J are other than $CO_2R_{15}$, can be prepared by reacting the sulfonamides of Formula IV with an appropriate methyl carbamate of Formula V in the presence of an equimolar or greater amount of trimethylaluminum, as shown in Equation 2.

Equation 2

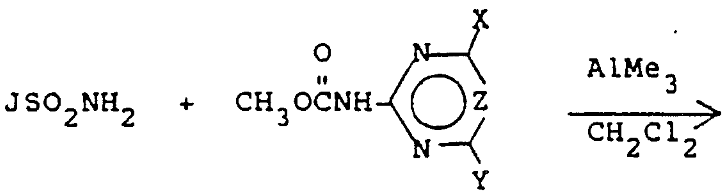


IV V

The reaction Equation 2 is preferably carried out at 25° to 40°C in a solvent such as methylene chloride under an inert atmosphere, as taught in our EP-A-84,244 (published July 27, 1983). The required carbamates V are prepared by reacting the corresponding amines III with dimethylcarbonate or methyl chloroformate in the presence of a base.

Alternatively, compounds of Formula I, where J is other than $J_{16}$, $J_{17}$, $J_{21}$, and $J_{22}$, and where L is $-CH_2-$, can be prepared by reacting a sulfonyl carbamate of Formula VI with an appropriate amine of Formula III, as shown in Equation 3.

Equation 3

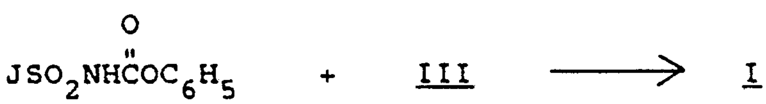

VI

The reaction is preferably carried out at 50° to 100°C in a solvent such as dioxane for 0.5 to 24 hours as taught for example in our EP-A-44,807. The required carbamates VI are prepared by reacting the corresponding sulfona mides IV with diphenylcarbonate in the presence of a strong base.

Additionally, compounds of Formula I, where J is other than $J_{16}$, $J_{17}$, $J_{21}$ and $J_{22}$, and L is $-CH_2-$, can be prepared by reacting a sulfonamide of Formula IV with an appropriate heterocyclic phenyl carbamate of Formula VII, in the presence of a nonnucleophilic base, such as 1,8-diazabicyclo-[5.4.0]undec-7-ene (DBU) as shown below in Equation 4.

Equation 4

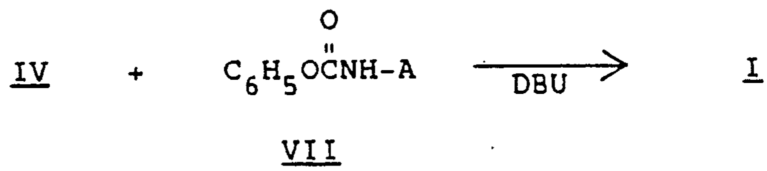

IV I VII

The reaction is preferably carried out at 20° to 100° C in an inert solvent, such as dioxane, for 0.5 to 24 hours by the methods taught in EP-A-44,807. The required carbamates, VII, are prepared by reacting the corresponding heterocyclic amines, III, with phenylchloroformate in the presence of a base.

Compounds of Formula I, where J is other than $J_1$, $J_2$, $J_3$, $J_4$, $J_5$, $J_6$, $J_7$, $J_8$, $J_9$, $J_{10}$, $J_{11}$, $J_{26}$, $J_{28}$ and $J_{30}$, and where L is 0, can be prepared as shown below in Equation 5. The intermediate chlorosulfonylurea of Formula VIII is formed by treatment of the corresponding heterocyclic amine, III, with commercially available chlorosulfonyl isocyanate and is then allowed to react with an appropriate phenol of Formula IX.

Equation 5

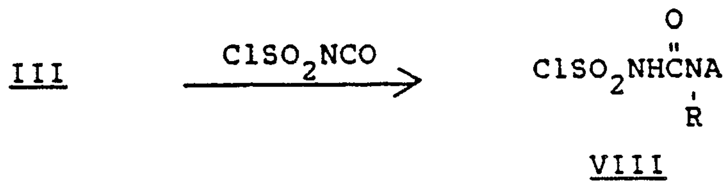

III VIII

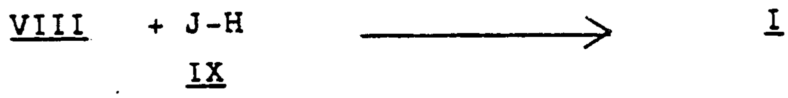
VIII IX I

wherein
J and A are as previously defined.

The reaction shown in Equation 5 is best carried out according to the methods taught in United States Patent 4,391,976.

The intermediate sulfonyl isocyanates of Formula II in Equation 1 can be prepared as shown in Equations 6 and 7.

As shown in Equation 6, sulfonyl isocyanates of Formula II where J is other than $J_1$, $J_2$, $J_3$ and $J_{30}$, and $R_{21}$ is $C_1$-$C_3$ alkyl, $R_{22}$ is other than hydrogen and hydroxy, and where L is $-CH_2-$, can be prepared by the reaction of sulfonamides of Formula IV with phosgene, in the presence of n-butyl isocyanate and a tertiary amine catalyst, such as diazabicyclo-[2.2.2]octane (DABCO®), at reflux in a solvent such as xylene by the method of U.S. Patent 4,238,621.

Equation 6

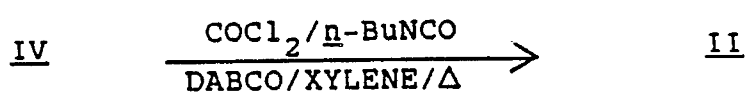

IV II

The sulfonyl isocyanates can also be prepared from the sulfonamides by a two step procedure involving (a) reacting the sulfonamides with n-butyl isocyanate in the presence of a base such as $K_2CO_3$ at reflux in an inert solvent such as 2-butanone forming a n-butyl sulfonylurea; and (b) reacting this compound with phosgene and a tertiary amine catalyst at reflux in xylene solvent. The method is similar to a procedure taught by Ulrich and Sayigh, Newer Methods of Preparative Organic Chemistry, Vol. VI, p. 223-241, Academic Press, New York and London, W. Foerst Ed.

Alternatively, as shown in Equation 7, the sulfonyl isocyanates of Formula II can be prepared by reacting the corresponding sulfonyl chlorides X with cyanic acid salts.

Equation 7

$$\underset{\underline{X}}{JSO_2Cl} \xrightarrow{M^{\oplus}OCN^{\ominus}} \underline{II}$$

The reaction is carried out at 25° to 100° C in an inert aprotic solvent such as acetonitrile for 0.5-24 hours in the presence of phosphorus pentoxide and an alkali metal salt such as lithium iodide according to the teachings of Japanese Patent No. 76/26,816 (Chem. Abst., 85:77892e (1976)).

Another method for the preparation of sulfonyl isocyanates II is shown in Equation 8. Treatment of sulfonamides of Formula IV with thionyl chloride gives intermediate N-sulfinylsulfonamides, XI, which afford sulfonyl isocyanates of Formula II upon exposure to phosgene in the presence of a catalytic amount of pyridine.

Equation 8

$$\underset{\underline{IV}}{JSO_2NH_2} \xrightarrow[\Delta]{SOCl_2} \underset{\underline{XI}}{JSO_2NSO}$$

$$\underline{XI} \xrightarrow[\text{cat. pyridine, toluene}]{COCl_2} \underset{\underline{II}}{JSO_2NCO}$$

wherein

J is as previously defined.

The reaction of Equation 8 can best be performed according to the procedure of H. Ulrich, B. Tucker, and A. Sayigh, J. Org. Chem., 34, 3200 (1969).

Sulfonyl isocyanates of Formula II, where J is other than $J_1$-$J_{11}$, $J_{26}$, $J_{28}$ or $J_{30}$, $R_{21}$ is $C_1$-$C_3$ alkyl, $R_{22}$ is other than hydrogen and hydroxy, and L is 0, can be most easily prepared as shown below in Equation 9 by the reaction of appropriately substituted phenols of Formula IX with chlorosulfonyl isocyanate. This procedure is taught in United States Patents 4,191,553 and 4,394,153, and by Lohaus in Chem. Ber., 105, 2791 (1972).

Equation 9

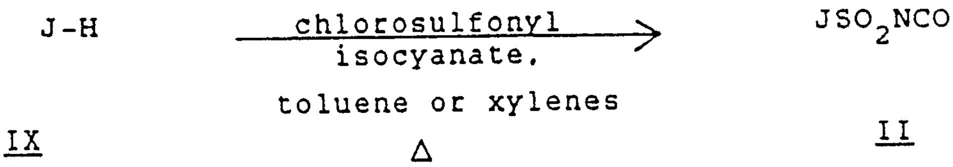


IX II

wherein
J is as previously defined.

As shown in Equation 10, sulfonamides of Formula IV can be prepared from the corresponding sulfonyl chlorides of Formula X by contacting with either anhydrous or aqueous ammonia.

Equation 10

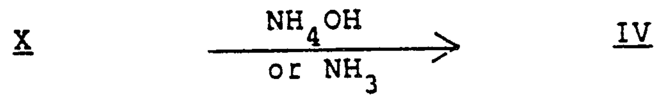


The preparation of sulfonamides from sulfonyl chlorides is widely reported in the literature, for reviews see: F. Hawking and J. S. Lawrence, "The Sulfonamides," H. K. Lewis and Co., London, 1950 and E. H. Northey, "The Sulfonamides and Allied Compounds," Reinhold Publishing Corp., New York, 1948.

The requisite methylsulfonyl chlorides of Formula X may be synthesized from appropriately substituted benzylic type chlorides or benzylic type bromides, XII, by the two-step sequence of reactions outlined below in Equation 11 and Equation 12.

Equation 11

(a)

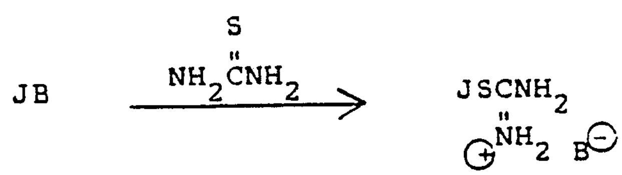

XII XIII

(b)

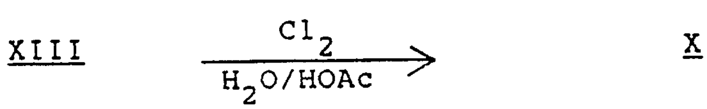


wherein
B is Cl or Br; and
J is $J_1$-$J_{11}$, $J_{12}$, $J_{13}$, $J_{14}$, $J_{24}$, $J_{25}$, $J_{26}$, $J_{27}$, $J_{31}$ or $J_{32}$;
L is $-CH_2-$, and
when J is $J_{26}$ or $J_{27}$, $Q_1$ is $SO_2$.

Equation 12

(a)

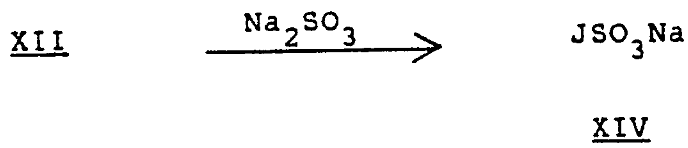


(b)

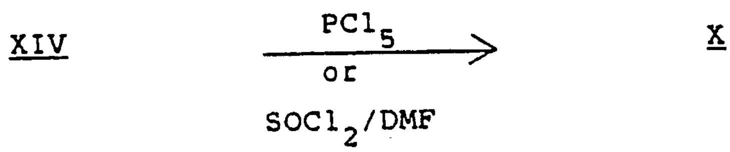


B is Cl or Br;
J is other than $J_{13}$, $J_{16}$, $J_{17}$, $J_{21}$ or $J_{22}$; and
L is $-CH_2-$.

Equation 11(a)

The conversion of alkyl halides to isothiouronium salts is well precedented in the chemical literature. For relevant examples, see T. B. Johnson and J. M. Sprague, J. Am. Chem. Soc., 58, (1936); ibid, 59, 1837 and 2439 (1937); ibid, 61, 176 (1939). In a typical procedure, a benzylic type halide of Formula XII is treated with thiourea in protic solvents such as methanol or ethanol, or aprotic solvents such as methylene chloride or benzene. Temperatures of 40-80° over one-half to 24 hours are typically required to complete the reaction. The product salts, XIII, are isolated by cooling and filtration or by concentration to remove the solvent. The salts, XIII, are generally sufficiently pure to be carried on directly to step (11b) without further purification.

Equation 11(b)

The oxidative chlorination of isothiouronium salts to afford sulfonyl chlorides is most efficiently carried out according to the procedure of Johnson as described in J. Am. Chem. Soc., 61, 2548 (1939). Thus, the salts of Formula XIII (B = chlorine or bromine) are dissolved or suspended in water or aqueous acetic acid and treated with at least three equivalents of chlorine at temperatures between 5-20°C. When the hydrobromide salts XIII (B = bromine) are used, it is sometimes advantageous to exchange the bromide ion for nitrate ion before chlorination by treatment with an aqueous solution of one equivalent of silver nitrate; the precipitated silver bromide is removed by filtration and the filtrate treated as described above. The product sulfonyl chlorides are isolated either by simple filtration or extraction into a suitable solvent such as methylene chloride or n-butyl chloride followed by drying and evaporation of the combined organic extracts. No further purification of the sulfonyl chlorides, X, is necessary.

The conversion of a benzylic type chloride or bromide of Formula XII to a sulfonic acid salt of Formula XIV as shown in Equation 12(a) is well known in the art. For a review, see Gilbert "Sulfonation and Related Reactions" Interscience Publishers, New York, 1965, pp. 136-148 and 161-163. The methods of conversion of a sulfonic acid salt of Formula XIV to a sulfonyl chloride of Formula X is well known to those skilled in the art.

Benzylic type bromides of Formula XII (B = bromine) may be prepared as shown below in Equation 13 by treating the appropriate methyl aryl compound IX, with N-bromosuccinimide (NBS).

Equation 13

J-H → (NBS / Catalyst, $CCl_4$, Δ) → J-Br

IX    XII (B is Br)

wherein

J is other than $J_{12}$, $J_{13}$, $J_{14}$, $J_{15}$, $J_{16}$, $J_{17}$, $J_{21}$, $J_{22}$, $J_{24}$, $J_{25}$, $J_{26}$ or $J_{27}$;

L is $-CH_2-$;

$R_{20}$ is H, F, Cl, Br, $OCH_3$; and

when J is $J_{19}$ or $J_{23}$, $R_{23}$ is H, and when J is $J_{28}$, $R_{23}$ and $R_{24}$ are H.

The reaction represented in Equation 13 can be most conveniently effected by heating a solution of the aryl methane derivatives, IX, and N-bromosuccinimide in a suitable solvent such as carbon tetrachloride at reflux temperature. A free radical catalyst such as azoisobutyronitrile (AIBN) or benzoyl peroxide is usually employed to initiate this reaction. When bromination is complete, the cooled reaction mixture is filtered to remove the by-product succinimide and the filtrate is concentrated in vacuo. The benzylic type bromides of Formula XII are often obtained in a sufficiently pure condition for further transformations. If not, they can be purified by vacuum distillation or column chromatography.

Benzylic type chlorides of Formula XII (L = chlorine) may be most efficiently synthesized by one of two methods, both of which are well known in the chemical literature. First, benzylic type chlorides, XII, can be prepared from the appropriate aryl methane derivatives of Formula IX by reaction with N-chlorosuccinimide (NCS). This reaction is depicted below in Equation 14.

Equation 14

IX → (NCS / Catalyst, $CCl_4$, Δ) → XII (B is Cl)

The reaction of Equation 14 can be carried out in a manner analogous to that described for the NBS bromination in Equation 13.

In compounds of Formula IX where J is J-1, J-2 or J-3, ring halogenation may occur together with benzylic-type halogenation in Equations 13 and 14. Subsequent removal of the ring halogen after conversion to the desired sulfonamide IV may be carried out by methods known in the art.

Alternatively, benzylic type chlorides of Formula XII may be prepared from the appropriate benzylic type alcohols of Formula XV as shown below in Equation 15.

Equation 15

JOH → ($SOCl_2$) → XII (B is Cl)

XV

There exists a variety of well known methods for converting alkyl alcohols to the corresponding chlorides. One of the most common procedures involves reaction of the alcohol with thionyl chloride either alone or in the presence of a trace of a suitable base such as pyridine. For relevant examples, see the following references: H. Gilman and J. E. Kirby, J. Am. Chem. Soc., 51, 3475 (1929); H. Gilman and A. P. Hewlett, Rec. Trav. Chim., 51, 93 (1932); and M. S. Newman, J. Am. Chem. Soc., 62, 2295 (1940).

The requisite aryl methanols of Formula XV (shown below in Equation 16 for the specific example of J = $J_{27}$), may be prepared by reduction of an appropriately substituted aryl carboxylic acid of Formula XVI with diborane in a solvent such as tetrahydrofuran.

Equation 16

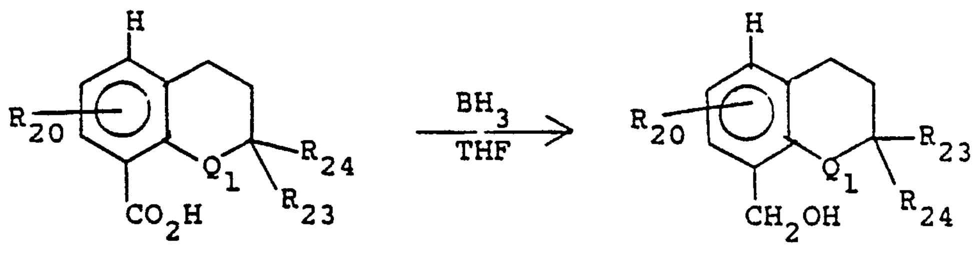


XVI

XV

wherein

$R_{20}$, $R_{23}$, $R_{24}$ and $Q_1$ are as previously defined.

Although the reaction of Equation 16 is shown specifically for $J = J_{27}$, this reduction is applicable to ring systems where J is other than $J_{16}$-$J_{23}$ or $J_{28}$, and where L is $-CH_2-$. For a description of the use of diborane for reduction of benzoic acid derivatives, see H. C. Brown, J. Org. Chem., 38, 2786 (1973).

Alternatively, benzyl alcohols of Formula XV (shown below in Equation 17 for the specific example of $J = J_{27}$) can be synthesized by reduction of the appropriately substituted benzaldehyde derivatives of Formula XVII with sodium borohydride in a solvent such as ethanol.

Equation 17

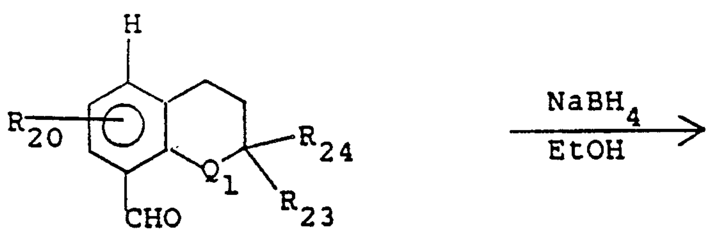


XVII

XV

wherein

$R_{20}$, $R_{23}$, $R_{24}$ and $Q_1$ are as previously defined.

Although the reaction of Equation 17 is shown specifically for $J = J_{27}$, this reduction is applicable to ring systems $J_1$-$J_{15}$, $J_{18}$, $J_{19}$, $J_{20}$, $J_{23}$, $J_{24}$, $J_{25}$, $J_{26}$, $J_{28}$, $J_{29}$, $J_{30}$, $J_{31}$ and $J_{32}$ where L is $-CH_2-$. For a description of the use of sodium borohydride for the selective reduction of aldehydes, see S. Chaikin and W. Brown, J. Am. Chem. Soc., 71, 122 (1949).

The aldehydes of Formula XVII can be prepared by the procedure shown in Equation 18 starting with the appropriate nitriles of Formula XVIII (shown for the specific example of $J = J_{27}$).

Equation 18

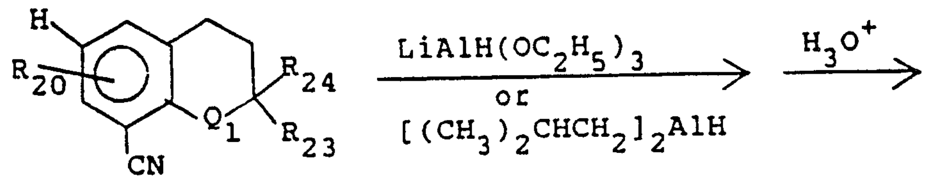

XVII

XVIII

wherein

$R_{20}$, $R_{33}$, $R_{34}$ and $Q_1$ are as previously defined.

Although the reaction of Equation 18 is shown specifically for $J = J_{27}$, this transformation is equally applicable to all of the ring systems ($L = -CH_2-$) except $J_{16}$, $J_{17}$, $J_{21}$, and $J_{22}$. The reaction shown in Equation 18 can be carried out according to the procedures of H. C. Brown, C. J. Shoaf, and C. P. Garg, Tetrahedron Lett., 9 (1959), and J. A. Marshall, N. H. Andersen, and J. W. Schlicher, J. Org. Chem., 35, 858 (1970).

Benzoic acid derivatives of Formula XVI (shown below in Equation 19 for the specific example of $J = J_{27}$) can be prepared by hydrolysis of the appropriate nitriles of Formula XVIII which are, in turn, prepared by a Sandmeyer reaction on the corresponding aniline derivatives XIX.

Equation 19

(a)

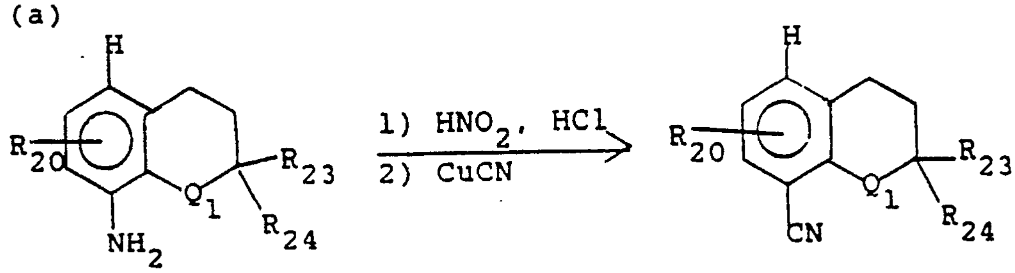

XIX ($J = J_{16}$)

XVIII

(b)

$$\text{XVIII} \xrightarrow{H_3O^+} \text{XVI}$$

wherein

$R_{20}$, $R_{23}$, $R_{24}$ and $Q_1$ are as previously defined.

As before, while the reactions of Equation 19 are shown specifically for $J = J_{27}$, these steps are applicable to all ring systems where J is other than $J_{16}$, $J_{17}$, $J_{21}$ or $J_{22}$, and L is $-CH_2-$.

Equation 19(a)

The diazotization and cuprous cyanide coupling reaction of Equation 19(a) is best carried out according to the procedure described in Organic Syntheses, Coll. Vol. I, p. 514.

Equation 19(b)

Nitriles of Formula XVIII are most easily hydrolyzed to the desired carboxylic acids XVI by heating at reflux temperature in concentrated solutions of sulfuric acid or by treatment with 100% phosphoric acid and

subsequent exposure to nitrous acid. The products are typically isolated by extraction into aqueous base solution followed by washing with a suitable organic solvent such as ether and acidification of the water layer. For a compilation of references dealing with this process, see R. Wagner and H. Zook, "Synthetic Organic Chemistry," John Wiley & Sons, Inc., New York, 1953, p. 412.

A method similar to that described in Equation 19 above can be applied to the synthesis of phenols of Formula IX as shown below in Equation 20 (shown for the specific example of $J = J_{27}$).

Equation 20

**XIX**
1) $HNO_2$, $H_2SO_4$
2) $H_2O$, Δ
**IX**

wherein

$R_{20}$, $R_{23}$, $R_{24}$ and $Q_1$ are as previously defined.

The reaction of Equation 20 is applicable to ring systems $J_1$-$J_{25}$, $J_{27}$, $J_{29}$, $J_{31}$ and $J_{32}$, where L is 0. The diazotization and hydrolysis reactions shown in Equation 20 can be carried out according to the following procedures: Stevens and Beutel, J. Am. Chem. Soc., 63, 311 (1941); Grillot and Gormley, J. Am. Chem. Soc., 67, 1968 (1945); and Mosettig and Stuart, J. Am. Chem. Soc., 61, 1 (1939).

The requisite aryl amine derivatives of Formula XIX can be prepared in a straightforward manner by reduction of the corresponding nitro compounds of Formula XX as shown in Equation 21 (for the example where $J = J_{27}$).

Equation 21

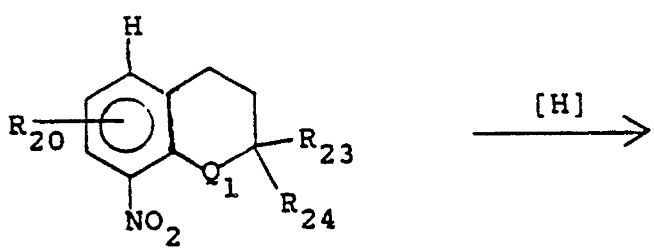


**XIX**

**XX**

wherein

$R_{20}$, $R_{23}$, $R_{24}$, and Q are as previously defined.

Although the reaction of Equation 21 is shown specifically for $J = J_{27}$, this reduction is applicable to all ring systems $J_1$-$J_{32}$. There exists a wide variety of methods for effecting the reduction of aromatic nitro groups to the corresponding aryl amine derivatives. One of the more common procedures involves treating the nitro compounds of Formula XX with a slight excess of stannous chloride dihydrate in concentrated hydrochloric acid solution at temperatures between 25° and 80°C. Alternatively, reduction may be accomplished with iron powder in glacial acetic acid solution as described by Hazlet and Dornfeld, J. Am. Chem. Soc., 66, 1781 (1944), and by West in J. Chem. Soc., 127, 494 (1925). For a general review, see Groggins, "Unit Processes In Organic Synthesis," McGraw-Hill Book Co., New York, 1947, pp. 73-128.

Many of the substituted aromatic nitro compounds of Formula XX (where it is understood that all ring systems defined in the Summary of Invention are represented) can be synthesized by any of several possible synthetic routes known to one skilled in the art.

The intermediate functionalized aryl methane derivatives of Formula IX (L = $-CH_2-$) and aryl methanols of Formula XV (L = $-CH_2-$) may also be prepared by straightforward synthetic routes known to one skilled in the art.

The heterocyclic amines of Formula III can abe prepared by methods known in the literature or simple modifications thereof, by those skilled in the art. For instance, EP-A 84,224 (published July 27, 1983) and W. Braker et al, J. Am. Chem. Soc. 1947, 69, 3072 describe methods for preparing aminopyrimidines and triazines substituted by acetal groups. Also, South African Patent Application Nos. 825,045 and 825,671 describe methods for preparing aminopyrimidines and triazines substituted by haloalkyl or haloalkylthio groups such as $OCH_2CH_2F$, $OCH_2CF_3$, $SCF_2H$, and $OCF_2H$ among other groups. South African Patent Application 837,434 (published October 5, 1983) describes methods for the synthesis of cyclopropylpyrimidines and triazines substituted by such groups as alkyl, haloalkyl, alkoxy, haloalkoxy, alkylamino, dialkylamino, and alkoxyalkyl.

The 5,6-dihydrofuro[2.3-d]pyrimidin-2-amines, and the cyclopenta[d]pyrimidin-2-amines, of formula III, where A is A-2, and the 6,7-dihydro-5H-pyrano[2.3-d]pyrimidin-2-amines, of formula III, where A is A-3, can be prepared as described in EP-A No. 15,863. The furo[2.3-d]-pyrimidin-2-amines, of formula III, where A is A-4, are described in EP-A No. 46,677. Heterocycles of formula III, where A is A-5, may be prepared as described in EP-A No. 73,562. Heterocycles of Formula III, where A is A-6, may be prepared by methods taught in EP-A 94,260.

In addition, general methods for preparing aminopyrimidines and triazines have been reviewed in the following publications.

- "The Chemistry of Heterocyclic Compounds", a series published by Interscience Publishers, Inc., New York and London;
- "Pyrimidines", Vol. 16 of the same series by D. J. Brown;
- "s-Triazines and Derivatives", Vol. 13 of the same series by E. M. Smolin and L. Rappaport; and
- F. C. Schaefer, U.S. Patent 3,154,547 and K. R. Huffman and F. C. Schaefer, J. Org. Chem., 28, 1812 (1963), which describe the synthesis of triazines.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydride). Quaternary amine salts can be made by similar techniques.

Salts of compounds of Formula I can also be prepared by exchange of one cation for another. Cationic exchange can be effected by direct contacting of an aqueous solution of a salt of a compound of Formula I (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., p-toluenesulfonic acid, trichloroacetic acid or the like.

The preparation of the compounds of this invention is illustrated by the following examples.

## Example 1

### Ethyl 5-(chloromethyl)-1,3-dimethyl-1H-pyrazole-4-carboxylate

To 5.3 g (26.8 mmol) of 4-ethoxycarbonyl-5-(1-hydroxymethyl)-1,3-dimethylpyrazole prepared by the method of S. Gelin, et al., J. Heterocyclic Chem., 16, (1979) 1117-20, was added 1.95 ml (26.8 mmol) of thionyl chloride, dropwise. Gas evolution and an exotherm occurred. The solution was cooled with an ice bath. After ten minutes the solution gave a solid, m.p. 81-83 °C, $^1H$ NMR (90 MHz, $CDCl_3$): δ 1.4 (t, $OCCH_3$, 3H); 2.65 (s, $CH_3$, 3H); 4.2 (s, $NCH_3$, 3H); 4.4 (q, $OCH_2C$, 2H); 5.2 (s, $CH_2Cl$, 2H).

This material was combined with the product of a second reaction (5.0 g of 4-ethoxycarbonyl-5-(1-hydroxymethyl)-1,3-dimethylpyrazole and 1.93 ml thionyl chloride), dissolved in dichloromethane, washed with brine, dried ($MgSO_4$) and concentrated to give 10.7 g of a solid, mp. 81-83 °C.

Example 2

Ethyl 5-[[(amino)(imino)methyl]thiomethyl]-1,3-dimethyl-1H-pyrazole-4-carboxylate

A solution under nitrogen was prepared from 10.7 g of the compound produced in Example 1 and 3.95 g of thiourea in 200 ml of ethanol. After refluxing the solution four hours, it was allowed to stir at room temperature overnight. The solvent was removed under reduced pressure. Then, the residue was triturated with hexane to give 16 g of solid (wet weight), m.p. 175-180° C.

Example 3

Ethyl 5-methanesulfonamide-1,3-dimethyl-1H-pyrazole-4-carboxylate

To 14 g of the compound produced in Example 2, dissolved in 90 ml of water at 5° C was added 10 ml of chlorine dropwise. The rate of addition was monitored so that the temperature did not exceed 20° C. A precipitate formed. The aqueous portion was decanted off and the residue was dissolved in dichloromethane, washed with a saturated sodium bicarbonate solution, dried ($MgSO_4$) and concentrated. The residue was dissolved in 100 ml of THF. To a 75 ml portion of the THF solution, cooled to 5° C, was added 1.5 ml of concentrated ammonium hydroxide solution in 10 ml of THF. A suspension formed, which was stirred for six hours. The reaction mixture was filtered and the mother liquor was concentrated under reduced pressure. The residue, triturated with ethyl acetatediethyl ether (40:60) gave 3.0 g of a light yellow solid, m.p. 135-138° C; $^1H$ NMR (90 MHz, $d_6$-DMSO): $\delta$ 1.2 (t, $CH_3$, 3H); 2.3 (s, $CH_3$, 3H); 3.8 (s, $NCH_3$, 3H); 4.2 (q, $OCH_2$, 2H); 4.8 (s, $CH_2SO_2$, 2H); 7.1 (bs, $NH_2$, 2H).

Example 4

Ethyl 5-[[[[4,6-dimethoxypyrimidin-2-yl]aminocarbonyl]aminosulfonyl]methyl]-1,3-dimethyl-1H-pyrazole-4-carboxylate

In a dry flask under nitrogen was stirred 0.33 g of the sulfonamide from Example 3 and 0.35 g of phenyl (4,6-dimethoxypyrimidin-2-yl)carbamate and 30 ml of dry acetonitrile. Then 0.19 ml of 1,8-diazabicyclo-[5.4.0]undec-7-ene was added via syringe and the solution stirred overnight. Then 50 ml of water was added, followed by a dropwise addition of 1 NHCl until a precipitate formed. The suspension was filtered. The filtrant, after being triturated with diethyl ether, gave 0.4 g of solid, m.p. 190° C; IR (Nujol) 1711 and 1680 (C=0) $cm^{-1}$; $^1H$ NMR (200 MHz, $CDCl_3$): $\delta$ 1.25 (t, $CH_3$, 3H); 2.38 (s, $CH_3$, 3H); 3.80 (s, $OCH_3$, 6H); 3.94 (s, $NCH_3$, 3H); 4.15 (q, $OCH_2$, 2H); 5.23 (s, $CH_2SO_2$, 2H); 5.71 (s, bs, CH, 1H); 7.35 (bs, NH, 1H) and 12.4 (bs, NH, 1H).

Example 5

2,6-Dimethylbenzoic acid, methyl ester

A solution of 10 g of 2,6-dimethylbenzoic acid in 22 g thionyl chloride was stirred at reflux temperature for 2 hours. Removal of the excess thionyl chloride afforded the crude acid chloride as a yellow oil. This intermediate was added to a stirred solution of 4 mL of methanol in 10.8 mL pyridine at 65-70° C. After completion of the addition, the resulting mixture was stirred at room temperature for 2 hours and then poured into 75 mL ice-water. The aqueous layer was extracted with four 50-mL portions of ether and the

combined organic extracts were back-washed with saturated sodium bicarbonate solution, 5% aqueous hydrochloric acid, and water. Drying and evaporation of the solvent gave a pale yellow oil which was purified by bulb-to-bulb distillation (40-50° C, 0.5 mm Hg) to afford 9.5 g of pure 2,6-dimethylbenzoic acid, methyl ester as a colorless liquid; IR (film)1725 $cm^{-1}$; NMR($CDCl_3$) δ 2.3 (s, 6H), 3.9 (s, 3H), 7.0-7.4 (m, 3H).

Example 6

2,6-Bis(α,α′-bromomethyl)benzoic acid, methyl ester

A mixture of 5.0 g of the product from Example 5, 11.1 g of N-bromosuccinimide and 0.1 g of azoisobutyronitrile in 76 mL carbon tetrachloride was heated at reflux temperature for one hour. The solution was cooled to room temperature, filtered, and the filtrate concentrated in vacuo to give a light orange oil. Treatment with ether-hexane (1:1, v/v) afforded 3.0 g of pure 2,6-bis(α,α′-bromomethyl)benzoic acid, methyl ester as a white solid, m.p. 67-70° C; IR(nujol) 1720 $cm^{-1}$; NMR($CDCl_3$) δ 4.0 (s, 3H), 4.65 (s, 4H), 7.5 (s, 3H).

Example 7

7-Bromomethylphthalide

The product from Example 6 (2.0g) was added to 8 mL of concentrated sulfuric acid and the suspension was warmed to about 60° C for 3 hours. The solution was allowed to cool and was poured into ice-water. The resulting white precipitate was collected by filtration, washed well with water and dried. Recrystallization from acetone gave 1.0 g of pure 7-bromomethylphthalide as a white powder, m.p. 127.5-131° C; IR(KBr) 1755 $cm^{-1}$; NMR($CDCl_3$) δ 5.0 (s, 2H), 5.29 (s, 2H), 7.4 (br d, 1H), 7.5 (br d, 1H), 7.6 (dd, 1H).

Example 8

7-[[(Amino)(imino)methyl]thiomethyl]phthalide

A solution of 1.0 g of the product from Example 7 in 10 mL tetrahydrofuran was treated with 0.33 g of thiourea and the suspension was warmed to reflux temperature for 15 minutes as a thick, white precipitate formed. The reaction mixture was allowed to cool and was filtered. The collected solid was washed with 1-chlorobutane and dried. The yield of the pure title compound was 1.2 g as a white powder, m.p. 206-211° C (dec.); NMR(DMSO-$d_6$) δ 4.8(s, 2H), 5.4 (s, 2H), 7.61 (br t, 2H), 7.73 (br t, 1H), 9.1 (brs, 1H), 9.23 (br s, 2H).

Example 9

1,3-Dihydro-1-oxo-benzo[c]furan-7-methanesulfonyl chloride

A solution of 9.4 g of the product from Example 8 in 77 mL of 50% aqueous acetic acid was cooled to 0° C and treated with 8 mL of liquid chlorine. Following the addition, the mixture was stirred at 0-10° C for 2 hours, filtered, and the solid was washed well with water. The yield of 1,3-dihydro-1-oxo-benzo[c]furan-7-methanesulfonyl chloride was 5.6 g as an off-white solid, m.p. 103-105.5° C (dec); IR(KBr) 1740, 1355, 1200

$cm^{-1}$.

## Example 10

### 1,3-Dihydro-1-oxo-benzo[c]furan-7-methanesulfonamide

A solution of 5.6 g of the product from Example 9 in 50 mL tetrahydrofuran was cooled to 0 °C under an atmosphere of nitrogen and was treated with 1.5 mL of anhydrous ammonia. The resulting suspension was stirred at room temperature for 3 hours. The insoluble solids were collected by filtration and were washed with water and dried. The yield of the title compound was 3.8 g as a white powder, m.p. 192-195 °C; IR(KBr) 3340, 3240, 1740, 1130 $cm^{-1}$; NMR(DMSO-$d_6$/$CDCl_3$) $\delta$ 4.9 (s, 2H), 5.4 (s, 2H), 6.7 (br s, 2H) 7.5-7.8 (m, 3H).

## Example 11

### 1,3-Dihydro-1-oxo-benzo[c]furan-7-methanesulfonyl isocyanate

A solution of 2.0 g of the product from Example 19 in 25 mL thionyl chloride was heated at reflux temperature for 18 hours. The reaction solution was allowed to cool to room temperature and excess thionyl chloride was removed in vacuo. The resulting residue was treated with 16 g of 10% phosgene in toluene and 5 drops of pyridine. This mixture was stirred at reflux temperature for 3 hours. The solution was allowed to cool, filtered under a stream of nitrogen, and the filtrate was concentrated to give 1,3-dihydro-1-oxo-benzo[c]furan-7-methanesulfonyl isocyanate as an orange oil. The infrared spectrum of this compound displayed a characteristic isocyanate stretching absorption at 2240 $cm^{-1}$ as well as a carbonyl stretching signal at 1750 $cm^{-1}$.

## Example 12

### 1,3-Dihydro-N-[(4,6-dimethoxyprimidine-2-yl)aminocarbonyl]-1-oxo-benzo[c]furan-7-methanesulfonamide

A solution of 8 mmole of the product from Example 11 in 16 mL dry acetonitrile was treated with 1.2 g of 2-amino-4,6-dimethoxypyrimidine and the mixture was heated to 45-50 °C under an atmosphere of nitrogen for 2 hours. After being stirred at room temperature for an additional 12 hours, the suspension was filtered and the collected solid was washed well with warm acetonitrile. The yield of the title compound was 1.0 g as a yellow powder, m.p. 181-184 °C (dec.); IR(KBr) 1765, 1710, 1620, 1355 $cm^{-1}$; NMR($CDCl_3$) $\delta$ 3.83 (s, 6H), 5.27 (s, 2H), 5.41 (s, 2H), 5.76 (s, 1H), 7.35 (br s, 1H), 7.5 (m, 1H), 7.7 (m, 2H), 12.4 (br s, 1H).

The invention is further exemplified, but not limited to, the compounds in Tables I-XXII. The compounds depicted in these tables may be prepared by methods described in Examples 1-4, or by modifications thereof apparent to those skilled in the art.

## General Formulas for Tables

General Formula 1

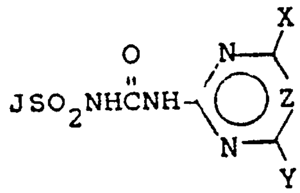

General Formula 2

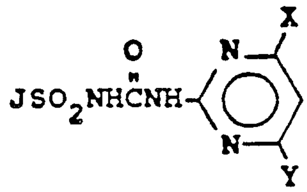

General Formula 2a

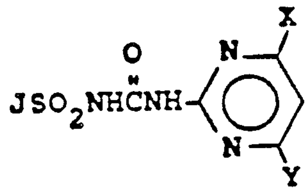

General Formula 3

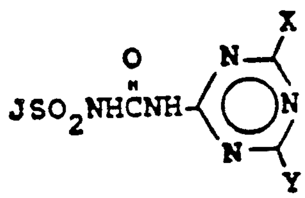

General Formula 3a

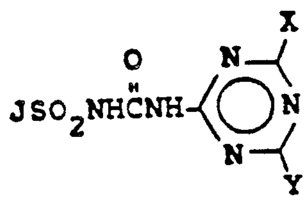

General Formula 4

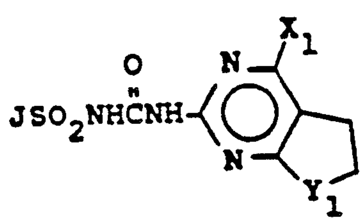

General Formulas for Tables (continued)

General Formula 5

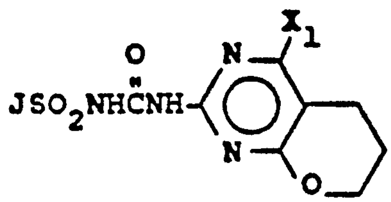

General Formula 6

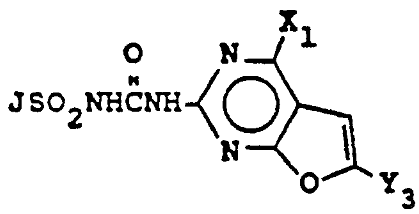

General Formula 7

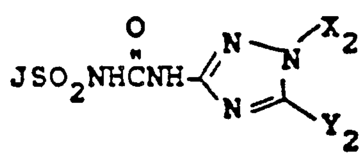

General Formula 8

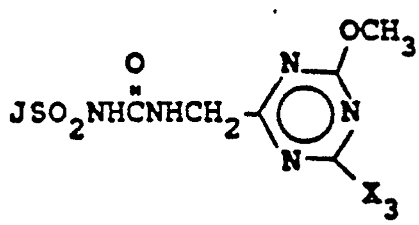

Table I

General Formula 1

$(J=J_1)$

| $R_1$ | $R_2$ | $R_3$ | X | Y | Z | m.p.(°C) |
|---|---|---|---|---|---|---|
| H | H | H | $CH_3$ | $CH_3$ | CH | |
| H | H | H | $CH_3$ | $OCH_3$ | CH | |
| $CH_3$ | H | H | $OCH_3$ | $OCH_3$ | CH | |
| $CH_3$ | H | H | $CH_3$ | $CH_3$ | N | |
| $CH_3$ | H | H | $CH_3$ | $OCH_3$ | N | |
| $CH_2CH_3$ | H | H | $OCH_3$ | $OCH_3$ | N | |
| $CH_2CH_2CH_3$ | H | H | $CH_3$ | $OCH_2CH_3$ | CH | |
| $CH(CH_3)_2$ | H | H | $CH_3$ | $OCH_3$ | N | |
| $CO_2CH_3$ | H | H | $OCH_3$ | $OCH_3$ | N | |
| $CO_2CH_3$ | H | H | Br | $OCH_3$ | CH | |
| $CO_2CH_3$ | H | H | $CH_3$ | $OCH_3$ | CH | |
| $CO_2CH_3$ | H | H | $OCH_3$ | $OCH_3$ | CH | |
| $CO_2CH_3$ | H | H | $CH_3$ | $CH_3$ | N | |
| $CO_2CH_3$ | H | H | $CH_3$ | $OCH_3$ | N | |
| $CO_2CH_2CH_3$ | H | H | $OCH_3$ | $OCH_3$ | N | |
| $CO_2CH_2CH_3$ | H | H | $CH_3$ | $OCH_3$ | CH | |
| $SO_2N(CH_3)_2$ | H | H | $OCH_3$ | $OCH_3$ | CH | |
| $SO_2N(CH_3)CH_2CH_3$ | H | H | $CH_3$ | $CH_3$ | N | |
| $SO_2N(CH_2CH_3)_2$ | H | H | $CH_3$ | $OCH_3$ | N | |
| $SO_2CH_3$ | H | H | $OCH_3$ | $OCH_3$ | N | |
| $SO_2CH_3$ | H | H | $CH_3$ | $CH_2F$ | CH | |

Table I (continued)

| $R_1$ | $R_2$ | $R_3$ | X | Y | Z | m.p.(°C) |
|---|---|---|---|---|---|---|
| $SO_2CH_3$ | H | H | $OCH_3$ | $OCH_3$ | CH | |
| $SO_2CH_3$ | H | H | Cl | $OCH_3$ | CH | |
| $SO_2CH_2CH_3$ | H | H | $OCH_3$ | $OCH_3$ | CH | |
| H | $CH_3$ | H | $CH_3$ | $CH_3$ | CH | |
| H | $CH_3$ | H | $CH_3$ | $OCH_3$ | CH | |
| $CH_3$ | $CH_3$ | H | $OCH_3$ | $OCH_3$ | CH | |
| $CH_3$ | $CH_3$ | H | $CH_3$ | $CH_3$ | N | |
| $CH_3$ | $CH_3$ | H | $CH_3$ | $OCH_3$ | N | |
| $CH_2CH_3$ | $CH_3$ | H | $OCH_3$ | $OCH_3$ | N | |
| $CH_2CH_2CH_3$ | $CH_3$ | H | $CH_3$ | $OCH_2CH_3$ | CH | |
| $CH_2CH_2CH_3$ | $CH_3$ | H | $CH_3$ | $CH_3$ | N | |
| $CH(CH_3)_2$ | $CH_3$ | H | $CH_3$ | $OCH_3$ | N | |
| $CO_2CH_3$ | $CH_3$ | H | $OCH_3$ | $OCH_3$ | N | |
| $CO_2CH_3$ | $CH_3$ | H | $OCH_2CF_3$ | $CH_3$ | CH | |
| $CO_2CH_3$ | $CH_3$ | H | $CH_3$ | $OCH_3$ | CH | |
| $CO_2CH_3$ | $CH_3$ | H | $OCH_3$ | $OCH_3$ | CH | |
| $CO_2CH_3$ | $CH_3$ | H | $CH_3$ | $OCH_3$ | N | |
| $CO_2CH_2CH_3$ | $CH_3$ | H | $OCH_3$ | $OCH_3$ | N | |
| ~~$CO_2CH_2CH_3$~~ | ~~$CH_3$~~ | ~~H~~ | ~~$OCH_3$~~ | ~~$CH_2OCH_3$~~ | ~~CH~~ | |
| $CO_2CH_2CH_3$ | $CH_3$ | H | $CH_3$ | $OCH_3$ | CH | |
| $SO_2N(CH_3)_2$ | $CH_3$ | H | $OCH_3$ | $OCH_3$ | CH | |
| $SO_2N(CH_3)CH_2CH_3$ | $CH_3$ | H | $CH_3$ | $CH_3$ | N | |
| $SO_2N(CH_2CH_3)_2$ | $CH_3$ | H | $CH_3$ | $OCH_3$ | N | |
| $SO_2CH_3$ | $CH_3$ | H | $OCH_3$ | $OCH_3$ | N | |
| $SO_2CH_3$ | $CH_3$ | H | Cl | $OCH_3$ | CH | |
| $SO_2CH_2CH_3$ | $CH_3$ | H | $OCH_3$ | $OCH_3$ | CH | |

## Table I (continued)

| $R_1$ | $R_2$ | $R_3$ | X | Y | Z | m.p.(°C) |
|---|---|---|---|---|---|---|
| H | H | $CH_3$ | $CH_3$ | $CH_3$ | CH | |
| H | H | $CH_3$ | $CH_3$ | $OCH_3$ | CH | |
| $CH_3$ | H | $CH_3$ | $OCH_3$ | $OCH_3$ | CH | |
| $CH_3$ | H | $CH_3$ | $CH_3$ | $CH_3$ | N | |
| $CH_3$ | H | $CH_3$ | $CH_3$ | $OCH_3$ | N | |
| $CH_2CH_3$ | H | $CH_3$ | $OCH_3$ | Δ | N | |
| $CH_2CH_2CH_3$ | H | $CH_3$ | $OCH_3$ | $OCH_3$ | CH | |
| $CH_2CH_2CH_3$ | H | $CH_3$ | $CH_3$ | $CH_3$ | N | |
| $CH(CH_3)_2$ | H | $CH_3$ | $CH_3$ | $OCH_3$ | N | |
| $CO_2CH_3$ | H | $CH_3$ | $CH_3$ | $CH_3$ | CH | |
| $CO_2CH_3$ | H | $CH_3$ | $CH_3$ | $OCH_3$ | CH | |
| $CO_2CH_3$ | H | $CH_3$ | $OCH_3$ | $OCH_3$ | CH | |
| $CO_2CH_3$ | H | $CH_3$ | $CH_3$ | $CH_3$ | N | |
| $CO_2CH_3$ | H | $CH_3$ | $CH_3$ | $OCH_3$ | N | |
| $CO_2CH_2CH_3$ | H | $CH_3$ | $OCH_3$ | $OCH_3$ | N | |
| ~~$CO_2CH_2CH_3$~~ | ~~H~~ | ~~$CH_3$~~ | ~~$OCH_3$~~ | ~~$CH_2OCH_3$~~ | ~~CH~~ | |
| $CO_2CH_2CH_3$ | H | $CH_3$ | $CH_3$ | $OCH_3$ | CH | |
| $SO_2N(CH_3)_2$ | H | $CH_3$ | $OCH_3$ | $OCH_3$ | CH | |
| $SO_2N(CH_3)CH_2CH_3$ | H | $CH_3$ | $CH_3$ | $CH_3$ | N | |
| $SO_2N(CH_2CH_3)_2$ | H | $CH_3$ | $CH_3$ | $OCH_2CH_2F$ | N | |
| $SO_2CH_3$ | H | $CH_3$ | $OCH_3$ | $OCH_3$ | N | |
| $SO_2CH_3$ | H | $CH_3$ | $OCH_3$ | $OCH_3$ | CH | |
| $SO_2CH_3$ | H | $CH_3$ | Cl | $OCH_3$ | CH | |
| $SO_2CH_2CH_3$ | H | $CH_3$ | $OCH_3$ | $OCH_3$ | CH | |
| H | $CH_3$ | $CH_3$ | $CH_3$ | $CF_3$ | CH | |
| H | $CH_3$ | $CH_3$ | $CH_3$ | $OCH_3$ | CH | |
| $CH_3$ | $CH_3$ | $CH_3$ | $OCH_3$ | $OCH_3$ | CH | |

Table I (continued)

| $R_1$ | $R_2$ | $R_3$ | X | Y | Z | m.p.(°C) |
|---|---|---|---|---|---|---|
| $CH_3$ | $CH_3$ | $CH_3$ | $CH_3$ | $SCH_3$ | N | |
| $CH_3$ | $CH_3$ | $CH_3$ | $CH_3$ | $OCH_3$ | N | |
| $CH_2CH_3$ | $CH_3$ | $CH_3$ | $OCH_3$ | $OCH_3$ | N | |
| $CH_2CH_2CH_3$ | $CH_3$ | $CH_3$ | $CH_3$ | $OCH_2CH_3$ | CH | |
| $CH_2CH_2CH_3$ | $CH_3$ | $CH_3$ | $CH_3$ | $OCH_3$ | CH | |
| $CH_2CH_2CH_3$ | $CH_3$ | $CH_3$ | $OCH_3$ | $OCH_2C\equiv CH$ | CH | |
| $CH_2CH_2CH_3$ | $CH_3$ | $CH_3$ | $CH_3$ | $CH_3$ | N | |
| $CH(CH_3)_2$ | $CH_3$ | $CH_3$ | $CH_3$ | $OCH_3$ | N | |
| $CO_2CH_3$ | $CH_3$ | $CH_3$ | $OCH_3$ | $OCH_3$ | N | |
| $CO_2CH_3$ | $CH_3$ | $CH_3$ | $CH_3$ | $OCH_2CH_3OCH_3$ | CH | |
| $CO_2CH_3$ | $CH_3$ | $CH_3$ | $CH_3$ | $OCH_3$ | CH | |
| $CO_2CH_3$ | $CH_3$ | $CH_3$ | $OCH_3$ | $OCH_3$ | CH | |
| $CO_2CH_3$ | $CH_3$ | $CH_3$ | $CH_3$ | $CH_3$ | N | |
| $CO_2CH_3$ | $CH_3$ | $CH_3$ | $CH_3$ | $OCH_3$ | N | |
| ~~$CO_2CH_2CH_3$~~ | ~~$CH_3$~~ | ~~$CH_3$~~ | ~~$OCH_3$~~ | ~~$CH_2OCH_3$~~ | ~~CH~~ | |
| $CO_2CH_2CH_3$ | $CH_3$ | $CH_3$ | $CH_3$ | $OCH_3$ | CH | |
| ~~$SO_2N(CH_3)_2$~~ | ~~$CH_3$~~ | ~~$CH_3$~~ | ~~$OCH_3$~~ | ~~$CH(OCH_3)_2$~~ | ~~CH~~ | |
| $SO_2N(CH_3)CH_2CH_3$ | $CH_3$ | $CH_3$ | $CH_3$ | $CH_3$ | N | |
| $SO_2N(CH_2CH_3)_2$ | $CH_3$ | $CH_3$ | $CH_3$ | $OCH_3$ | N | |
| $SO_2CH_3$ | $CH_3$ | $CH_3$ | $OCH_3$ | $OCH_3$ | N | |
| $SO_2CH_3$ | $CH_3$ | $CH_3$ | $CH_3$ | $CH_3$ | CH | |
| $SO_2CH_3$ | $CH_3$ | $CH_3$ | $CH_3$ | $OCH_3$ | CH | |
| $SO_2CH_3$ | $CH_3$ | $CH_3$ | $OCH_3$ | $OCH_3$ | CH | |
| $SO_2CH_3$ | $CH_3$ | $CH_3$ | Cl | $OCH_3$ | CH | |
| ~~$SO_2CH_2CH_3$~~ | ~~$CH_3$~~ | ~~$CH_3$~~ | ~~$OCH_3$~~ | ~~1,3-dioxolan-2-yl~~ | ~~CH~~ | |

Table II

General Formula 1

$(J=J_2)$

| $R_4$ | $R_5$ | $R_6$ | X | Y | Z | m.p.(°C) |
|---|---|---|---|---|---|---|
| H | $CH_3$ | H | $CH_3$ | $OCH_3$ | CH | |
| H | $CH_3$ | H | $OCH_3$ | $OCH_3$ | CH | |
| H | $CO_2CH_3$ | H | $CH_3$ | $CH_3$ | N | |
| H | $CO_2CH_3$ | H | $CH_3$ | $OCH_3$ | N | |
| H | $CO_2CH_3$ | H | $OCH_3$ | $OCH_3$ | N | |
| H | $CO_2CH_3$ | H | $CH_3$ | $OCF_2H$ | CH | |
| H | $CO_2CH_3$ | H | $CH_3$ | $OCH_3$ | CH | |
| H | $CO_2CH_3$ | H | $OCH_3$ | $OCH_3$ | CH | |
| H | $CO_2CH_2CH_3$ | H | $CH_3$ | $CH_3$ | N | |
| H | $CO_2CH_2CH_3$ | H | $CH_3$ | $OCH_2CH_3$ | N | |
| H | $SO_2N(CH_3)_2$ | H | $OCH_3$ | $NHCH_3$ | N | |
| H | $SO_2N(CH_3)_2$ | H | $CH_3$ | $CH_3$ | CH | |
| H | $SO_2N(CH_3)_2$ | H | $CH_3$ | C≡CH | CH | |
| H | $SO_2N(CH_3)_2$ | H | $OCH_3$ | $OCH_3$ | CH | |
| H | $SO_2N(CH_3)_2$ | H | $CH_3$ | $CH_3$ | N | |
| H | $SO_2N(CH_3)_2$ | H | $CH_3$ | $OCH_3$ | N | |
| H | $SO_2N(CH_3)CH_2CH_3$ | H | $OCH_3$ | $OCH_3$ | N | |
| H | $SO_2N(OCH_3)CH_3$ | H | $CH_3$ | $CH_3$ | CH | |
| H | $SO_2N(CH_2CH_3)_2$ | H | $CH_3$ | $OCH_3$ | CH | |
| H | $SO_2N(CH_2CH_3)_2$ | H | Br | $OCH_3$ | CH | |
| HH | Cl | H | $OCH_3$ | $OCH_3$ | N | |
| H | $SO_2CH_3$ | H | $CH_3$ | $CH_2CH_3$ | CH | |
| H | $SO_2CH_3$ | H | $CH_3$ | $OCH_3$ | CH | |
| ~~H~~ | ~~$SO_2CH_3$~~ | ~~H~~ | ~~$OCH_3$~~ | ~~$CH(OCH_3)_2$~~ | ~~CH~~ | |

Table II (continued)

| $R_4$ | $R_5$ | $R_6$ | X | Y | Z | m.p. (°C) |
|---|---|---|---|---|---|---|
| H | $SO_2CH_2CH_3$ | H | Cl | $OCH_3$ | CH | |
| H | $SO_2CH_2CH_3$ | H | $OCH_3$ | $OCH_3$ | N | |
| H | H | $CH_3$ | $CH_3$ | $CH_3$ | CH | |
| H | $CH_3$ | $CH_3$ | $CH_3$ | $OCH_3$ | CH | |
| H | $CH_3$ | $CH_3$ | $OCH_3$ | $OCH_3$ | CH | |
| H | $CO_2CH_3$ | $CH_3$ | $CH_3$ | $CH_3$ | N | |
| H | $CO_2CH_3$ | $CH_3$ | $CH_3$ | $OCH_3$ | N | |
| H | $CO_2CH_3$ | $CH_3$ | $OCH_3$ | $OCH_3$ | N | |
| H | $CO_2CH_3$ | $CH_3$ | $CH_3$ | $CH_3$ | CH | |
| H | $CO_2CH_3$ | $CH_3$ | $CH_3$ | $OCH_3$ | CH | |
| H | $CO_2CH_3$ | $CH_3$ | $OCH_3$ | $OCH_3$ | CH | |
| H | $CO_2CH_2CH_3$ | $CH_3$ | $CH_3$ | $CH_3$ | N | |
| H | $CO_2CH_2CH_3$ | $CH_3$ | $CH_3$ | $OCH_2CH_3$ | N | |
| H | $SO_2N(CH_3)_2$ | $CH_3$ | $OCH_3$ | $OCH_3$ | N | |
| H | $SO_2N(CH_3)_2$ | $CH_3$ | $CH_3$ | $CH_3$ | CH | |
| H | $SO_2N(CH_3)_2$ | $CH_3$ | $CH_3$ | $OCH_3$ | N | |
| H | $SO_2N(CH_3)CH_2CH_3$ | $CH_3$ | $OCH_3$ | $OCH_3$ | N | |
| H | $SO_2N(OCH_3)CH_3$ | $CH_3$ | $CH_3$ | $CH_3$ | CH | |
| H | $SO_2N(CH_2CH_3)_2$ | $CH_3$ | $CH_3$ | $OCH_2CH=CH_2$ | CH | |
| H | $SO_2N(CH_2CH_3)_2$ | $CH_3$ | $OCH_3$ | $OCH_3$ | CH | |
| H | $SO_2CH_3$ | $CH_3$ | $CH_3$ | $CH_3$ | N | |
| H | $SO_2CH_3$ | $CH_3$ | $CH_3$ | $OCH_3$ | N | |
| H | $SO_2CH_3$ | $CH_3$ | $OCH_3$ | $OCH_3$ | N | |
| H | $SO_2CH_3$ | $CH_3$ | $CH_3$ | $CH_2CH_3$ | CH | |
| H | Br | $CH_3$ | $CH_3$ | $OCH_3$ | CH | |
| H | $SO_2CH_3$ | $CH_3$ | $OCH_3$ | $OCH_3$ | CH | |
| H | $SO_2CH_2CH_3$ | $CH_3$ | Cl | $OCH_3$ | CH | |
| H | $SO_2CH_2CH_3$ | $CH_3$ | $OCH_3$ | $OCH_3$ | N | |

Table II (continued)

| $R_4$ | $R_5$ | $R_6$ | X | Y | Z | m.p. (°C) |
|---|---|---|---|---|---|---|
| H | H | $CH_2CH_2CH_3$ | $CH_3$ | $CH_3$ | CH | |
| H | $CH_3$ | $CH_2CH_2CH_3$ | $CH_3$ | $OCH_3$ | CH | |
| H | $CH_3$ | $CH_2CH_2CH_3$ | $OCH_3$ | $OCH_3$ | CH | |
| H | $CO_2CH_3$ | $CH_2CH_2CH_3$ | $CH_3$ | $CH_3$ | N | |
| H | $CO_2CH_3$ | $CH_2CH_2CH_3$ | $CH_3$ | $OCH_3$ | N | |
| H | $CO_2CH_3$ | $CH_2CH_2CH_3$ | $OCH_3$ | $OCH_3$ | N | |
| H | $CO_2CH_3$ | $CH_2CH_2CH_3$ | $CH_3$ | $CH_3$ | CH | |
| H | $CO_2CH_3$ | $CH_2CH_2CH_3$ | $CH_3$ | $OCH_3$ | CH | |
| H | $CO_2CH_3$ | $CH_2CH_2CH_3$ | $OCH_3$ | $SCH_3$ | CH | |
| H | $CO_2CH_2CH_3$ | $CH_2CH_2CH_3$ | $CH_3$ | $CH_3$ | N | |
| H | $CO_2CH_2CH_3$ | $CH_2CH_2CH_3$ | $CH_3$ | $OCH_2CH_3$ | N | |
| H | $SO_2N(CH_3)_2$ | $CH_2CH_2CH_3$ | $OCH_2CF_3$ | $OCH_3$ | N | |
| H | $SO_2N(CH_3)_2$ | $CH_2CH_2CH_3$ | $CH_3$ | $OCH_3$ | CH | |
| H | $SO_2N(CH_3)_2$ | $CH_2CH_2CH_3$ | $OCH_3$ | $OCH_3$ | CH | |
| H | $SO_2N(CH_3)_2$ | $CH_2CH_2CH_3$ | $CH_3$ | $OCH_3$ | N | |
| H | $SO_2N(CH_3)CH_2CH_3$ | $CH_2CH_2CH_3$ | $OCH_3$ | $OCH_3$ | N | |
| H | $SO_2N(OCH_3)CH_3$ | $CH_2CH_2CH_3$ | $CH_3$ | $CH_3$ | CH | |
| H | $SO_2N(CH_2CH_3)_2$ | $CH_2CH_2CH_3$ | $CH_3$ | $OCH_3$ | CH | |
| H | $SO_2N(CH_2CH_3)_2$ | $CH_2CH_2CH_3$ | $OCH_3$ | $OCH_3$ | CH | |
| H | $SO_2CH_3$ | $CH_2CH_2CH_3$ | $CH_3$ | $OCH_3$ | N | |
| H | $SO_2CH_3$ | $CH_2CH_2CH_3$ | $OCH_3$ | $OCH_3$ | N | |
| H | $SO_2CH_3$ | $CH_2CH_2CH_3$ | $CH_3$ | $CH_2CH_3$ | CH | |
| H | $SO_2CH_3$ | $CH_2CH_2CH_3$ | $CH_3$ | $OCH_3$ | CH | |
| H | $SO_2CH_3$ | $CH_2CH_2CH_3$ | $OCH_3$ | $OCH_3$ | CH | |
| H | $SO_2CH_2CH_3$ | $CH_2CH_2CH_3$ | Cl | $CH_2F$ | CH | |
| H | $SO_2CH_2CH_3$ | $CH_2CH_2CH_3$ | $OCH_3$ | $OCH_3$ | N | |
| $CH_3$ | H | $CH_3$ | $CH_3$ | $CH_3$ | CH | |
| $CH_3$ | $CH_3$ | $CH_3$ | $CH_3$ | $OCH_3$ | CH | |

Table II (continued)

| $R_4$ | $R_5$ | $R_6$ | X | Y | Z | m.p. (°C) |
|---|---|---|---|---|---|---|
| $CH_3$ | $CH_3$ | $CH_3$ | $OCH_3$ | $OCH_3$ | CH | |
| $CH_3$ | $CO_2CH_3$ | $CH_3$ | $CH_3$ | $CH_3$ | N | |
| $CH_3$ | $CO_2CH_3$ | $CH_3$ | $CH_3$ | $OCH_3$ | N | |
| $CH_3$ | $CO_2CH_3$ | $CH_3$ | $OCH_3$ | $OCH_3$ | N | |
| $CH_3$ | $CO_2CH_3$ | $CH_3$ | $CH_3$ | $CH_3$ | CH | |
| $CH_3$ | $CO_2CH_3$ | $CH_3$ | $CH_3$ | $OCH_3$ | CH | |
| $CH_3$ | $CO_2CH_3$ | $CH_3$ | $OCH_3$ | $NH_2$ | CH | |
| $CH_3$ | $CO_2CH_2CH_3$ | $CH_3$ | $CH_3$ | $CH_3$ | N | |
| $CH_3$ | $CO_2CH_2CH_3$ | $CH_3$ | $CH_3$ | $OCH_2CH_3$ | N | |
| $CH_3$ | $SO_2N(CH_3)_2$ | $CH_3$ | $OCH_3$ | $OCH_3$ | N | |
| $CH_3$ | $SO_2N(CH_3)_2$ | $CH_3$ | $CH_3$ | $OCH_3$ | CH | |
| $CH_3$ | $SO_2N(CH_3)_2$ | $CH_3$ | $OCH_3$ | $OCH_3$ | CH | |
| $CH_3$ | Br | $CH_3$ | $CH_3$ | $OCH_3$ | N | |
| $CH_3$ | $SO_2N(CH_3)CH_2CH_3$ | $CH_3$ | $OCH_3$ | $OCH_3$ | N | |
| $CH_3$ | $SO_2N(OCH_3)CH_3$ | $CH_3$ | $CH_3$ | $CH_3$ | CH | |
| $CH_3$ | $SO_2N(CH_2CH_3)_2$ | $CH_3$ | $CH_3$ | $OCH_3$ | CH | |
| $CH_3$ | $SO_2N(CH_2CH_3)_2$ | $CH_3$ | $OCH_3$ | $OCH_3$ | CH | |
| $CH_3$ | $SO_2CH_3$ | $CH_3$ | $OCH_3$ | $OCH_3$ | N | |
| $CH_3$ | $SO_2CH_3$ | $CH_3$ | $CH_3$ | $CH_2CH_3$ | CH | |
| $CH_3$ | $SO_2CH_3$ | $CH_3$ | $CH_3$ | $OCH_3$ | CH | |
| $CH_3$ | $SO_2CH_3$ | $CH_3$ | $OCF_2H$ | $CH_3$ | CH | |
| $CH_3$ | $SO_2CH_2CH_3$ | $CH_3$ | Cl | $OCH_3$ | CH | |
| $CH_3$ | $SO_2CH_2CH_3$ | $CH_3$ | $OCH_3$ | $OCH_3$ | N | |
| $CH_3$ | H | $CH_2CH_2CH_3$ | $CH_3$ | $CH_3$ | CH | |
| $CH_3$ | $CH_3$ | $CH_2CH_2CH_3$ | $OCH_3$ | $OCH_3$ | CH | |
| $CH_3$ | $CO_2CH_3$ | $CH_2CH_2CH_3$ | $CH_3$ | $CH_3$ | N | |

Table II (continued)

| $R_4$ | $R_5$ | $R_6$ | X | Y | Z | m.p. (°C) |
|---|---|---|---|---|---|---|
| $CH_3$ | $CO_2CH_3$ | $CH_2CH_2CH_3$ | $CH_3$ | $OCH_3$ | N | |
| $CH_3$ | $CO_2CH_3$ | $CH_2CH_2CH_3$ | $OCH_3$ | $OCH_3$ | N | |
| $CH_3$ | $CO_2CH_3$ | $CH_2CH_2CH_3$ | $CH_3$ | $CH_3$ | CH | |
| $CH_3$ | $CO_2CH_3$ | $CH_2CH_2CH_3$ | $CH_3$ | $OCH_3$ | CH | |
| $CH_3$ | $CO_2CH_3$ | $CH_2CH_2CH_3$ | $OCH_3$ | $OCH_3$ | CH | |
| $CH_3$ | $CO_2CH_2CH_3$ | $CH_2CH_2CH_3$ | $CH_3$ | $CH_3$ | N | |
| $CH_3$ | $CO_2CH_2CH_3$ | $CH_2CH_2CH_3$ | $CH_3$ | $OCH_2CH_3$ | N | |
| $CH_3$ | $SO_2N(CH_3)_2$ | $CH(CH_3)_2$ | $OCH_3$ | $OCH_3$ | N | |
| $CH_3$ | $SO_2N(CH_3)_2$ | $CH_2CH_2CH_3$ | $CH_3$ | $CH_3$ | CH | |
| $CH_3$ | $SO_2N(CH_3)_2$ | $CH_2CH_2CH_3$ | $CH_3$ | $OCH_3$ | CH | |
| $CH_3$ | $SO_2N(CH_3)_2$ | $CH_2CH_2CH_3$ | $OCH_3$ | $OCH_3$ | CH | |
| $CH_3$ | $SO_2N(CH_3)_2$ | $CH_2CH_2CH_3$ | $CH_3$ | $CH_3$ | N | |
| $CH_3$ | $SO_2N(CH_3)_2$ | $CH_2CH_2CH_3$ | $CH_3$ | $OCH_3$ | N | |
| $CH_3$ | $SO_2N(CH_3)CH_2CH_3$ | $CH_2CH_2CH_3$ | $OCH_3$ | $OCH_3$ | N | |
| $CH_3$ | $SO_2N(OCH_3)CH_3$ | $CH_2CH_2CH_3$ | $CH_3$ | $CH_3$ | CH | |
| $CH_3$ | $SO_2N(CH_2CH_3)_2$ | $CH_2CH_2CH_3$ | $CH_3$ | $OCH_3$ | CH | |
| $CH_3$ | $SO_2N(CH_2CH_3)_2$ | $CH_2CH_2CH_3$ | $OCH_3$ | $OCH_3$ | CH | |
| $CH_3$ | $SO_2CH_3$ | $CH_2CH_2CH_3$ | $OCH_3$ | $OCH_3$ | N | |
| $CH_3$ | $SO_2CH_3$ | $CH_2CH_2CH_3$ | $CH_3$ | $CH_2CH_3$ | CH | |
| $CH_3$ | $SO_2CH_3$ | $CH_2CH_2CH_3$ | $CH_3$ | $OCH_3$ | CH | |
| $CH_3$ | $SO_2CH_3$ | $CH_2CH_2CH_3$ | $OCH_3$ | $OCH_2CH_2F$ | CH | |
| $CH_3$ | $SO_2CH_2CH_3$ | $CH_2CH_2CH_3$ | Cl | $OCH_3$ | CH | |
| $CH_3$ | $SO_2CH_2CH_3$ | $CH_2CH_3$ | $OCH_3$ | $OCH_3$ | N | |
| H | H | $CO_2CH_3$ | $CH_3$ | $CH_2CH_3$ | CH | |
| H | H | $CO_2CH_3$ | $CH_3$ | $OCH_3$ | CH | |
| H | H | $CO_2CH_3$ | $OCH_3$ | $OCH_3$ | CH | |
| H | H | $CO_2CH_3$ | $CH_3$ | $CH_3$ | N | |
| H | H | $CO_2CH_3$ | $CH_3$ | $OCH_3$ | N | |
| H | H | $CO_2CH_3$ | $OCH_3$ | $OCH_3$ | N | |

Table II (continued)

| $R_4$ | $R_5$ | $R_6$ | X | Y | Z | m.p. (°C) |
|---|---|---|---|---|---|---|
| H | H | $CO_2CH_2CH_3$ | $CH_3$ | $CH_3$ | CH | |
| H | H | $CO_2CH_2CH_3$ | $CH_3$ | $SCH_3$ | CH | |
| H | H | $CO_2CH_2CH_3$ | $OCH_3$ | $OCH_3$ | CH | |
| H | H | $CO_2CH_2CH_3$ | $CH_3$ | $CH_2CH_2CH_3$ | N | |
| H | H | $CO_2CH_2CH_3$ | $CH_3$ | $OCH_3$ | N | |
| ~~H~~ | ~~H~~ | ~~$CO_2CH_2CH_3$~~ | ~~$OCH_3$~~ | ~~$CH_2OCH_3$~~ | ~~CH~~ | |
| H | H | $SO_2N(CH_3)_2$ | $CH_3$ | $OCH_3$ | CH | |
| H | H | $SO_2N(CH_3)_2$ | $OCH_3$ | $OCH_3$ | CH | |
| H | H | $SO_2N(CH_3)_2$ | $CH_3$ | $CH_2CH_3$ | N | |
| H | H | $SO_2N(CH_3)_2$ | $CH_3$ | $OCH_3$ | N | |
| H | H | $SO_2N(CH_3)_2$ | $OCH_3$ | $OCH_3$ | N | |
| H | H | $SO_2N(CH_3)CH_2CH_3$ | $CH_3$ | $CH_3$ | CH | |
| H | H | $SO_2N(CH_2CH_3)_2$ | $CH_3$ | $OCH_3$ | CH | |
| H | H | $SO_2CH_3$ | $OCH_3$ | $OCH_3$ | CH | |
| H | H | $SO_2CH_3$ | $CH_3$ | $OCH_2CH_3$ | N | |
| H | H | $SO_2CH_3$ | $CH_3$ | $OCH_3$ | N | |
| H | H | $SO_2CH_3$ | $OCH_3$ | $OCH_3$ | N | |
| H | H | $SO_2CH_3$ | $CH_3$ | $CH_3$ | CH | |
| H | H | $SO_2CH_3$ | $CH_3$ | $OCH_3$ | CH | |
| H | H | $SO_2CH_2CH_3$ | $OCH_3$ | $OCH_3$ | CH | |
| H | H | $SO_2CH_2CH_3$ | Cl | $OCH_2CH_2OCH_3$ | CH | |
| $CH_3$ | H | $CO_2CH_3$ | $CH_3$ | $CH_3$ | CH | |
| $CH_3$ | H | $CO_2CH_3$ | $CH_3$ | $OCH_3$ | CH | |
| $CH_3$ | H | $CO_2CH_3$ | $OCH_3$ | $OCH_3$ | CH | |
| $CH_3$ | H | $CO_2CH_3$ | $CH_3$ | $CH_3$ | N | |
| $CH_3$ | H | $CO_2CH_3$ | $CH_3$ | $OCH_3$ | N | |
| $CH_3$ | H | $CO_2CH_3$ | $OCH_3$ | $OCH_3$ | N | |
| $CH_3$ | H | $CO_2CH_2CH_3$ | $CH_3$ | $CH_3$ | CH | |
| $CH_3$ | H | $CO_2CH_2CH_3$ | $CH_3$ | $OCH_3$ | CH | |

Table II (continued)

| $R_4$ | $R_5$ | $R_6$ | X | Y | Z | m.p. (°C) |
|---|---|---|---|---|---|---|
| $CH_3$ | H | $CO_2CH_2CH_3$ | $OCH_3$ | $OCH_3$ | CH | |
| $CH_3$ | H | $CO_2CH_2CH_3$ | $CH_3$ | $CH_3$ | N | |
| $CH_3$ | H | $CO_2CH_2CH_3$ | $CH_3$ | $OCH_3$ | N | |
| ~~$CH_3$~~ | ~~H~~ | ~~$CO_2CH_2CH_3$~~ | ~~$OCH_3$~~ | ~~$CH_2OCH_3$~~ | ~~CH~~ | |
| $CH_3$ | H | $SO_2N(CH_3)_2$ | $CH_3$ | $CH_3$ | CH | |
| $CH_3$ | H | $SO_2N(CH_3)_2$ | $CH_3$ | $OCH_3$ | CH | |
| $CH_3$ | H | $SO_2N(CH_3)_2$ | $OCH_3$ | $OCH_3$ | CH | |
| $CH_3$ | H | $SO_2N(CH_3)_2$ | $CH_3$ | $CH_2CH_3$ | N | |
| $CH_3$ | H | $SO_2N(CH_3)_2$ | $CH_3$ | $OCH_3$ | N | |
| $CH_3$ | H | $SO_2N(CH_3)_2$ | $OCH_3$ | $OCH_3$ | N | |
| $CH_3$ | H | $SO_2N(CH_3)CH_2CH_3$ | $CH_3$ | $CH_3$ | CH | |
| $CH_3$ | H | $SO_2N(CH_2CH_3)_2$ | $CH_3$ | $OCH_3$ | CH | |
| $CH_3$ | H | $SO_2CH_3$ | $OCH_3$ | $OCH_3$ | CH | |
| $CH_3$ | H | $SO_2CH_3$ | $CH_3$ | $OCH_2CH_3$ | N | |
| $CH_3$ | H | $SO_2CH_3$ | $CH_3$ | $OCH_3$ | N | |
| $CH_3$ | H | $SO_2CH_3$ | $OCH_3$ | $OCH_3$ | N | |
| $CH_3$ | H | $SO_2CH_3$ | $CH_3$ | $CH_3$ | CH | |
| $CH_3$ | H | $SO_2CH_3$ | $CH_3$ | $OCH_3$ | CH | |
| $CH_3$ | H | $SO_2CH_2CH_3$ | $OCH_3$ | $OCH_3$ | CH | |
| $CH_3$ | H | $SO_2CH_2CH_3$ | $OCH_3$ | $CH_2CH_3$ | CH | |
| $CH_3$ | H | $SO_2CH_2CH_3$ | Cl | $OCH_3$ | CH | |
| $CH_3$ | $NO_2$ | H | $CH_3$ | $OCH_3$ | CH | |
| $CH_3$ | $NO_2$ | $CH_3$ | $CH_3$ | $OCH_3$ | N | |
| ~~$CH_3$~~ | ~~$NO_2$~~ | ~~$CH_3$~~ | ~~$OCH_3$~~ | ~~1,3-dioxolan-2-yl~~ | ~~N~~ | |

Table III

General Formula 1

($J=J_3$)

| $R_7$ | $R_8$ | X | Y | Z | m.p. (°C) |
|---|---|---|---|---|---|
| H | $CO_2CH_3$ | $CH_3$ | $CH_3$ | CH | |
| H | $CO_2CH_3$ | $CH_3$ | $OCH_3$ | CH | |
| H | $CO_2CH_3$ | $OCH_3$ | $OCH_3$ | CH | |
| H | $CO_2CH_3$ | $CH_3$ | $CH_3$ | N | |
| H | $CO_2CH_3$ | $CH_3$ | $OCH_3$ | N | |
| H | $CO_2CH_3$ | $OCH_3$ | $OCH_3$ | N | |
| H | $CO_2CH_2CH_3$ | $CH_3$ | $CH_3$ | CH | |
| H | $SO_2N(CH_3)_2$ | $OCH_3$ | $CH_2CH_3$ | N | |
| H | $SO_2N(CH_3)_2$ | $CH_3$ | $OCH_3$ | N | |
| H | $SO_2N(CH_3)_2$ | $OCH_3$ | $OCH_3$ | N | |
| H | $SO_2N(CH_3)_2$ | $CH_3$ | $CH_3$ | CH | |
| H | $SO_2N(CH_3)_2$ | Br | $OCH_3$ | CH | |
| H | $SO_2N(CH_3)_2$ | $OCH_3$ | $OCH_3$ | CH | |
| H | $SO_2N(CH_3)OCH_3$ | $CH_3$ | $CH_3$ | N | |
| H | $SO_2N(CH_3)CH_2CH_3$ | $CH_3$ | $OCH_2CH_3$ | N | |
| H | $SO_2CH_3$ | $OCH_3$ | $OCH_3$ | N | |
| H | $SO_2CH_3$ | $CH_3$ | $CH_3$ | CH | |
| H | $SO_2CH_3$ | $CH_3$ | $OCH_3$ | CH | |
| H | $SO_2CH_3$ | $OCH_3$ | $OCH_3$ | CH | |
| H | $SO_2CH_3$ | $CH_3$ | $CH_3$ | N | |
| H | $SO_2CH_3$ | $CH_3$ | $OCH_3$ | N | |
| H | $SO_2CH_2CH_3$ | $OCH_3$ | $SCH_3$ | N | |

Table III (continued)

| $R_7$ | $R_8$ | X | Y | Z | m.p. (°C) |
|---|---|---|---|---|---|
| H | $NO_2$ | $CH_3$ | $CH_3$ | CH | |
| H | $NO_2$ | $CH_3$ | $OCH_3$ | CH | |
| H | $NO_2$ | $OCH_3$ | $OCH_3$ | CH | |
| H | $NO_2$ | Cl | $OCH_3$ | CH | |
| ~~H~~ | ~~$NO_2$~~ | ~~$OCH_3$~~ | ~~$CH_2OCH_3$~~ | ~~CH~~ | |
| $CH_3$ | $CO_2CH_3$ | $CH_3$ | $CH_3$ | CH | |
| $CH_3$ | $CO_2CH_3$ | $CH_3$ | $OCH_3$ | CH | |
| $CH_3$ | $CO_2CH_3$ | $OCH_3$ | $OCH_3$ | CH | |
| $CH_3$ | $CO_2CH_3$ | $CH_3$ | $CH_3$ | N | |
| $CH_3$ | $CO_2CH_3$ | $CH_3$ | $OCH_3$ | N | |
| $CH_3$ | $CO_2CH_3$ | $OCH_3$ | $OCH_2CH_2F$ | N | |
| $CH_3$ | $CO_2CH_2CH_3$ | $CH_3$ | $CH_3$ | CH | |
| $CH_3$ | $CO_2CH_2CH_3$ | $CH_3$ | $OCH_3$ | CH | |
| $CH_3$ | $CO_2CH_2CH_3$ | $OCF_2H$ | $OCH_3$ | CH | |
| $CH_3$ | $SO_2N(CH_3)_2$ | $OCH_3$ | $CH_2CH_3$ | N | |
| $CH_3$ | $SO_2N(CH_3)_2$ | $CH_3$ | $OCH_3$ | N | |
| $CH_3$ | $SO_2N(CH_3)_2$ | $OCH_3$ | $OCH_3$ | N | |
| $CH_3$ | $SO_2N(CH_3)_2$ | $OCH_3$ | $OCH_3$ | CH | |
| $CH_3$ | $SO_2N(CH_3)OCH_3$ | $CH_3$ | $CH_3$ | N | |
| $CH_3$ | $SO_2N(CH_3)CH_2CH_3$ | $CH_3$ | $OCH_2CH_3$ | N | |
| $CH_3$ | $SO_2CH_3$ | $OCH_3$ | $OCH_3$ | N | |
| $CH_3$ | $SO_2CH_3$ | $CH_3$ | $CH_3$ | CH | |
| $CH_3$ | $SO_2CH_3$ | $CH_3$ | $OCH_3$ | CH | |
| $CH_3$ | $SO_2CH_3$ | $OCH_2CF_3$ | $OCH_3$ | CH | |
| $CH_3$ | $SO_2CH_3$ | $CH_3$ | $CH_3$ | N | |
| $CH_3$ | $SO_2CH_3$ | $CH_3$ | $OCH_3$ | N | |
| $CH_3$ | $SO_2CH_2CH_3$ | $OCH_3$ | $OCH_3$ | N | |
| $CH_3$ | $NO_2$ | $CH_3$ | △ | CH | |

## Table III (continued)

| $R_7$ | $R_8$ | X | Y | Z | m.p. (°C) |
|---|---|---|---|---|---|
| $CH_3$ | $NO_2$ | $OCH_3$ | $OCH_3$ | CH | |
| $CH_3$ | $NO_2$ | Cl | $OCH_3$ | CH | |
| $CH_3$ | $NO_2$ | $OCH_3$ | $CH_2OCH_3$ | CH | |
| ~~$CH_3$~~ | ~~$NO_2$~~ | ~~$OCH_3$~~ | ~~1,3-dioxolan-2-yl~~ | ~~CH~~ | |
| H | $CO_2CH_3$ | $OCH_3$ | $CH_2F$ | CH | |
| H | $CO_2CH_3$ | $OCH_3$ | $C{\equiv}CH$ | CH | |
| H | $CO_2CH_3$ | $OCH_3$ | $CF_3$ | CH | |
| H | $CO_2CH_3$ | $OCH_3$ | $NHCH_3$ | CH | |
| H | $CO_2CH_3$ | $OCH_3$ | $N(CH_3)_2$ | CH | |
| H | $CO_2CH_3$ | $OCH_3$ | $OCH_2CH{=}CH_2$ | CH | |
| ~~H~~ | ~~$CO_2CH_3$~~ | ~~$OCH_3$~~ | ~~$CH(OCH_2CH_3)_2$~~ | ~~CH~~ | |

Table IV

General Formula 1

(J is $J_4$)

| $R_9$ | $R_{10}$ | $R_{11}$ | X | Y | Z | m.p.(°C) |
|---|---|---|---|---|---|---|
| H | $CO_2CH_3$ | H | $CH_3$ | $CH_3$ | CH | |
| H | $CO_2CH_3$ | H | $CH_3$ | $OCH_3$ | CH | |
| H | $CO_2CH_3$ | H | $OCH_3$ | $OCH_3$ | CH | |
| H | $CO_2CH_3$ | H | $CH_3$ | $CH_3$ | N | |
| H | $CO_2CH_3$ | H | $CH_3$ | $OCH_3$ | N | |
| H | $CO_2CH_2CH_3$ | H | $OCH_3$ | $OCH_3$ | N | |
| H | $CO_2CH_3$ | $CH_3$ | $CH_3$ | $CF_3$ | CH | |
| $CH_3$ | $CO_2CH_3$ | H | $CH_3$ | $OCH_3$ | CH | |
| $CH_3$ | $CO_2CH_3$ | H | $CH_3$ | $OCH_2CH_3$ | CH | |
| $CH_3$ | $CO_2CH_3$ | H | Cl | $OCH_3$ | CH | |
| $CH_3$ | $CO_2CH_3$ | $CH_3$ | $CH_3$ | $CH_3$ | CH | |
| $CH_3$ | $CO_2CH_3$ | $CH_3$ | $CH_3$ | $OCH_3$ | CH | |
| $CH_3$ | $CO_2CH_3$ | $CH_3$ | $OCH_3$ | $OCH_2CH_2OCH_3$ | CH | |
| $CH_3$ | $CO_2CH_3$ | $CH_3$ | $CH_3$ | $CH_3$ | N | |
| $CH_3$ | $CO_2CH_3$ | $CH_3$ | $CH_3$ | $OCH_3$ | N | |
| $CH_3$ | $CO_2CH_3$ | $CH_3$ | $OCH_3$ | $OCH_3$ | N | |
| $CH_3$ | $CO_2CH_3CH$ | H | $CH_3$ | $OCH_3$ | CH | |
| $CH_3$ | $CO_2CH_3CH$ | H | $OCH_3$ | $OCH_3$ | CH | 198-200 |
| $CH_3$ | $CO_2CH_3CH$ | H | $CH_3$ | $OCH_3$ | N | |
| $CH_3$ | $CO_2CH_3CH$ | H | $OCH_3$ | $OCH_3$ | N | 199-201 |
| $CH_3$ | $CO_2CH_3CH$ | H | Cl | $OCH_3$ | CH | |
| $CH_3$ | $CO_2CH_2CH_3$ | $CH_3$ | $OCF_2H$ | $CH_3$ | CH | |
| $CH_3$ | $CO_2CH_2CH_3$ | $CH_3$ | $CH_3$ | $OCH_3$ | CH | |

Table IV (continued)

| $R_9$ | $R_{10}$ | $R_{11}$ | X | Y | Z | m.p. (°C) |
|---|---|---|---|---|---|---|
| $CH_3$ | $CO_2CH_2CH_3$ | $CH_3$ | $OCH_3$ | $OCH_3$ | CH | 190 |
| $CH_3$ | $CO_2CH_2CH_3$ | $CH_3$ | $CH_3$ | $CH_3$ | N | |
| $CH_3$ | $CO_2CH_2CH_3$ | $CH_3$ | $CH_3$ | $OCH_3$ | N | 183-184 |
| $CH_3$ | $CO_2CH_2CH_3$ | $CH_3$ | $OCH_3$ | $OCH_3$ | N | 165-167 |
| $CH_3$ | $CO_2CH_2CH_3$ | $CH_3$ | Cl | $OCH_3$ | CH | 140.5-144.5 |
| $CH_3$ | $CO_2CH_2CH_3$ | $CH_3$ | $OCH_3$ | $OCH_2CH_3$ | CH | |
| $CH_2CH_2CH_3$ | $CO_2CH_2CH_3$ | $CH_3$ | $OCH_3$ | $OCH_3$ | N | |
| $CH_2CH_2CH_3$ | $CO_2CH_2CH_3$ | $CH_3$ | $OCH_3$ | $OCH_3$ | CH | |
| $CH_2CH_2CH_2$ | $CO_2CH_2CH_3$ | $CH_3$ | $CH_3$ | $OCH_3$ | CH | |
| $CH_2CH_2CH_2$ | $CO_2CH_2CH_3$ | H | $OCH_3$ | $CH_2F$ | CH | |
| $CH_2CH_2CH_3$ | $CO_2CH_2CH_3$ | H | $OCH_3$ | $OCH_3$ | N | |
| $CH_2CH_2CH_2$ | $CO_2CH_3$ | H | $OCH_3$ | $OCH_3$ | CH | |
| $CH_2CH_2CH_3$ | $CO_2CH_3$ | $CH_3$ | $OCH_3$ | $OCH_3$ | N | |
| $C_6H_5$ | $CO_2CH_3$ | H | $OCH_3$ | $OCH_3$ | CH | |
| $C_6H_5$ | $CO_2Et$ | $CH_3$ | $CH_3$ | $OCH_3$ | N | |
| $C_6H_5$ | $CO_2Et$ | $CH_3$ | $OCH_3$ | $OCH_3$ | N | |
| $C_6H_5$ | $CO_2CH_3$ | $CH_3$ | $CH_3$ | $CH_3$ | CH | |
| $C_6H_5$ | $CO_2Et$ | $CH_3$ | $CH_3$ | $SCH_3$ | CH | |
| $C_6H_5$ | $CO_2Et$ | $CH_3$ | $OCH_3$ | $OCH_3$ | CH | |
| H | $SO_2N(CH_3)_2$ | H | Br | $OCH_3$ | CH | |
| H | $SO_2N(CH_2CH_3)_2$ | H | $OCH_3$ | $OCH_2CH_3$ | N | |
| H | $SO_2N(CH_3)OCH_3$ | H | $CH_3$ | $OCH_3$ | CH | |
| H | $SO_2N(CH_3)_2$ | $CH_3$ | $OCH_3$ | $OCH_3$ | CH | |
| H | $SO_2N(CH_2CH_3)_2$ | $CH_3$ | $CH_3$ | $CH_2F$ | N | |
| H | $SO_2N(OCH_3)CH_3$ | $CH_3$ | $CH_3$ | $OCH_3$ | N | |
| H | $NO_2$ | $CH_3$ | $OCH_3$ | $OCH_3$ | N | |
| $CH_3$ | $SO_2N(CH_3)_2$ | H | $CH_3$ | $CH_3$ | CH | |
| $CH_3$ | $SO_2N(CH_3)_2$ | $CH_3$ | $CH_3$ | $OCH_3$ | CH | |
| ~~$CH_3$~~ | ~~$SO_2N(CH_3)OCH_3$~~ | ~~H~~ | ~~$OCH_3$~~ | ~~$CH(OCH_3)_2$~~ | ~~CH~~ | |
| $CH_3$ | $SO_2N(CH_3)CH_2CH_3$ | $CH_3$ | $CH_3$ | $CH_3$ | N | |

Table IV (continued)

| $R_9$ | $R_{10}$ | $R_{11}$ | X | Y | Z | m.p. (°C) |
|---|---|---|---|---|---|---|
| $CH_3$ | $NO_2$ | H | $CH_3$ | $OCH_2CH_3$ | N | |
| $CH_3$ | $NO_2$ | $CH_3$ | $OCH_3$ | $OCH_3$ | N | |
| $CH_2CH_2CH_3$ | $SO_2N(CH_3)_2$ | H | $CH_3$ | $CH_3$ | CH | |
| $CH_2CH_2CH_3$ | $SO_2N(CH_3)_2$ | $CH_3$ | $CH_3$ | $OCH_3$ | CH | |
| $CH_2CH_2CH_3$ | $SO_2N(CH_3)OCH_3$ | H | $OCH_3$ | $OCH_3$ | CH | |
| $CH_2CH_2CH_3$ | $SO_2N(CH_3)OCH_3$ | $CH_3$ | $CH_3$ | $CH_3$ | N | |
| $CH_2CH_2CH_3$ | $NO_2$ | H | $CH_3$ | $OCH_3$ | N | |
| $CH_2CH_2CH_3$ | $NO_2$ | $CH_3$ | $OCH_3$ | $OCH_3$ | N | |
| $C_6H_5$ | $SO_2N(CH_3)_2$ | H | $CH_3$ | $CH_3$ | CH | |
| $C_6H_5$ | $SO_2N(CH_3)CH_2CH_3$ | $CH_3$ | $CH_3$ | $OCH_3$ | CH | |
| $C_6H_5$ | $SO_2N(CH_3)OCH_3$ | H | $OCH_3$ | $OCH_3$ | CH | |
| $C_6H_5$ | $SO_2N(CH_3)OCH_3$ | $CH_3$ | $CH_3$ | $CH_2CH_3$ | N | |
| $C_6H_5$ | $NO_2$ | H | $CH_3$ | $OCH_3$ | N | |
| $C_6H_5$ | $NO_2$ | $CH_3$ | $OCH_3$ | $OCH_3$ | N | |
| $C_6H_5$ | $NO_2$ | $CH_3$ | $CH_3$ | $CH_3$ | CH | |
| H | $SO_2CH_2CH_3$ | H | $CH_3$ | $N(CH_3)_2$ | N | |
| H | $SO_2CH_2CH_3$ | $CH_3$ | $OCH_3$ | $OCH_3$ | N | |
| $CH_3$ | $SO_2CH_2CH_3$ | H | $OCH_2CF_3$ | $CH_3$ | CH | |
| $CH_2CH_3$ | $SO_2CH_2CH_3$ | $CH_3$ | $CH_3$ | $OCH_3$ | CH | |
| $CH(CH_3)_2$ | $SO_2CH_2CH_3$ | H | $OCH_3$ | $OCH_3$ | CH | |
| $C_6H_5$ | $SO_2CH_2CH_3$ | $CH_3$ | $CH_3$ | $CH_2CH_2CH_3$ | N | |

## Table V

General Formula 1

(J is $J_5$)

| $R_{10}$ | $R_{11}$ | X | Y | Z | m.p.(°C) |
|---|---|---|---|---|---|
| $CO_2CH_3$ | H | $CH_3$ | $CH_3$ | CH | |
| $CO_2CH_3$ | H | $CH_3$ | $OCH_3$ | CH | |
| $CO_2CH_3$ | H | $OCH_3$ | $OCH_3$ | CH | |
| $CO_2CH_3$ | H | Cl | $OCH_3$ | CH | |
| $CO_2CH_3$ | H | $CH_3$ | $OCH_3$ | N | |
| $CO_2CH_3$ | H | $OCH_3$ | $OCH_3$ | N | |
| $CO_2CH_3$ | $CH_3$ | $CH_3$ | $CH_3$ | CH | |
| $CO_2CH_3$ | $CH_3$ | $CH_3$ | $OCH_3$ | CH | |
| $CO_2CH_3$ | $CH_3$ | $OCH_3$ | $OCH_3$ | CH | |
| $CO_2CH_3$ | $CH_3$ | $CH_3$ | $OCH_2CH_3$ | CH | |
| $CO_2CH_3$ | $CH_3$ | $CH_3$ | $OCH_3$ | N | |
| $CO_2CH_3$ | $CH_3$ | $OCH_3$ | $OCH_3$ | N | |
| $CO_2Et$ | H | $CH_3$ | $CH_3$ | CH | |
| $CO_2Et$ | $CH_3$ | $CH_3$ | $OCH_3$ | CH | |
| $CO_2Et$ | $CH_3$ | $OCH_3$ | $OCH_3$ | CH | 180-183 |
| $CO_2Et$ | $CH_3$ | Br | $OCH_3$ | CH | |
| $CO_2Et$ | $CH_3$ | $CH_3$ | $OCH_2CH_3$ | CH | |
| $CO_2Et$ | $CH_3$ | $CH_3$ | $CH_3$ | N | |
| $CO_2Et$ | $CH_3$ | $CH_3$ | $OCH_3$ | N | 154-157 |
| $CO_2Et$ | $CH_3$ | $OCH_3$ | $OCH_3$ | N | 188-188.5 |
| $CO_2Et$ | $CH_3$ | $OCH_3$ | $OCH_2CH_3$ | N | |
| ~~$CO_2Et$~~ | ~~$CH_3$~~ | ~~$CH_3$~~ | ~~$CH_2OCH_3$~~ | ~~CH~~ | |
| ~~$CO_2Et$~~ | ~~$CH_3$~~ | ~~$OCH_3$~~ | ~~$CH_2OCH_3$~~ | ~~CH~~ | |

Table V (continued)

| $R_{10}$ | $R_{11}$ | X | Y | Z | m.p.(°C) |
|---|---|---|---|---|---|
| $SO_2N(CH_3)_2$ | H | $OCF_2H$ | $CH_3$ | CH | |
| $SO_2N(CH_3)_2$ | H | $CH_3$ | $OCH_3$ | CH | |
| $SO_2N(CH_3)_2$ | H | $OCH_3$ | $OCH_3$ | CH | |
| $SO_2N(CH_3)_2$ | H | $CH_3$ | $CH_3$ | N | |
| $SO_2N(CH_3)_2$ | H | $CH_3$ | $OCH_3$ | N | |
| $SO_2NCH_3(OCH_3)_2$ | H | $OCH_3$ | $OCH_3$ | N | |
| $SO_2N(CH_2CH_3)_2$ | $CH_3$ | $CH_3$ | $CH_3$ | CH | |
| $SO_2N(CH_2CH_3)_2$ | H | $CH_3$ | $OCH_3$ | CH | |
| $SO_2N(CH_2CH_3)_2$ | H | $OCH_3$ | $OCH_3$ | CH | |
| $SO_2N(CH_2CH_3)_2$ | H | $CH_3$ | $CH_3$ | N | |
| $SO_2N(CH_2CH_3)_2$ | H | $CH_3$ | $OCH_3$ | N | |
| $SO_2N(CH_3)CH_2CH_3$ | H | $OCH_3$ | $OCH_3$ | N | |
| $SO_2CH_3$ | H | $CH_3$ | $CH_3$ | CH | |
| $SO_2CH_3$ | H | $CH_3$ | $OCH_3$ | CH | |
| $SO_2CH_3$ | H | $OCH_3$ | $OCH_3$ | CH | |
| $SO_2CH_3$ | H | $OCH_3$ | $CH_3$ | N | |
| $SO_2CH_3$ | H | $CH_3$ | $NHCH_3$ | N | |
| $SO_2CH_3$ | $CH_3$ | $OCH_2CF_3$ | $OCH_3$ | N | |
| $SO_2CH_3$ | $CH_3$ | $OCH_3$ | $OCH_3$ | N | |
| $SO_2CH_2CH_3$ | H | $OCH_3$ | $OCH_3$ | CH | |
| $SO_2CH_2CH_3$ | H | $CH_3$ | $OCH_3$ | CH | |
| $SO_2CH_2CH_3$ | $CH_3$ | $OCH_3$ | $OCH_3$ | CH | |
| $SO_2CH_2CH_3$ | $CH_3$ | $CH_3$ | $CH_3$ | N | |
| $SO_2CH_2CH_3$ | $CH_3$ | $CH_3$ | $OCH_3$ | N | |
| $SO_2CH_2CH_3$ | $CH_3$ | $OCH_3$ | $OCH_3$ | N | |
| $SO_2CH_2CH_3$ | $CH_3$ | $OCH_3$ | $CH_2CH_3$ | CH | |
| $SO_2CH_2CH_3$ | $CH_3$ | $CH_3$ | $OCH_2CH_3$ | CH | |
| $SO_2CH_2CH_3$ | $CH_3$ | Cl | $OCH_3$ | CH | |
| $NO_2$ | H | $OCH_3$ | $CH_3$ | CH | |
| $NO_2$ | H | $OCH_3$ | $CH_3$ | N | |
| $NO_2$ | H | $OCH_3$ | $OCH_2C{\equiv}CH$ | CH | |

Table VI

General Formula 1

(J is $J_6$)

| $R_{12}$ | $R_{13}$ | $R_{14}$ | X | Y | Z | m.p. (°C) |
|---|---|---|---|---|---|---|
| $CO_2CH_3$ | H | H | $CH_3$ | $OCH_3$ | CH | |
| $CO_2CH_3$ | H | H | $OCH_3$ | $OCH_3$ | CH | |
| $CO_2CH_3$ | H | H | $CH_3$ | $CH_3$ | N | |
| $CO_2CH_3$ | H | H | $CH_3$ | $OCH_3$ | N | |
| $CO_2CH_3$ | H | H | $OCH_3$ | $OCH_3$ | N | |
| $CO_2CH_3$ | H | H | $CH_3$ | $CH_3$ | CH | |
| $CO_2CH_2CH_3$ | H | H | $CH_3$ | $OCH_3$ | CH | |
| $CO_2CH_2CH_3$ | H | H | $OCH_2CF_3$ | $OCH_3$ | CH | |
| $CO_2CH_2CH_3$ | H | H | $CH_3$ | $CH_3$ | N | |
| $CO_2CH_2CH_3$ | H | H | $CH_3$ | $OCH_3$ | N | |
| $CO_2CH_2CH_3$ | H | H | $OCH_3$ | $OCH_3$ | N | |
| $SO_2N(CH_3)_2$ | H | H | $CH_3$ | $OCH_3$ | CH | |
| $SO_2N(CH_3)_2$ | H | H | $OCH_3$ | $OCH_3$ | CH | |
| $SO_2N(CH_3)_2$ | H | H | $CH_3$ | $CH_3$ | N | |
| $SO_2N(CH_3)_2$ | H | H | $CH_3$ | $OCH_3$ | N | |
| $SO_2N(CH_3)_2$ | H | H | $OCH_3$ | $OCH_3$ | N | |
| $SO_2N(CH_3)_2$ | H | H | $OCH_3$ | $CH_2CH_3$ | N | |
| $SO_2N(CH_3)OCH_3$ | H | H | $OCH_3$ | $OCH_2CH_3$ | CH | |
| $SO_2N(CH_3)OCH_3$ | H | H | Cl | $OCH_3$ | CH | |
| ~~$SO_2N(CH_3)OCH_3$~~ | ~~H~~ | ~~H~~ | ~~$OCH_3$~~ | ~~$CH_2OCH_3$~~ | ~~CH~~ | |
| $CO_2CH_3$ | H | $CH_3$ | $CH_3$ | $OCH_3$ | CH | |
| $CO_2CH_3$ | H | $CH_3$ | $OCH_3$ | $OCH_3$ | CH | |
| $CO_2CH_3$ | H | $CH_3$ | $CH_3$ | $CH_3$ | N | |

Table VI (continued)

| $R_{12}$ | $R_{13}$ | $R_{14}$ | X | Y | Z | m.p. (°C) |
|---|---|---|---|---|---|---|
| $CO_2CH_3$ | H | $CH_3$ | $CH_3$ | $OCH_3$ | N | |
| $CO_2CH_3$ | H | $CH_3$ | $OCH_3$ | $OCH_3$ | N | |
| $CO_2CH_3$ | H | $CH_3$ | $CH_3$ | $CH_3$ | CH | |
| $CO_2CH_2CH_3$ | H | $CH_3$ | $CH_3$ | $CH_3$ | CH | |
| $CO_2CH_2CH_3$ | H | $CH_3$ | $CH_3$ | $OCH_3$ | CH | |
| $CO_2CH_2CH_3$ | H | $CH_3$ | $OCH_3$ | $OCH_3$ | CH | |
| $CO_2CH_2CH_3$ | H | $CH_3$ | $CH_3$ | $CH_3$ | N | |
| $CO_2CH_2CH_3$ | H | $CH_3$ | $CH_3$ | $OCH_3$ | N | |
| $CO_2CH_2CH_3$ | H | $CH_3$ | $OCH_3$ | $OCH_3$ | N | |
| $SO_2N(CH_3)_2$ | H | $CH_3$ | $CH_3$ | $OCH_3$ | CH | |
| $SO_2N(CH_3)_2$ | H | $CH_3$ | $OCH_3$ | $OCH_3$ | CH | |
| $SO_2N(CH_3)_2$ | H | $CH_3$ | $CH_3$ | $CH_3$ | N | |
| $SO_2N(CH_3)_2$ | H | $CH_3$ | $CH_3$ | $OCH_3$ | N | |
| $SO_2N(CH_3)_2$ | H | $CH_3$ | $OCH_3$ | $OCH_3$ | N | |
| $SO_2N(CH_3)_2$ | H | $CH_3$ | $OCH_3$ | $CH_2CH_3$ | N | |
| $SO_2N(CH_3)OCH_3$ | H | $CH_3$ | $OCH_3$ | $OCH_2CH_2F$ | CH | |
| $SO_2N(CH_3)OCH_3$ | H | $CH_3$ | Cl | $OCH_3$ | CH | |
| ~~$SO_2N(CH_3)OCH_3$~~ | ~~H~~ | ~~$CH_3$~~ | ~~$OCH_3$~~ | ~~$CH_2OCH_3$~~ | ~~CH~~ | |
| $SO_2N(CH_2CH_3)_2$ | H | $CH_3$ | $OCH_3$ | $OCH_3$ | CH | |
| $SO_2CH_3$ | H | $CH_3$ | $OCH_3$ | $CH_3$ | CH | |
| $SO_2CH_2CH_3$ | H | $CH_3$ | $OCH_3$ | $OCH_3$ | N | |
| $NO_2$ | H | $CH_3$ | $OCH_3$ | $OCH_2CH_3$ | CH | |
| $SO_2CH_3$ | H | H | $OCH_3$ | $OCH_3$ | N | |
| ~~$SO_2CH_2CH_3$~~ | ~~H~~ | ~~H~~ | ~~$OCH_3$~~ | ~~$CH_2OCH_3$~~ | ~~CH~~ | |
| $NO_2$ | H | H | $OCH_3$ | $OCH_3$ | N | |
| $NO_2$ | H | H | $OCH_3$ | $\Delta$ | N | |
| H | $CO_2CH_3$ | H | $CH_3$ | $OCH_3$ | CH | |
| H | $CO_2CH_3$ | H | $OCH_3$ | $OCH_3$ | CH | |
| H | $CO_2CH_3$ | H | $CH_3$ | $CH_3$ | N | |
| H | $CO_2CH_3$ | H | $CH_3$ | $OCH_3$ | N | |
| H | $CO_2CH_3$ | H | $OCH_3$ | $OCH_3$ | N | |
| H | $CO_2CH_3$ | H | $CH_3$ | $CH_2F$ | CH | |

Table VI (continued)

| $R_{12}$ | $R_{13}$ | $R_{14}$ | X | Y | Z | m.p. (°C) |
|---|---|---|---|---|---|---|
| H | $CO_2CH_2CH_3$ | H | $CH_3$ | $OCH_3$ | CH | |
| H | $CO_2CH_2CH_3$ | H | $OCH_3$ | $OCH_3$ | CH | |
| H | $CO_2CH_2CH_3$ | H | $CH_3$ | $CH_3$ | N | |
| H | $CO_2CH_2CH_3$ | H | $CH_3$ | $OCH_3$ | N | |
| H | $CO_2CH_2CH_3$ | H | $OCH_3$ | $OCH_3$ | N | |
| H | $CO_2CH_2CH_3$ | H | $CH_3$ | $CH_3$ | CH | |
| H | $SO_2N(CH_3)_2$ | H | $CH_3$ | $OCH_3CH_2OCH_3$ | CH | |
| H | $SO_2N(CH_3)_2$ | H | $OCH_3$ | $OCH_3$ | CH | |
| H | $SO_2N(CH_3)_2$ | H | $CH_3$ | $CH_3$ | N | |
| H | $SO_2N(CH_3)_2$ | H | $CH_3$ | $N(CH_3)_2$ | N | |
| H | $SO_2N(CH_3)_2$ | H | $OCH_3$ | $OCH_3$ | N | |
| H | $SO_2N(CH_3)_2$ | H | $OCH_3$ | $CH_2CH_3$ | N | |
| H | $SO_2N(CH_3)OCH_3$ | H | $OCH_3$ | $OCH_2CH_3$ | CH | |
| H | $SO_2N(CH_3)OCH_3$ | H | Cl | $OCH_3$ | CH | |
| ~~H~~ | ~~$SO_2N(CH_3)OCH_3$~~ | ~~H~~ | ~~$OCH_3$~~ | ~~$CH_2OCH_3$~~ | ~~CH~~ | |
| H | $CO_2CH_3$ | $CH_3$ | $CH_3$ | $OCH_2C{\equiv}CH$ | CH | |
| H | $CO_2CH_3$ | $CH_3$ | $OCH_3$ | $OCH_3$ | CH | |
| H | $CO_2CH_3$ | $CH_3$ | $CH_3$ | $CH_3$ | N | |
| H | $CO_2CH_3$ | $CH_3$ | $CH_3$ | $OCH_3$ | N | |
| H | $CO_2CH_3$ | $CH_3$ | $OCH_3$ | $OCH_3$ | N | |
| H | $CO_2CH_3$ | $CH_3$ | $CH_3$ | $\Delta$ | CH | |
| H | $CO_2CH_2CH_3$ | $CH_3$ | $CH_3$ | $CF_3$ | CH | |
| H | $CO_2CH_2CH_3$ | $CH_3$ | $CH_3$ | $OCH_3$ | CH | |
| H | $CO_2CH_2CH_3$ | $CH_3$ | $OCH_3$ | $OCH_3$ | CH | |
| H | $CO_2CH_2CH_3$ | $CH_3$ | $CH_3$ | $CH_3$ | N | |
| H | $SO_2N(CH_3)_2$ | $CH_3$ | $CH_3$ | $OCH_3$ | CH | |
| H | $SO_2N(CH_3)_2$ | $CH_3$ | $OCH_3$ | $OCH_3$ | CH | |
| H | $SO_2N(CH_3)_2$ | $CH_3$ | $CH_3$ | $CH_3$ | N | |
| H | $SO_2N(CH_3)_2$ | $CH_3$ | $CH_3$ | $OCH_3$ | N | |
| H | $SO_2N(CH_3)_2$ | $CH_3$ | $OCH_3$ | $OCH_3$ | N | |

Table VI (continued)

| $R_{12}$ | $R_{13}$ | $R_{14}$ | X | Y | Z | m.p. (°C) |
|---|---|---|---|---|---|---|
| H | $SO_2N(CH_3)_2$ | $CH_3$ | $OCH_3$ | $CH_2CH_3$ | N | |
| H | $SO_2N(CH_3)OCH_3$ | $CH_3$ | $OCH_3$ | $OCH_2CH_3$ | CH | |
| H | $SO_2N(CH_3)OCH_3$ | $CH_3$ | Cl | $OCH_3$ | CH | |
| H | $SO_2N(CH_3)OCH_3$ | $CH_3$ | $OCH_3$ | $CH_2OCH_3$ | CH | |
| H | $SO_2N(CH_2CH_3)_2$ | $CH_3$ | $OCH_3$ | $OCH_3$ | CH | |
| H | $SO_2CH_3$ | $CH_3$ | $OCH_3$ | $CH_3$ | CH | |
| H | $SO_2CH_2CH_3$ | $CH_3$ | $OCH_3$ | $OCH_3$ | N | |
| H | $NO_2$ | $CH_3$ | $OCH_3$ | $OCH_2CH_3$ | CH | |
| H | $SO_2CH_3$ | H | $OCH_3$ | $OCH_3$ | N | |
| H | $SO_2CH_2CH_3$ | H | $OCH_3$ | $CH_2OCH_3$ | CH | |
| H | $NO_2$ | H | $OCH_3$ | $SCH_3$ | N | |
| H | $NO_2$ | H | $OCH_3$ | 1,3-dioxo-lan-2-yl | CH | |

## Table VII

## General Formula 1

| J | $R_5$ | $R_{19}$ | R | X | Y | Z | m.p.(°C) |
|---|---|---|---|---|---|---|---|
| J-7 | H | $CH_3$ | H | $OCH_3$ | $CH_3$ | CH | |
| J-7 | H | Cl | H | $OCH_3$ | $CH_3$ | CH | |
| J-7 | H | $NO_2$ | H | $OCH_3$ | $CH_3$ | CH | |
| J-7 | H | $SCH_2CH_3$ | H | $OCH_3$ | $N(CH_3)_2$ | CH | |
| J-7 | H | $SO_2CH_3$ | H | $OCH_2CF_3$ | $CH_3$ | N | |
| J-7 | $CH_3$ | $SO_2CH_2CH_3$ | H | $CH_3$ | C≡CH | N | |
| J-7 | $CO_2CH_3$ | H | H | $OCH_3$ | $OCH_3$ | CH | 192-194 |
| J-7 | $CO_2CH_3$ | H | H | $CH_3$ | $OCH_3$ | CH | 155-162 |
| J-7 | $CO_2CH_3$ | H | H | $CH_3$ | $CH_3$ | CH | 153-156 |
| J-7 | $CO_2CH_3$ | H | H | $OCH_3$ | $OCH_3$ | N | 167-169 |
| J-7 | $CO_2CH_3$ | H | H | $CH_3$ | $OCH_3$ | N | 166-167 |
| J-7 | $CO_2CH_3$ | H | H | Cl | $OCH_3$ | CH | 185-186 |
| J-7 | $CO_2CH_3$ | H | $CH_3$ | $OCH_3$ | $OCH_3$ | N | 165-166 |
| J-7 | Cl | H | H | $OCH_3$ | $CH_2CH_2CH_3$ | CH | |
| J-7 | Cl | $CH_3$ | H | $OCH_3$ | $OCH_2CH_3$ | CH | |
| J-7 | $SO_2N(CH_3)_2$ | H | H | $OCH_3$ | $CF_3$ | CH | |
| J-7 | $SO_2N(CH_3)_2$ | H | H | $OCH_3$ | $SCH_3$ | CH | |
| ~~J-7~~ | ~~$SO_2CH_3$~~ | ~~Cl~~ | ~~H~~ | ~~$OCH_3$~~ | ~~$CH(OCH_3)_2$~~ | ~~CH~~ | |
| J-7 | $SO_2CH_3$ | H | H | $OCH_3$ | $OCH_2CH=CH_2$ | CH | |
| J-7 | $SO_2CH_2CH_3$ | H | H | $OCHF_2$ | $CH_3$ | CH | |
| J-8 | H | - | H | $OCH_3$ | $CH_3$ | CH | |
| J-8 | $CH_3$ | - | H | $OCH_3$ | $CH_3$ | N | |
| J-8 | $CO_2CH_3$ | - | H | $OCH_3$ | $CH_3$ | CH | |
| J-8 | $CO_2CH_3$ | - | H | $OCH_3$ | $CH_3$ | N | |
| J-8 | Cl | - | H | $OCH_3$ | $OCH_2CH_2F$ | CH | |
| J-8 | $SO_2N(CH_3)_2$ | - | H | $OCH_3$ | $OCH_2CH_3$ | N | |

Table VII (Continued)

| J | $R_5$ | $R_{19}$ | R | X | Y | Z |
|---|---|---|---|---|---|---|
| J-8 | $SO_2CH_3$ | - | H | $OCH_3$ | $CH_3$ | CH |
| J-8 | $NO_2$ | - | H | $OCH_3$ | $NHCH_3$ | CH |
| J-9 | H | Br | H | $OCF_2H$ | $CH_3$ | CH |
| J-9 | H | $CH_3$ | H | $CH_3$ | $OCH_3$ | N |
| J-9 | $CH_3$ | $NO_2$ | H | $OCH_3$ | $OCF_2H$ | CH |
| J-9 | Cl | $SCH_3$ | H | $CH_3$ | $OCH_3$ | N |
| J-9 | $CO_2CH_3$ | Cl | H | Cl | $OCH_3$ | CH |
| J-9 | $CO_2CH_3$ | $CH_3$ | H | $OCH_3$ | $OCH_3$ | N |
| J-9 | $CO_2CH_3$ | $SO_2CH_3$ | H | Br | $OCH_3$ | CH |
| J-9 | $SO_2N(CH_3)_2$ | H | H | $CH_3$ | $CH_3$ | N |
| J-9 | $SO_2CH_3$ | H | H | $OCH_3$ | $CH_2CH_3$ | CH |
| J-9 | $SO_2CH_2CH_3$ | H | H | $OCH_3$ | 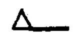 | N |
| J-10 | H | $SO_2CH_2CH_3$ | H | $OCH_3$ | $CH_3$ | CH |
| J-10 | H | $NO_2$ | H | $OCH_3$ | $CH_3$ | N |
| ~~J-10~~ | ~~$CH_3$~~ | ~~Cl~~ | ~~H~~ | ~~$OCH_3$~~ | ~~$CH_2OCH_3$~~ | ~~CH~~ |
| J-10 | Cl | Cl | H | $OCH_3$ | $OCH_3$ | N |
| J-10 | $CO_2CH_3$ | $SCH_3$ | H | $OCH_3$ | $OCH_3$ | CH |
| J-10 | $CO_2CH_3$ | H | H | $OCH_3$ | $OCH_3$ | N |
| ~~J-10~~ | ~~$CO_2CH_2CH_3$~~ | ~~H~~ | ~~H~~ | ~~$OCH_3$~~ | ~~$CH(OEt)_2$~~ | ~~CH~~ |
| J-10 | $SO_2N(CH_3)Et$ | H | H | $OCH_3$ | $OCH_3$ | N |
| J-10 | $SO_2CH_3$ | H | $CH_3$ | $OCH_3$ | $OCH_3$ | CH |
| J-10 | $SO_2CH_3$ | $CH_3$ | H | $OCH_3$ | $OCH_3$ | N |
| J-11 | Cl | - | H | $OCH_3$ | $OCH_3$ | CH |
| J-11 | Br | - | H | $OCH_3$ | $OCH_3$ | N |
| J-11 | $CH_3$ | - | H | $OCH_3$ | $OCH_2C\equiv CH$ | CH |
| J-11 | $CO_2CH_3$ | - | H | $OCH_3$ | $CH_3$ | N |
| J-11 | $CO_2CH_3$ | - | H | $OCH_3$ | $CF_3$ | CH |
| J-11 | $CO_2CH_2CH_3$ | - | H | $OCH_3$ | $OCH_3$ | N |
| J-11 | $SO_2N(CH_3)_2$ | - | H | $CH_3$ | $C\equiv CH$ | CH |
| J-11 | $SO_2CH_3$ | - | H | $CH_3$ | $N(CH_3)_2$ | N |
| J-11 | $SO_2CH_2CH_3$ | - | H | $CH_3$ | $CH_2F$ | CH |

Table VIII

| General Formula 4 | | | | | | |
|---|---|---|---|---|---|---|
| J | $R_4$ | $R_5$ | $R_6$ | $R_{19}$ | $X_1$ | $Y_1$ |
| $J_2$ | H | $CH_3$ | $CH_3$ | - | $CH_3$ | O |
| $J_2$ | $CH_3$ | Cl | $CH_3$ | - | $OCH_3$ | $CH_2$ |
| $J_2$ | H | Br | $CH_2CH_2CH_3$ | - | $OCH_3$ | O |
| $J_2$ | $CH_3$ | $CO_2CH_3$ | $CH_2CH_2CH_3$ | - | $OCH_3$ | O |
| $J_2$ | $CH_3$ | $SO_2N(CH_3)_2$ | $CH_3$ | - | $CH_3$ | O |
| $J_2$ | $CH_3$ | H | $SO_2CH_3$ | - | $OCH_3$ | $CH_2$ |
| $J_2$ | $CH_3$ | $SO_2CH_2CH_3$ | $CH_3$ | - | $CH_3$ | O |
| $J_7$ | - | $CH_3$ | - | $SCH_3$ | $OCH_3$ | O |
| $J_7$ | - | Cl | - | $CH_3$ | $OCH_3$ | $CH_2$ |
| $J_7$ | - | $CO_2CH_3$ | - | $CH_3$ | $CH_3$ | O |
| $J_7$ | - | H | - | $SO_2CH_3$ | $OCH_3$ | O |
| $J_7$ | - | $SO_2CH_3$ | - | Cl | $OCH_3$ | O |
| $J_7$ | - | $SO_2N(CH_3)_2$ | - | H | $CH_3$ | O |
| $J_8$ | - | $CH_3$ | - | - | $OCH_3$ | $CH_2$ |
| $J_8$ | - | Cl | - | - | $CH_3$ | O |
| $J_8$ | - | $CO_2CH_3$ | - | - | $OCH_3$ | O |
| $J_8$ | - | $CO_2CH_2CH_3$ | - | - | $OCH_2CH_3$ | O |
| $J_8$ | - | $NO_2$ | - | - | $CH_3$ | O |
| $J_8$ | - | $SO_2CH_3$ | - | - | $OCF_2H$ | $CH_2$ |
| $J_8$ | - | $SO_2CH_3$ | - | - | $OCH_3$ | $CH_2$ |
| $J_9$ | - | Cl | - | $CH_3$ | $OCH_3$ | O |
| $J_9$ | - | $CO_2CH_3$ | - | $CH_3$ | $OCH_3$ | O |
| $J_9$ | - | $SO_2CH_2CH_3$ | - | $CH_3$ | $CH_3$ | O |
| $J_{10}$ | - | $SO_2CH_3$ | - | $CH_3$ | $CH_3$ | O |
| $J_{10}$ | - | Cl | - | $CH_3$ | $OCH_3$ | $CH_2$ |
| $J_{10}$ | - | $CO_2CH_3$ | - | $SCH_3$ | $OCH_3$ | O |
| $J_{10}$ | - | $SO_2CH_3$ | - | Cl | $CH_3$ | $CH_2$ |
| $J_{11}$ | - | Cl | - | - | $OCH_3$ | O |
| $J_{11}$ | - | $CO_2CH_3$ | - | - | $OCH_3$ | O |
| $J_{11}$ | - | $SO_2N(CH_3)_2$ | - | - | $OCH_3$ | $CH_2$ |
| $J_{11}$ | - | $SO_2CH_3$ | - | - | $CH_3$ | O |

Table IX

| General Formula 4 | | | | | | | |
|---|---|---|---|---|---|---|---|
| J | $R_1$ | $R_2$ | $R_3$ | $R_7$ | $R_8$ | $X_1$ | $Y_1$ |
| $J_1$ | $CH_3$ | H | H | - | - | $CH_3$ | O |
| $J_1$ | $CH_3$ | $CH_3$ | $CH_3$ | - | - | $OCH_3$ | $CH_2$ |
| $J_1$ | $CO_2CH_3$ | $CH_3$ | $CH_3$ | - | - | $CH_3$ | $CH_2$ |
| $J_1$ | $CO_2CH_3$ | H | H | - | - | $OCH_3$ | O |
| $J_1$ | $CO_2CH_3$ | H | H | - | - | $OCF_2H$ | $CH_2$ |
| $J_1$ | $CO_2CH_3$ | $CH_3$ | H | - | - | $OCH_3$ | O |
| $J_1$ | $CO_2CH_2CH_3$ | H | H | - | - | $CH_3$ | O |
| $J_1$ | $CO_2CH_2CH_3$ | $CH_3$ | H | - | - | $CH_3$ | O |
| $J_1$ | $SO_2N(CH_3)_2$ | H | H | - | - | $CH_3$ | O |
| $J_1$ | $SO_2N(CH_3)_2$ | $CH_3$ | $CH_3$ | - | - | $OCH_2CH_3$ | $CH_2$ |
| $J_1$ | $SO_2N(CH_2CH_3)_2$ | H | H | - | - | $OCH_3$ | O |
| $J_1$ | $SO_2CH_3$ | H | H | - | - | $OCH_3$ | $CH_2$ |
| $J_1$ | $SO_2CH_3$ | H | $CH_3$ | - | - | $OCH_3$ | O |
| $J_1$ | $SO_2CH_3$ | $CH_3$ | $CH_3$ | - | - | $CH_3$ | O |
| $J_1$ | $SO_2CH_2CH_3$ | $CH_3$ | $CH_3$ | - | - | $CH_3$ | $CH_2$ |
| $J_3$ | - | - | - | H | $CO_2CH_3$ | $CH_3$ | O |
| $J_3$ | - | - | - | H | $CO_2CH_2CH_3$ | $CH_3$ | O |
| $J_3$ | - | - | - | $CH_3$ | $SO_2N(CH_3)_2$ | $OCH_3$ | $CH_2$ |
| $J_3$ | - | - | - | $CH_3$ | $SO_2N(OCH_3)CH_3$ | $OCH_3$ | $CH_2$ |
| $J_3$ | - | - | - | H | $SO_2CH_3$ | $OCH_3$ | O |
| $J_3$ | - | - | - | H | $SO_2CH_3$ | $OCH_3$ | $CH_2$ |
| $J_3$ | - | - | - | $CH_3$ | $SO_2CH_2CH_3$ | $CH_3$ | O |
| $J_3$ | - | - | - | $CH_3$ | $NO_2$ | $CH_3$ | O |

Table X

| General Formula 4 | | | | | | | | |
|---|---|---|---|---|---|---|---|---|
| J | $R_9$ | $R_{10}$ | $R_{11}$ | $R_{12}$ | $R_{13}$ | $R_{14}$ | $X_1$ | $Y_1$ |
| $J_4$ | H | $CO_2CH_3$ | H | - | - | - | $OCH_3$ | $CH_2$ |
| $J_4$ | $CH_3$ | $CO_2CH_3$ | $CH_3$ | - | - | - | $CH_3$ | O |
| $J_4$ | $CH_3$ | $CO_2CH_3$ | $CH_3$ | - | - | - | $CH_3$ | O |
| $J_4$ | $CH_3$ | $CO_2CH_2CH_3$ | H | - | - | - | $OCH_3$ | O |
| $J_4$ | $CH_3$ | $SO_2N(CH_3)_2$ | $CH_3$ | - | - | - | $OCH_3$ | O |
| $J_4$ | $CH_3$ | $SO_2N(OCH_3)CH_3$ | $CH_3$ | - | - | - | $OCH_2CH_3$ | $CH_2$ |
| $J_4$ | $CH_3$ | $SO_2CH_3$ | $CH_3$ | - | - | - | $CH_3$ | O |
| $J_4$ | $CH_3$ | $SO_2CH_3$ | $CH_3$ | - | - | - | $OCF_2H$ | O |
| $J_4$ | $CH_2CH_2CH_3$ | $CO_2CH_3$ | $CH_3$ | - | - | - | $OCH_3$ | $CH_2$ |
| $J_4$ | $CH_3$ | $NO_2$ | $CH_3$ | - | - | - | $OCH_3$ | O |
| $J_4$ | $CH_3$ | $SO_2CH_2CH_3$ | H | - | - | - | $CH_3$ | O |
| $J_4$ | Ph | $CO_2CH_3$ | H | - | - | - | $OCH_3$ | $CH_2$ |
| $J_5$ | - | $CO_2CH_3$ | H | - | - | - | $OCH_3$ | O |
| $J_5$ | - | $CO_2CH_3$ | $CH_3$ | - | - | - | $OCH_3$ | O |
| $J_5$ | - | $CO_2H_3$ | H | - | - | - | $CH_3$ | O |
| $J_5$ | - | $SO_2N(CH_3)_2$ | H | - | - | - | $OCH_3$ | $CH_2$ |
| $J_5$ | - | $SO_2N(CH_3)Et$ | H | - | - | - | $OCH_3$ | O |
| $J_5$ | - | $SO_2CH_3$ | $CH_3$ | - | - | - | $CH_3$ | O |
| $J_5$ | - | $SO_2CH_3$ | $CH_3$ | - | - | - | $OCH_3$ | O |
| $J_5$ | - | $SO_2CH_2CH_3$ | $CH_3$ | - | - | - | $OCH_3$ | $CH_2$ |
| $J_5$ | - | $NO_2$ | H | - | - | - | $OCH_3$ | O |
| $J_6$ | - | - | - | H | $CO_2CH_3$ | H | $CH_3$ | O |
| $J_6$ | - | - | - | H | $CO_2CH_3$ | $CH_3$ | $OCH_3$ | $CH_2$ |
| $J_6$ | - | - | - | $CH_3$ | H | $CH_3$ | $CH_3$ | O |
| $J_6$ | - | - | - | H | $SO_2N(CH_3)_2$ | $CH_3$ | $OCH_3$ | O |
| $J_6$ | - | - | - | $NO_2$ | H | $CH_3$ | $OCH_3$ | O |
| $J_6$ | - | - | - | H | $SO_2CH_3$ | $CH_3$ | $OCH_3$ | $CH_2$ |
| $J_6$ | - | - | - | $CO_2CH_3$ | H | H | $CH_3$ | O |
| $J_6$ | - | - | - | $CO_2CH_3$ | H | $CH_3$ | $CH_3$ | O |
| $J_6$ | - | - | - | $SO_2CH_3$ | H | $CH_3$ | $OCH_3$ | $CH_2$ |

Table XI

| General Formula 5 | | | | | | |
|---|---|---|---|---|---|---|
| J | $R_1$ | $R_2$ | $R_3$ | $R_7$ | $R_8$ | $X_1$ |
| $J_1$ | $CH_3$ | H | H | - | - | $CH_3$ |
| $J_1$ | $CH_3$ | $CH_3$ | $CH_3$ | - | - | $OCH_3$ |
| $J_1$ | $CO_2CH_3$ | $CH_3$ | $CH_3$ | - | - | $CH_3$ |
| $J_1$ | $CO_2CH_3$ | H | H | - | - | $OCH_3$ |
| $J_1$ | $CO_2CH_3$ | H | H | - | - | $OCF_2H$ |
| $J_1$ | $CO_2CH_3$ | $CH_3$ | H | - | - | $OCH_3$ |
| $J_1$ | $CO_2CH_2CH_3$ | H | H | - | - | $CH_3$ |
| $J_1$ | $CO_2CH_2CH_3$ | $CH_3$ | H | - | - | $CH_3$ |
| $J_1$ | $SO_2N(CH_3)_2$ | H | H | - | - | $CH_3$ |
| $J_1$ | $SO_2N(CH_3)_2$ | H | $CH_3$ | - | - | $OCH_3$ |
| $J_1$ | $SO_2N(CH_3)_2$ | $CH_3$ | $CH_3$ | - | - | $OCH_2CH_3$ |
| $J_1$ | $SO_2N(CH_2CH_3)_2$ | H | H | - | - | $OCH_3$ |
| $J_1$ | $SO_2CH_3$ | H | H | - | - | $OCH_3$ |
| $J_1$ | $SO_2CH_3$ | H | $CH_3$ | - | - | $OCH_3$ |
| $J_1$ | $SO_2CH_3$ | $CH_3$ | $CH_3$ | - | - | $CH_3$ |
| $J_1$ | $SO_2CH_2CH_3$ | $CH_3$ | $CH_3$ | - | - | $CH_3$ |
| $J_3$ | - | - | - | H | $CO_2CH_3$ | $CH_3$ |
| $J_3$ | - | - | - | H | $CO_2CH_2CH_3$ | $CH_3$ |
| $J_3$ | - | - | - | H | $SO_2N(CH_3)_2$ | $OCH_3$ |
| $J_3$ | - | - | - | $CH_3$ | $SO_2N(OCH_3)CH_3$ | $OCH_3$ |
| $J_3$ | - | - | - | H | $SO_2CH_3$ | $OCH_3$ |
| $J_3$ | - | - | - | H | $SO_2CH_3$ | $OCH_2CH_3$ |
| $J_3$ | - | - | - | $CH_3$ | $SO_2CH_3$ | $CH_3$ |
| $J_3$ | - | - | - | $CH_3$ | $NO_2$ | $CH_3$ |

## Table XII

## General Formula 5

| J | $R_4$ | $R_5$ | $R_6$ | $R_{19}$ | $X_1$ |
|---|---|---|---|---|---|
| $J_2$ | H | $CH_3$ | $CH_3$ | - | $CH_3$ |
| $J_2$ | $CH_3$ | Cl | $CH_3$ | - | $OCH_3$ |
| $J_2$ | H | Br | $CH_2CH_2CH_3$ | - | $OCF_2H$ |
| $J_2$ | $CH_3$ | $CO_2CH_3$ | $CH_3$ | - | $OCH_3$ |
| $J_2$ | $CH_3$ | $SO_2N(CH_3)_2$ | $CH_3$ | - | $CH_3$ |
| $J_2$ | $CH_3$ | $SO_2CH_3$ | $SO_2CH_3$ | - | $OCH_3$ |
| $J_2$ | $CH_3$ | $SO_2CH_2CH_3$ | $CH_3$ | - | $CH_3$ |
| $J_7$ | - | $CH_3$ | - | $SCH_3$ | $OCH_3$ |
| $J_7$ | - | Cl | - | $CH_3$ | $OCH_3$ |
| $J_7$ | - | $CO_2CH_3$ | - | $CH_3$ | $CH_3$ |
| $J_7$ | - | H | - | $SO_2CH_3$ | $OCH_3$ |
| $J_7$ | - | $SO_2CH_3$ | - | Cl | $OCH_3$ |
| $J_7$ | - | $SO_2N(CH_3)_2$ | - | $SCH_3$ | $CH_3$ |
| $J_8$ | - | $CH_3$ | - | - | $OCH_3$ |
| $J_8$ | - | Cl | - | - | $CH_3$ |
| $J_8$ | - | $CO_2CH_3$ | - | - | $OCH_3$ |
| $J_8$ | - | $CO_2CH_2CH_3$ | - | - | $OCH_2CH_3$ |
| $J_8$ | - | $NO_2$ | - | - | $CH_3$ |
| $J_8$ | - | $SO_2CH_3$ | - | - | $OCH_3$ |
| $J_9$ | - | Cl | - | $CH_3$ | $OCH_3$ |
| $J_9$ | - | $CO_2CH_3$ | - | H | $OCH_3$ |
| $J_9$ | - | $SO_2CH_3$ | - | $CH_3$ | $CH_3$ |
| $J_{10}$ | - | Cl | - | $CH_3$ | $OCH_3$ |
| $J_{10}$ | - | $CO_2CH_3$ | - | $SCH_3$ | $OCH_3$ |
| $J_{10}$ | - | $SO_2CH_3$ | - | Cl | $CH_3$ |
| $J_{11}$ | - | Cl | - | - | $OCH_3$ |

Table XII (Continued)

| J | $R_4$ | $R_5$ | $R_6$ | $R_{19}$ | $X_1$ |
|---|---|---|---|---|---|
| $J_{11}$ | - | $CO_2CH_3$ | - | - | $OCH_3$ |
| $J_{11}$ | - | $SO_2N(CH_3)_2$ | - | - | $OCH_3$ |
| $J_{11}$ | - | $SO_2CH_3$ | - | - | $CH_3$ |

Table XIII

| General Formula 5 | | | | | | | |
|---|---|---|---|---|---|---|---|
| J | $R_9$ | $R_{10}$ | $R_{11}$ | $R_{12}$ | $R_{13}$ | $R_{14}$ | $X_1$ |
| $J_4$ | H | $CO_2CH_3$ | H | - | - | - | $OCH_3$ |
| $J_4$ | $CH_3$ | $CO_2CH_3$ | $CH_3$ | - | - | - | $CH_3$ |
| $J_4$ | $CH_3$ | $CO_2CH_2CH_3$ | H | - | - | - | $OCH_3$ |
| $J_4$ | $CH_3$ | $SO_2N(CH_3)_2$ | $CH_3$ | - | - | - | $OCH_2CH_3$ |
| $J_4$ | $CH_3$ | $SO_2CH_3$ | $CH_3$ | - | - | - | $CH_3$ |
| $J_4$ | $CH_3$ | $SO_2CH_3$ | $CH_3$ | - | - | - | $OCF_2H$ |
| $J_4$ | $CH_2CH_2CH_2$ | $CO_2CH_3$ | $CH_3$ | - | - | - | $OCH_3$ |
| $J_4$ | $CH_3$ | $NO_2$ | $CH_3$ | - | - | - | $OCH_3$ |
| $J_4$ | $CH_3$ | $SO_2CH_2CH_3$ | H | - | - | - | $CH_3$ |
| $J_4$ | Ph | $CO_2CH_3$ | H | - | - | - | $OCH_3$ |
| $J_5$ | - | $CO_2CH_3$ | $CH_3$ | - | - | - | $OCH_3$ |
| $J_5$ | - | $CO_2CH_3$ | H | - | - | - | $OCH_3$ |
| $J_5$ | - | $CO_2H_3$ | H | - | - | - | $CH_3$ |
| $J_5$ | - | $SO_2N(CH_3)_2$ | H | - | - | - | $OCH_3$ |
| $J_5$ | - | $SO_2N(CH_3)Et$ | H | - | - | - | $OCH_3$ |
| $J_5$ | - | $SO_2CH_3$ | $CH_3$ | - | - | - | $CH_3$ |
| $J_5$ | - | $SO_2CH_3$ | $CH_3$ | - | - | - | $OCH_3$ |
| $J_5$ | - | $SO_2CH_2CH_3$ | $CH_3$ | - | - | - | $OCH_3$ |
| $J_5$ | - | $NO_2$ | H | - | - | - | $OCH_3$ |
| $J_6$ | - | - | - | H | $CO_2CH_3$ | H | $CH_3$ |
| $J_6$ | - | - | - | H | $CO_2CH_3$ | $CH_3$ | $OCH_3$ |
| $J_6$ | - | - | - | $CH_3$ | H | $CH_3$ | $CH_3$ |
| $J_6$ | - | - | - | H | $SO_2N(CH_3)_2$ | $CH_3$ | $OCH_3$ |
| $J_6$ | - | - | - | $NO_2$ | H | $CH_3$ | $OCH_3$ |
| $J_6$ | - | - | - | H | $SO_2CH_3$ | $CH_3$ | $OCH_3$ |
| $J_6$ | - | - | - | $CO_2CH_3$ | H | $CH_3$ | $CH_3$ |
| $J_6$ | - | - | - | $CO_2CH_3$ | H | $CH_3$ | $CH_3$ |
| $J_6$ | - | - | - | $SO_2CH_3$ | H | $CH_3$ | $OCH_3$ |

Table XIV

| General Formula 6 | | | | | | | |
|---|---|---|---|---|---|---|---|
| J | $R_1$ | $R_2$ | $R_3$ | $R_7$ | $R_8$ | $X_1$ | $Y_3$ |
| $J_1$ | $CH_3$ | H | H | - | - | $CH_3$ | H |
| $J_1$ | $CH_3$ | $CH_3$ | $CH_3$ | - | - | $OCH_3$ | H |
| $J_1$ | $CO_2CH_3$ | $CH_3$ | $CH_3$ | - | - | $CH_3$ | H |
| $J_1$ | $CO_2CH_3$ | H | H | - | - | $OCH_3$ | H |
| $J_1$ | $CO_2CH_3$ | H | H | - | - | $OCF_2H$ | $CH_3$ |
| $J_1$ | $CO_2CH_3$ | $CH_3$ | H | - | - | $OCH_3$ | $CH_3$ |
| $J_1$ | $CO_2CH_2CH_3$ | H | H | - | - | $CH_3$ | $CH_3$ |
| $J_1$ | $CO_2CH_2CH_3$ | $CH_3$ | H | - | - | $CH_3$ | H |
| $J_1$ | $SO_2N(CH_3)_2$ | H | H | - | - | $CH_3$ | H |
| $J_1$ | $SO_2N(CH_3)_2$ | H | $CH_3$ | - | - | $OCH_3$ | H |
| $J_1$ | $SO_2N(CH_3)_2$ | $CH_3$ | $CH_3$ | - | - | $OCH_2CH_3$ | $CH_3$ |
| $J_1$ | $SO_2N(CH_2CH_3)_2$ | H | H | - | - | $OCH_3$ | $CH_3$ |
| $J_1$ | $SO_2CH_3$ | H | H | - | - | $OCH_3$ | $CH_3$ |
| $J_1$ | $SO_2CH_3$ | H | $CH_3$ | - | - | $OCH_3$ | H |
| $J_1$ | $SO_2CH_3$ | $CH_3$ | $CH_3$ | - | - | $CH_3$ | H |
| $J_1$ | $SO_2CH_2CH_3$ | $CH_3$ | $CH_3$ | - | - | $CH_3$ | H |
| $J_3$ | - | - | - | H | $CO_2CH_3$ | $CH_3$ | $CH_3$ |
| $J_3$ | - | - | - | H | $CO_2CH_2CH_3$ | $CH_3$ | $CH_3$ |
| $J_3$ | - | - | - | $CH_3$ | $SO_2N(CH_3)_2$ | $OCH_3$ | $CH_3$ |
| $J_3$ | - | - | - | $CH_3$ | $SO_2N(OCH_3)CH_3$ | $OCH_3$ | H |
| $J_3$ | - | - | - | H | $SO_2CH_3$ | $OCH_3$ | H |
| $J_3$ | - | - | - | $CH_3$ | $SO_2CH_3$ | $OCH_3$ | H |
| $J_3$ | - | - | - | $CH_3$ | $SO_2CH_3$ | $CH_3$ | $CH_3$ |
| $J_3$ | - | - | - | $CH_3$ | $NO_2$ | $CH_3$ | $CH_3$ |

Table XV

| General Formula 6 | | | | | | |
|---|---|---|---|---|---|---|
| J | $R_4$ | $R_5$ | $R_6$ | $R_{19}$ | $X_1$ | $Y_3$ |
| $J_2$ | H | $CH_3$ | $CH_3$ | - | $CH_3$ | H |
| $J_2$ | $CH_3$ | Cl | $CH_3$ | - | $OCH_3$ | H |
| $J_2$ | H | Br | $CH_2CH_2CH_3$ | - | $OCH_3$ | H |
| $J_2$ | $CH_3$ | $CO_2CH_3$ | $CH_3$ | - | $OCH_3$ | $CH_3$ |
| $J_2$ | $CH_3$ | $SO_2N(CH_3)_2$ | $CH_3$ | - | $CH_3$ | $CH_3$ |
| $J_2$ | $CH_3$ | H | $SO_2CH_3$ | - | $OCH_3$ | $CH_3$ |
| $J_2$ | $CH_3$ | $SO_2CH_2CH_3$ | $CH_3$ | - | $CH_3$ | H |
| $J_7$ | - | $CH_3$ | - | $SCH_3$ | $OCH_3$ | $CH_3$ |
| $J_7$ | - | Cl | - | $CH_3$ | $OCH_3$ | $CH_3$ |
| $J_7$ | - | $CO_2CH_3$ | - | $CH_3$ | $CH_3$ | $CH_3$ |
| $J_7$ | - | H | - | $SO_2CH_3$ | $OCH_3$ | $CH_3$ |
| $J_7$ | - | $SO_2CH_3$ | - | Cl | $OCH_3$ | $CH_3$ |
| $J_7$ | - | $SO_2N(CH_3)_2$ | - | $SCH_3$ | $CH_3$ | H |
| $J_8$ | - | $CH_3$ | - | - | $OCH_3$ | H |
| $J_8$ | - | Cl | - | - | $CH_3$ | H |
| $J_8$ | - | $CO_2CH_3$ | - | - | $OCH_3$ | H |
| $J_8$ | - | $CO_2CH_2CH_3$ | - | - | $OCH_2CH_3$ | $CH_3$ |
| $J_8$ | - | $NO_2$ | - | - | $CH_3$ | $CH_3$ |
| $J_8$ | - | $SO_2CH_3$ | - | - | $OCH_3$ | $CH_3$ |
| $J_9$ | - | Cl | - | $CH_3$ | $OCH_3$ | H |
| $J_9$ | - | $CO_2CH_3$ | - | $CH_3$ | $OCH_3$ | H |
| $J_9$ | - | $SO_2CH_3$ | - | $CH_3$ | $CH_3$ | $CH_3$ |
| $J_{10}$ | - | Cl | - | $CH_3$ | $OCH_3$ | H |
| $J_{10}$ | - | $CO_2CH_3$ | - | $SCH_3$ | $OCH_3$ | $CH_3$ |
| $J_{10}$ | - | $SO_2CH_3$ | - | Cl | $CH_3$ | $CH_3$ |
| $J_{11}$ | - | Cl | - | - | $OCF_2H$ | $CH_3$ |
| $J_{11}$ | - | $CO_2CH_3$ | - | - | $OCH_3$ | H |
| $J_{11}$ | - | $SO_2N(CH_3)_2$ | - | - | $OCH_3$ | H |
| $J_{11}$ | - | $SO_2CH_3$ | - | - | $CH_3$ | H |

Table XVI

| General Formula 6 | | | | | | | | |
|---|---|---|---|---|---|---|---|---|
| J | $R_9$ | $R_{10}$ | $R_{11}$ | $R_{12}$ | $R_{13}$ | $R_{14}$ | $X_1$ | $Y_3$ |
| $J_4$ | H | $CO_2CH_3$ | H | - | - | - | $OCH_3$ | H |
| $J_4$ | $CH_3$ | $CO_2CH_3$ | $CH_3$ | - | - | - | $CH_3$ | H |
| $J_4$ | $CH_3$ | $CO_2CH_2CH_3$ | H | - | - | - | $OCH_3$ | H |
| $J_4$ | $CH_3$ | $SO_2N(CH_3)_2$ | $CH_3$ | - | - | - | $OCH_3$ | H |
| $J_4$ | $CH_3$ | $SO_2N(OCH_3)CH_3$ | $CH_3$ | - | - | - | $OCH_2CH_3$ | $CH_3$ |
| $J_4$ | $CH_3$ | $SO_2CH_3$ | $CH_3$ | - | - | - | $OCF_2H$ | $CH_3$ |
| $J_4$ | $CH_2CH_2CH_3$ | $CO_2CH_3$ | $CH_3$ | - | - | - | $OCH_3$ | $CH_3$ |
| $J_4$ | $CH_3$ | $NO_2$ | $CH_3$ | - | - | - | $OCH_3$ | $CH_3$ |
| $J_4$ | $CH_3$ | $SO_2CH_2CH_3$ | H | - | - | - | $CH_3$ | $CH_3$ |
| $J_4$ | Ph | $CO_2CH_3$ | H | - | - | - | $OCH_3$ | $CH_3$ |
| $J_5$ | - | $CO_2CH_3$ | H | - | - | - | $OCH_2CH_3$ | H |
| $J_5$ | - | $CO_2CH_3$ | H | - | - | - | $OCH_3$ | H |
| $J_5$ | - | $CO_2CH_3$ | H | - | - | - | $CH_3$ | H |
| $J_5$ | - | $SO_2N(CH_3)_2$ | H | - | - | - | $OCH_3$ | H |
| $J_5$ | - | $SO_2N(CH_3)Et$ | H | - | - | - | $OCH_3$ | H |
| $J_5$ | - | $SO_2CH_3$ | $CH_3$ | - | - | - | $CH_3$ | H |
| $J_5$ | - | $SO_2CH_3$ | $CH_3$ | - | - | - | $OCH_3$ | H |
| $J_5$ | - | $SO_2CH_2CH_3$ | $CH_3$ | - | - | - | $OCH_3$ | $CH_3$ |
| $J_5$ | - | $NO_2$ | H | - | - | - | $OCH_3$ | $CH_3$ |
| $J_6$ | - | - | - | H | $CO_2CH_3$ | H | $CH_3$ | $CH_3$ |
| $J_6$ | - | - | - | H | $CO_2CH_3$ | $CH_3$ | $OCH_3$ | $CH_3$ |
| $J_6$ | - | - | - | $CH_3$ | H | $CH_3$ | $CH_3$ | $CH_3$ |
| $J_6$ | - | - | - | H | $SO_2N(CH_3)_2$ | $CH_3$ | $OCH_3$ | $CH_3$ |
| $J_6$ | - | - | - | $NO_2$ | H | $CH_3$ | $OCH_3$ | H |
| $J_6$ | - | - | - | H | $SO_2CH_3$ | $CH_3$ | $OCH_3$ | H |
| $J_6$ | - | - | - | $CO_2CH_3$ | H | H | $CH_3$ | H |
| $J_6$ | - | - | - | $CO_2CH_3$ | H | $CH_3$ | $CH_3$ | H |
| $J_6$ | - | - | - | $SO_2CH_3$ | $CH_3$ | $CH_3$ | $OCH_3$ | H |

Table XVII

| General Formula 7 | | | | | | |
|---|---|---|---|---|---|---|
| J | $R_4$ | $R_5$ | $R_6$ | $R_{19}$ | $X_2$ | $Y_2$ |
| $J_2$ | H | $CH_3$ | $CH_3$ | - | $CH_3$ | $OCH_3$ |
| $J_2$ | $CH_3$ | Cl | $CH_3$ | - | $CH_3$ | $OCH_3$ |
| $J_2$ | H | Br | $CH_2CH_2CH_3$ | - | $CH_3$ | $OCH_3$ |
| $J_2$ | $CH_3$ | $CO_2CH_3$ | $CH_3$ | - | $CH_3$ | $OCH_3$ |
| $J_2$ | $CH_3$ | $SO_2N(CH_3)_2$ | $CH_3$ | - | $CH_3$ | $OCH_3$ |
| $J_2$ | $CH_3$ | H | $SO_2CH_3$ | - | $CH_3$ | $CH_3$ |
| $J_2$ | $CH_3$ | $SO_2CH_2CH_3$ | $CH_3$ | - | $C_2H_5$ | $OCH_2CH_3$ |
| $J_7$ | - | $CH_3$ | - | $SCH_3$ | $CH_2CF_3$ | $OCH_3$ |
| $J_7$ | - | Cl | - | $CH_3$ | $CH_3$ | $OCH_3$ |
| $J_7$ | - | $CO_2CH_3$ | - | $CH_3$ | $CH_3$ | $OCH_3$ |
| $J_7$ | - | H | - | $SO_2CH_3$ | $CH_3$ | $OCH_3$ |
| $J_7$ | - | $SO_2CH_3$ | - | Cl | $CH_3$ | $SCH_3$ |
| $J_7$ | - | $SO_2N(CH_3)_2$ | - | $SCH_3$ | $C_2H_5$ | $OCH_3$ |
| $J_8$ | - | $CH_3$ | - | - | $CH_2CF_3$ | $OCH_3$ |
| $J_8$ | - | Cl | - | - | $CH_3$ | $OCH_3$ |
| $J_8$ | - | $CO_2CH_3$ | - | - | $CH_3$ | $SCF_2H$ |
| $J_8$ | - | $CO_2CH_2CH_3$ | - | - | $CH_3$ | $OCH_3$ |
| $J_8$ | - | $NO_2$ | - | - | $CH_3$ | $OCH_3$ |
| $J_8$ | - | $SO_2CH_3$ | - | - | $CH_2CH_3$ | $OCH_3$ |
| $J_9$ | - | Cl | - | $CH_3$ | $CH_3$ | $OCH_3$ |
| $J_9$ | - | $CO_2CH_3$ | - | $SO_2CH_3$ | $CH_3$ | $CH_3$ |
| $J_9$ | - | $SO_2CH_3$ | - | $CH_3$ | $CH_3$ | $OCH_3$ |
| $J_{10}$ | - | Cl | - | $CH_3$ | $CH_3$ | $SCH_2CH_3$ |
| $J_{10}$ | - | $CO_2CH_3$ | - | $SCH_3$ | $C_2H_5$ | $OCH_3$ |
| $J_{10}$ | - | $SO_2CH_3$ | - | Cl | $CH_2CF_3$ | $OCH_3$ |
| $J_{11}$ | - | Cl | - | - | $CH_3$ | $OCH_3$ |
| $J_{11}$ | - | $CO_2CH_3$ | - | - | $CH_3$ | $SC_2H_5$ |
| $J_{11}$ | - | $SO_2N(CH_3)_2$ | - | - | $CH_3$ | $OCH_3$ |
| $J_{11}$ | - | $SO_2CH_3$ | - | - | $CH_3$ | $OCH_3$ |

Table XVIII

| General Formula 7 | | | | | | | |
|---|---|---|---|---|---|---|---|
| J | $R_1$ | $R_2$ | $R_3$ | $R_7$ | $R_8$ | $X_2$ | $Y_2$ |
| $J_1$ | $CH_3$ | H | H | - | - | $CH_3$ | $OCH_3$ |
| $J_1$ | $CH_3$ | $CH_3$ | $CH_3$ | - | - | $CH_3$ | $OCH_3$ |
| $J_1$ | $CO_2CH_3$ | $CH_3$ | $CH_3$ | - | - | $CH_3$ | $OCH_3$ |
| $J_1$ | $CO_2CH_3$ | H | H | - | - | $CH_3$ | $OCH_3$ |
| $J_1$ | $CO_2CH_3$ | H | H | - | - | $CH_3$ | $OCH_3$ |
| $J_1$ | $CO_2CH_3$ | $CH_3$ | H | - | - | $C_2H_5$ | $OCH_3$ |
| $J_1$ | $CO_2CH_2CH_3$ | H | H | - | - | $CH_2CF_3$ | $OCH_3$ |
| $J_1$ | $CO_2CH_2CH_3$ | $CH_3$ | H | - | - | $CH_3$ | $OCH_3$ |
| $J_1$ | $SO_2N(CH_3)_2$ | H | H | - | - | $CH_3$ | $CH_3$ |
| $J_1$ | $SO_2N(CH_3)_2$ | H | $CH_3$ | - | - | $CH_3$ | OEt |
| $J_1$ | $SO_2N(CH_3)_2$ | $CH_3$ | $CH_3$ | - | - | $CH_3$ | $OCH_3$ |
| $J_1$ | $SO_2N(CH_2CH_3)_2$ | H | H | - | - | $CH_3$ | $OCH_3$ |
| $J_1$ | $SO_2CH_3$ | H | H | - | - | $CH_3$ | $OCH_3$ |
| $J_1$ | $SO_2CH_3$ | H | $CH_3$ | - | - | $CH_3$ | $SCH_3$ |
| $J_1$ | $SO_2CH_3$ | $CH_3$ | $CH_3$ | - | - | $CH_3$ | $CH_2CH_3$ |
| $J_1$ | $SO_2CH_2CH_3$ | $CH_3$ | $CH_3$ | - | - | $C_2H_5$ | $OCH_3$ |
| $J_3$ | - | - | - | H | $CO_2CH_3$ | $CH_2CF_3$ | $OCH_3$ |
| $J_3$ | - | - | - | H | $CO_2CH_2CH_3$ | $CH_3$ | $OCH_3$ |
| $J_3$ | - | - | - | $CH_3$ | $SO_2N(CH_3)_2$ | $CH_3$ | $SCF_2H$ |
| $J_3$ | - | - | - | $CH_3$ | $SO_2N(OCH_3)CH_3$ | $CH_3$ | $OCH_3$ |
| $J_3$ | - | - | - | H | $SO_2CH_3$ | $CH_3$ | $OCH_3$ |
| $J_3$ | - | - | - | H | $SO_2CH_3$ | $CH_3$ | $OCH_3$ |
| $J_3$ | - | - | - | $CH_3$ | $SO_2CH_2CH_3$ | $CH_3$ | $SCH_2CH_3$ |
| $J_3$ | - | - | - | $CH_3$ | $NO_2$ | $CH_3$ | $OCH_3$ |

Table XIX

| General Formula 7 | | | | | | | | |
|---|---|---|---|---|---|---|---|---|
| J | $R_9$ | $R_{10}$ | $R_{11}$ | $R_{12}$ | $R_{13}$ | $R_{14}$ | $X_2$ | $Y_2$ |
| $J_4$ | H | $CO_2CH_3$ | H | - | - | - | $CH_3$ | $OCH_3$ |
| $J_4$ | $CH_3$ | $CO_2CH_3$ | $CH_3$ | - | - | - | $CH_3$ | $OCH_3$ |
| $J_4$ | $CH_3$ | $CO_2CH_2CH_3$ | H | - | - | - | $CH_3$ | $OCH_3$ |
| $J_4$ | $CH_3$ | $SO_2N(CH_3)_2$ | $CH_3$ | - | - | - | $CH_3$ | $OCH_3$ |
| $J_4$ | $CH_3$ | $SO_2N(OCH_3)CH_3$ | $CH_3$ | - | - | - | $CH_3$ | $OCH_3$ |
| $J_4$ | $CH_3$ | $SO_2CH_3$ | $CH_3$ | - | - | - | $C_2H_5$ | $OCH_3$ |
| $J_4$ | $CH_3$ | $SO_2CH_3$ | $CH_3$ | - | - | - | $CH_3$ | $OCH_3$ |
| $J_4$ | $CH_2CH_2CH_3$ | $CO_2CH_3$ | $CH_3$ | - | - | - | $CH_3$ | $CH_3$ |
| $J_4$ | $CH_3$ | $NO_2$ | $CH_3$ | - | - | - | $OCH_3$ | $OCH_2CH_3$ |
| $J_4$ | $CH_3$ | $SO_2CH_2CH_3$ | H | - | - | - | $CH_3$ | $OCH_3$ |
| $J_4$ | Ph | $CO_2CH_3$ | H | - | - | - | $CH_2CF_3$ | $CH_3$ |
| $J_5$ | - | $CO_2CH_3$ | H | - | - | - | $CH_3$ | $OCH_3$ |
| $J_5$ | - | $CO_2CH_3$ | H | - | - | - | $CH_3$ | $CH_2CH_3$ |
| $J_5$ | - | $CO_2CH_3$ | H | - | - | - | $CH_3$ | $OCH_3$ |
| $J_5$ | - | $SO_2N(CH_3)_2$ | H | - | - | - | $CH_3$ | $OCH_3$ |
| $J_5$ | - | $SO_2N(CH_3)Et$ | H | - | - | - | $CH_3$ | $SCHF_2$ |
| $J_5$ | - | $SO_2CH_3$ | $CH_3$ | - | - | - | $CH_3$ | $OCH_3$ |
| $J_5$ | - | $SO_2CH_3$ | $CH_3$ | - | - | - | $CH_2CH_3$ | $OCH_3$ |
| $J_5$ | - | $SO_2CH_2CH_3$ | $CH_3$ | - | - | - | $CH_3$ | $OCH_3$ |
| $J_5$ | - | $NO_2$ | H | - | - | - | $CH_3$ | $OCH_3$ |
| $J_6$ | - | - | - | H | $CO_2CH_3$ | H | $CH_3$ | $SCH_2CH_3$ |
| $J_6$ | - | - | - | H | $CO_2CH_3$ | $CH_3$ | $CH_3$ | $OCH_3$ |
| $J_6$ | - | - | - | $CH_3$ | $CO_2CH_3$ | $CH_3$ | $CH_3$ | $OCH_3$ |
| $J_6$ | - | - | - | H | $SO_2N(CH_3)_2$ | $CH_3$ | $CH_3$ | $OCH_3$ |
| $J_6$ | - | - | - | $CH_3$ | $SO_2N(CH_3)_2$ | $CH_3$ | $CH_3$ | $OCH_3$ |
| $J_6$ | - | - | - | H | $SO_2CH_3$ | $CH_3$ | $CH_2CF_3$ | $OCH_3$ |
| $J_6$ | - | - | - | $CO_2CH_3$ | H | H | $CH_3$ | $OCH_3$ |
| $J_6$ | - | - | - | $CO_2CH_3$ | H | $CH_3$ | $CH_3$ | $OCH_3$ |
| $J_6$ | - | - | - | $SO_2CH_3$ | $CH_3$ | $CH_3$ | $CH_3$ | $OCH_3$ |

Table XX

| General Formula 8 | | | | | | | |
|---|---|---|---|---|---|---|---|
| J | $R_9$ | $R_{10}$ | $R_{11}$ | $R_{12}$ | $R_{13}$ | $R_{14}$ | $X_3$ |
| $J_4$ | H | $CO_2CH_3$ | H | - | - | - | $CH_3$ |
| $J_4$ | $CH_3$ | $CO_2CH_3$ | $CH_3$ | - | - | - | $OCH_3$ |
| $J_4$ | $CH_3$ | $CO_2CH_2CH_3$ | H | - | - | - | $CH_3$ |
| $J_4$ | $CH_3$ | $SO_2N(CH_3)_2$ | $CH_3$ | - | - | - | $OCH_3$ |
| $J_4$ | $CH_3$ | $SO_2N(OCH_3)CH_3$ | $CH_3$ | - | - | - | $CH_3$ |
| $J_4$ | $CH_3$ | $SO_2CH_3$ | $CH_3$ | - | - | - | $OCH_3$ |
| $J_4$ | $CH_3$ | $SO_2CH_3$ | $CH_3$ | - | - | - | $CH_3$ |
| $J_4$ | $CH_2CH_2CH_3$ | $CO_2CH_3$ | $CH_3$ | - | - | - | $OCH_3$ |
| $J_4$ | $CH_3$ | $NO_2$ | $CH_3$ | - | - | - | $CH_3$ |
| $J_4$ | $CH_3$ | $SO_2CH_2CH_3$ | H | - | - | - | $OCH_3$ |
| $J_4$ | Ph | $CO_2CH_3$ | H | - | - | - | $CH_3$ |
| $J_5$ | - | $CO_2CH_3$ | H | - | - | - | $OCH_3$ |
| $J_5$ | - | $CO_2CH_3$ | H | - | - | - | $CH_3$ |
| $J_5$ | - | $CO_2CH_3$ | $CH_3$ | - | - | - | $OCH_3$ |
| $J_5$ | - | $SO_2N(CH_3)_2$ | H | - | - | - | $CH_3$ |
| $J_5$ | - | $SO_2N(CH_3)Et$ | H | - | - | - | $OCH_3$ |
| $J_5$ | - | $SO_2CH_3$ | $CH_3$ | - | - | - | $CH_3$ |
| $J_5$ | - | $SO_2CH_3$ | $CH_3$ | - | - | - | $OCH_3$ |
| $J_5$ | - | $SO_2CH_2CH_3$ | $CH_3$ | - | - | - | $CH_3$ |
| $J_5$ | - | $NO_2$ | H | - | - | - | $OCH_3$ |
| $J_6$ | - | - | - | H | $CO_2CH_3$ | H | $CH_3$ |
| $J_6$ | - | - | - | H | $CO_2CH_3$ | $CH_3$ | $OCH_3$ |
| $J_6$ | - | - | - | $CH_3$ | H | $CH_3$ | $CH_3$ |
| $J_6$ | - | - | - | H | $SO_2N(CH_3)_2$ | $CH_3$ | $OCH_3$ |
| $J_6$ | - | - | - | $NO_2$ | H | $CH_3$ | $CH_3$ |
| $J_6$ | - | - | - | H | $SO_2CH_3$ | $CH_3$ | $OCH_3$ |
| $J_6$ | - | - | - | $CO_2CH_3$ | H | H | $CH_3$ |
| $J_6$ | - | - | - | $CO_2CH_3$ | H | $CH_3$ | $OCH_3$ |
| $J_6$ | - | - | - | $SO_2CH_3$ | $CH_3$ | $CH_3$ | $CH_3$ |

Table XXI

| General Formula 8 | | | | | | |
|---|---|---|---|---|---|---|
| J | $R_1$ | $R_2$ | $R_3$ | $R_7$ | $R_8$ | $X_3$ |
| $J_1$ | $CH_3$ | H | H | - | - | $CH_3$ |
| $J_1$ | $CH_3$ | $CH_3$ | $CH_3$ | - | - | $OCH_3$ |
| $J_1$ | $CO_2CH_3$ | $CH_3$ | $CH_3$ | - | - | $CH_3$ |
| $J_1$ | $CO_2CH_3$ | H | H | - | - | $OCH_3$ |
| $J_1$ | $CO_2CH_3$ | H | H | - | - | $CH_3$ |
| $J_1$ | $CO_2CH_3$ | $CH_3$ | H | - | - | $OCH_3$ |
| $J_1$ | $CO_2CH_2CH_3$ | H | H | - | - | $CH_3$ |
| $J_1$ | $CO_2CH_2CH_3$ | $CH_3$ | H | - | - | $CH_3$ |
| $J_1$ | $SO_2N(CH_3)_2$ | H | H | - | - | $OCH_3$ |
| $J_1$ | $SO_2N(CH_3)_2$ | H | $CH_3$ | - | - | $CH_3$ |
| $J_1$ | $SO_2N(CH_3)_2$ | $CH_3$ | $CH_3$ | - | - | $OCH_3$ |
| $J_1$ | $SO_2N(CH_2CH_3)_2$ | H | H | - | - | $CH_3$ |
| $J_1$ | $SO_2CH_3$ | H | H | - | - | $OCH_3$ |
| $J_1$ | $SO_2CH_3$ | H | $CH_3$ | - | - | $CH_3$ |
| $J_1$ | $SO_2CH_3$ | $CH_3$ | $CH_3$ | - | - | $CH_3$ |
| $J_1$ | $SO_2CH_2CH_3$ | $CH_3$ | $CH_3$ | - | - | $OCH_3$ |
| $J_3$ | - | - | - | H | $CO_2CH_3$ | $CH_3$ |
| $J_3$ | - | - | - | H | $CO_2CH_2CH_3$ | $OCH_3$ |
| $J_3$ | - | - | - | $CH_3$ | $SO_2N(CH_3)_2$ | $CH_3$ |
| $J_3$ | - | - | - | $CH_3$ | $SO_2N(OCH_3)CH_3$ | $OCH_3$ |
| $J_3$ | - | - | - | H | $SO_2CH_3$ | $CH_3$ |
| $J_3$ | - | - | - | H | $SO_2CH_3$ | $OCH_3$ |
| $J_3$ | - | - | - | $CH_3$ | $SO_2CH_3$ | $CH_3$ |
| $J_3$ | - | - | - | $CH_3$ | $NO_2$ | $OCH_3$ |

Table XXII

| General Formula 8 | | | | | |
|---|---|---|---|---|---|
| J | $R_4$ | $R_5$ | $R_6$ | $R_{19}$ | $X_3$ |
| $J_2$ | H | $CH_3$ | $CH_3$ | - | $CH_3$ |
| $J_2$ | $CH_3$ | Cl | $CH_3$ | - | $OCH_3$ |
| $J_2$ | H | Br | $CH_2CH_2CH_3$ | - | $CH_3$ |
| $J_2$ | $CH_3$ | $CO_2CH_3$ | $CH_3$ | - | $OCH_3$ |
| $J_2$ | $CH_3$ | $SO_2N(CH_3)_2$ | $CH_3$ | - | $CH_3$ |
| $J_2$ | $CH_3$ | H | $SO_2CH_3$ | - | $OCH_3$ |
| $J_2$ | $CH_3$ | $SO_2CH_2CH_3$ | $CH_3$ | - | $CH_3$ |
| $J_7$ | - | $CH_3$ | - | $SCH_3$ | $OCH_3$ |
| $J_7$ | - | Cl | - | $CH_3$ | $CH_3$ |
| $J_7$ | - | $CO_2CH_3$ | - | $CH_3$ | $OCH_3$ |
| $J_7$ | - | H | - | $SO_2CH_3$ | $CH_3$ |
| $J_7$ | - | $SO_2CH_3$ | - | Cl | $OCH_3$ |
| $J_7$ | - | $SO_2N(CH_3)_2$ | - | $SCH_3$ | $CH_3$ |
| $J_8$ | - | $CH_3$ | - | - | $OCH_3$ |
| $J_8$ | - | Cl | - | - | $CH_3$ |
| $J_8$ | - | $CO_2CH_3$ | - | - | $OCH_3$ |
| $J_8$ | - | $CO_2CH_2CH_3$ | - | - | $CH_3$ |
| $J_8$ | - | $NO_2$ | - | - | $OCH_3$ |
| $J_8$ | - | $SO_2CH_3$ | - | - | $CH_3$ |
| $J_9$ | - | Cl | - | $CH_3$ | $OCH_3$ |
| $J_9$ | - | $CO_2CH_3$ | - | $CH_3$ | $CH_3$ |
| $J_9$ | - | $SO_2CH_3$ | - | $CH_3$ | $OCH_3$ |
| $J_{10}$ | - | Cl | - | $CH_3$ | $CH_3$ |
| $J_{10}$ | - | $CO_2CH_3$ | - | $SCH_3$ | $OCH_3$ |
| $J_{10}$ | - | $SO_2CH_3$ | - | Cl | $CH_3$ |
| $J_{11}$ | - | Cl | - | - | $OCH_3$ |
| $J_{11}$ | - | $CO_2CH_3$ | - | - | $CH_3$ |
| $J_{11}$ | - | $SO_2N(CH_3)_2$ | - | - | $OCH_3$ |
| $J_{11}$ | - | $SO_2CH_3$ | - | - | $CH_3$ |

## Table XXIII

## General Formula 2

| J | n | L | $R_{20}$ | $R_{22}$ | X | Y | m.p. (°C) |
|---|---|---|---|---|---|---|---|
| $J_{12}$ | 0 | $CH_2$ | H | $CH_3$ | $CH_3$ | $OCH_3$ | ~~179-183~~ |
| $J_{12}$ | 0 | $CH_2$ | H | $CH_3$ | $OCH_3$ | $OCH_3$ | ~~184-187~~ |
| $J_{12}$ | 0 | $CH_2$ | H | $CH_3$ | $CH_3$ | $CH_3$ | ~~191-198~~ |
| $J_{12}$ | 0 | $CH_2$ | H | $CH_3$ | $OCH_3$ | $C_2H_5$ | ~~179-180~~ |
| $J_{12}$ | 0 | $CH_2$ | H | $CH_3$ | $CH_3$ | $OC_2H_5$ | ~~170-172~~ |
| ~~$J_{12}$~~ | ~~0~~ | ~~$CH_2$~~ | ~~H~~ | ~~$CH_3$~~ | ~~$OCH_3$~~ | ~~$CH_2OCH_3$~~ | ~~156-161~~ |
| $J_{12}$ | 0 | $CH_2$ | H | $CH_3$ | Cl | $OCH_3$ | |
| $J_{12}$ | 0 | $CH_2$ | H | $CH_3$ | Br | $OCH_3$ | |
| $J_{12}$ | 0 | $CH_2$ | H | $CH_3$ | $OCH_3$ | $CH_2F$ | |
| $J_{12}$ | 0 | $CH_2$ | H | $CH_3$ | $OCH_3$ | $CF_3$ | |
| $J_{12}$ | 1 | $CH_2$ | H | $CH_3$ | $OCH_3$ | $OCH_3$ | |
| $J_{12}$ | 0 | $CH_2$ | H | $C_2H_5$ | $OCH_3$ | $OCH_3$ | |
| $J_{12}$ | 0 | $CH_2$ | H | $\underline{n}$-$C_3H_7$ | $OCH_3$ | $OCH_3$ | |
| $J_{12}$ | 0 | $CH_2$ | H | $CH(CH_3)_2$ | $OCH_3$ | $OCH_3$ | |
| $J_{12}$ | 0 | $CH_2$ | H | H | $CH_3$ | $CH_3$ | |
| $J_{12}$ | 0 | $CH_2$ | H | H | $OCH_3$ | $OCH_3$ | |
| $J_{12}$ | 0 | $CH_2$ | H | H | $CH_3$ | $OCH_3$ | |
| $J_{12}$ | 0 | $CH_2$ | H | H | $OCH_3$ | Cl | |
| $J_{12}$ | 0 | O | H | H | $CH_3$ | $CH_3$ | |
| $J_{12}$ | 0 | O | H | H | $CH_3$ | $OCH_3$ | |
| $J_{12}$ | 0 | O | H | H | $OCH_3$ | $OCH_3$ | |
| $J_{12}$ | 0 | O | H | H | $OCH_3$ | Cl | |
| $J_{12}$ | 0 | O | H | $CH_3$ | $OCH_3$ | $OCH_3$ | |
| $J_{12}$ | 0 | O | H | $CH_3$ | $CH_3$ | $OCH_3$ | |
| $J_{12}$ | 0 | O | H | $CH_3$ | $CH_3$ | $CH_3$ | |
| $J_{12}$ | 0 | O | H | $CH_3$ | Cl | $OCH_3$ | |
| $J_{12}$ | 0 | O | H | $CH_3$ | Br | $OCH_3$ | |
| $J_{12}$ | 0 | O | H | $C_2H_5$ | $OCH_3$ | $OCH_3$ | |
| $J_{12}$ | 0 | O | H | $\underline{n}$-$C_3H_7$ | $OCH_3$ | $OCH_3$ | |

Table XXIII (continued)

| J | n | L | $R_{20}$ | $R_{22}$ | X | Y | m.p. (°C) |
|---|---|---|---|---|---|---|---|
| $J_{12}$ | 0 | O | H | $CH(CH_3)_2$ | $OCH_3$ | $OCH_3$ | |
| $J_{12}$ | 1 | O | H | $CH_3$ | $OCH_3$ | $OCH_3$ | |
| $J_{12}$ | 0 | $CH_2$ | 5-F | $CH_3$ | $OCH_3$ | $OCH_3$ | |
| $J_{12}$ | 0 | $CH_2$ | 6-Cl | $CH_3$ | $OCH_3$ | $OCH_3$ | |
| $J_{12}$ | 0 | $CH_2$ | 6-Br | $CH_3$ | $OCH_3$ | $OCH_3$ | |
| $J_{12}$ | 0 | $CH_2$ | 6-$CH_3$ | $CH_3$ | $OCH_3$ | $OCH_3$ | |
| $J_{12}$ | 0 | $CH_2$ | 6-$OCH_3$ | $CH_3$ | $OCH_3$ | $OCH_3$ | |
| $J_{12}$ | 0 | O | H | $CH_3$ | $OCH_2CF_3$ | $CH_3$ | |
| $J_{12}$ | 0 | O | H | $CH_3$ | $OCHF_2$ | $OCH_3$ | |
| $J_{12}$ | 0 | O | H | $CH_3$ | $OCH_3$ | $CH(CH_3)_2$ | |
| $J_{12}$ | 0 | O | H | $CH_3$ | $OCH_3$ | cyclopropyl | |
| $J_{12}$ | 0 | O | H | $CH_3$ | $OCH_3$ | C≡CH | |
| $J_{12}$ | 0 | O | H | $CH_3$ | $OCH_3$ | $NH_2$ | |
| $J_{12}$ | 0 | O | H | $CH_3$ | $OCH_3$ | $N(CH_3)_2$ | |
| $J_{12}$ | 0 | O | H | $CH_3$ | $OCH_3$ | $OCH_2CH_2F$ | |
| $J_{12}$ | 0 | O | H | $CH_3$ | $OCH_3$ | $SCH_3$ | |
| $J_{12}$ | 0 | O | H | $CH_3$ | $OCH_3$ | $OCH_2CH=CH_2$ | |
| $J_{12}$ | 0 | O | H | $CH_3$ | $OCH_3$ | $OCH_2CH_2OCH_3$ | |
| $J_{12}$ | 0 | O | H | $CH_3$ | $OCH_3$ | $OCH_2C≡CH$ | |
| ~~$J_{12}$~~ | ~~0~~ | ~~O~~ | ~~H~~ | ~~$CH_3$~~ | ~~$OCH_3$~~ | ~~$CH(OCH_3)_2$~~ | |
| $J_{12}$ | 0 | O | H | $CH_3$ | $OCH_3$ | $C(CH_3)(OCH_3)_2$ | |
| $J_{12}$ | 0 | O | H | $CH_3$ | $OCH_3$ | 1,3-dioxolan-2-yl | |
| $J_{12}$ | 0 | O | H | $CH_3$ | $OCH_3$ | 2-methyl-1,3-dioxolan-2-yl | |
| $J_{12}$ | 0 | O | H | $CH_3$ | $OCH_3$ | $CH(OC_2H_5)_2$ | |
| ~~$J_{12}$~~ | ~~0~~ | ~~O~~ | ~~H~~ | ~~$CH_3$~~ | ~~$OCH_3$~~ | ~~$C(CH_3)(OC_2H_5)_2$~~ | |
| $J_{12}$ | 0 | O | H | $CH_3$ | $OCH_3$ | $NHCH_3$ | |
| $J_{12}$ | 0 | O | H | $CH_3$ | Cl | $NHCH_3$ | |
| $J_{12}$ | 0 | O | H | $-CH_2CN$ | $OCH_3$ | $OCH_3$ | |
| $J_{12}$ | 0 | O | H | $-(CH_2)_3CN$ | $CH_3$ | $OCH_3$ | |
| $J_{12}$ | 0 | $CH_2$ | H | OH | $CH_3$ | $OCH_3$ | |
| $J_{12}$ | 0 | $CH_2$ | H | $-(CH_2)_3CH(CH_3)_2$ | Cl | $OCH_3$ | |

Table XXIII (continued)

| J | n | L | $R_{20}$ | $R_{22}$ | X | Y | m.p. (°C) |
|---|---|---|---|---|---|---|---|
| $J_{12}$ | 0 | $CH_2$ | H | $-CF_2H$ | $OCH_3$ | $OCH_3$ | |
| $J_{12}$ | 0 | $CH_2$ | H | $-CH_2CH_2Cl$ | Cl | $OCH_3$ | |
| $J_{12}$ | 0 | $CH_2$ | H | $-(CH_2)_5Cl$ | $CH_3$ | $OCH_3$ | |
| $J_{12}$ | 0 | $CH_2$ | H | $-CH_2OCH_3$ | Cl | $OCH_3$ | |
| $J_{12}$ | 0 | $CH_2$ | H | $-(CH_2)_4OCH_3$ | $CH_3$ | $OCH_3$ | |
| $J_{12}$ | 0 | $CH_2$ | H | $-CH_2CH{=}CH_2$ | $OCH_3$ | $OCH_3$ | |
| $J_{12}$ | 0 | $CH_2$ | H | $-CH_2CH{=}CHCH_3$ | $CH_3$ | $OCH_3$ | |
| $J_{12}$ | 0 | $CH_2$ | H | $-CH_2C{\equiv}CH$ | $OCH_3$ | $OCH_3$ | |
| $J_{12}$ | 0 | $CH_2$ | H | $-CH_2C{\equiv}C-CH_3$ | Cl | $OCH_3$ | |
| $J_{12}$ | 0 | $CH_2$ | H | $-(CH_2)_4C{\equiv}CH$ | $CH_3$ | $OCH_3$ | |
| $J_{12}$ | 0 | $CH_2$ | H | $-OCH_3$ | $CH_3$ | $OCH_3$ | |
| $J_{12}$ | 0 | $CH_2$ | H | $-O(CH_2)_4CH_3$ | Cl | $OCH_3$ | |
| $J_{12}$ | 0 | $CH_2$ | H | $-C_6H_5$ | $CH_3$ | $OCH_3$ | |
| $J_{12}$ | 0 | $CH_2$ | H | cyclopropyl | $OCH_3$ | $OCH_3$ | |
| $J_{12}$ | 0 | $CH_2$ | H | cyclohexyl | $OCH_3$ | $OCH_3$ | |
| $J_{12}$ | 0 | $CH_2$ | H | cyclopropylmethyl | $OCH_3$ | $OCH_3$ | |
| $J_{12}$ | 0 | $CH_2$ | H | cyclohexylmethyl | Cl | $OCH_3$ | |
| $J_{12}$ | 0 | $CH_2$ | H | $-C(O)CH_3$ | $OCH_3$ | $OCH_3$ | |
| $J_{12}$ | 0 | $CH_2$ | H | $-C(O)(CH_2)_5H$ | Cl | $OCH_3$ | |
| $J_{12}$ | 0 | $CH_2$ | H | $-CO_2C_2H_5$ | Br | $OCH_3$ | |
| $J_{12}$ | 0 | $CH_2$ | H | $-CO_2(CH_2)_4H$ | Cl | $OCH_3$ | |
| $J_{12}$ | 0 | $CH_2$ | H | $-CH_2C_6H_5$ | $OCH_3$ | $OCH_3$ | |
| $J_{12}$ | 0 | $CH_2$ | H | $-CH_2-CH{=}CH-CH_2Cl$ | $CH_3$ | $OCH_3$ | |
| $J_{12}$ | 0 | $CH_2$ | H | $-CH_2C{\equiv}C-CH_2Cl$ | Cl | $OCH_3$ | |
| $J_{12}$ | 0 | $CH_2$ | H | $-CH_2C(O)CH_3$ | $OCH_3$ | $OCH_3$ | |
| $J_{12}$ | 0 | $CH_2$ | H | $-CH(CH_3)CO_2C_2H_5$ | $OCH_3$ | $OCH_3$ | |
| $J_{12}$ | 0 | $CH_2$ | H | H | $CH_3$ | $OCH_3$ | |
| $J_{12}$ | 0 | $CH_2$ | H | H | $CH_3$ | $CH_3$ | |
| $J_{12}$ | 0 | $CH_2$ | H | H | Cl | $OCH_3$ | |
| $J_{12}$ | 0 | O | H | H | $OCH_3$ | $OCH_3$ | |
| $J_{12}$ | 0 | O | H | H | $CH_3$ | $OCH_3$ | |
| $J_{12}$ | 0 | O | H | H | $CH_3$ | $CH_3$ | |
| $J_{12}$ | 0 | O | H | H | Cl | $OCH_3$ | |

Table XXIII (continued)

| J | n | L | $R_{20}$ | $R_{22}$ | X | Y | m.p. (°C) |
|---|---|---|---|---|---|---|---|
| $J_{12}$ | 0 | $CH_2$ | H | $n$-$C_4H_9$ | $CH_3$ | $CH_3$ | |
| $J_{12}$ | 0 | $CH_2$ | H | $n$-$C_4H_9$ | $CH_3$ | $OCH_3$ | |
| $J_{12}$ | 0 | $CH_2$ | H | $n$-$C_4H_9$ | $OCH_3$ | $OCH_3$ | |
| $J_{12}$ | 0 | $CH_2$ | H | $n$-$C_4H_9$ | Cl | $OCH_3$ | |
| $J_{12}$ | 0 | O | H | $n$-$C_4H_9$ | $CH_3$ | $CH_3$ | |
| $J_{12}$ | 0 | O | H | $n$-$C_4H_9$ | $CH_3$ | $OCH_3$ | |
| $J_{12}$ | 0 | O | H | $n$-$C_4H_9$ | $OCH_3$ | $OCH_3$ | |
| $J_{12}$ | 0 | O | H | $n$-$C_4H_9$ | Cl | $OCH_3$ | |
| $J_{18}$ | - | $CH_2$ | H | $CH_3$ | $OCH_3$ | $OCH_3$ | |
| $J_{18}$ | - | $CH_2$ | H | $CH_3$ | $OCH_3$ | $CH_3$ | |
| $J_{18}$ | - | $CH_2$ | H | $CH_3$ | $CH_3$ | $CH_3$ | |
| $J_{18}$ | - | $CH_2$ | H | $CH_3$ | Cl | $OCH_3$ | |
| $J_{18}$ | - | $CH_2$ | H | $CH_3$ | Br | $OCH_3$ | |
| $J_{18}$ | - | $CH_2$ | H | $CH_3$ | $OCH_3$ | $C_2H_5$ | |
| $J_{18}$ | - | $CH_2$ | H | $CH_3$ | $CH_3$ | $OC_2H_5$ | |
| ~~$J_{18}$~~ | ~~-~~ | ~~$CH_2$~~ | ~~H~~ | ~~$CH_3$~~ | ~~$OCH_3$~~ | ~~$CH_2OCH_3$~~ | |
| $J_{18}$ | - | $CH_2$ | H | $CH_3$ | $OCH_3$ | $CH_2F$ | |
| $J_{18}$ | - | $CH_2$ | H | $CH_3$ | $OCH_3$ | $CF_3$ | |
| $J_{18}$ | - | $CH_2$ | H | H | $OCH_3$ | $OCH_3$ | |
| $J_{18}$ | - | $CH_2$ | H | $C_2H_5$ | $OCH_3$ | $OCH_3$ | |
| $J_{18}$ | - | $CH_2$ | H | $n$-$C_3H_7$ | $OCH_3$ | $OCH_3$ | |
| $J_{18}$ | - | $CH_2$ | H | $CH(CH_3)_2$ | $OCH_3$ | $OCH_3$ | |
| $J_{18}$ | - | O | H | $CH_3$ | $OCH_3$ | $OCH_3$ | |
| $J_{18}$ | - | $CH_2$ | 7-Cl | $CH_3$ | $OCH_3$ | $OCH_3$ | |
| $J_{18}$ | - | O | 7-Br | $CH_3$ | $OCH_3$ | $OCH_3$ | |
| $J_{18}$ | - | $CH_2$ | 6-F | $CH_3$ | $OCH_3$ | $OCH_3$ | |
| $J_{18}$ | - | O | 7-$CH_3$ | $CH_3$ | $OCH_3$ | $OCH_3$ | |
| $J_{18}$ | - | O | 7-$OCH_3$ | $CH_3$ | $OCH_3$ | $OCH_3$ | |
| $J_{18}$ | - | $CH_2$ | H | $CH_3$ | $OCH_2CF_3$ | $OCH_3$ | |
| $J_{18}$ | - | $CH_2$ | H | $CH_3$ | $OCHF_2$ | $OCH_3$ | |
| $J_{18}$ | - | $CH_2$ | H | $CH_3$ | $OCH_3$ | cyclopropyl | |
| $J_{18}$ | - | $CH_2$ | H | $CH_3$ | $OCH_3$ | $NHCH_3$ | |
| $J_{18}$ | - | $CH_2$ | H | $CH_3$ | $OCH_3$ | $N(CH_3)_2$ | |

Table XXIII (continued)

| J | n | L | $R_{20}$ | $R_{22}$ | X | Y | m.p. (°C) |
|---|---|---|---|---|---|---|---|
| $J_{18}$ | - | $CH_2$ | H | $CH_3$ | $OCH_3$ | $OCH_2CH{=}CH_2$ | |
| ~~$J_{18}$~~ | ~~-~~ | ~~$CH_2$~~ | ~~H~~ | ~~$CH_3$~~ | ~~$OCH_3$~~ | ~~$CH(OCH_3)_2$~~ | |
| $J_{18}$ | - | $CH_2$ | H | $CH_3$ | $OCH_3$ | 1,3-dioxolan-2-yl | |
| ~~$J_{18}$~~ | ~~-~~ | ~~$CH_2$~~ | ~~H~~ | ~~$CH_3$~~ | ~~$OCH_3$~~ | ~~$CH(OC_2H_5)_2$~~ | |
| $J_{18}$ | - | $CH_2$ | H | $CH_3$ | Cl | $NHCH_3$ | |
| $J_{18}$ | - | $CH_2$ | H | $CH_3$ | $OCH_3$ | $-C{\equiv}CH$ | |
| ~~$J_{18}$~~ | ~~-~~ | ~~$CH_2$~~ | ~~H~~ | ~~$CH_3$~~ | ~~$OCH_3$~~ | ~~$C(CH_3)(OCH_3)_2$~~ | |
| $J_{18}$ | - | $CH_2$ | H | OH | $OCH_3$ | $OCH_3$ | |
| $J_{18}$ | - | $CH_2$ | H | $(CH_2)_2CH(CH_3)_2$ | Cl | $OCH_3$ | |
| $J_{18}$ | - | $CH_2$ | H | $CH_2CH_2Cl$ | $OCH_3$ | $OCH_3$ | |
| $J_{18}$ | - | $CH_2$ | H | $(CH_2)_5Cl$ | $CH_3$ | $OCH_3$ | |
| $J_{18}$ | - | $CH_2$ | H | $CH_2OCH_3$ | Cl | $OCH_3$ | |
| $J_{18}$ | - | $CH_2$ | H | $(CH_2)_2OCH_3$ | Cl | $OCH_3$ | |
| $J_{18}$ | - | $CH_2$ | H | $CH_2CH{=}CH_2$ | $OCH_3$ | $OCH_3$ | |
| $J_{18}$ | - | $CH_2$ | H | $CH_2CH{=}CHCH_2Cl$ | $CH_3$ | $OCH_3$ | |
| $J_{18}$ | - | $CH_2$ | H | $CH_2CH{=}CHCH_3$ | $CH_3$ | $OCH_3$ | |
| $J_{18}$ | - | $CH_2$ | H | $CH_2C{\equiv}CCH_3$ | $OCH_3$ | $OCH_3$ | |
| $J_{18}$ | - | $CH_2$ | H | $OCH_3$ | Cl | $OCH_3$ | |
| $J_{18}$ | - | $CH_2$ | H | $O(CH_2)_4CH_3$ | Cl | $OCH_3$ | |
| $J_{18}$ | - | $CH_2$ | H | $C_6H_5$ | $OCH_3$ | $OCH_3$ | |
| $J_{18}$ | - | $CH_2$ | H | cyclopropyl | Cl | $OCH_3$ | |
| $J_{18}$ | - | $CH_2$ | H | cyclopentyl | $OCH_3$ | $OCH_3$ | |
| $J_{18}$ | - | $CH_2$ | H | $C(O)CH_3$ | $OCH_3$ | $OCH_3$ | |
| $J_{18}$ | - | $CH_2$ | H | $CO_2CH_3$ | $OCH_3$ | $OCH_3$ | |
| $J_{18}$ | - | $CH_2$ | H | $CH_2C_6H_5$ | Cl | $OCH_3$ | |
| $J_{18}$ | - | $CH_2$ | H | $CH_2CH{=}CHCl$ | Cl | $OCH_3$ | |
| $J_{18}$ | - | $CH_2$ | H | $CH(CH_3)CO_2CH_3$ | Cl | $OCH_3$ | |

Table XXIIIa

General Formula 2a

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{13}$ | 0 | $CH_2$ | - | - | - | H | $CH_3$ | - | - | - | - | - | - | $OCH_3$ | $OCH_3$ | |
| $J_{13}$ | 0 | $CH_2$ | - | - | - | H | $CH_3$ | - | - | - | - | - | - | $OCH_3$ | $CH_3$ | |
| $J_{13}$ | 0 | $CH_2$ | - | - | - | H | $CH_3$ | - | - | - | - | - | - | Cl | $OCH_3$ | |
| $J_{13}$ | 0 | $CH_2$ | - | - | - | H | $CH_3$ | - | - | - | - | - | - | Br | $OCH_3$ | |
| $J_{13}$ | 0 | $CH_2$ | - | - | - | H | $CH_3$ | - | - | - | - | - | - | $OCH_3$ | $C_2H_5$ | |
| $J_{13}$ | 0 | $CH_2$ | - | - | - | H | $CH_3$ | - | - | - | - | - | - | $CH_3$ | $OC_2H_5$ | |
| ~~$J_{13}$~~ | ~~0~~ | ~~$CH_2$~~ | | | | ~~H~~ | ~~$CH_3$~~ | | | | | | | ~~$OCH_3$~~ | ~~$CH_2OCH_3$~~ | |
| $J_{13}$ | 0 | $CH_2$ | - | - | - | H | $CH_3$ | - | - | - | - | - | - | $OCH_3$ | $CH_2F$ | |
| $J_{13}$ | 0 | $CH_2$ | - | - | - | H | $CH_3$ | - | - | - | - | - | - | $OCH_3$ | $CF_3$ | |
| $J_{13}$ | 0 | $CH_2$ | - | - | - | H | H | - | - | - | - | - | - | $OCH_3$ | $OCH_3$ | |
| $J_{13}$ | 0 | $CH_2$ | - | - | - | H | $C_2H_5$ | - | - | - | - | - | - | $OCH_3$ | $OCH_3$ | |
| $J_{13}$ | 0 | $CH_2$ | - | - | - | H | n-$C_3H_7$ | - | - | - | - | - | - | $OCH_3$ | $OCH_3$ | |
| $J_{13}$ | 0 | $CH_2$ | - | - | - | H | $CH(CH_3)_2$ | - | - | - | - | - | - | $OCH_3$ | $OCH_3$ | |
| $J_{13}$ | 1 | $CH_2$ | - | - | - | H | $CH_3$ | - | - | - | - | - | - | $OCH_3$ | $OCH_3$ | |
| $J_{13}$ | 0 | O | - | - | - | H | $CH_3$ | - | - | - | - | - | - | $OCH_3$ | $OCH_3$ | |
| $J_{13}$ | 0 | O | - | - | - | H | $CH_3$ | - | - | - | - | - | - | $OCH_3$ | $CH_3$ | |
| $J_{13}$ | 0 | O | - | - | - | H | $CH_3$ | - | - | - | - | - | - | Cl | $OCH_3$ | |
| $J_{13}$ | 0 | O | - | - | - | H | $CH_3$ | - | - | - | - | - | - | Br | $OCH_3$ | |
| $J_{13}$ | 1 | O | - | - | - | H | $CH_3$ | - | - | - | - | - | - | $OCH_3$ | $OCH_3$ | |
| $J_{13}$ | 0 | O | - | - | - | H | $C_2H_5$ | - | - | - | - | - | - | $OCH_3$ | $OCH_3$ | |
| $J_{13}$ | 0 | $CH_2$ | - | - | - | 6-Br | $CH_3$ | - | - | - | - | - | - | $OCH_3$ | $OCH_3$ | |
| $J_{13}$ | 0 | $CH_2$ | - | - | - | 6-Cl | $CH_3$ | - | - | - | - | - | - | $OCH_3$ | $OCH_3$ | |
| $J_{13}$ | 0 | $CH_2$ | - | - | - | 5-F | $CH_3$ | - | - | - | - | - | - | $OCH_3$ | $OCH_3$ | |
| $J_{13}$ | 0 | O | - | - | - | 6-$CH_3$ | $CH_3$ | - | - | - | - | - | - | $OCH_3$ | $OCH_3$ | |
| $J_{13}$ | 0 | O | - | - | - | 6-$OCH_3$ | $CH_3$ | - | - | - | - | - | - | $OCH_3$ | $OCH_3$ | |
| $J_{14}$ | 0 | $CH_2$ | - | - | - | H | - | H | H | - | - | - | - | $OCH_3$ | $OCH_3$ | 181-184,d |
| $J_{14}$ | 0 | $CH_2$ | - | - | - | H | - | H | H | - | - | - | - | $CH_3$ | $OCH_3$ | 180-185,d |
| $J_{14}$ | 0 | $CH_2$ | - | - | - | H | - | H | H | - | - | - | - | $CH_3$ | $CH_3$ | |
| $J_{14}$ | 0 | $CH_2$ | - | - | - | H | - | H | H | - | - | - | - | Cl | $OCH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{13}$ | 0 | $CH_2$ | - | - | - | H | $CH_3$ | - | - | - | - | - | - | $OCH_3$ | $CH(CH_3)_2$ | |
| $J_{13}$ | 0 | $CH_2$ | - | - | - | H | $CH_3$ | - | - | - | - | - | - | Cl | $NHCH_3$ | |
| $J_{13}$ | 0 | $CH_2$ | - | - | - | H | $CH_3$ | - | - | - | - | - | - | Cl | $OCF_2H$ | |
| $J_{13}$ | 0 | $CH_2$ | - | - | - | H | $CH_3$ | - | - | - | - | - | - | $OCH_2CF_3$ | $OCH_3$ | |
| $J_{13}$ | 0 | $CH_2$ | - | - | - | H | $CH_3$ | - | - | - | - | - | - | $OCF_2H$ | $OCF_2H$ | |
| $J_{13}$ | 0 | $CH_2$ | - | - | - | H | $CH_3$ | - | - | - | - | - | - | $OCH_3$ | cyclopropyl | |
| $J_{13}$ | 0 | $CH_2$ | - | - | - | H | $CH_3$ | - | - | - | - | - | - | $OCH_3$ | $NHCH_3$ | |
| $J_{13}$ | 0 | $CH_2$ | - | - | - | H | $CH_3$ | - | - | - | - | - | - | $OCH_3$ | $N(CH_3)_2$ | |
| $J_{13}$ | 0 | $CH_2$ | - | - | - | H | $CH_3$ | - | - | - | - | - | - | $OCH_3$ | $OCH_2CH=CH_2$ | |
| ~~$J_{13}$~~ | ~~0~~ | ~~$CH_2$~~ | | | | ~~H~~ | ~~$CH_3$~~ | | | | | | | ~~$OCH_3$~~ | ~~$CH(OCH_3)_2$~~ | |
| $J_{13}$ | 0 | $CH_2$ | - | - | - | H | $CH_3$ | - | - | - | - | - | - | $OCH_3$ | 1,3-dioxolan-2-yl | |
| ~~$J_{13}$~~ | ~~0~~ | ~~$CH_2$~~ | ~~-~~ | ~~-~~ | ~~-~~ | ~~H~~ | ~~$CH_3$~~ | ~~-~~ | ~~-~~ | ~~-~~ | ~~-~~ | ~~-~~ | ~~-~~ | ~~$OCH_3$~~ | ~~$CH(OC_2H_5)_2$~~ | |
| $J_{13}$ | 0 | $CH_2$ | - | - | - | H | $CH_3$ | - | - | - | - | - | - | $OCH_3$ | C≡CH | |
| $J_{13}$ | 0 | $CH_2$ | - | - | - | H | $CH_3$ | - | - | - | - | - | - | $OCH_3$ | $OCH_2CH_2OCH_3$ | |
| $J_{13}$ | 0 | $CH_2$ | - | - | - | H | $CH_3$ | - | - | - | - | - | - | $OCH_3$ | $SCH_3$ | |
| $J_{13}$ | 0 | $CH_2$ | - | - | - | H | $CH_3$ | - | - | - | - | - | - | $OCF_2H$ | $OCH_3$ | |
| ~~$J_{13}$~~ | ~~0~~ | ~~$CH_2$~~ | | | | ~~H~~ | ~~$CH_3$~~ | | | | | | | ~~$CH_3$~~ | ~~1,3-dioxolan-2-yl~~ | |
| $J_{14}$ | 0 | $CH_2$ | - | - | - | H | - | H | H | - | - | - | - | $CH_3$ | $OCH_2C≡CH$ | |
| $J_{14}$ | 0 | $CH_2$ | - | - | - | H | - | H | H | - | - | - | - | Br | $OCH_3$ | |
| $J_{14}$ | 0 | $CH_2$ | - | - | - | H | - | H | H | - | - | - | - | $OCH_3$ | $C_2H_5$ | |
| $J_{14}$ | 0 | $CH_2$ | - | - | - | H | - | H | H | - | - | - | - | $CH_3$ | $OC_2H_5$ | |
| ~~$J_{14}$~~ | ~~0~~ | ~~$CH_2$~~ | | | | ~~H~~ | | ~~H~~ | ~~H~~ | | | | | ~~$CH_3$~~ | ~~$CH_2OCH_3$~~ | |
| $J_{14}$ | 0 | $CH_2$ | - | - | - | H | - | H | H | - | - | - | - | $OCH_3$ | $CH_2F$ | |
| $J_{14}$ | 0 | $CH_2$ | - | - | - | H | - | H | H | - | - | - | - | $OCH_3$ | $CF_3$ | |
| $J_{14}$ | 1 | $CH_2$ | - | - | - | H | - | H | H | - | - | - | - | $OCH_3$ | $OCH_3$ | |
| $J_{14}$ | 1 | $CH_2$ | - | - | - | H | - | $CH_3$ | H | - | - | - | - | $OCH_3$ | $OCH_3$ | |
| $J_{14}$ | 0 | O | - | - | - | H | - | H | H | - | - | - | - | $OCH_3$ | $CH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{14}$ | 1 | $CH_2$ | - | - | - | H | - | $CH_3$ | H | - | - | - | - | $CH_3$ | $CH_3$ | |
| $J_{14}$ | 1 | $CH_2$ | - | - | - | H | - | $CH_3$ | H | - | - | - | - | $CH_3$ | $OCH_3$ | |
| $J_{14}$ | 1 | $CH_2$ | - | - | - | H | - | $CH_3$ | H | - | - | - | | $OCH_3$ | $OCH_3$ | |
| $J_{14}$ | 1 | $CH_2$ | - | - | - | H | - | $CH_3$ | H | - | - | - | - | Cl | $OCH_3$ | |
| $J_{14}$ | 1 | O | - | - | - | H | - | $CH_3$ | H | - | - | - | - | $CH_3$ | $CH_3$ | |
| $J_{14}$ | 1 | O | - | - | - | H | - | $CH_3$ | H | - | - | - | - | $OCH_3$ | $CH_3$ | |
| $J_{14}$ | 1 | O | - | - | - | H | - | $CH_3$ | H | - | - | - | - | Cl | $OCH_3$ | |
| $J_{14}$ | 0 | $CH_2$ | - | - | - | H | - | $CH_3$ | H | - | - | - | - | $OCH_3$ | $OCH_3$ | |
| $J_{14}$ | 0 | O | - | - | - | H | - | H | H | - | - | - | - | $OCH_3$ | $OCH_3$ | |
| $J_{14}$ | 0 | O | - | - | - | H | - | H | H | - | - | - | - | $OCH_3$ | $CH_3$ | |
| $J_{14}$ | 0 | O | - | - | - | H | - | H | H | - | - | - | - | Cl | $OCH_3$ | |
| $J_{14}$ | 0 | O | - | - | - | H | - | H | H | - | - | - | - | Br | $OCH_3$ | |
| $J_{14}$ | 0 | O | - | - | - | H | - | H | H | - | - | - | - | $CH_3$ | $CH_3$ | |
| $J_{14}$ | 0 | $CH_2$ | - | - | - | H | - | H | H | - | - | - | - | $CH_3$ | $CH_3$ | |
| $J_{14}$ | 0 | $CH_2$ | - | - | - | H | - | H | H | - | - | - | - | $OCH_3$ | $CH_3$ | |
| $J_{14}$ | 0 | $CH_2$ | - | - | - | H | - | H | H | - | - | - | - | $OCH_3$ | $OCH_3$ | |
| $J_{14}$ | 0 | $CH_2$ | - | - | - | H | - | H | H | - | - | - | - | Cl | $OCH_3$ | |
| $J_{14}$ | 0 | $CH_2$ | - | - | - | 5-F | - | H | H | - | - | - | - | $OCH_3$ | $OCH_3$ | |
| $J_{14}$ | 0 | $CH_2$ | - | - | - | 6-Cl | - | H | H | - | - | - | - | $OCH_3$ | $OCH_3$ | |
| $J_{14}$ | 0 | $CH_2$ | - | - | - | 6-Br | - | H | H | - | - | - | - | $OCH_3$ | $OCH_3$ | |
| $J_{14}$ | 0 | O | - | - | - | 5-$CH_3$ | - | H | H | - | - | - | - | $OCH_3$ | $OCH_3$ | |
| $J_{14}$ | 0 | O | - | - | - | 6-$OCH_3$ | - | H | H | - | - | - | - | $OCH_3$ | $OCH_3$ | |
| $J_{14}$ | 1 | O | - | - | - | H | - | H | H | - | - | - | - | $OCH_3$ | $OCH_3$ | |
| $J_{15}$ | 0 | $CH_2$ | - | - | - | H | - | - | - | - | - | - | - | $OCH_3$ | $OCH_3$ | |
| $J_{15}$ | 1 | $CH_2$ | - | - | - | H | - | - | - | - | - | - | - | $OCH_3$ | $CH_3$ | |
| $J_{15}$ | 0 | O | - | - | - | H | - | - | - | - | - | - | - | $OCH_3$ | $OCH_3$ | |
| $J_{15}$ | 1 | O | - | - | - | H | - | - | - | - | - | - | - | $OCH_3$ | $OCH_3$ | |
| $J_{15}$ | 0 | $CH_2$ | - | - | - | H | - | - | - | - | - | - | - | Cl | $OCH_3$ | |
| $J_{15}$ | 0 | O | - | - | - | H | - | - | - | - | - | - | - | Cl | $OCH_3$ | |
| $J_{16}$ | 0 | - | - | - | - | H | $CH_3$ | - | - | - | - | - | - | $OCH_3$ | $OCH_3$ | |
| $J_{16}$ | 0 | - | - | - | - | H | $CH_3$ | - | - | - | - | - | - | $CH_3$ | $OCH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{16}$ | 0 | - | - | - | - | H | $CH_3$ | - | - | - | - | - | - | $CH_3$ | $CH_3$ | |
| $J_{16}$ | 0 | - | - | - | - | H | $CH_3$ | - | - | - | - | - | - | Cl | $OCH_3$ | |
| $J_{16}$ | 1 | - | - | - | - | H | $CH_3$ | - | - | - | - | - | - | $OCH_3$ | $OCH_3$ | |
| $J_{16}$ | 0 | - | - | - | - | H | H | - | - | - | - | - | - | $OCH_3$ | $OCH_3$ | |
| $J_{17}$ | 0 | - | - | - | - | H | $CH_3$ | - | - | - | - | - | - | $OCH_3$ | $OCH_3$ | |
| $J_{17}$ | 0 | - | - | - | - | H | $CH_3$ | - | - | - | - | - | - | Cl | $OCH_3$ | |
| $J_{17}$ | 0 | - | - | - | - | H | $CH_3$ | - | - | - | - | - | - | $CH_3$ | $OCH_3$ | |
| $J_{17}$ | 0 | - | - | - | - | H | H | - | - | - | - | - | - | $OCH_3$ | $OCH_3$ | |
| $J_{17}$ | 1 | - | - | - | - | H | $CH_3$ | - | - | - | - | - | - | $OCH_3$ | $OCH_3$ | |
| $J_{19}$ | - | $CH_2$ | - | - | - | H | - | H | - | - | - | - | - | $OCH_3$ | $OCH_3$ | |
| $J_{19}$ | - | $CH_2$ | - | - | - | H | - | H | - | - | - | - | - | $OCH_3$ | $CH_3$ | |
| $J_{19}$ | - | $CH_2$ | - | - | - | H | - | H | - | - | - | - | - | Cl | $OCH_3$ | |
| $J_{19}$ | - | $CH_2$ | - | - | - | H | - | H | - | - | - | - | - | Br | $OCH_3$ | |
| $J_{19}$ | - | $CH_2$ | - | - | - | H | - | H | - | - | - | - | - | $OCH_3$ | $C_2H_5$ | |
| $J_{19}$ | - | $CH_2$ | - | - | - | H | - | H | - | - | - | - | - | $CH_3$ | $OC_2H_5$ | |
| ~~$J_{19}$~~ | | ~~$CH_2$~~ | | | | ~~H~~ | | ~~H~~ | | | | | | ~~$OCH_3$~~ | ~~$CH_2OCH_3$~~ | |
| $J_{19}$ | - | $CH_2$ | - | - | - | H | - | H | - | - | - | - | - | $OCH_3$ | $CH_2F$ | |
| $J_{19}$ | - | $CH_2$ | - | - | - | H | - | H | - | - | - | - | - | $OCH_3$ | $CF_3$ | |
| $J_{19}$ | - | O | - | - | - | H | - | H | - | - | - | - | - | $OCH_3$ | $OCH_3$ | |
| $J_{19}$ | - | O | - | - | - | H | - | H | - | - | - | - | - | Cl | $OCH_3$ | |
| $J_{19}$ | - | $CH_2$ | - | - | - | 7-Cl | - | H | - | - | - | - | - | $OCH_3$ | $OCH_3$ | |
| $J_{20}$ | - | $CH_2$ | - | - | - | H | - | - | - | - | - | - | - | $OCH_3$ | $OCH_3$ | |
| $J_{20}$ | - | $CH_2$ | - | - | - | H | - | - | - | - | - | - | - | Cl | $OCH_3$ | |
| $J_{20}$ | - | O | - | - | - | H | - | - | - | - | - | - | - | $OCH_3$ | $OCH_3$ | |
| $J_{21}$ | - | - | - | - | - | H | $CH_3$ | - | - | - | - | - | - | $OCH_3$ | $OCH_3$ | |
| $J_{21}$ | - | - | - | - | - | H | $CH_3$ | - | - | - | - | - | - | Cl | $OCH_3$ | |
| $J_{21}$ | - | - | - | - | - | H | $C_2H_5$ | - | - | - | - | - | - | $OCH_3$ | $OCH_3$ | |
| $J_{22}$ | - | - | - | - | - | H | $CH_3$ | - | - | - | - | - | - | $OCH_3$ | $OCH_3$ | |
| $J_{22}$ | - | - | - | - | - | H | $C_2H_5$ | - | - | - | - | - | - | $OCH_3$ | $OCH_3$ | |
| $J_{23}$ | - | $CH_2$ | - | - | - | H | - | H | - | - | - | - | - | $OCH_3$ | $OCH_3$ | |
| $J_{23}$ | - | $CH_2$ | - | - | - | H | - | H | - | - | - | - | - | $OCH_3$ | $CH_3$ | |
| $J_{23}$ | - | $CH_2$ | - | - | - | H | - | H | - | - | - | - | - | Cl | $OCH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{23}$ | - | $CH_2$ | - | - | - | H | - | H | - | - | - | - | - | Br | $OCH_3$ | |
| $J_{23}$ | - | $CH_2$ | - | - | - | H | - | $CH_3$ | - | - | - | - | - | $OCH_3$ | $OCH_3$ | |
| $J_{23}$ | - | O | - | - | - | H | - | H | - | - | - | - | - | $OCH_3$ | $OCH_3$ | |
| $J_{23}$ | - | O | - | - | - | H | - | H | - | - | - | - | - | Cl | $OCH_3$ | |
| $J_{23}$ | - | O | - | - | - | H | - | $CH_3$ | - | - | - | - | - | $OCH_3$ | $OCH_3$ | |
| $J_{24}$ | - | $CH_2$ | O | - | - | H | - | - | - | - | - | - | - | $OCH_3$ | $OCH_3$ | |
| $J_{24}$ | - | $CH_2$ | O | - | - | H | - | - | - | - | - | - | - | Cl | $OCH_3$ | |
| $J_{24}$ | - | O | O | - | - | H | - | - | - | - | - | - | - | $OCH_3$ | $OCH_3$ | |
| $J_{24}$ | - | O | O | - | - | H | - | - | - | - | - | - | - | Cl | $OCH_3$ | |
| $J_{24}$ | - | $CH_2$ | $CH_2$ | - | - | H | - | - | - | - | - | - | - | $OCH_3$ | $OCH_3$ | |
| $J_{24}$ | - | $CH_2$ | $CH_2$ | - | - | H | - | - | - | - | - | - | - | Cl | $OCH_3$ | |
| $J_{24}$ | - | O | $CH_2$ | - | - | H | - | - | - | - | - | - | - | $OCH_3$ | $OCH_3$ | |
| $J_{24}$ | - | O | $CH_2$ | - | - | H | - | - | - | - | - | - | - | Cl | $OCH_3$ | |
| $J_{25}$ | - | $CH_2$ | - | - | - | H | - | - | - | - | - | - | - | $OCH_3$ | $OCH_3$ | |
| $J_{25}$ | - | $CH_2$ | - | - | - | H | - | - | - | - | - | - | - | Cl | $OCH_3$ | |
| $J_{25}$ | - | O | - | - | - | H | - | - | - | - | - | - | - | $OCH_3$ | $OCH_3$ | |
| $J_{25}$ | - | O | - | - | - | H | - | - | - | - | - | - | - | Cl | $OCH_3$ | |
| $J_{26}$ | - | - | - | O | - | H | - | $CH_3$ | H | H | - | - | - | $OCH_3$ | $OCH_3$ | |
| $J_{26}$ | - | - | - | O | - | H | - | $CH_3$ | H | H | - | - | - | $OCH_3$ | $CH_3$ | |
| $J_{26}$ | - | - | - | O | - | H | - | H | H | $CH_3$ | - | - | - | $CH_3$ | $CH_3$ | |
| $J_{26}$ | - | - | - | O | - | H | - | H | H | $CH_3$ | - | - | - | Cl | $OCH_3$ | |
| $J_{26}$ | - | - | - | O | - | H | - | $CH_3$ | H | H | - | - | - | Br | $OCH_3$ | |
| $J_{26}$ | - | - | - | O | - | H | - | H | H | $CH_3$ | - | - | | $OCH_3$ | $C_2H_5$ | |
| $J_{26}$ | - | - | - | O | - | H | - | $CH_3$ | H | H | - | - | - | $CH_3$ | $OC_2H_5$ | |
| ~~$J_{26}$~~ | ~~-~~ | ~~-~~ | ~~-~~ | ~~O~~ | ~~-~~ | ~~H~~ | ~~-~~ | ~~$CH_3$~~ | ~~H~~ | ~~H~~ | ~~-~~ | ~~-~~ | ~~-~~ | ~~$OCH_3$~~ | ~~$CH_2OCH_3$~~ | |
| $J_{26}$ | - | - | - | O | - | H | - | H | H | $C_2H_5$ | - | - | - | $OCH_3$ | $CF_3$ | |
| $J_{26}$ | - | - | - | O | - | H | - | $CH_3$ | $CH_3$ | H | - | - | - | $OCH_3$ | $OCH_3$ | |
| $J_{26}$ | - | - | - | O | - | H | - | $CH_3$ | H | $CH_3$ | - | - | - | $OCH_3$ | $OCH_3$ | |
| $J_{26}$ | - | - | - | O | - | H | - | $CH_3$ | H | $CH_3$ | - | - | - | $OCH_3$ | $CH_3$ | |
| $J_{26}$ | - | - | - | O | - | H | - | $CH_3$ | H | $CH_3$ | - | - | - | $CH_3$ | $CH_3$ | |
| $J_{26}$ | - | - | - | O | - | H | - | $CH_3$ | H | $CH_3$ | - | - | - | Cl | $OCH_3$ | |
| $J_{26}$ | - | - | - | O | - | H | - | $CH_3$ | H | $CH_3$ | - | - | - | Br | $OCH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{26}$ | - | - | - | O | - | H | - | $CH_3$ | H | $CH_3$ | - | - | - | $OCH_3$ | $C_2H_5$ | |
| $J_{26}$ | - | - | - | O | - | H | - | $CH_3$ | H | $CH_3$ | - | | - | $CH_3$ | $OC_2H_5$ | |
| ~~$J_{26}$~~ | | | | ~~O~~ | | ~~H~~ | | ~~$CH_3$~~ | ~~H~~ | ~~$CH_3$~~ | | | | ~~$OCH_3$~~ | ~~$CH_2OCH_3$~~ | |
| $J_{26}$ | - | - | - | O | - | H | - | $CH_3$ | H | $CH_3$ | - | - | - | $OCH_3$ | $CH_2F$ | |
| $J_{26}$ | - | - | - | O | - | H | - | $CH_3$ | H | $CH_3$ | - | | - | $OCH_3$ | $CF_3$ | |
| $J_{26}$ | - | - | - | S | - | H | - | $CH_3$ | H | H | - | - | - | $OCH_3$ | $OCH_3$ | |
| $J_{26}$ | - | - | - | S | - | H | - | $CH_3$ | H | H | - | - | - | $OCH_3$ | $CH_3$ | |
| $J_{26}$ | - | - | - | S | - | H | - | $CH_3$ | H | H | - | - | - | $CH_3$ | $CH_3$ | |
| $J_{26}$ | - | - | - | S | - | H | - | $CH_3$ | H | H | - | - | - | Cl | $OCH_3$ | |
| $J_{26}$ | - | - | - | S | - | H | - | $CH_3$ | H | H | - | - | - | Br | $OCH_3$ | |
| $J_{26}$ | - | - | - | S | - | H | - | H | $CH_3$ | $CH_3$ | - | - | - | $OCH_3$ | $CH_3$ | |
| $J_{26}$ | - | - | - | $SO_2$ | - | H | - | $CH_3$ | H | H | - | - | - | $OCH_3$ | $OCH_3$ | 222-222.5 |
| $J_{26}$ | - | - | - | $SO_2$ | - | H | - | $CH_3$ | H | H | - | - | - | $OCH_3$ | $CH_3$ | 208-209 |
| $J_{26}$ | - | - | - | $SO_2$ | - | H | - | $CH_3$ | H | H | - | - | - | $CH_3$ | $CH_3$ | 192-193 |
| $J_{26}$ | - | - | - | $SO_2$ | - | H | - | $CH_3$ | H | H | - | - | - | Cl | $OCH_3$ | |
| $J_{26}$ | - | - | - | $SO_2$ | - | H | - | $CH_3$ | H | H | - | - | - | Br | $OCH_3$ | |
| $J_{26}$ | - | - | - | $SO_2$ | - | H | - | $CH_3$ | H | H | - | - | - | $OCH_3$ | $C_2H_5$ | |
| $J_{26}$ | - | - | - | $SO_2$ | - | H | - | $CH_3$ | H | H | - | - | - | $CH_3$ | $OC_2H_5$ | |
| ~~$J_{26}$~~ | | | | ~~$SO_2$~~ | | ~~H~~ | | ~~$CH_3$~~ | ~~H~~ | ~~H~~ | | | | ~~$OCH_3$~~ | ~~$CH_2OCH_3$~~ | |
| $J_{26}$ | - | - | - | $SO_2$ | - | H | - | $CH_3$ | H | H | - | - | - | $OCH_3$ | $CH_2F$ | |
| $J_{26}$ | - | - | - | $SO_2$ | - | H | - | $CH_3$ | H | H | - | - | - | $OCH_3$ | $CF_3$ | |
| $J_{26}$ | - | - | - | $SO_2$ | - | H | - | $CH_3$ | $CH_3$ | H | - | | - | $OCH_3$ | $OCH_3$ | |
| $J_{26}$ | - | - | - | $SO_2$ | - | H | - | $CH_3$ | H | $CH_3$ | - | - | - | $OCH_3$ | $OCH_3$ | |
| $J_{26}$ | - | - | - | $SO_2$ | - | H | - | $CH_3$ | H | $CH_3$ | - | - | - | $OCH_3$ | $CH_3$ | |
| $J_{26}$ | - | - | - | $SO_2$ | - | H | - | $CH_3$ | H | $CH_3$ | - | - | - | $CH_3$ | $CH_3$ | |
| $J_{26}$ | - | - | - | $SO_2$ | - | H | - | $CH_3$ | H | $CH_3$ | - | - | - | Cl | $OCH_3$ | |
| $J_{26}$ | - | - | - | $SO_2$ | - | H | - | $CH_3$ | H | $CH_3$ | - | | - | Br | $OCH_3$ | |
| $J_{26}$ | - | - | - | $SO_2$ | - | H | - | $CH_3$ | H | $CH_3$ | - | | - | $OCH_3$ | $C_2H_5$ | |
| $J_{26}$ | - | - | - | $SO_2$ | - | H | - | $CH_3$ | H | $CH_3$ | - | | - | $CH_3$ | $OC_2H_5$ | |
| ~~$J_{26}$~~ | | | | ~~$SO_2$~~ | | ~~H~~ | | ~~$CH_3$~~ | ~~H~~ | ~~$CH_3$~~ | | | | ~~$OCH_3$~~ | ~~$CH_2OCH_3$~~ | |
| $J_{26}$ | - | - | - | $SO_2$ | - | H | - | $CH_3$ | H | $CH_3$ | - | - | - | $OCH_3$ | $CH_2F$ | |
| $J_{26}$ | - | - | - | $SO_2$ | - | H | - | $CH_3$ | H | $CH_3$ | - | - | - | $OCH_3$ | $CF_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{26}$ | - | - | - | $SO_2$ | - | H | - | H | H | H | - | - | - | $CH_3$ | $CH_3$ | |
| $J_{26}$ | - | - | - | $SO_2$ | - | H | - | H | H | H | - | - | - | $CH_3$ | $OCH_3$ | |
| $J_{26}$ | - | - | - | $SO_2$ | - | H | - | H | H | H | - | - | - | $OCH_3$ | $OCH_3$ | |
| $J_{26}$ | - | - | - | $SO_2$ | - | H | - | H | H | H | - | - | - | Cl | $OCH_3$ | |
| $J_{26}$ | - | - | - | $SO_2$ | - | 6-Cl | $CH_3$ | H | H | H | - | - | - | $CH_3$ | $CH_3$ | |
| $J_{26}$ | - | - | - | $SO_2$ | - | 6-Cl | $CH_3$ | H | H | H | - | - | - | $CH_3$ | $OCH_3$ | |
| $J_{26}$ | - | - | - | $SO_2$ | - | 6-Cl | $CH_3$ | H | H | H | - | - | - | $OCH_3$ | $OCH_3$ | |
| $J_{26}$ | - | - | - | $SO_2$ | - | 6-Cl | $CH_3$ | H | H | H | - | - | - | Cl | $OCH_3$ | |
| $J_{26}$ | - | - | - | S | - | H | - | $CH_3$ | H | $CH_3$ | - | - | - | $OCH_3$ | $OCH_3$ | |
| $J_{26}$ | - | - | - | S | - | H | - | $CH_3$ | H | $CH_3$ | - | - | - | $OCH_3$ | $CH_3$ | |
| $J_{26}$ | - | - | - | S | - | H | - | $CH_3$ | H | $CH_3$ | - | - | - | $CH_3$ | $CH_3$ | |
| $J_{26}$ | - | - | - | S | - | H | - | $CH_3$ | H | $CH_3$ | - | - | - | Cl | $OCH_3$ | |
| $J_{26}$ | - | - | - | S | - | H | - | $CH_3$ | H | $CH_3$ | - | - | - | Br | $OCH_3$ | |
| $J_{26}$ | - | - | - | O | - | 6-Cl | - | $CH_3$ | H | H | - | - | - | $OCH_3$ | $OCH_3$ | |
| $J_{26}$ | - | - | - | O | - | 5-Br | - | H | H | $CH_3$ | - | - | - | $OCH_3$ | $OCH_3$ | |
| $J_{26}$ | - | - | - | O | - | 6-F | - | $CH_3$ | H | H | - | - | - | $OCH_3$ | $OCH_3$ | |
| $J_{26}$ | - | - | - | O | - | 5-$CH_3$ | - | $CH_3$ | H | H | - | - | - | $OCH_3$ | $OCH_3$ | |
| $J_{26}$ | - | - | - | O | - | 6-$OCH_3$ | - | $CH_3$ | H | H | - | - | - | $OCH_3$ | $OCH_3$ | |
| $J_{26}$ | - | - | - | S | - | 6-Cl | - | $CH_3$ | H | H | - | - | - | $OCH_3$ | $OCH_3$ | |
| $J_{27}$ | - | $CH_2$ | - | O | - | H | - | $CH_3$ | H | - | - | - | - | $OCH_3$ | $OCH_3$ | |
| $J_{27}$ | - | $CH_2$ | - | S | - | H | - | H | $CH_3$ | - | - | - | - | $OCH_3$ | $OCH_3$ | |
| $J_{27}$ | - | O | - | $SO_2$ | - | H | - | $CH_3$ | H | - | - | - | - | $OCH_3$ | $OCH_3$ | |
| $J_{27}$ | - | O | - | $SO_2$ | - | H | - | $CH_3$ | H | - | - | - | - | Cl | $OCH_3$ | |
| $J_{28}$ | - | - | - | - | O | H | - | $CH_3$ | H | - | - | - | - | $OCH_3$ | $OCH_3$ | |
| $J_{28}$ | - | - | - | - | S | H | - | $CH_3$ | H | - | - | - | - | $OCH_3$ | $OCH_3$ | |
| $J_{28}$ | - | - | - | - | O | H | - | $CH_3$ | $CH_3$ | - | - | - | - | $OCH_3$ | $OCH_3$ | |
| $J_{28}$ | - | | - | - | O | H | - | H | H | - | - | - | - | $OCH_3$ | $OCH_3$ | |
| $J_{29}$ | - | $CH_2$ | - | - | - | H | - | H | H | - | H | H | - | $OCH_3$ | $OCH_3$ | |
| $J_{29}$ | - | $CH_2$ | - | - | - | H | - | H | H | - | $CH_3$ | H | - | Cl | $OCH_3$ | |
| $J_{29}$ | - | O | - | - | - | H | - | H | H | - | H | $CH_3$ | - | $OCH_3$ | $OCH_3$ | |
| $J_{30}$ | - | - | - | - | - | H | - | $CH_3$ | $CH_3$ | - | - | - | - | $OCH_3$ | $OCH_3$ | |
| $J_{30}$ | - | - | - | - | - | H | - | H | H | - | - | - | - | $OCH_3$ | $OCH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{30}$ | - | - | - | - | - | H | - | H | H | - | - | - | - | Cl | $OCH_3$ | |
| $J_{30}$ | - | - | - | - | - | H | - | $CH_3$ | $CH_3$ | - | - | - | - | Cl | $OCH_3$ | |
| $J_{31}$ | - | $CH_2$ | - | - | - | H | - | - | | - | - | - | - | $OCH_3$ | $OCH_3$ | |
| $J_{31}$ | - | $CH_2$ | - | - | - | H | - | - | - | - | - | - | - | $OCH_3$ | $CH_3$ | |
| $J_{31}$ | - | $CH_2$ | - | - | - | H | - | - | - | - | - | - | - | Cl | $OCH_3$ | |
| $J_{31}$ | - | $CH_2$ | - | - | - | H | - | - | - | - | - | - | - | $CH_3$ | $CH_3$ | |
| $J_{31}$ | - | $CH_2$ | - | - | - | 7-Cl | | - | - | - | - | - | - | $OCH_3$ | $OCH_3$ | |
| $J_{31}$ | - | O | - | - | - | H | - | - | - | - | - | - | - | $OCH_3$ | $OCH_3$ | |
| $J_{31}$ | - | O | - | - | - | H | - | - | - | - | - | - | - | $OCH_3$ | $CH_3$ | |
| $J_{31}$ | - | O | - | - | - | H | - | - | - | - | - | - | - | Cl | $OCH_3$ | |
| $J_{31}$ | - | O | - | - | - | H | - | - | - | - | - | - | - | $CH_3$ | $CH_3$ | |
| $J_{32}$ | - | $CH_2$ | - | - | - | - | - | - | - | - | - | - | F | $OCH_3$ | $OCH_3$ | |
| $J_{32}$ | - | $CH_2$ | - | - | - | - | - | - | - | - | - | - | Cl | Cl | $OCH_3$ | |
| $J_{32}$ | - | $CH_2$ | - | - | - | - | - | - | - | - | - | - | Br | $OCH_3$ | $CH_3$ | |
| $J_{32}$ | - | $CH_2$ | - | - | - | - | - | - | - | - | - | - | $OCH_3$ | $OCH_3$ | $OCH_3$ | |
| $J_{32}$ | - | $CH_2$ | - | - | - | - | - | - | - | - | - | - | $SCH_3$ | $OCH_3$ | $OCH_3$ | |
| $J_{32}$ | - | $CH_2$ | - | - | - | - | - | - | - | - | - | - | $SO_2CH_3$ | $OCH_3$ | $OCH_3$ | |
| $J_{32}$ | - | $CH_2$ | - | - | - | - | - | - | - | - | - | - | $CO_2CH_3$ | $OCH_3$ | $OCH_3$ | |
| $J_{32}$ | - | $CH_2$ | - | - | - | - | - | - | - | - | - | - | $SO_2N(CH_3)_2$ | $OCH_3$ | $OCH_3$ | |
| $J_{32}$ | - | $CH_2$ | - | - | - | - | - | - | - | - | - | - | $OSO_2CH_3$ | $OCH_3$ | $OCH_3$ | |
| $J_{32}$ | - | $CH_2$ | - | - | - | - | - | | - | - | - | - | Cl | $OCH_3$ | $OCH_3$ | |
| $J_{32}$ | - | $CH_2$ | - | - | - | - | - | - | - | - | - | - | Cl | Br | $OCH_3$ | |
| $J_{32}$ | - | $CH_2$ | - | - | - | - | - | - | - | - | - | - | Cl | $OCH_3$ | $CH_2F$ | |
| $J_{32}$ | - | $CH_2$ | - | - | - | - | - | - | - | - | - | - | Cl | $CH_3$ | $OC_2H_5$ | |
| $J_{32}$ | - | $CH_2$ | - | - | - | - | - | - | - | - | - | - | Cl | $OCH_3$ | $C_2H_5$ | |
| $J_{32}$ | - | O | - | - | - | - | - | - | - | - | - | - | F | $OCH_3$ | $OCH_3$ | |
| $J_{32}$ | - | O | - | - | - | - | - | - | - | - | - | - | Cl | $OCH_3$ | $OCH_3$ | |
| $J_{32}$ | - | O | - | - | - | - | - | - | - | - | - | - | Br | Cl | $OCH_3$ | |
| $J_{32}$ | - | O | - | - | - | - | - | - | - | - | - | - | $OCH_3$ | $OCH_3$ | $OCH_3$ | |
| $J_{32}$ | - | O | - | - | - | - | - | - | - | - | - | - | $SCH_3$ | $OCH_3$ | $OCH_3$ | |
| $J_{32}$ | - | O | - | - | - | - | - | | - | - | - | - | $SO_2CH_3$ | $OCH_3$ | $OCH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{32}$ | - | O | - | - | - | - | - | - | - | - | - | - | $CO_2CH_3$ | $OCH_3$ | $OCH_3$ | |
| $J_{32}$ | - | O | - | - | - | - | - | - | - | - | - | - | $SO_2N(CH_3)_2$ | $OCH_3$ | $OCH_3$ | |
| $J_{32}$ | - | O | - | - | - | - | - | - | - | - | - | - | $OSO_2CH_3$ | $OCH_3$ | $CH_3$ | |
| $J_{32}$ | - | $CH_2$ | - | - | - | - | - | - | - | - | - | - | $CO_2CH_3$ | $CH_3$ | $OCH_3$ | |
| $J_{32}$ | - | $CH_2$ | - | - | - | - | - | - | - | - | - | - | $CO_2CH_3$ | $CH_3$ | $CH_3$ | |
| $J_{32}$ | - | $CH_2$ | - | - | - | - | - | - | - | - | - | - | $CO_2CH_3$ | Cl | $OCH_3$ | |
| $J_{32}$ | - | $CH_2$ | - | - | - | - | - | - | - | - | - | - | $CO_2CH_3$ | Br | $OCH_3$ | |
| $J_{32}$ | - | $CH_2$ | - | - | - | - | - | - | - | - | - | - | $CO_2CH_3$ | $OCH_3$ | $C_2H_5$ | |
| $J_{32}$ | - | $CH_2$ | - | - | - | - | - | - | - | - | - | - | $CO_2CH_3$ | $CH_3$ | $OC_2H_5$ | |
| $J_{32}$ | - | $CH_2$ | - | - | - | - | - | - | - | - | - | - | $CO_2CH_3$ | $OCH_3$ | $CH_2OCH_3$ | |
| $J_{32}$ | - | $CH_2$ | - | - | - | - | - | - | - | - | - | - | $CO_2CH_3$ | $OCH_3$ | $CH_2F$ | |
| $J_{32}$ | - | $CH_2$ | - | - | - | - | - | - | - | - | - | - | $CO_2CH_3$ | $OCH_3$ | $CF_3$ | |
| $J_{15}$ | 0 | $CH_2$ | - | - | - | H | - | H | H | - | - | - | - | $OCH_3$ | $CH_2CH_2CH_3$ | |
| $J_{15}$ | 0 | $CH_2$ | - | - | - | H | - | H | H | - | - | - | - | Cl | $NHCH_3$ | |
| $J_{15}$ | 0 | $CH_2$ | - | - | - | H | - | H | H | - | - | - | - | Cl | $OCF_2H$ | |
| $J_{15}$ | 0 | $CH_2$ | - | - | - | H | - | H | H | - | - | - | - | Cl | $N(CH_3)_2$ | |
| $J_{15}$ | 0 | $CH_2$ | - | - | - | H | - | H | H | - | - | - | - | $OCH_3$ | cyclopropyl | |
| $J_{15}$ | 0 | $CH_2$ | - | - | - | H | - | H | H | - | - | - | - | $OCH_3$ | $C\equiv CH$ | |
| $J_{15}$ | 0 | $CH_2$ | - | - | - | H | - | H | H | - | - | - | - | $OCH_3$ | $OCH_2CH=CH_2$ | |
| $J_{15}$ | 0 | $CH_2$ | - | - | - | H | - | H | H | - | - | - | - | $OCH_3$ | $OCH_2CH_2F$ | |
| $J_{16}$ | 0 | - | - | - | - | H | $CH_3$ | - | - | - | - | - | - | $OCH_3$ | $CH(CH_3)_2$ | |
| $J_{16}$ | 0 | - | - | - | - | H | $CH_3$ | - | - | - | - | - | - | Cl | $NHCH_3$ | |
| $J_{16}$ | 0 | - | - | - | - | H | $CH_3$ | - | - | - | - | | - | Cl | $OCF_2H$ | |
| $J_{16}$ | 0 | - | - | - | - | H | $CH_3$ | - | - | - | - | - | - | Cl | $N(CH_3)_2$ | |
| $J_{16}$ | 0 | - | - | - | - | H | $CH_3$ | - | - | - | - | - | - | $OCH_3$ | cyclopropyl | |
| $J_{16}$ | 0 | - | - | - | - | H | $CH_3$ | - | - | - | - | - | - | $OCH_3$ | $C\equiv CH$ | |
| $J_{16}$ | 0 | - | - | - | - | H | $CH_3$ | - | - | | - | - | - | $OCH_3$ | $OCH_2C\equiv CH$ | |
| $J_{16}$ | 0 | - | - | - | - | H | $CH_3$ | - | - | | | | | $OCH_3$ | $OCH_2CH_2F$ | |
| $J_{16}$ | 0 | - | - | - | - | H | $CH_3$ | - | - | - | - | - | - | Cl | $OCF_2H$ | |
| $J_{16}$ | 0 | - | - | - | - | H | $CH_3$ | - | - | | - | - | - | $OCH_3$ | $SCH_3$ | |
| $J_{16}$ | 0 | - | - | - | - | H | $CH_3$ | - | - | - | - | - | - | $OCH_3$ | $CH(OCH_3)_2$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| ~~$J_{16}$~~ | ~~0~~ | | | | | ~~H~~ | ~~$CH_3$~~ | | | | | | | ~~$OCH_3$~~ | ~~1,3-dioxolan-2-yl~~ | |
| $J_{17}$ | 0 | $CH_2$ | - | - | - | H | $CH_3$ | - | - | - | - | - | - | $OCH_3$ | $CH(CH_3)_2$ | |
| $J_{17}$ | 0 | $CH_2$ | - | - | - | H | $CH_3$ | - | - | - | - | - | - | $OCH_3$ | cyclopropyl | |
| $J_{17}$ | 0 | $CH_2$ | - | - | - | H | $CH_3$ | - | - | - | - | - | - | $OCH_3$ | C≡CH | |
| $J_{17}$ | 0 | $CH_2$ | - | - | - | H | $CH_3$ | - | - | - | - | - | - | Cl | $N(CH_3)_2$ | |
| $J_{17}$ | 0 | $CH_2$ | - | - | - | H | $CH_3$ | - | - | - | - | - | - | Cl | $N(CH_3)_2$ | |
| $J_{17}$ | 0 | $CH_2$ | - | - | - | H | $CH_3$ | - | - | - | - | - | - | $OCH_3$ | $OCH_2CH_2F$ | |
| $J_{17}$ | 0 | $CH_2$ | - | - | - | H | $CH_3$ | - | - | - | - | - | - | $OCH_3$ | $SCH_3$ | |
| ~~$J_{17}$~~ | ~~0~~ | ~~$CH_2$~~ | | | | ~~H~~ | ~~$CH_3$~~ | | | | | | | ~~$OCH_3$~~ | ~~$CH(OCH_3)_2$~~ | |
| $J_{19}$ | - | $CH_2$ | - | - | - | H | - | - | - | - | - | - | - | $OCH_3$ | $NHCH_3$ | |
| $J_{19}$ | - | $CH_2$ | - | - | - | H | - | - | - | - | - | - | - | $OCH_3$ | C≡CH | |
| $J_{19}$ | - | $CH_2$ | - | - | - | H | - | - | - | - | - | - | - | Cl | $NHCH_3$ | |
| ~~$J_{19}$~~ | | ~~$CH_2$~~ | | | | ~~H~~ | | | | | | | | ~~$OCH_3$~~ | ~~$CH(OCH_3)_2$~~ | |
| $J_{19}$ | - | $CH_2$ | - | - | - | H | - | - | - | - | - | - | - | $OCH_3$ | $OCH_2C≡CH$ | |
| $J_{19}$ | - | $CH_2$ | - | - | - | H | - | - | - | - | - | - | - | $OCH_3$ | $OCH_2CH_2F$ | |
| $J_{19}$ | - | $CH_2$ | - | - | - | H | - | - | - | - | - | - | - | $OCH_3$ | $SCH_3$ | |
| ~~$J_{19}$~~ | | ~~$CH_2$~~ | | | | ~~H~~ | | | | | | | | ~~$OCH_3$~~ | ~~1,3-dioxolan-2-yl~~ | |
| $J_{19}$ | - | $CH_2$ | - | - | - | H | - | - | - | - | - | - | - | $OCH_3$ | cyclopropyl | |
| ~~$J_{20}$~~ | | ~~O~~ | | | | ~~H~~ | | | | | | | | ~~$OCH_3$~~ | ~~$CH(OCH_3)_2$~~ | |
| $J_{20}$ | - | O | - | - | - | H | - | - | - | - | - | - | - | Cl | $N(CH_3)_2$ | |
| $J_{20}$ | - | O | - | - | - | H | - | - | - | - | - | - | - | $OCH_3$ | $NHCH_3$ | |
| $J_{21}$ | - | - | - | - | - | H | $CH_3$ | - | - | - | - | - | - | Cl | $NHCH_3$ | |
| $J_{21}$ | - | - | - | - | - | H | $CH_3$ | - | - | - | - | - | - | Cl | $OCH_3$ | |
| $J_{22}$ | - | - | - | - | - | H | $CH_3$ | - | - | - | - | - | - | Cl | $OCH_3$ | |
| $J_{22}$ | - | - | - | - | - | H | H | - | - | - | - | - | - | $OCH_3$ | $NHCH_3$ | |
| $J_{23}$ | - | O | - | - | - | H | - | H | - | - | - | - | - | Cl | $NHCH_3$ | |
| $J_{23}$ | - | O | - | - | - | H | - | $CH_3$ | - | - | - | - | - | $OCH_3$ | $N(CH_3)_2$ | |
| ~~$J_{23}$~~ | | ~~O~~ | | | | ~~H~~ | | ~~$CH_3$~~ | | | | | | ~~$OCH_3$~~ | ~~1,3-dioxolan-2-yl~~ | |
| $J_{24}$ | - | $CH_2$ | $CH_2$ | - | | H | - | - | - | - | - | - | - | $OCH_3$ | $NHCH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{24}$ | - | $CH_2$ | $CH_2$ | - | - | H | - | - | - | - | - | - | - | $OCH_3$ | $SCH_3$ | |
| ~~$J_{24}$~~ | ~~-~~ | ~~$CH_2$~~ | ~~$CH_2$~~ | ~~-~~ | ~~-~~ | ~~H~~ | ~~-~~ | ~~-~~ | ~~-~~ | ~~-~~ | ~~-~~ | ~~-~~ | ~~-~~ | ~~$OCH_3$~~ | ~~$CH(OCH_3)_2$~~ | |
| $J_{24}$ | - | $CH_2$ | $CH_2$ | - | - | H | - | - | - | - | - | - | - | $OCH_3$ | $OCH_2CH{\equiv}CH$ | |
| $J_{25}$ | - | $CH_2$ | - | - | - | H | - | - | - | - | - | - | - | $OCH_3$ | $OCH_2CH{=}CH_2$ | |
| $J_{25}$ | - | $CH_2$ | - | - | - | H | - | - | - | - | - | - | - | $CH_3$ | $OCH_2CH_2F$ | |
| $J_{25}$ | - | $CH_2$ | - | - | - | H | - | - | - | - | - | - | - | $OCH_2CF_3$ | $OCH_3$ | |
| $J_{25}$ | - | $CH_2$ | - | - | - | H | - | - | - | - | - | - | - | $OCHF_2$ | $OCHF_2$ | |
| $J_{25}$ | - | $CH_2$ | - | - | - | H | - | - | - | - | - | - | - | $OCH_3$ | $OCH_2CH_2OCH_3$ | |
| $J_{26}$ | - | - | - | O | - | H | - | $CH_3$ | H | H | - | - | - | $OCH_3$ | $NHCH_3$ | |
| $J_{26}$ | - | - | - | O | - | H | - | $CH_3$ | H | H | - | - | - | $OCH_3$ | $OCH_2CH{=}CH_2$ | |
| $J_{26}$ | - | - | - | O | - | H | - | H | H | H | - | - | - | $OCH_3$ | $C{\equiv}CH$ | |
| $J_{26}$ | - | - | - | O | - | H | - | H | H | H | - | - | - | $OCH_3$ | $OCH_2CH_2F$ | |
| $J_{26}$ | - | - | - | O | - | H | - | $CH_3$ | H | H | - | - | - | $OCH_3$ | $OCH_2CH{=}CHCl$ | |
| $J_{26}$ | - | - | - | O | - | H | - | H | H | H | - | - | - | Cl | $NHCH_3$ | |
| $J_{26}$ | - | - | - | O | - | H | - | H | H | $CH_3$ | - | - | - | Cl | $N(CH_3)_2$ | |
| $J_{26}$ | - | - | - | O | - | H | - | H | H | $CH_3$ | - | - | - | $OCH_3$ | cyclopropyl | |
| ~~$J_{26}$~~ | | | | ~~O~~ | | ~~H~~ | | ~~H~~ | ~~H~~ | ~~$CH_3$~~ | | | | ~~$OCH_3$~~ | ~~$CH(OCH_3)_2$~~ | |
| $J_{26}$ | - | - | - | O | - | H | - | $CH_3$ | $CH_3$ | H | - | - | - | $OCH_3$ | $NHCH_3$ | |
| ~~$J_{26}$~~ | ~~-~~ | ~~-~~ | ~~-~~ | ~~O~~ | ~~-~~ | ~~H~~ | ~~-~~ | ~~$CH_3$~~ | ~~$CH_3$~~ | ~~H~~ | ~~-~~ | ~~-~~ | ~~-~~ | ~~$OCH_3$~~ | ~~$CH(OCH_3)_2$~~ | |
| $J_{26}$ | - | - | - | O | - | H | - | $CH_3$ | H | H | - | - | - | $OCH_3$ | $OCH_2C{\equiv}CH$ | |
| $J_{26}$ | - | - | - | O | - | H | - | $CH_3$ | $CH_3$ | H | - | - | - | $OCH_3$ | $SCH_3$ | |
| $J_{26}$ | - | - | - | S | - | H | - | $CH_3$ | H | H | - | - | | $OCH_3$ | $NHCH_3$ | |
| $J_{26}$ | - | - | - | S | - | H | - | $CH_3$ | H | H | - | - | - | $OCH_3$ | $N(CH_3)_2$ | |
| $J_{26}$ | - | - | - | S | - | H | - | $CH_3$ | H | H | - | - | - | $OCH_3$ | $OCH_2CH{=}CH_2$ | |
| $J_{26}$ | - | - | - | S | - | H | - | $CH_3$ | H | H | - | - | - | $OCH_3$ | cyclopropyl | |
| $J_{26}$ | - | - | - | S | - | H | - | $CH_3$ | H | H | - | - | - | Cl | $N(CH_3)_2$ | |
| ~~$J_{26}$~~ | ~~-~~ | ~~-~~ | ~~-~~ | ~~S~~ | ~~-~~ | ~~H~~ | ~~-~~ | ~~$CH_3$~~ | ~~$CH_3$~~ | ~~$CH_3$~~ | ~~-~~ | ~~-~~ | ~~-~~ | ~~$OCH_3$~~ | ~~$CH(OCH_3)_2$~~ | |
| $J_{26}$ | - | - | - | $SO_2$ | - | H | - | $CH_3$ | H | H | - | - | - | $OCH_3$ | $NHCH_3$ | |
| $J_{26}$ | - | - | - | $SO_2$ | - | H | - | $CH_3$ | H | H | - | - | - | Cl | $N(CH_3)_2$ | |
| $J_{26}$ | - | - | - | $SO_2$ | - | H | - | $CH_3$ | H | H | - | - | - | $OCH_3$ | $OCH_2CH{=}CH_2$ | |
| $J_{26}$ | - | - | - | $SO_2$ | - | H | - | $CH_3$ | H | H | - | | - | $OCHF_2$ | $SCH_3$ | |
| $J_{26}$ | - | - | - | $SO_2$ | - | H | - | $CH_3$ | H | H | - | - | - | $CH_3$ | $OCH_2CH_2OCH_3$ | |

Table XXIIIa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| ~~$J_{26}$~~ | | | | ~~$SO_2$~~ | | ~~H~~ | | ~~$CH_3$~~ | ~~H~~ | ~~H~~ | | | | ~~$CH_3$~~ | ~~$CH(OCH_3)_2$~~ | |
| $J_{27}$ | - | $CH_2$ | - | O | - | H | - | $CH_3$ | H | - | - | - | - | $OCH_3$ | $N(CH_3)_2$ | |
| $J_{27}$ | - | $CH_2$ | - | S | - | H | - | H | $CH_3$ | - | - | - | - | $OCH_3$ | $OCH_2CH{=}CH_2$ | |
| $J_{27}$ | - | O | - | $SO_2$ | - | H | - | $CH_3$ | H | - | - | - | - | $OCH_3$ | $OCH_2CH_2F$ | |
| $J_{27}$ | - | O | - | $SO_2$ | - | H | - | $CH_3$ | H | - | - | - | - | $OCH_3$ | $OCH_2CH_2OCH_3$ | |
| $J_{28}$ | - | - | - | - | O | H | - | $CH_3$ | H | - | - | - | - | Cl | $N(CH_3)_2$ | |
| ~~$J_{28}$~~ | | | | | ~~O~~ | ~~H~~ | | ~~$CH_3$~~ | ~~H~~ | | | | | ~~$CH_3$~~ | ~~$CH(OCH_3)_2$~~ | |
| $J_{28}$ | - | - | - | - | S | H | - | $CH_3$ | H | - | - | - | - | $OCH_3$ | $NHCH_3$ | |
| $J_{29}$ | - | $CH_2$ | - | - | - | H | - | H | H | - | H | H | - | $OCH_3$ | $NHCH_3$ | |
| $J_{29}$ | - | $CH_2$ | - | - | - | H | - | H | H | - | $CH_3$ | H | - | Cl | $N(CH_3)_2$ | |
| $J_{29}$ | - | O | - | - | - | H | - | H | H | - | H | $CH_3$ | - | $CH_3$ | $OCH_2CH_2OCH_3$ | |
| $J_{30}$ | - | - | - | - | - | H | - | H | H | - | - | - | - | $CH_3$ | $OCH_2CH_2F$ | |
| $J_{30}$ | - | - | - | - | - | H | - | $CH_3$ | $CH_3$ | - | - | - | - | $OCH_3$ | $NHCH_3$ | |
| $J_{30}$ | - | - | - | - | - | H | - | H | $CH_3$ | - | - | - | - | $OCH_3$ | $C{\equiv}CH$ | |
| $J_{31}$ | - | $CH_2$ | - | - | - | H | - | - | - | - | - | - | - | $OCH_3$ | $NHCH_3$ | |
| $J_{31}$ | - | $CH_2$ | - | - | - | H | - | - | - | - | - | - | - | $CH_3$ | $OCH_2CH_2F$ | |
| $J_{31}$ | - | O | - | - | - | H | - | - | - | - | - | - | - | $OCH_3$ | $C{\equiv}CH$ | |
| ~~$J_{31}$~~ | | ~~O~~ | | | | ~~H~~ | | | | | | | | ~~$OCH_3$~~ | ~~$CH(OCH_3)_2$~~ | |
| $J_{32}$ | - | $CH_2$ | - | - | - | - | - | - | - | - | - | - | Cl | $OCH_3$ | $NHCH_3$ | |
| $J_{32}$ | - | $CH_2$ | - | - | - | - | - | - | - | - | - | - | Br | $OCH_3$ | $N(CH_3)_2$ | |
| $J_{32}$ | - | $CH_2$ | - | - | - | - | - | - | - | - | - | - | F | $OCH_3$ | $NHCH_3$ | |
| $J_{32}$ | - | $CH_2$ | - | - | - | - | - | - | - | - | - | - | $OCH_3$ | $OCH_3$ | $NHCH_3$ | |
| $J_{32}$ | - | $CH_2$ | - | - | - | - | - | - | - | - | - | - | $SO_2N(CH_3)_2$ | $OCH_3$ | $C{\equiv}CH$ | |
| $J_{32}$ | - | $CH_2$ | - | - | - | - | - | - | - | - | - | - | Cl | $OCH_3$ | $OCH_2CH_2F$ | |
| $J_{32}$ | - | O | - | - | - | - | - | - | - | - | - | - | Cl | Cl | $NHCH_3$ | |
| $J_{32}$ | - | O | - | - | - | - | - | - | - | - | - | - | Br | $OCH_3$ | $NHCH_3$ | |
| $J_{32}$ | - | O | - | - | - | - | - | - | - | - | - | - | $OCH_3$ | $OCH_3$ | $NHCH_3$ | |
| $J_{32}$ | - | O | - | - | - | - | - | - | - | - | - | - | $SO_2N(CH_3)_2$ | $OCH_3$ | $NHCH_3$ | |
| $J_{32}$ | - | O | - | - | - | - | - | - | - | - | - | - | $SO_2CH_3$ | $OCH_3$ | $NHCH_3$ | |
| $J_{32}$ | - | $CH_2$ | - | - | - | - | - | - | - | - | - | - | $CO_2CH_3$ | $OCH_3$ | $OCH_3$ | 148-151.5 |
| $J_{32}$ | - | $CH_2$ | - | - | - | - | - | - | - | - | - | - | $CO_2CH_3$ | $CH_3$ | $CH_3$ | 191-193.5 |
| $J_{32}$ | - | $CH_2$ | - | - | - | - | - | - | - | - | - | - | $CO_2CH_3$ | $OCH_3$ | $CH_3$ | |

## Table XXIV

## General Formula 3

| J | n | L | $R_{20}$ | $R_{22}$ | X | Y | m.p. (°C) |
|---|---|---|---|---|---|---|---|
| $J_{12}$ | 0 | $CH_2$ | H | $CH_3$ | $CH_3$ | $OCH_3$ | |
| $J_{12}$ | 0 | $CH_2$ | H | $CH_3$ | $OCH_3$ | $OCH_3$ | |
| $J_{12}$ | 0 | $CH_2$ | H | $CH_3$ | $CH_3$ | $CH_3$ | |
| $J_{12}$ | 0 | $CH_2$ | H | $CH_3$ | $OCH_3$ | $C_2H_5$ | |
| $J_{12}$ | 0 | $CH_2$ | H | $CH_3$ | $CH_3$ | $OC_2H_5$ | |
| ~~$J_{12}$~~ | ~~0~~ | ~~$CH_2$~~ | ~~H~~ | ~~$CH_3$~~ | ~~$OCH_3$~~ | ~~$CH_2OCH_3$~~ | |
| $J_{12}$ | 0 | $CH_2$ | H | $CH_3$ | $OCH_3$ | $CH_2F$ | |
| $J_{12}$ | 0 | $CH_2$ | H | $CH_3$ | $OCH_3$ | $CF_3$ | |
| $J_{12}$ | 1 | $CH_2$ | H | $CH_3$ | $OCH_3$ | $CH_3$ | |
| $J_{12}$ | 0 | $CH_2$ | H | $C_2H_5$ | $OCH_3$ | $CH_3$ | |
| $J_{12}$ | 0 | $CH_2$ | H | $\underline{n}$-$C_3H_7$ | $OCH_3$ | $CH_3$ | |
| $J_{12}$ | 0 | $CH_2$ | H | $CH(CH_3)_2$ | $OCH_3$ | $CH_3$ | |
| $J_{12}$ | 0 | $CH_2$ | H | H | $OCH_3$ | $OCH_3$ | |
| $J_{12}$ | 0 | $CH_2$ | H | H | $OCH_3$ | $CH_3$ | |
| $J_{12}$ | 0 | $CH_2$ | H | H | $CH_3$ | $CH_3$ | |
| $J_{12}$ | 0 | O | H | H | $CH_3$ | $CH_3$ | |
| $J_{12}$ | 0 | O | H | H | $CH_3$ | $OCH_3$ | |
| $J_{12}$ | 0 | O | H | H | $OCH_3$ | $OCH_3$ | |
| $J_{12}$ | 0 | O | H | $CH_3$ | $OCH_3$ | $OCH_3$ | |
| $J_{12}$ | 0 | O | H | $CH_3$ | $CH_3$ | $OCH_3$ | |
| $J_{12}$ | 0 | O | H | $CH_3$ | $CH_3$ | $CH_3$ | |
| $J_{12}$ | 0 | O | H | $C_2H_5$ | $OCH_3$ | $OCH_3$ | |
| $J_{12}$ | 0 | O | H | $\underline{n}$-$C_3H_7$ | $OCH_3$ | $CH_3$ | |
| $J_{12}$ | 0 | O | H | $CH(CH_3)_2$ | $OCH_3$ | $CH_3$ | |
| $J_{12}$ | 1 | O | H | $CH_3$ | $OCH_3$ | $CH_3$ | |
| $J_{12}$ | 0 | $CH_2$ | 5-F | $CH_3$ | $OCH_3$ | $CH_3$ | |
| $J_{12}$ | 0 | $CH_2$ | 6-Cl | $CH_3$ | $OCH_3$ | $CH_3$ | |
| $J_{12}$ | 0 | $CH_2$ | 6-Br | $CH_3$ | $OCH_3$ | $OCH_3$ | |
| $J_{12}$ | 0 | $CH_2$ | 6-$CH_3$ | $CH_3$ | $OCH_3$ | $OCH_3$ | |
| $J_{12}$ | 0 | $CH_2$ | 6-$OCH_3$ | $CH_3$ | $OCH_3$ | $OCH_3$ | |
| $J_{12}$ | 0 | O | H | $CH_3$ | $OCH_2CF_3$ | $CH_3$ | |
| $J_{12}$ | 0 | O | H | $CH_3$ | $OCH_3$ | $CH(CH_3)_2$ | |

Table XXIV (continued)

| J | n | L | $R_{20}$ | $R_{22}$ | X | Y | m.p. (°C) |
|---|---|---|---|---|---|---|---|
| $J_{12}$ | 0 | O | H | $CH_3$ | $OCH_3$ | cyclopropyl | |
| $J_{12}$ | 0 | O | H | $CH_3$ | $OCH_3$ | C≡CH | |
| $J_{12}$ | 0 | O | H | $CH_3$ | $OCH_3$ | $NH_2$ | |
| $J_{12}$ | 0 | O | H | $CH_3$ | $OCH_3$ | $N(CH_3)_2$ | |
| $J_{12}$ | 0 | O | H | $CH_3$ | $OCH_3$ | $OCH_2CH_2F$ | |
| $J_{12}$ | 0 | O | H | $CH_3$ | $OCH_3$ | $SCH_3$ | |
| $J_{12}$ | 0 | O | H | $CH_3$ | $OCH_3$ | $OCH_2CH=CH_2$ | |
| $J_{12}$ | 0 | O | H | $CH_3$ | $OCH_3$ | $OCH_2CH_2OCH_3$ | |
| $J_{12}$ | 0 | O | H | $CH_3$ | $OCH_3$ | $OCH_2C≡CH$ | |
| ~~$J_{12}$~~ | ~~0~~ | ~~O~~ | ~~H~~ | ~~$CH_3$~~ | ~~$OCH_3$~~ | ~~$CH(OCH_3)_2$~~ | |
| ~~$J_{12}$~~ | ~~0~~ | ~~O~~ | ~~H~~ | ~~$CH_3$~~ | ~~$OCH_3$~~ | ~~$C(CH_3)(OCH_3)_2$~~ | |
| $J_{12}$ | 0 | O | H | $CH_3$ | $OCH_3$ | 1,3-dioxolan-2-yl | |
| $J_{12}$ | 0 | O | H | $CH_3$ | $OCH_3$ | 2-methyl-1,3-dioxolan-2-yl | |
| $J_{12}$ | 0 | O | H | $CH_3$ | $OCH_3$ | $CH(OC_2H_5)_2$ | |
| ~~$J_{12}$~~ | ~~0~~ | ~~O~~ | ~~H~~ | ~~$CH_3$~~ | ~~$OCH_3$~~ | ~~$C(CH_3)(OC_2H_5)_2$~~ | |
| $J_{12}$ | 0 | O | H | $CH_3$ | $OCH_3$ | $NHCH_3$ | |
| $J_{12}$ | 0 | O | H | $-CH_2CN$ | $OCH_3$ | $OCH_3$ | |
| $J_{12}$ | 0 | O | H | $-(CH_2)_3CN$ | $CH_3$ | $OCH_3$ | |
| $J_{12}$ | 0 | $CH_2$ | H | OH | $CH_3$ | $OCH_3$ | |
| $J_{12}$ | 0 | $CH_2$ | H | $-(CH_2)_3CH(CH_3)_2$ | Cl | $OCH_3$ | |
| $J_{12}$ | 0 | $CH_2$ | H | $-CF_2H$ | $OCH_3$ | $OCH_3$ | |
| $J_{12}$ | 0 | $CH_2$ | H | $-CH_2CH_2Cl$ | Cl | $OCH_3$ | |
| $J_{12}$ | 0 | $CH_2$ | H | $-(CH_2)_5Cl$ | $CH_3$ | $OCH_3$ | |
| $J_{12}$ | 0 | $CH_2$ | H | $-CH_2OCH_3$ | Cl | $OCH_3$ | |
| $J_{12}$ | 0 | $CH_2$ | H | $-(CH_2)_4OCH_3$ | $CH_3$ | $OCH_3$ | |
| $J_{12}$ | 0 | $CH_2$ | H | $-CH_2CH=CH_2$ | $OCH_3$ | $OCH_3$ | |
| $J_{12}$ | 0 | $CH_2$ | H | $-CH_2CH=CHCH_3$ | $CH_3$ | $OCH_3$ | |
| $J_{12}$ | 0 | $CH_2$ | H | $-CH_2C≡CH$ | $OCH_3$ | $OCH_3$ | |
| $J_{12}$ | 0 | $CH_2$ | H | $-(CH_2)_4C≡CH$ | $CH_3$ | $OCH_3$ | |
| $J_{12}$ | 0 | $CH_2$ | H | $-OCH_3$ | $CH_3$ | $OCH_3$ | |
| $J_{12}$ | 0 | $CH_2$ | H | $-C_6H_5$ | $CH_3$ | $OCH_3$ | |
| $J_{12}$ | 0 | $CH_2$ | H | cyclopropyl | $OCH_3$ | $OCH_3$ | |

Table XXIV (continued)

| J | n | L | $R_{20}$ | $R_{22}$ | X | Y | m.p. (°C) |
|---|---|---|---|---|---|---|---|
| $J_{12}$ | 0 | $CH_2$ | H | cyclohexyl | $OCH_3$ | $OCH_3$ | |
| $J_{12}$ | 0 | $CH_2$ | H | cyclopropylmethyl | $OCH_3$ | $OCH_3$ | |
| $J_{12}$ | 0 | $CH_2$ | H | cyclohexylmethyl | $CH_3$ | $OCH_3$ | |
| $J_{12}$ | 0 | $CH_2$ | H | $-C(O)CH_3$ | $OCH_3$ | $OCH_3$ | |
| $J_{12}$ | 0 | $CH_2$ | H | $-C(O)(CH_2)_5H$ | $OCH_3$ | $OCH_3$ | |
| $J_{12}$ | 0 | $CH_2$ | H | $-CO_2C_2H_5$ | $CH_3$ | $OCH_3$ | |
| $J_{12}$ | 0 | $CH_2$ | H | $-CO_2(CH_2)_4H$ | $CH_3$ | $OCH_3$ | |
| $J_{12}$ | 0 | $CH_2$ | H | $-CH_2C_6H_5$ | $OCH_3$ | $OCH_3$ | |
| $J_{12}$ | 0 | $CH_2$ | H | $-CH_2-CH=CH-CH_2Cl$ | $CH_3$ | $OCH_3$ | |
| $J_{12}$ | 0 | $CH_2$ | H | $-CH_2C\equiv C-CH_2Cl$ | $CH_3$ | $OCH_3$ | |
| $J_{12}$ | 0 | $CH_2$ | H | $-CH_2C(O)CH_3$ | $OCH_3$ | $OCH_3$ | |
| $J_{12}$ | 0 | $CH_2$ | H | $-CH(CH_3)CO_2C_2H_5$ | $OCH_3$ | $OCH_3$ | |
| $J_{12}$ | 0 | $CH_2$ | H | H | $OCH_3$ | $OCH_3$ | |
| $J_{12}$ | 0 | $CH_2$ | H | H | $CH_3$ | $CH_3$ | |
| $J_{12}$ | 0 | O | H | H | $CH_3$ | $CH_3$ | |
| $J_{12}$ | 0 | O | H | H | $OCH_3$ | $CH_3$ | |
| $J_{12}$ | 0 | O | H | H | $OCH_3$ | $OCH_3$ | |
| $J_{12}$ | 0 | $CH_2$ | H | $\underline{n}-C_4H_9$ | $CH_3$ | $CH_3$ | |
| $J_{12}$ | 0 | $CH_2$ | H | $\underline{n}-C_4H_9$ | $CH_3$ | $OCH_3$ | |
| $J_{12}$ | 0 | $CH_2$ | H | $\underline{n}-C_4H_9$ | $OCH_3$ | $OCH_3$ | |
| $J_{12}$ | 0 | O | H | $\underline{n}-C_4H_9$ | $CH_3$ | $CH_3$ | |
| $J_{12}$ | 0 | O | H | $\underline{n}-C_4H_9$ | $OCH_3$ | $CH_3$ | |
| $J_{12}$ | 0 | O | H | $\underline{n}-C_4H_9$ | $OCH_3$ | $OCH_3$ | |
| $J_{18}$ | - | $CH_2$ | H | $\underline{n}-C_3H_7$ | $CH_3$ | $OCH_3$ | |
| $J_{18}$ | - | $CH_2$ | H | $CH(CH_3)_2$ | $CH_3$ | $OCH_3$ | |
| $J_{18}$ | - | O | H | $CH_3$ | $CH_3$ | $OCH_3$ | |
| $J_{18}$ | - | $CH_2$ | 7-Cl | $CH_3$ | $CH_3$ | $OCH_3$ | |
| $J_{18}$ | - | O | 7-Br | $CH_3$ | $CH_3$ | $OCH_3$ | |
| $J_{18}$ | - | $CH_2$ | 6-F | $CH_3$ | $CH_3$ | $OCH_3$ | |
| $J_{18}$ | - | O | 7-$CH_3$ | $CH_3$ | $CH_3$ | $OCH_3$ | |
| $J_{18}$ | - | O | 7-$OCH_3$ | $CH_3$ | $CH_3$ | $OCH_3$ | |
| $J_{18}$ | - | $CH_2$ | H | $CH_3$ | $OCH_3$ | $OCH_3$ | |
| $J_{18}$ | - | $CH_2$ | H | $CH_3$ | $OCH_3$ | $CH_3$ | |

Table XXIV (continued)

| J | n | L | $R_{20}$ | $R_{22}$ | X | Y | m.p. (°C) |
|---|---|---|---|---|---|---|---|
| $J_{18}$ | - | $CH_2$ | H | $CH_3$ | $CH_3$ | $CH_3$ | |
| $J_{18}$ | - | $CH_2$ | H | $CH_3$ | $OCH_3$ | $C_2H_5$ | |
| $J_{18}$ | - | $CH_2$ | H | $CH_3$ | $CH_3$ | $OC_2H_5$ | |
| ~~$J_{18}$~~ | ~~-~~ | ~~$CH_2$~~ | ~~H~~ | ~~$CH_3$~~ | ~~$OCH_3$~~ | ~~$CH_2OCH_3$~~ | |
| $J_{18}$ | - | $CH_2$ | H | $CH_3$ | $OCH_3$ | $CH_2F$ | |
| $J_{18}$ | - | $CH_2$ | H | $CH_3$ | $OCH_3$ | $CF_3$ | |
| $J_{18}$ | - | $CH_2$ | H | H | $CH_3$ | $OCH_3$ | |
| $J_{18}$ | - | $CH_2$ | H | $C_2H_5$ | $CH_3$ | $OCH_3$ | |
| $J_{18}$ | - | $CH_2$ | H | $CH_3$ | $OCH_2CF_3$ | $OCH_3$ | |
| $J_{18}$ | - | $CH_2$ | H | $CH_3$ | $OCH_3$ | cyclopropyl | |
| $J_{18}$ | - | $CH_2$ | H | $CH_3$ | $OCH_3$ | $NHCH_3$ | |
| $J_{18}$ | - | $CH_2$ | H | $CH_3$ | $OCH_3$ | $N(CH_3)_2$ | |
| $J_{18}$ | - | $CH_2$ | H | $CH_3$ | $OCH_3$ | $OCH_2CH{=}CH_2$ | |
| ~~$J_{18}$~~ | ~~-~~ | ~~$CH_2$~~ | ~~H~~ | ~~$CH_3$~~ | ~~$OCH_3$~~ | ~~$CH(OCH_3)_2$~~ | |
| $J_{18}$ | - | $CH_2$ | H | $CH_3$ | $OCH_3$ | 1,3-dioxolan-2-yl | |
| ~~$J_{18}$~~ | ~~-~~ | ~~$CH_2$~~ | ~~H~~ | ~~$CH_3$~~ | ~~$OCH_3$~~ | ~~$CH(OC_2H_5)_2$~~ | |
| $J_{18}$ | - | $CH_2$ | H | $CH_3$ | $OCH_3$ | $-C{\equiv}CH$ | |
| ~~$J_{18}$~~ | ~~-~~ | ~~$CH_2$~~ | ~~H~~ | ~~$CH_3$~~ | ~~$OCH_3$~~ | ~~$C(CH_3)(OCH_3)_2$~~ | |
| $J_{18}$ | - | $CH_2$ | H | OH | $OCH_3$ | $OCH_3$ | |
| $J_{18}$ | - | $CH_2$ | H | $(CH_2)_2CH(CH_3)_2$ | $CH_3$ | $OCH_3$ | |
| $J_{18}$ | - | $CH_2$ | H | $CH_2CH_2Cl$ | $OCH_3$ | $OCH_3$ | |
| $J_{18}$ | - | $CH_2$ | H | $(CH_2)_5Cl$ | $CH_3$ | $OCH_3$ | |
| $J_{18}$ | - | $CH_2$ | H | $CH_2OCH_3$ | $CH_3$ | $OCH_3$ | |
| $J_{18}$ | - | $CH_2$ | H | $(CH_2)_2OCH_3$ | $CH_3$ | $OCH_3$ | |
| $J_{18}$ | - | $CH_2$ | H | $CH_2CH{=}CH_2$ | $OCH_3$ | $OCH_3$ | |
| $J_{18}$ | - | $CH_2$ | H | $CH_2CH{=}CHCH_2Cl$ | $CH_3$ | $OCH_3$ | |
| $J_{18}$ | - | $CH_2$ | H | $CH_2CH{=}CHCH_3$ | $CH_3$ | $OCH_3$ | |
| $J_{18}$ | - | $CH_2$ | H | $CH_2C{\equiv}CCH_3$ | $OCH_3$ | $OCH_3$ | |
| $J_{18}$ | - | $CH_2$ | H | $OCH_3$ | $OCH_3$ | $OCH_3$ | |
| $J_{18}$ | - | $CH_2$ | H | $O(CH_2)_4CH_3$ | $CH_3$ | $OCH_3$ | |
| $J_{18}$ | - | $CH_2$ | H | $C_6H_5$ | $OCH_3$ | $OCH_3$ | |

Table XXIV (continued)

| J | n | L | $R_{20}$ | $R_{22}$ | X | Y | m.p. (°C) |
|---|---|---|---|---|---|---|---|
| $J_{18}$ | - | $CH_2$ | H | cyclopropyl | $CH_3$ | $OCH_3$ | |
| $J_{18}$ | - | $CH_2$ | H | cyclopentyl | $OCH_3$ | $OCH_3$ | |
| $J_{18}$ | - | $CH_2$ | H | $COCH_3$ | $OCH_3$ | $OCH_3$ | |
| $J_{18}$ | - | $CH_2$ | H | $CO_2CH_3$ | $OCH_3$ | $OCH_3$ | |
| $J_{18}$ | - | $CH_2$ | H | $CH_2C_6H_5$ | $CH_3$ | $OCH_3$ | |
| $J_{18}$ | - | $CH_2$ | H | $CH_2CH=CHCl$ | $CH_3$ | $OCH_3$ | |
| $J_{18}$ | - | $CH_2$ | H | $CH(CH_3)CO_2CH_3$ | $CH_3$ | $OCH_3$ | |

Table XXIVa

General Formula 3a

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{13}$ | 0 | $CH_2$ | - | - | - | H | H | - | - | - | - | - | - | $OCH_3$ | $OCH_3$ | |
| $J_{13}$ | 0 | $CH_2$ | - | - | - | H | $CH_3$ | - | - | - | - | - | - | $OCH_3$ | $CH_3$ | |
| $J_{13}$ | 0 | $CH_2$ | - | - | - | H | $CH_3$ | - | - | - | - | - | - | $OCH_3$ | $C_2H_5$ | |
| $J_{13}$ | 0 | $CH_2$ | - | - | - | H | $CH_3$ | - | - | - | - | - | - | $CH_3$ | $OC_2H_5$ | |
| ~~$J_{13}$~~ | ~~0~~ | ~~$CH_2$~~ | ~~-~~ | ~~-~~ | ~~-~~ | ~~H~~ | ~~$CH_3$~~ | ~~-~~ | ~~-~~ | ~~-~~ | ~~-~~ | ~~-~~ | ~~-~~ | ~~$OCH_3$~~ | ~~$CH_2OCH_3$~~ | |
| $J_{13}$ | 0 | $CH_2$ | - | - | - | H | $CH_3$ | - | - | - | - | - | - | $OCH_3$ | $CH_2F$ | |
| $J_{13}$ | 0 | $CH_2$ | - | - | - | H | $CH_3$ | - | - | - | - | - | - | $OCH_3$ | $CF_3$ | |
| $J_{13}$ | 0 | $CH_2$ | - | - | - | H | H | - | - | - | - | - | - | $CH_3$ | $OCH_3$ | |
| $J_{13}$ | 0 | $CH_2$ | - | - | - | H | $C_2H_5$ | - | - | - | - | - | - | $CH_3$ | $OCH_3$ | |
| $J_{13}$ | 0 | $CH_2$ | - | - | - | H | n-$C_3H_7$ | - | - | - | - | - | - | $CH_3$ | $OCH_3$ | |
| $J_{13}$ | 0 | $CH_2$ | - | - | - | H | $CH(CH_3)_2$ | - | - | - | - | - | - | $CH_3$ | $OCH_3$ | |
| $J_{13}$ | 1 | $CH_2$ | - | - | - | H | $CH_3$ | - | - | - | - | - | - | $CH_3$ | $OCH_3$ | |
| $J_{13}$ | 0 | O | - | - | - | H | $CH_3$ | - | - | - | - | - | - | $OCH_3$ | $OCH_3$ | |
| $J_{13}$ | 0 | O | - | - | - | H | $CH_3$ | - | - | - | - | - | - | $OCH_3$ | $CH_3$ | |
| $J_{13}$ | 1 | O | - | - | - | H | $CH_3$ | - | - | - | - | - | - | $CH_3$ | $OCH_3$ | |
| $J_{13}$ | 0 | O | - | - | - | H | $C_2H_5$ | - | - | - | - | - | - | $CH_3$ | $OCH_3$ | |
| $J_{13}$ | 0 | $CH_2$ | - | - | - | 6-Br | $CH_3$ | - | - | - | - | - | - | $CH_3$ | $OCH_3$ | |
| $J_{13}$ | 0 | $CH_2$ | - | - | - | 6-Cl | $CH_3$ | - | - | - | - | - | - | $CH_3$ | $OCH_3$ | |
| $J_{13}$ | 0 | $CH_2$ | - | - | - | 5-F | $CH_3$ | - | - | - | - | - | - | $CH_3$ | $OCH_3$ | |
| $J_{13}$ | 0 | O | - | | - | 6-$CH_3$ | $CH_3$ | - | - | - | - | - | - | $CH_3$ | $OCH_3$ | |
| $J_{13}$ | 0 | O | - | - | - | 6-$OCH_3$ | $CH_3$ | - | - | - | - | - | - | $CH_3$ | $OCH_3$ | |
| $J_{14}$ | 0 | $CH_2$ | - | - | - | H | - | H | H | - | - | - | - | $OCH_3$ | $OCH_3$ | |
| $J_{14}$ | 0 | $CH_2$ | - | - | - | H | - | H | H | - | - | - | - | $CH_3$ | $OCH_3$ | 175-183,d |
| $J_{14}$ | 0 | $CH_2$ | - | - | - | H | - | H | H | - | - | - | - | $CH_3$ | $CH_3$ | |
| $J_{14}$ | 0 | $CH_2$ | - | - | - | H | - | H | H | - | - | - | - | $OCH_3$ | $C_2H_5$ | |
| $J_{14}$ | 0 | $CH_2$ | - | - | - | H | - | H | H | - | - | - | - | $CH_3$ | $OC_2H_5$ | |
| ~~$J_{14}$~~ | ~~0~~ | ~~$CH_2$~~ | ~~-~~ | ~~-~~ | ~~-~~ | ~~H~~ | ~~-~~ | ~~H~~ | ~~H~~ | ~~-~~ | ~~-~~ | ~~-~~ | ~~-~~ | ~~$CH_3$~~ | ~~$CH_2OCH_3$~~ | |
| $J_{14}$ | 0 | $CH_2$ | - | - | - | H | - | H | H | - | - | - | - | $OCH_3$ | $CH_2F$ | |
| $J_{14}$ | 0 | $CH_2$ | - | - | - | H | - | H | H | - | - | - | - | $OCH_3$ | $CF_3$ | |
| $J_{14}$ | 1 | $CH_2$ | - | - | - | H | - | H | H | - | - | - | - | $CH_3$ | $OCH_3$ | |
| $J_{14}$ | 0 | $CH_2$ | - | - | - | H | - | $CH_3$ | H | - | - | - | - | $CH_3$ | $OCH_3$ | |

Table XXIVa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{14}$ | 0 | $CH_2$ | - | - | - | H | - | H | $CH_3$ | - | - | - | - | $CH_3$ | $OCH_3$ | |
| $J_{14}$ | 0 | O | - | - | - | H | - | H | H | - | - | - | - | $OCH_3$ | $OCH_3$ | |
| $J_{14}$ | 0 | O | - | - | - | H | - | H | H | - | - | - | - | $CH_3$ | $CH_3$ | |
| $J_{14}$ | 0 | O | - | - | - | H | - | H | H | - | - | - | - | $OCH_3$ | $CH_3$ | |
| $J_{14}$ | 0 | O | - | - | - | H | - | H | H | - | - | - | - | $CH_3$ | $CH_3$ | |
| $J_{14}$ | 0 | $CH_2$ | - | - | - | 5-F | - | H | H | - | - | - | - | $CH_3$ | $OCH_3$ | |
| $J_{14}$ | 0 | $CH_2$ | - | - | - | 6-Cl | - | H | H | - | - | - | - | $OCH_3$ | $OCH_3$ | |
| $J_{14}$ | 0 | $CH_2$ | - | - | - | 6-Br | - | H | H | - | - | - | - | $OCH_3$ | $CH_3$ | |
| $J_{14}$ | 0 | O | - | - | - | 5-$CH_3$ | - | H | H | - | - | - | - | $OCH_3$ | $CH_3$ | |
| $J_{14}$ | 0 | O | - | - | - | 6-$OCH_3$ | - | H | H | - | - | - | - | $OCH_3$ | $CH_3$ | |
| $J_{14}$ | 1 | O | - | - | - | H | - | H | H | - | - | - | - | $OCH_3$ | $CH_3$ | |
| $J_{14}$ | 1 | $CH_2$ | - | - | - | H | - | $CH_3$ | H | - | - | - | - | $CH_3$ | $CH_3$ | |
| $J_{14}$ | 1 | $CH_2$ | - | - | - | H | - | $CH_3$ | H | - | - | - | - | $CH_3$ | $OCH_3$ | |
| $J_{14}$ | 1 | $CH_2$ | - | - | - | H | - | $CH_3$ | H | - | - | - | - | $OCH_3$ | $OCH_3$ | |
| $J_{14}$ | 1 | O | - | - | - | H | - | $CH_3$ | H | - | - | - | - | $CH_3$ | $CH_3$ | |
| $J_{14}$ | 1 | O | - | - | - | H | - | $CH_3$ | H | - | - | - | - | $CH_3$ | $OCH_3$ | |
| $J_{14}$ | 1 | O | - | - | - | H | - | $CH_3$ | H | - | - | - | - | $OCH_3$ | $OCH_3$ | |
| $J_{15}$ | 0 | $CH_2$ | - | - | - | H | - | - | - | - | - | - | - | $CH_3$ | $OCH_3$ | |
| $J_{15}$ | 1 | $CH_2$ | - | - | - | H | - | - | - | - | - | - | - | $CH_3$ | $CH_3$ | |
| $J_{15}$ | 0 | O | - | - | - | H | - | - | - | - | - | - | - | $CH_3$ | $OCH_3$ | |
| $J_{15}$ | 1 | O | - | - | - | H | - | - | - | - | - | - | - | $CH_3$ | $OCH_3$ | |
| $J_{15}$ | 0 | - | - | - | - | H | - | - | - | - | - | - | - | $OCH_3$ | $OCH_3$ | |
| $J_{16}$ | 0 | - | - | - | - | H | $CH_3$ | - | - | - | - | - | - | $CH_3$ | $OCH_3$ | |
| $J_{16}$ | 0 | - | - | - | - | H | $CH_3$ | - | - | - | - | - | - | $CH_3$ | $CH_3$ | |
| $J_{16}$ | 1 | - | - | - | - | H | $CH_3$ | - | - | - | - | - | - | $CH_3$ | $OCH_3$ | |
| $J_{16}$ | 0 | - | - | - | - | H | H | - | - | - | - | - | - | $CH_3$ | $OCH_3$ | |
| $J_{17}$ | 0 | - | - | - | - | H | $CH_3$ | - | - | - | - | - | - | $OCH_3$ | $OCH_3$ | |
| $J_{17}$ | 0 | - | - | - | - | H | $CH_3$ | - | - | - | - | - | - | $CH_3$ | $OCH_3$ | |
| $J_{17}$ | 0 | - | - | - | - | H | H | - | - | - | - | - | - | $CH_3$ | $OCH_3$ | |
| $J_{17}$ | 1 | - | - | - | - | H | $CH_3$ | - | - | - | - | - | - | $CH_3$ | $OCH_3$ | |
| $J_{19}$ | - | $CH_2$ | - | - | - | H | - | H | - | - | - | - | - | $OCH_3$ | $OCH_3$ | |
| $J_{19}$ | - | $CH_2$ | - | - | - | H | - | H | - | - | - | - | - | $OCH_3$ | $CH_3$ | |
| $J_{19}$ | - | $CH_2$ | - | - | - | H | - | $CH_3$ | - | - | - | - | - | $OCH_3$ | $CH_3$ | |

Table XXIVa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{19}$ | - | $CH_2$ | - | - | - | H | - | H | - | - | - | - | - | $OCH_3$ | $C_2H_5$ | |
| $J_{19}$ | - | $CH_2$ | - | - | - | H | - | H | - | - | - | - | - | $CH_3$ | $OC_2H_5$ | |
| ~~$J_{19}$~~ | ~~-~~ | ~~$CH_2$~~ | ~~-~~ | ~~-~~ | ~~-~~ | ~~H~~ | ~~-~~ | ~~H~~ | ~~-~~ | ~~-~~ | ~~-~~ | ~~-~~ | ~~-~~ | ~~$OCH_3$~~ | ~~$CH_2OCH_3$~~ | |
| $J_{19}$ | - | $CH_2$ | - | - | - | H | - | H | - | - | - | - | - | $OCH_3$ | $CH_2F$ | |
| $J_{19}$ | - | $CH_2$ | - | - | - | H | - | H | - | - | - | - | - | $OCH_3$ | $CF_3$ | |
| $J_{19}$ | - | O | - | - | - | H | - | H | - | - | - | - | - | $OCH_3$ | $OCH_3$ | |
| $J_{19}$ | - | O | - | - | - | H | - | H | - | - | - | - | - | $OCH_3$ | $CH_3$ | |
| $J_{19}$ | - | $CH_2$ | - | - | - | 7-Cl | - | H | - | - | - | - | - | $CH_3$ | $OCH_3$ | |
| $J_{20}$ | - | $CH_2$ | - | - | - | H | - | - | - | - | - | - | - | $OCH_3$ | $OCH_3$ | |
| $J_{20}$ | - | $CH_2$ | - | - | - | H | - | - | - | - | - | - | - | $OCH_3$ | $CH_3$ | |
| $J_{20}$ | - | O | - | - | - | H | - | - | - | - | - | - | - | $CH_3$ | $OCH_3$ | |
| $J_{21}$ | - | - | - | - | - | H | $CH_3$ | - | - | - | - | - | - | $OCH_3$ | $OCH_3$ | |
| $J_{21}$ | - | - | - | - | - | H | $CH_3$ | - | - | - | - | - | - | $OCH_3$ | $CH_3$ | |
| $J_{21}$ | - | - | - | - | - | H | $C_2H_5$ | - | - | - | - | - | - | $CH_3$ | $OCH_3$ | |
| $J_{22}$ | - | - | - | - | - | H | $CH_3$ | - | - | - | - | - | - | $CH_3$ | $OCH_3$ | |
| $J_{22}$ | - | - | - | - | - | H | $C_2H_5$ | - | - | - | - | - | - | $CH_3$ | $OCH_3$ | |
| $J_{23}$ | - | $CH_2$ | - | - | - | H | - | H | - | - | - | - | - | $OCH_3$ | $OCH_3$ | |
| $J_{23}$ | - | $CH_2$ | - | - | - | H | - | H | - | - | - | - | - | $OCH_3$ | $CH_3$ | |
| $J_{23}$ | - | $CH_2$ | - | - | - | H | - | $CH_3$ | - | - | - | - | - | $OCH_3$ | $OCH_3$ | |
| $J_{23}$ | - | O | - | - | - | H | - | H | - | - | - | - | - | $OCH_3$ | $OCH_3$ | |
| $J_{23}$ | - | O | - | - | - | H | - | H | - | - | - | - | - | $OCH_3$ | $CH_3$ | |
| $J_{23}$ | - | O | - | - | - | H | - | $CH_3$ | - | - | - | - | - | $OCH_3$ | $CH_3$ | |
| $J_{24}$ | - | $CH_2$ | O | - | - | H | - | - | - | - | - | - | - | $OCH_3$ | $OCH_3$ | |
| $J_{24}$ | - | $CH_2$ | O | - | - | H | - | - | - | - | - | - | - | $OCH_3$ | $CH_3$ | |
| $J_{24}$ | - | O | O | - | - | H | - | - | - | - | - | - | - | $OCH_3$ | $OCH_3$ | |
| $J_{24}$ | - | O | O | - | - | H | - | - | - | - | - | - | - | $OCH_3$ | $CH_3$ | |
| $J_{24}$ | - | $CH_2$ | $CH_2$ | - | - | H | - | - | - | - | - | - | - | $OCH_3$ | $OCH_3$ | |
| $J_{24}$ | - | $CH_2$ | $CH_2$ | - | - | H | - | - | - | - | - | - | - | $OCH_3$ | $CH_3$ | |
| $J_{24}$ | - | O | $CH_2$ | - | - | H | - | - | - | - | - | - | - | $OCH_3$ | $OCH_3$ | |
| $J_{24}$ | - | O | $CH_2$ | - | - | H | - | - | - | - | - | - | - | $OCH_3$ | $CH_3$ | |
| $J_{25}$ | - | $CH_2$ | - | - | - | H | - | - | - | - | - | - | - | $OCH_3$ | $OCH_3$ | |
| $J_{25}$ | - | $CH_2$ | - | - | - | H | - | - | - | - | - | - | - | $OCH_3$ | $CH_3$ | |

Table XXIVa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{25}$ | - | O | - | - | - | H | - | - | - | - | - | - | - | $OCH_3$ | $OCH_3$ | |
| $J_{25}$ | - | O | - | - | - | H | - | - | - | - | - | - | - | $OCH_3$ | $CH_3$ | |
| $J_{26}$ | - | - | - | O | - | H | - | $CH_3$ | H | H | - | - | - | $OCH_3$ | $OCH_3$ | |
| $J_{26}$ | - | - | - | O | - | H | - | $CH_3$ | H | H | - | - | - | $OCH_3$ | $CH_3$ | |
| $J_{26}$ | - | - | - | O | - | H | - | H | H | $CH_3$ | - | - | - | $CH_3$ | $CH_3$ | |
| $J_{26}$ | - | - | - | O | - | H | - | H | H | $CH_3$ | - | - | - | $OCH_3$ | $C_2H_5$ | |
| $J_{26}$ | - | - | - | O | - | H | - | $CH_3$ | H | H | - | - | - | $CH_3$ | $OC_2H_5$ | |
| ~~$J_{26}$~~ | ~~-~~ | ~~-~~ | ~~-~~ | ~~O~~ | ~~-~~ | ~~H~~ | ~~-~~ | ~~$CH_3$~~ | ~~H~~ | ~~H~~ | ~~-~~ | ~~-~~ | ~~-~~ | ~~$OCH_3$~~ | ~~$CH_2OCH_3$~~ | |
| $J_{26}$ | - | - | - | O | - | H | - | H | H | $C_2H_5$ | - | - | - | $OCH_3$ | $CF_3$ | |
| $J_{26}$ | - | - | - | O | - | H | - | $CH_3$ | $CH_3$ | H | - | - | - | $CH_3$ | $OCH_3$ | |
| $J_{26}$ | - | - | - | $SO_2$ | - | H | - | H | H | H | - | - | | $CH_3$ | $CH_3$ | |
| $J_{26}$ | - | - | - | $SO_2$ | - | H | - | H | H | H | - | - | - | $CH_3$ | $OCH_3$ | |
| $J_{26}$ | - | - | - | $SO_2$ | - | H | - | H | H | H | - | - | - | $OCH_3$ | $OCH_3$ | |
| $J_{26}$ | - | - | - | O | - | H | - | $CH_3$ | H | $CH_3$ | - | - | - | $OCH_3$ | $OCH_3$ | |
| $J_{26}$ | - | - | - | O | - | H | - | $CH_3$ | H | $CH_3$ | - | - | - | $OCH_3$ | $CH_3$ | |
| $J_{26}$ | - | - | - | O | - | H | - | $CH_3$ | H | $CH_3$ | - | - | - | $CH_3$ | $CH_3$ | |
| $J_{26}$ | - | - | - | O | - | H | - | $CH_3$ | H | $CH_3$ | - | - | - | $OCH_3$ | $C_2H_5$ | |
| $J_{26}$ | - | - | - | O | - | H | - | $CH_3$ | H | $CH_3$ | - | - | - | $CH_3$ | $OC_2H_5$ | |
| ~~$J_{26}$~~ | ~~-~~ | ~~-~~ | ~~-~~ | ~~O~~ | ~~-~~ | ~~H~~ | ~~-~~ | ~~$CH_3$~~ | ~~H~~ | ~~$CH_3$~~ | ~~-~~ | ~~-~~ | ~~-~~ | ~~$OCH_3$~~ | ~~$CH_2OCH_3$~~ | |
| $J_{26}$ | - | - | - | O | - | H | - | $CH_3$ | H | $CH_3$ | - | | - | $OCH_3$ | $CH_2F$ | |
| $J_{26}$ | - | - | - | O | - | H | - | $CH_3$ | H | $CH_3$ | - | - | | $OCH_3$ | $CF_3$ | |
| $J_{26}$ | - | - | - | S | - | H | - | $CH_3$ | H | H | - | - | - | $OCH_3$ | $OCH_3$ | |
| $J_{26}$ | - | - | - | S | - | H | - | $CH_3$ | H | H | - | - | - | $OCH_3$ | $CH_3$ | |
| $J_{26}$ | - | - | - | S | - | H | - | $CH_3$ | H | H | - | - | - | $CH_3$ | $CH_3$ | |
| $J_{26}$ | - | - | - | S | - | H | - | H | $CH_3$ | $CH_3$ | - | - | - | $OCH_3$ | $CH_3$ | |
| $J_{26}$ | - | - | - | $SO_2$ | - | H | - | H | H | H | - | - | | $CH_3$ | $CH_3$ | |
| $J_{26}$ | - | - | - | $SO_2$ | - | H | - | H | H | H | - | - | - | $CH_3$ | $OCH_3$ | |
| $J_{26}$ | - | - | - | $SO_2$ | - | H | - | H | H | H | - | - | | $OCH_3$ | $OCH_3$ | |
| $J_{26}$ | - | - | - | $SO_2$ | - | H | - | $CH_3$ | H | H | - | | - | $OCH_3$ | $OCH_3$ | 192-194 |
| $J_{26}$ | - | - | - | $SO_2$ | - | H | - | $CH_3$ | H | H | - | - | - | $OCH_3$ | $CH_3$ | 186-187 |
| $J_{26}$ | - | - | - | $SO_2$ | - | H | - | $CH_3$ | H | H | - | - | - | $CH_3$ | $CH_3$ | |
| $J_{26}$ | - | - | - | $SO_2$ | - | H | - | $CH_3$ | H | H | - | - | - | $OCH_3$ | $C_2H_5$ | |
| $J_{26}$ | - | - | - | $SO_2$ | - | H | - | $CH_3$ | H | H | - | | | $CH_3$ | $OC_2H_5$ | |

Table XXIVa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| ~~$J_{26}$~~ | | | | ~~$SO_2$~~ | | ~~H~~ | | ~~$CH_3$~~ | ~~H~~ | ~~H~~ | | | | ~~$OCH_3$~~ | ~~$CH_2OCH_3$~~ | |
| $J_{26}$ | - | - | - | $SO_2$ | - | H | - | $CH_3$ | H | H | - | - | - | $OCH_3$ | $CH_2F$ | |
| $J_{26}$ | - | - | - | $SO_2$ | - | H | - | $CH_3$ | H | H | - | - | - | $OCH_3$ | $CF_3$ | |
| $J_{26}$ | - | - | - | $SO_2$ | - | H | - | $CH_3$ | $CH_3$ | H | - | - | - | $OCH_3$ | $OCH_3$ | |
| $J_{26}$ | - | - | - | $SO_2$ | - | H | - | $CH_3$ | H | $CH_3$ | - | - | | $OCH_3$ | $OCH_3$ | |
| $J_{26}$ | - | - | - | $SO_2$ | - | H | - | $CH_3$ | H | $CH_3$ | - | - | - | $OCH_3$ | $CH_3$ | |
| $J_{26}$ | - | - | - | $SO_2$ | - | H | - | $CH_3$ | H | $CH_3$ | - | - | - | $CH_3$ | $CH_3$ | |
| $J_{26}$ | - | - | - | $SO_2$ | - | H | - | $CH_3$ | H | $CH_3$ | - | - | - | $OCH_3$ | $C_2H_5$ | |
| $J_{26}$ | - | - | - | $SO_2$ | - | H | - | $CH_3$ | H | $CH_3$ | - | - | - | $CH_3$ | $OC_2H_5$ | |
| ~~$J_{26}$~~ | | | | ~~$SO_2$~~ | ~~-~~ | ~~H~~ | ~~-~~ | ~~$CH_3$~~ | ~~H~~ | ~~$CH_3$~~ | ~~-~~ | ~~-~~ | ~~-~~ | ~~$OCH_3$~~ | ~~$CH_2OCH_3$~~ | |
| $J_{26}$ | - | - | - | $SO_2$ | - | H | - | $CH_3$ | H | $CH_3$ | - | - | - | $OCH_3$ | $CH_2F$ | |
| $J_{26}$ | - | - | - | $SO_2$ | - | H | - | $CH_3$ | H | $CH_3$ | - | - | - | $OCH_3$ | $CF_3$ | |
| $J_{26}$ | - | - | - | S | - | H | - | $CH_3$ | H | $CH_3$ | - | - | - | $OCH_3$ | $OCH_3$ | |
| $J_{26}$ | - | - | - | S | - | H | - | $CH_3$ | H | $CH_3$ | - | - | - | $OCH_3$ | $CH_3$ | |
| $J_{26}$ | - | - | - | S | - | H | - | $CH_3$ | H | $CH_3$ | - | - | - | $CH_3$ | $CH_3$ | |
| $J_{26}$ | - | - | - | $SO_2$ | - | 6-Cl | - | $CH_3$ | H | H | - | - | - | $CH_3$ | $CH_3$ | |
| $J_{26}$ | - | - | - | $SO_2$ | - | 6-Cl | - | $CH_3$ | H | H | - | - | - | $OCH_3$ | $OCH_3$ | |
| $J_{26}$ | - | - | - | O | - | 6-Cl | - | $CH_3$ | H | H | - | - | - | $CH_3$ | $OCH_3$ | |
| $J_{26}$ | - | - | - | O | - | 5-Br | - | H | H | $CH_3$ | - | - | - | $CH_3$ | $OCH_3$ | |
| $J_{26}$ | - | - | - | O | - | 6-F | - | $CH_3$ | H | H | - | - | - | $CH_3$ | $OCH_3$ | |
| $J_{26}$ | - | - | - | O | - | 5-$CH_3$ | - | $CH_3$ | H | H | - | - | - | $CH_3$ | $OCH_3$ | |
| $J_{26}$ | - | - | - | O | - | 6-$OCH_3$ | - | $CH_3$ | H | H | - | - | - | $CH_3$ | $OCH_3$ | |
| $J_{26}$ | - | - | - | $SO_2$ | - | 6-Cl | - | $CH_3$ | H | H | - | - | - | $CH_3$ | $OCH_3$ | |
| $J_{26}$ | - | - | - | S | - | 6-Cl | - | $CH_3$ | H | H | - | - | - | $CH_3$ | $OCH_3$ | |
| $J_{27}$ | - | $CH_2$ | - | O | - | H | - | $CH_3$ | H | - | - | - | - | $CH_3$ | $OCH_3$ | |
| $J_{27}$ | - | $CH_2$ | - | S | - | H | - | H | $CH_3$ | - | - | - | - | $CH_3$ | $OCH_3$ | |
| $J_{27}$ | - | O | - | $SO_2$ | - | H | - | $CH_3$ | H | - | - | - | - | $CH_3$ | $OCH_3$ | |
| $J_{27}$ | - | O | - | $SO_2$ | - | H | - | $CH_3$ | H | - | - | - | | $CH_3$ | $CH_3$ | |
| $J_{28}$ | - | - | - | - | O | H | - | $CH_3$ | H | - | | | - | $CH_3$ | $OCH_3$ | |
| $J_{28}$ | - | - | - | - | S | H | - | $CH_3$ | H | - | - | - | - | $CH_3$ | $OCH_3$ | |
| $J_{28}$ | - | - | - | - | O | H | - | $CH_3$ | $CH_3$ | - | - | - | - | $CH_3$ | $OCH_3$ | |
| $J_{28}$ | - | - | - | - | O | H | - | H | H | - | - | - | - | $CH_3$ | $OCH_3$ | |

Table XXIVa (continued)

| J | n | L | Q | Q1 | Q2 | R20 | R21 | R23 | R24 | R25 | R26 | R27 | R28 | X | Y | m.p. (°C) |
|---|---|---|---|---|---|---|---|---|---|---|---|---|---|---|---|---|
| $J_{29}$ | - | $CH_2$ | - | - | - | H | - | H | H | - | H | H | - | $CH_3$ | $OCH_3$ | |
| $J_{29}$ | - | $CH_2$ | - | - | - | H | - | H | H | - | $CH_3$ | H | - | $OCH_3$ | $CH_3$ | |
| $J_{29}$ | - | O | - | - | - | H | - | H | H | - | H | $CH_3$ | - | $CH_3$ | $OCH_3$ | |
| $J_{30}$ | - | - | - | - | - | H | - | $CH_3$ | $CH_3$ | - | - | - | | $CH_3$ | $OCH_3$ | |
| $J_{30}$ | - | - | - | - | - | H | - | H | H | - | - | - | - | $OCH_3$ | $OCH_3$ | |
| $J_{30}$ | - | - | - | - | - | H | - | H | H | - | - | - | - | $OCH_3$ | $CH_3$ | |
| $J_{30}$ | - | - | - | - | - | H | - | $CH_3$ | $CH_3$ | - | - | - | - | $OCH_3$ | $CH_3$ | |
| $J_{31}$ | - | $CH_2$ | - | - | - | H | - | - | - | - | - | - | - | $OCH_3$ | $OCH_3$ | |
| $J_{31}$ | - | $CH_2$ | - | - | - | H | - | - | - | - | - | - | - | $OCH_3$ | $CH_3$ | |
| $J_{31}$ | - | $CH_2$ | - | - | - | H | - | - | - | - | - | - | - | $CH_3$ | $CH_3$ | |
| $J_{31}$ | - | $CH_2$ | - | - | - | 7-Cl | - | - | - | - | - | - | - | $OCH_3$ | $OCH_3$ | |
| $J_{31}$ | - | O | - | - | - | H | - | - | - | - | - | - | - | $OCH_3$ | $OCH_3$ | |
| $J_{31}$ | - | O | - | - | - | H | - | - | - | - | - | - | - | $OCH_3$ | $CH_3$ | |
| $J_{31}$ | - | O | - | - | - | H | - | - | - | - | - | - | - | $CH_3$ | $CH_3$ | |
| $J_{32}$ | - | $CH_2$ | - | - | - | - | - | - | - | - | - | - | F | $OCH_3$ | $CH_3$ | |
| $J_{32}$ | - | $CH_2$ | - | - | - | - | - | - | - | - | - | - | Cl | $OCH_3$ | $CH_3$ | |
| $J_{32}$ | - | $CH_2$ | - | - | - | - | - | - | - | - | - | - | Br | $OCH_3$ | $CH_3$ | |
| $J_{32}$ | - | $CH_2$ | - | - | - | - | - | - | - | - | - | - | $OCH_3$ | $OCH_3$ | $OCH_3$ | |
| $J_{32}$ | - | $CH_2$ | - | - | - | - | - | - | - | - | - | - | $SCH_3$ | $OCH_3$ | $CH_3$ | |
| $J_{32}$ | - | $CH_2$ | - | - | - | - | - | - | - | - | - | - | $SO_2CH_3$ | $OCH_3$ | $CH_3$ | |
| $J_{32}$ | - | $CH_2$ | - | - | - | - | - | - | - | - | - | - | $CO_2CH_3$ | $OCH_3$ | $OCH_3$ | 122-122.5 |
| $J_{32}$ | - | $CH_2$ | - | - | - | - | - | - | - | - | - | - | $SO_2N(CH_3)_2$ | $OCH_3$ | $CH_3$ | |
| $J_{32}$ | - | $CH_2$ | - | - | - | - | - | - | - | - | - | - | $OSO_2CH_3$ | $OCH_3$ | $CH_3$ | |
| $J_{32}$ | - | $CH_2$ | - | - | - | - | - | - | - | - | - | - | Cl | $OCH_3$ | $CF_3$ | |
| $J_{32}$ | - | $CH_2$ | - | - | - | - | - | - | - | - | - | - | Cl | $CH_3$ | $OC_2H_5$ | |
| $J_{32}$ | - | $CH_2$ | - | - | - | - | - | - | - | - | - | - | Cl | $OCH_3$ | $C_2H_5$ | |
| $J_{32}$ | - | O | - | - | - | - | - | - | - | - | - | - | F | $OCH_3$ | $CH_3$ | |
| $J_{32}$ | - | O | - | - | - | - | - | - | - | - | - | - | Cl | $OCH_3$ | $CH_3$ | |
| $J_{32}$ | - | O | - | - | - | - | - | - | - | - | - | - | Br | $OCH_3$ | $CH_3$ | |
| $J_{32}$ | - | O | - | - | - | - | - | - | - | - | - | - | $OCH_3$ | $OCH_3$ | $CH_3$ | |
| $J_{32}$ | - | O | - | - | - | - | - | - | - | - | - | - | $SCH_3$ | $OCH_3$ | $CH_3$ | |
| $J_{32}$ | - | O | - | - | - | - | - | - | - | - | - | - | $SO_2CH_3$ | $OCH_3$ | $CH_3$ | |

Table XXIVa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{32}$ | - | O | - | - | - | - | - | - | - | - | - | - | $CO_2CH_3$ | $OCH_3$ | $CH_3$ | |
| $J_{32}$ | - | O | - | - | - | - | - | - | - | - | - | - | $SO_2N(CH_3)_2$ | $OCH_3$ | $CH_3$ | |
| $J_{32}$ | - | O | - | - | - | - | - | - | - | - | - | - | $OSO_2CH_3$ | $OCH_3$ | $CH_3$ | |
| $J_{32}$ | - | $CH_2$ | - | - | - | - | - | - | - | - | - | - | $CO_2CH_3$ | $OCH_3$ | $CH_3$ | 187-190 |
| $J_{32}$ | - | $CH_2$ | - | - | - | - | - | - | - | - | - | - | $CO_2CH_3$ | $CH_3$ | $OC_2H_5$ | |
| $J_{32}$ | - | $CH_2$ | - | - | - | - | - | - | - | - | - | - | $CO_2CH_3$ | $OCH_3$ | $CF_3$ | |
| $J_{32}$ | - | $CH_2$ | - | - | - | - | - | - | - | - | - | - | $CO_2CH_3$ | $OCH_3$ | $C_2H_5$ | |
| $J_{13}$ | 0 | $CH_2$ | - | - | - | H | $CH_3$ | - | - | - | - | - | - | $OCH_3$ | $CH(CH_3)_2$ | |
| $J_{13}$ | 0 | $CH_2$ | - | - | - | H | $CH_3$ | - | - | - | - | - | - | $OCH_2CF_3$ | $OCH_3$ | |
| $J_{13}$ | 0 | $CH_2$ | - | - | - | H | $CH_3$ | - | - | - | - | - | - | $OCH_3$ | cyclo-propyl | |
| $J_{13}$ | 0 | $CH_2$ | - | - | - | H | $CH_3$ | - | - | - | - | - | - | $OCH_3$ | $NHCH_3$ | |
| $J_{13}$ | 0 | $CH_2$ | - | - | - | H | $CH_3$ | - | - | - | - | - | - | $OCH_3$ | $N(CH_3)_2$ | |
| $J_{13}$ | 0 | $CH_2$ | - | - | - | H | $CH_3$ | - | - | - | - | - | - | $OCH_3$ | $OCH_2CH=CH_2$ | |
| ~~$J_{13}$~~ | ~~0~~ | ~~$CH_2$~~ | | | | ~~H~~ | ~~$CH_3$~~ | | | | | | | ~~$OCH_3$~~ | ~~$CH(OCH_3)_2$~~ | |
| $J_{13}$ | 0 | $CH_2$ | - | - | - | H | $CH_3$ | - | - | - | - | - | - | $OCH_3$ | 1,3-dioxolan-2-yl | |
| ~~$J_{13}$~~ | ~~0~~ | ~~$CH_2$~~ | ~~-~~ | ~~-~~ | ~~-~~ | ~~H~~ | ~~$CH_3$~~ | ~~-~~ | ~~-~~ | ~~-~~ | ~~-~~ | ~~-~~ | ~~-~~ | ~~$OCH_3$~~ | ~~$CH(OC_2H_5)_2$~~ | |
| $J_{13}$ | 0 | $CH_2$ | - | - | - | H | $CH_3$ | - | - | - | - | - | - | $OCH_3$ | $C\equiv CH$ | |
| $J_{13}$ | 0 | $CH_2$ | - | - | - | H | $CH_3$ | - | - | - | - | - | - | $OCH_3$ | $OCH_2CH_2OCH_3$ | |
| $J_{13}$ | 0 | $CH_2$ | - | - | - | H | $CH_3$ | - | - | - | - | - | - | $OCH_3$ | $SCH_3$ | |
| $J_{14}$ | 0 | $CH_2$ | - | - | - | H | $CH_3$ | - | - | - | - | - | - | $OCH_3$ | $NHCH_3$ | |
| $J_{14}$ | 0 | $CH_2$ | - | - | - | H | $CH_3$ | - | - | - | - | - | - | $OCF_2H$ | $OCH_3$ | |
| $J_{14}$ | 0 | $CH_2$ | - | - | - | H | $CH_3$ | - | - | - | - | - | - | $OCH_3$ | cyclo-propyl | |
| ~~$J_{14}$~~ | ~~0~~ | ~~$CH_2$~~ | | | | ~~H~~ | ~~$CH_3$~~ | | | | | | | ~~$OCH_3$~~ | ~~$CH(OCH_3)_2$~~ | |
| $J_{14}$ | 0 | $CH_2$ | - | - | - | H | $CH_3$ | - | - | - | - | - | - | $OCH_3$ | $C\equiv CH$ | |
| $J_{14}$ | 0 | $CH_2$ | - | - | - | H | $CH_3$ | - | - | - | - | - | - | $OCH_3$ | $OCH_2CH_2OCH_3$ | |
| ~~$J_{14}$~~ | ~~0~~ | ~~$CH_2$~~ | | | | ~~H~~ | ~~$CH_3$~~ | | | | | | | ~~$CH_3$~~ | ~~1,3-dioxolan-2-yl~~ | |
| $J_{14}$ | 0 | $CH_2$ | - | - | - | H | $CH_3$ | - | - | - | - | - | - | $CH_3$ | $OCH_2C\equiv CH$ | |
| $J_{15}$ | 0 | $CH_2$ | - | - | - | H | - | H | H | - | - | - | - | $OCH_3$ | $CH_2CH_2CH_3$ | |
| $J_{15}$ | 0 | $CH_2$ | - | - | - | H | - | H | H | - | - | - | - | $OCH_3$ | cyclopropyl | |
| $J_{15}$ | 0 | $CH_2$ | - | - | - | H | - | H | H | - | | - | - | $OCH_3$ | $C\equiv CH$ | |
| $J_{15}$ | 0 | $CH_2$ | - | - | - | H | - | H | H | - | - | - | - | $OCH_3$ | $OCH_2CH=CH_2$ | |
| $J_{15}$ | 0 | $CH_2$ | - | - | - | H | - | H | H | - | - | - | - | $OCH_3$ | $OCH_2CH_2F$ | |

Table XXIVa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{16}$ | 0 | - | - | - | - | H | $CH_3$ | - | - | - | - | - | - | $OCH_3$ | $CH(CH_3)_2$ | |
| $J_{16}$ | 0 | - | - | - | - | H | $CH_3$ | - | - | - | - | - | - | $OCH_3$ | cyclopropyl | |
| $J_{16}$ | 0 | - | - | - | - | H | $CH_3$ | - | - | - | - | - | - | $OCH_3$ | C≡CH | |
| $J_{16}$ | 0 | - | - | - | - | H | $CH_3$ | - | - | - | - | - | - | $OCH_3$ | $OCH_2C≡CH$ | |
| $J_{16}$ | 0 | - | - | - | - | H | $CH_3$ | - | - | - | - | - | - | $OCH_3$ | $OCH_2CH_2F$ | |
| $J_{16}$ | 0 | - | - | - | - | H | $CH_3$ | - | - | - | - | - | - | $OCH_3$ | $SCH_3$ | |
| ~~$J_{16}$~~ | ~~0~~ | | | | | ~~H~~ | ~~$CH_3$~~ | | | | | | | ~~$OCH_3$~~ | ~~$CH(OCH_3)_2$~~ | |
| ~~$J_{16}$~~ | ~~0~~ | | | | | ~~H~~ | ~~$CH_3$~~ | ~~-~~ | ~~-~~ | ~~-~~ | ~~-~~ | ~~-~~ | ~~-~~ | ~~$OCH_3$~~ | ~~1,3-dioxolan-2-yl~~ | |
| $J_{17}$ | 0 | $CH_2$ | - | - | - | H | $CH_3$ | - | - | - | - | - | - | $OCH_3$ | $CH(CH_3)_2$ | |
| $J_{17}$ | 0 | $CH_2$ | - | - | - | H | $CH_3$ | - | - | - | - | - | - | $OCH_3$ | cyclopropyl | |
| $J_{17}$ | 0 | $CH_2$ | - | - | - | H | $CH_3$ | - | - | - | - | - | - | $OCH_3$ | C≡CH | |
| $J_{17}$ | 0 | $CH_2$ | - | - | - | H | $CH_3$ | - | - | - | - | - | - | $OCH_3$ | $OCH_2CH_2F$ | |
| $J_{17}$ | 0 | $CH_2$ | - | - | - | H | $CH_3$ | - | - | - | - | - | - | $OCH_3$ | $SCH_3$ | |
| ~~$J_{17}$~~ | ~~0~~ | ~~$CH_2$~~ | | | | ~~H~~ | ~~$CH_3$~~ | | | | | | | ~~$OCH_3$~~ | ~~$CH(OCH_3)_2$~~ | |
| $J_{19}$ | - | $CH_2$ | - | - | - | H | - | - | - | - | - | - | - | $OCH_3$ | $NHCH_3$ | |
| $J_{19}$ | - | $CH_2$ | - | - | - | H | - | - | - | - | - | - | - | $OCH_3$ | C≡CH | |
| $J_{19}$ | - | $CH_2$ | - | - | - | H | - | - | - | - | - | - | - | $OCH_3$ | $NHCH_3$ | |
| ~~$J_{19}$~~ | | ~~$CH_2$~~ | | | | ~~H~~ | | | | | | | | ~~$OCH_3$~~ | ~~$CH(OCH_3)_2$~~ | |
| $J_{19}$ | - | $CH_2$ | - | - | - | H | - | - | - | - | - | - | - | $OCH_3$ | $OCH_2C≡CH$ | |
| $J_{19}$ | - | $CH_2$ | - | - | - | H | - | - | - | - | - | - | - | $OCH_3$ | $OCH_2CH_2F$ | |
| $J_{19}$ | - | $CH_2$ | - | - | - | H | - | - | - | - | - | - | - | $OCH_3$ | $SCH_3$ | |
| ~~$J_{19}$~~ | | ~~$CH_2$~~ | | | | ~~H~~ | | | | | | | | ~~$OCH_3$~~ | ~~1,3-dioxolan-2-yl~~ | |
| $J_{19}$ | - | $CH_2$ | - | - | - | H | - | - | - | - | - | - | - | $OCH_3$ | cyclopropyl | |
| ~~$J_{20}$~~ | ~~-~~ | ~~O~~ | | | | ~~H~~ | | | | | | | | ~~$OCH_3$~~ | ~~$CH(OCH_3)_2$~~ | |
| $J_{20}$ | - | O | - | - | - | H | - | - | - | - | - | - | - | $OCH_3$ | $N(CH_3)_2$ | |
| $J_{20}$ | - | O | - | - | - | H | - | - | - | - | - | - | - | $OCH_3$ | $NHCH_3$ | |
| $J_{21}$ | - | - | - | - | - | H | $CH_3$ | - | - | - | - | - | - | $OCH_3$ | $NHCH_3$ | |
| ~~$J_{21}$~~ | | | | | | ~~H~~ | ~~$CH_3$~~ | | | | | | | ~~$CH_3$~~ | ~~$CH(OCH_3)_2$~~ | |
| ~~$J_{23}$~~ | | ~~$CH_2$~~ | | | | ~~H~~ | | | | | | | | ~~$OCH_3$~~ | ~~$CH(OCH_3)_2$~~ | |
| $J_{23}$ | - | $CH_2$ | - | - | - | H | - | - | - | - | - | - | - | $OCH_3$ | $NHCH_3$ | |
| $J_{23}$ | - | O | - | - | - | H | - | - | - | - | - | - | - | $CH_3$ | $NHCH_3$ | |

Table XXIVa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{23}$ | - | O | - | - | - | H | - | - | - | - | - | - | - | $OCH_3$ | $N(CH_3)_2$ | |
| ~~$J_{23}$~~ | | ~~O~~ | | | | ~~H~~ | | | | | | | | ~~$OCH_3$~~ | ~~1,3-dioxolan-2-yl~~ | |
| $J_{24}$ | - | $CH_2$ | $CH_2$ | - | - | H | - | - | - | - | - | - | - | $OCH_3$ | $NHCH_3$ | |
| $J_{24}$ | - | $CH_2$ | $CH_2$ | - | - | H | - | - | - | - | - | - | - | $OCH_3$ | $SCH_3$ | |
| ~~$J_{24}$~~ | ~~-~~ | ~~$CH_2$~~ | ~~$CH_2$~~ | ~~-~~ | ~~-~~ | ~~H~~ | | | | | | | | ~~$OCH_3$~~ | ~~$CH(OCH_3)_2$~~ | |
| $J_{24}$ | - | $CH_2$ | $CH_2$ | - | - | H | - | - | - | - | - | - | - | $OCH_3$ | $OCH_2CH{\equiv}CH$ | |
| $J_{25}$ | - | $CH_2$ | - | - | - | H | - | - | - | - | - | - | - | $OCH_3$ | $OCH_2CH{=}CH_2$ | |
| $J_{25}$ | - | $CH_2$ | - | - | - | H | - | - | - | - | - | - | - | $CH_3$ | $OCH_2CH_2F$ | |
| $J_{25}$ | - | $CH_2$ | - | - | - | H | - | - | - | - | - | - | - | $OCH_2CF_3$ | $OCH_3$ | |
| $J_{25}$ | - | $CH_2$ | - | - | - | H | - | - | - | - | - | - | - | $OCH_3$ | $OCH_2CH_2OCH_3$ | |
| $J_{26}$ | - | - | - | O | - | H | - | $CH_3$ | H | H | - | | - | $OCH_3$ | $NHCH_3$ | |
| $J_{26}$ | - | - | - | O | - | H | - | $CH_3$ | H | H | - | | - | $OCH_3$ | $OCH_2CH{=}CH_2$ | |
| $J_{26}$ | - | - | - | O | - | H | - | H | H | H | - | - | - | $OCH_3$ | $C{\equiv}CH$ | |
| $J_{26}$ | - | - | - | O | - | H | - | H | H | H | - | - | - | $OCH_3$ | $OCH_2CH_2F$ | |
| $J_{26}$ | - | - | - | O | - | H | - | $CH_3$ | H | H | - | - | - | $OCH_3$ | $OCH_2CH{=}CHCl$ | |
| $J_{26}$ | - | - | - | O | - | H | - | H | H | H | - | - | - | $CH_3$ | $NHCH_3$ | |
| $J_{26}$ | - | - | - | O | - | H | - | H | H | $CH_3$ | - | - | - | $OCH_3$ | $N(CH_3)_2$ | |
| $J_{26}$ | - | - | - | O | - | H | - | H | H | $CH_3$ | - | - | - | $OCH_3$ | cyclopropyl | |
| ~~$J_{26}$~~ | | | | ~~O~~ | | ~~H~~ | | ~~H~~ | ~~H~~ | ~~$CH_3$~~ | | | | ~~$OCH_3$~~ | ~~$CH(OCH_3)_2$~~ | |
| $J_{26}$ | - | - | - | O | - | H | - | $CH_3$ | $CH_3$ | H | - | - | - | $OCH_3$ | $NHCH_3$ | |
| ~~$J_{26}$~~ | | | | ~~O~~ | | ~~H~~ | | ~~$CH_3$~~ | ~~$CH_3$~~ | ~~H~~ | | | | ~~$OCH_3$~~ | ~~$CH(OCH_3)_2$~~ | |
| $J_{26}$ | - | - | - | O | - | H | - | $CH_3$ | H | H | - | - | - | $OCH_3$ | $OCH_2C{\equiv}CH$ | |
| $J_{26}$ | - | - | - | O | - | H | - | $CH_3$ | $CH_3$ | H | - | - | - | $OCH_3$ | $SCH_3$ | |
| $J_{26}$ | - | - | - | S | - | H | - | $CH_3$ | H | H | - | - | - | $OCH_3$ | $NHCH_3$ | |
| $J_{26}$ | - | - | - | S | - | H | - | $CH_3$ | H | H | - | - | - | $OCH_3$ | $N(CH_3)_2$ | |
| $J_{26}$ | - | - | - | S | - | H | - | $CH_3$ | H | H | - | - | - | $OCH_3$ | $OCH_2CH{=}CH_2$ | |
| $J_{26}$ | - | - | - | S | - | H | - | $CH_3$ | H | H | - | - | - | $OCH_3$ | cyclopropyl | |
| $J_{26}$ | - | - | - | S | - | H | - | $CH_3$ | H | H | - | | - | $OCH_3$ | $N(CH_3)_2$ | |
| ~~$J_{26}$~~ | | | | ~~S~~ | | ~~H~~ | | ~~$CH_3$~~ | ~~$CH_3$~~ | ~~$CH_3$~~ | | | | ~~$OCH_3$~~ | ~~$CH(OCH_3)_2$~~ | |
| $J_{26}$ | - | - | - | $SO_2$ | - | H | - | $CH_3$ | H | H | - | - | - | $OCH_3$ | $NHCH_3$ | |
| $J_{26}$ | - | - | - | $SO_2$ | - | H | - | $CH_3$ | H | H | - | - | - | $OCH_3$ | $N(CH_3)_2$ | |

Table XXIVa (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | X | Y | m.p. (°C) |
|---|---|---|---|---|---|---|---|---|---|---|---|---|---|---|---|---|
| $J_{26}$ | - | - | - | $SO_2$ | - | H | - | $CH_3$ | H | H | - | - | - | $OCH_3$ | $OCH_2CH{=}CH_2$ | |
| $J_{26}$ | - | - | - | $SO_2$ | - | H | - | $CH_3$ | H | H | - | - | - | $CH_3$ | $OCH_2CH_2OCH_3$ | |
| ~~$J_{26}$~~ | | | | ~~$SO_2$~~ | | ~~H~~ | | ~~$CH_3$~~ | ~~H~~ | ~~H~~ | | | | ~~$CH_3$~~ | ~~$CH(OCH_3)_2$~~ | |
| $J_{27}$ | - | $CH_2$ | - | O | - | H | - | $CH_3$ | H | - | | - | | $OCH_3$ | $N(CH_3)_2$ | |
| $J_{27}$ | - | $CH_2$ | - | S | - | H | - | H | $CH_3$ | - | - | - | - | $OCH_3$ | $OCH_2CH{=}CH_2$ | |
| $J_{27}$ | - | O | - | $SO_2$ | - | H | - | $CH_3$ | H | - | - | - | - | $OCH_3$ | $OCH_2CH_2F$ | |
| $J_{27}$ | - | O | - | $SO_2$ | - | H | - | $CH_3$ | H | - | - | - | - | $OCH_3$ | $OCH_2CH_2OCH_3$ | |
| $J_{28}$ | - | - | - | - | O | H | - | $CH_3$ | H | - | - | - | - | $OCH_3$ | $N(CH_3)_2$ | |
| ~~$J_{28}$~~ | | | | | ~~O~~ | ~~H~~ | | ~~$CH_3$~~ | ~~H~~ | - | - | - | - | ~~$CH_3$~~ | ~~$CH(OCH_3)_2$~~ | |
| $J_{28}$ | - | - | - | - | S | H | - | $CH_3$ | H | - | - | - | - | $OCH_3$ | $NHCH_3$ | |
| $J_{29}$ | - | $CH_2$ | - | - | - | H | - | H | H | - | H | H | - | $OCH_3$ | $NHCH_3$ | |
| $J_{29}$ | - | $CH_2$ | - | - | - | H | - | H | H | - | $CH_3$ | H | - | $OCH_3$ | $N(CH_3)_2$ | |
| $J_{29}$ | - | O | - | - | - | H | - | H | H | - | H | $CH_3$ | - | $CH_3$ | $OCH_2CH_2OCH_3$ | |
| $J_{30}$ | - | - | - | - | - | H | - | H | H | - | - | - | - | $CH_3$ | $OCH_2CH_2F$ | |
| $J_{30}$ | - | - | - | - | - | H | - | $CH_3$ | $CH_3$ | - | - | - | - | $OCH_3$ | $NHCH_3$ | |
| $J_{30}$ | - | - | - | - | - | H | - | H | $CH_3$ | - | - | - | - | $OCH_3$ | $C{\equiv}CH$ | |
| $J_{31}$ | - | $CH_2$ | - | - | - | H | - | - | - | - | - | - | - | $OCH_3$ | $NHCH_3$ | |
| $J_{31}$ | - | $CH_2$ | - | - | - | H | - | - | - | - | - | - | - | $CH_3$ | $OCH_2CH_2F$ | |
| $J_{31}$ | - | O | - | - | - | H | - | - | - | - | - | - | - | $OCH_3$ | $C{\equiv}CH$ | |
| ~~$J_{31}$~~ | ~~-~~ | ~~O~~ | ~~-~~ | ~~-~~ | ~~-~~ | ~~H~~ | ~~-~~ | ~~-~~ | ~~-~~ | ~~-~~ | ~~-~~ | ~~-~~ | ~~-~~ | ~~$OCH_3$~~ | ~~$CH(OCH_3)_2$~~ | |
| $J_{32}$ | - | $CH_2$ | - | - | - | - | - | - | - | - | - | - | Cl | $OCH_3$ | $NHCH_3$ | |
| $J_{32}$ | - | $CH_2$ | - | - | - | - | - | - | - | - | - | - | Br | $OCH_3$ | $N(CH_3)_2$ | |
| $J_{32}$ | - | $CH_2$ | - | - | - | - | - | - | - | - | - | - | F | $OCH_3$ | $NHCH_3$ | |
| $J_{32}$ | - | $CH_2$ | - | - | - | - | - | - | - | - | - | - | $OCH_3$ | $OCH_3$ | $NHCH_3$ | |
| $J_{32}$ | - | $CH_2$ | - | - | - | - | - | - | - | - | - | - | $SO_2N(CH_3)_2$ | $OCH_3$ | $C{\equiv}CH$ | |
| $J_{32}$ | - | $CH_2$ | - | - | - | - | - | - | - | - | - | - | Cl | $OCH_3$ | $OCH_2CH_2F$ | |
| $J_{32}$ | - | O | - | - | - | - | - | - | - | - | - | - | Cl | $OCH_3$ | $NHCH_3$ | |
| $J_{32}$ | - | O | - | - | - | - | - | - | - | - | - | - | Br | $OCH_3$ | $NHCH_3$ | |
| $J_{32}$ | - | O | - | - | - | - | - | - | - | - | - | - | $OCH_3$ | $OCH_3$ | $NHCH_3$ | |
| $J_{32}$ | - | O | - | - | - | - | - | - | - | - | - | - | $SO_2N(CH_3)_2$ | $OCH_3$ | $NHCH_3$ | |
| $J_{32}$ | - | O | - | - | - | - | - | - | - | - | - | - | $SO_2CH_3$ | $OCH_3$ | $NHCH_3$ | |

Table XXV

General Formula 4

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_1$ | $Y_1$ | m.p. (°C) |
|---|---|---|---|---|---|---|---|---|---|---|---|---|---|---|---|---|---|
| $J_{12}$ | 0 | $CH_2$ | - | - | - | H | - | - | - | - | - | - | - | $CH_3$ | $OCH_3$ | O | |
| $J_{12}$ | 0 | $CH_2$ | - | - | - | H | - | - | - | - | - | - | - | $CH_3$ | $CH_3$ | O | |
| $J_{12}$ | 0 | $CH_2$ | - | - | - | H | - | - | - | - | - | - | - | $CH_3$ | $OCH_3$ | $CH_2$ | |
| $J_{12}$ | 0 | $CH_2$ | - | - | - | H | - | - | - | - | - | - | - | $CH_3$ | $OC_2H_5$ | O | |
| $J_{12}$ | 0 | $CH_2$ | - | - | - | H | - | - | - | - | - | - | - | $CH_3$ | $OCF_2H$ | O | |
| $J_{12}$ | 0 | O | - | - | - | H | - | - | - | - | - | - | - | $CH_3$ | $OCH_3$ | $CH_2$ | |
| $J_{12}$ | 0 | O | - | - | - | H | - | - | - | - | - | - | - | $CH_3$ | $CH_3$ | O | |
| $J_{12}$ | 0 | O | - | - | - | H | - | - | - | - | - | - | - | $CH_3$ | $OCF_2H$ | O | |
| $J_{13}$ | 0 | $CH_2$ | - | - | - | H | $CH_3$ | - | - | - | - | - | - | - | $OCH_3$ | O | |
| $J_{13}$ | 0 | $CH_2$ | - | - | - | H | $CH_3$ | - | - | - | - | - | - | - | $CH_3$ | O | |
| $J_{13}$ | 0 | $CH_2$ | - | - | - | H | $CH_3$ | - | - | - | - | - | - | - | $OCH_3$ | $CH_2$ | |
| $J_{13}$ | 0 | O | - | - | - | H | $CH_3$ | - | - | - | - | - | - | - | $OCF_2H$ | O | |
| $J_{13}$ | 1 | $CH_2$ | - | - | - | H | $CH_3$ | - | - | - | - | - | - | - | $OCH_3$ | $CH_2$ | |
| $J_{13}$ | 1 | $CH_2$ | - | - | - | H | $CH_3$ | - | - | - | - | - | - | - | $CH_3$ | O | |
| $J_{13}$ | 1 | O | - | - | - | H | $CH_3$ | - | - | - | - | - | - | - | $OCH_3$ | O | |
| $J_{14}$ | 0 | $CH_2$ | - | - | - | H | - | H | H | - | - | - | - | - | $OCH_3$ | O | |
| $J_{14}$ | 0 | $CH_2$ | - | - | - | H | - | H | H | - | - | - | - | - | $CH_3$ | O | |
| $J_{14}$ | 0 | $CH_2$ | - | - | - | H | - | H | H | - | - | - | - | - | $OCH_3$ | $CH_2$ | |
| $J_{14}$ | 0 | $CH_2$ | - | - | - | H | - | H | H | - | - | - | - | - | $OCF_2H$ | O | |
| $J_{14}$ | 1 | $CH_2$ | - | - | - | H | - | H | H | - | - | - | - | - | $OCH_3$ | O | |
| $J_{14}$ | 1 | $CH_2$ | - | - | - | H | - | H | H | - | - | - | - | - | $OCH_3$ | $CH_2$ | |
| $J_{14}$ | 1 | $CH_2$ | - | - | - | H | - | H | H | - | - | - | - | - | $OC_2H_5$ | O | |
| $J_{14}$ | 1 | O | - | - | - | H | - | H | H | - | - | - | - | - | $OCH_3$ | O | |
| $J_{15}$ | 0 | $CH_2$ | - | - | - | H | - | - | - | - | - | - | - | - | $OCH_3$ | $CH_2$ | |
| $J_{15}$ | 0 | O | - | - | - | H | - | - | - | - | - | - | - | - | $OCH_3$ | O | |
| $J_{15}$ | 1 | $CH_2$ | - | - | - | H | - | - | - | - | - | - | - | - | $CH_3$ | O | |
| $J_{16}$ | 0 | - | - | - | - | H | $CH_3$ | - | - | - | - | - | - | - | $OCH_3$ | $CH_2$ | |
| $J_{16}$ | 0 | - | - | - | - | H | $CH_3$ | - | - | - | - | - | - | - | $OCH_3$ | O | |
| $J_{16}$ | 0 | - | - | - | - | H | $CH_3$ | - | - | - | - | - | - | - | $CH_3$ | O | |
| $J_{17}$ | 0 | - | - | - | - | H | $CH_3$ | - | - | - | - | - | - | - | $OCH_3$ | $CH_2$ | |

Table XXV (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_1$ | $Y_1$ | m.p. (°C) |
|---|---|---|---|---|---|---|---|---|---|---|---|---|---|---|---|---|---|
| $J_{18}$ | - | $CH_2$ | - | - | - | H | $CH_3$ | - | - | - | - | - | - | $CH_3$ | $OCH_3$ | $CH_2$ | |
| $J_{18}$ | - | $CH_2$ | - | - | - | H | $CH_3$ | - | - | - | - | - | - | $CH_3$ | $CH_3$ | O | |
| $J_{19}$ | - | $CH_2$ | - | - | - | H | - | H | - | - | - | - | - | - | $OCH_3$ | O | |
| $J_{19}$ | - | O | - | - | - | H | - | H | - | - | - | - | - | - | $CH_3$ | O | |
| $J_{20}$ | - | $CH_2$ | - | - | - | H | - | - | - | - | - | - | - | - | $OCH_3$ | $CH_2$ | |
| $J_{21}$ | - | - | - | - | - | H | $CH_3$ | - | - | - | - | - | - | - | $OCH_3$ | $CH_2$ | |
| $J_{21}$ | - | - | - | - | - | H | $CH_3$ | - | - | - | - | - | - | - | $OCH_3$ | O | |
| $J_{22}$ | - | - | - | - | - | H | $CH_3$ | - | - | - | - | - | - | - | $OCH_3$ | O | |
| $J_{23}$ | - | $CH_2$ | - | - | - | H | - | H | - | - | - | - | - | - | $OCH_3$ | $CH_2$ | |
| $J_{23}$ | - | $CH_2$ | - | - | - | H | - | H | - | - | - | - | - | - | $OCH_3$ | O | |
| $J_{23}$ | - | O | - | - | - | H | - | H | - | - | - | - | - | - | $CH_3$ | O | |
| $J_{23}$ | - | O | - | - | - | H | - | H | - | - | - | - | - | - | $OCH_3$ | $CH_2$ | |
| $J_{24}$ | - | $CH_2$ | O | - | - | H | - | - | - | - | - | - | - | - | $OCH_3$ | $CH_2$ | |
| $J_{24}$ | - | O | O | - | - | H | - | - | - | - | - | - | - | - | $CH_3$ | O | |
| $J_{24}$ | - | $CH_2$ | $CH_2$ | - | - | H | - | - | - | - | - | - | - | - | $OCH_3$ | O | |
| $J_{25}$ | - | $CH_2$ | - | - | - | H | - | - | - | - | - | - | - | - | $OCH_3$ | O | |
| $J_{25}$ | - | $CH_2$ | - | - | - | H | - | - | - | - | - | - | - | - | $CH_3$ | O | |
| $J_{25}$ | - | O | - | - | - | H | - | - | - | - | - | - | - | - | $OCH_3$ | $CH_2$ | |
| $J_{26}$ | - | - | - | O | - | H | - | $CH_3$ | H | H | - | - | - | - | $OCH_3$ | O | |
| $J_{26}$ | - | - | - | O | - | H | - | $CH_3$ | H | H | - | - | - | - | $OCH_3$ | $CH_2$ | |
| $J_{26}$ | - | - | - | O | - | H | - | $CH_3$ | H | H | - | - | - | - | $CH_3$ | O | |
| $J_{26}$ | - | - | - | S | - | H | - | $CH_3$ | H | H | - | - | - | - | $OCH_3$ | O | |
| $J_{26}$ | - | - | - | S | - | H | - | $CH_3$ | H | H | - | - | - | | $CH_3$ | O | |
| $J_{26}$ | - | - | - | $SO_2$ | - | H | - | $CH_3$ | H | H | - | - | - | - | $OCH_3$ | O | |
| $J_{26}$ | - | - | - | $SO_2$ | - | H | - | $CH_3$ | H | H | - | - | - | - | $OCH_3$ | $CH_2$ | |
| $J_{27}$ | - | $CH_2$ | - | O | - | H | - | $CH_3$ | H | - | - | - | - | - | $OCH_3$ | $CH_2$ | |
| $J_{27}$ | - | $CH_2$ | - | S | - | H | - | $CH_3$ | H | - | | | | | $OCH_3$ | O | |
| $J_{27}$ | - | $CH_2$ | - | $SO_2$ | - | H | - | $CH_3$ | H | - | - | - | - | - | $OCH_3$ | O | |
| $J_{27}$ | - | O | - | $SO_2$ | - | H | - | $CH_3$ | H | - | - | - | - | - | $CH_3$ | O | |
| $J_{28}$ | - | - | - | - | O | H | - | H | H | - | | - | - | - | $OCH_3$ | O | |
| $J_{28}$ | - | - | - | - | S | H | - | H | H | - | | | - | - | $OCH_3$ | O | |

Table XXV (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_1$ | $Y_1$ | m.p. (°C) |
|---|---|---|---|---|---|---|---|---|---|---|---|---|---|---|---|---|---|
| $J_{29}$ | - | $CH_2$ | - | - | - | H | - | H | H | - | H | H | - | - | $OCH_3$ | O | |
| $J_{29}$ | - | $CH_2$ | - | - | - | H | - | H | H | - | $CH_3$ | H | - | - | $CH_3$ | O | |
| $J_{30}$ | - | - | - | - | - | H | - | H | H | - | - | - | - | - | $OCH_3$ | O | |
| $J_{30}$ | - | - | - | - | - | H | - | H | H | - | - | - | - | - | $CH_3$ | O | |
| $J_{31}$ | - | $CH_2$ | - | - | - | H | - | - | - | - | - | - | - | - | $CH_3$ | O | |
| $J_{31}$ | - | $CH_2$ | - | - | - | H | - | - | - | - | - | - | - | - | $OCH_3$ | O | |
| $J_{31}$ | - | O | - | - | - | H | - | - | - | - | - | - | - | - | $OCH_3$ | $CH_2$ | |
| $J_{31}$ | - | O | - | - | - | H | - | - | - | - | - | - | - | - | $OCH_3$ | O | |
| $J_{32}$ | - | $CH_2$ | - | - | - | - | - | - | - | - | - | - | Cl | - | $OCH_3$ | O | |
| $J_{32}$ | - | $CH_2$ | - | - | - | - | - | - | - | - | - | - | F | - | $OCH_3$ | O | |
| $J_{32}$ | - | $CH_2$ | - | - | - | - | - | - | - | - | - | - | Br | - | $OCH_3$ | O | |
| $J_{32}$ | - | $CH_2$ | - | - | - | - | - | - | - | - | - | - | $SO_2CH_3$ | - | $OCH_3$ | O | |
| $J_{32}$ | - | $CH_2$ | - | - | - | - | - | - | - | - | - | - | $OSO_2CH_3$ | - | $OCH_3$ | $CH_2$ | |
| $J_{32}$ | - | $CH_2$ | - | - | - | - | - | - | - | - | - | - | $SO_2N(CH_3)_2$ | - | $OCH_3$ | $CH_2$ | |
| $J_{32}$ | - | $CH_2$ | - | - | - | - | - | - | - | - | - | - | $CO_2CH_3$ | - | $OCH_3$ | $CH_2$ | |
| $J_{32}$ | - | O | - | - | - | - | - | - | - | - | - | - | Cl | - | $OCH_3$ | O | |
| $J_{32}$ | - | O | - | - | - | - | - | - | - | - | - | - | F | - | $OCH_3$ | O | |
| $J_{32}$ | - | O | - | - | - | - | - | - | - | - | - | - | Br | - | $OCH_3$ | O | |
| $J_{32}$ | - | O | - | - | - | - | - | - | - | - | - | - | $SO_2CH_3$ | - | $OCH_3$ | O | |
| $J_{32}$ | - | O | - | - | - | - | - | - | - | - | - | - | $OSO_2CH_3$ | - | $OCH_3$ | O | |
| $J_{32}$ | - | O | - | - | - | - | - | - | - | - | - | - | $SO_2N(CH_3)_2$ | - | $OCH_3$ | O | |
| $J_{32}$ | - | O | - | - | - | - | - | - | - | - | - | - | $CO_2CH_3$ | - | $OCH_3$ | O | |

Table XXVI

General Formula 5

| J | n | L | Q | $Q_1$ | $Q_2$ | $R_{20}$ | $R_{21}$ | $R_{233}$ | $R_{24}$ | $R_{25}$ | $R_{26}$ | $R_{27}$ | $R_{28}$ | $R_{22}$ | $X_1$ | m.p. (°C) |
|---|---|---|---|---|---|---|---|---|---|---|---|---|---|---|---|---|
| $J_{12}$ | 0 | $CH_2$ | - | - | - | H | - | - | - | - | - | - | - | $CH_3$ | $CH_3$ | |
| $J_{12}$ | 0 | $CH_2$ | - | - | - | H | - | - | - | - | - | - | - | $CH_3$ | $OCH_3$ | |
| $J_{12}$ | 0 | $CH_2$ | - | - | - | H | - | - | - | - | - | - | - | $CH_3$ | $OC_2H_5$ | |
| $J_{12}$ | 0 | $CH_2$ | - | - | - | H | - | - | - | - | - | - | - | $CH_3$ | $OCF_2H$ | |
| $J_{12}$ | 0 | O | - | - | - | H | - | - | - | - | - | - | - | $CH_3$ | $CH_3$ | |
| $J_{12}$ | 0 | O | - | - | - | H | - | - | - | - | - | - | - | $CH_3$ | $OCH_3$ | |
| $J_{12}$ | 0 | O | - | - | - | H | - | - | - | - | - | - | - | $CH_3$ | $OCF_2H$ | |
| $J_{13}$ | 0 | $CH_2$ | - | - | - | H | $CH_3$ | - | - | - | - | - | - | - | $CH_3$ | |
| $J_{13}$ | 0 | $CH_2$ | - | - | - | H | $CH_3$ | - | - | - | - | - | - | - | $OCH_3$ | |
| $J_{13}$ | 0 | O | - | - | - | H | $CH_3$ | - | - | - | - | - | - | - | $CH_3$ | |
| $J_{13}$ | 1 | $CH_2$ | - | - | - | H | $CH_3$ | - | - | - | - | - | - | - | $OCH_3$ | |
| $J_{13}$ | 1 | $CH_2$ | - | - | - | H | $CH_3$ | - | - | - | - | - | - | - | $OC_2H_5$ | |
| $J_{13}$ | 1 | O | - | - | - | H | $CH_3$ | - | - | - | - | - | - | - | $OCH_3$ | |
| $J_{14}$ | 0 | $CH_2$ | - | - | - | H | - | H | H | - | - | - | - | - | $OCH_3$ | |
| $J_{14}$ | 0 | $CH_2$ | - | - | - | H | - | H | H | - | - | - | - | - | $CH_3$ | |
| $J_{14}$ | 0 | $CH_2$ | - | - | - | H | - | H | H | - | - | - | - | - | $OC_2H_5$ | |
| $J_{14}$ | 1 | $CH_2$ | - | - | - | H | - | H | H | - | - | - | - | - | $OCH_3$ | |
| $J_{14}$ | 1 | $CH_2$ | - | - | - | H | - | H | H | - | - | - | - | - | $CH_3$ | |
| $J_{14}$ | 1 | O | - | - | - | H | - | H | H | - | - | - | - | - | $OCH_3$ | |
| $J_{15}$ | 0 | $CH_2$ | - | - | - | H | - | - | - | - | - | - | - | - | $OCH_3$ | |
| $J_{15}$ | 0 | O | - | - | - | H | - | - | - | - | - | - | - | - | $OCH_3$ | |
| $J_{15}$ | 1 | $CH_2$ | - | - | - | H | - | - | - | - | - | - | - | - | $OCH_3$ | |
| $J_{16}$ | 0 | - | - | - | - | H | $CH_3$ | - | - | - | - | - | - | - | $CH_3$ | |
| $J_{16}$ | 0 | - | - | - | - | H | $CH_3$ | - | - | - | - | - | - | - | $OCH_3$ | |
| $J_{16}$ | 0 | - | - | - | - | H | $CH_3$ | - | - | - | - | - | - | - | $OC_2H_5$ | |
| $J_{17}$ | 0 | - | - | - | - | H | $CH_3$ | - | - | - | - | - | - | - | $OCF_2H$ | |
| $J_{18}$ | - | $CH_2$ | - | - | - | H | $CH_3$ | - | - | - | - | - | - | $CH_3$ | $OCH_3$ | |
| $J_{18}$ | - | $CH_2$ | - | - | - | H | $CH_3$ | - | - | - | - | - | - | $CH_3$ | $CH_3$ | |
| $J_{19}$ | - | $CH_2$ | - | - | - | H | - | H | - | - | - | - | - | - | $OCH_3$ | |
| $J_{19}$ | - | O | - | - | - | H | - | H | - | - | - | - | - | - | $OCH_3$ | |

Table XXVI (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_1$ | m.p. (°C) |
|---|---|---|---|---|---|---|---|---|---|---|---|---|---|---|---|---|
| $J_{20}$ | - | $CH_2$ | - | - | - | H | - | - | - | - | - | - | - | - | $OCH_3$ | |
| $J_{21}$ | - | - | - | - | - | H | $CH_3$ | - | - | - | - | - | - | - | $OCH_3$ | |
| $J_{21}$ | - | - | - | - | - | H | $CH_3$ | - | - | - | - | - | - | - | $CH_3$ | |
| $J_{22}$ | - | - | - | - | - | H | $CH_3$ | - | - | - | - | - | - | - | $OC_2H_5$ | |
| $J_{23}$ | - | $CH_2$ | - | - | - | H | - | H | - | - | - | - | - | - | $CH_3$ | |
| $J_{23}$ | - | $CH_2$ | - | - | - | H | - | H | - | - | - | - | - | - | $OCH_3$ | |
| $J_{23}$ | - | O | - | - | - | H | - | H | - | - | - | - | - | - | $OCH_3$ | |
| $J_{23}$ | - | O | - | - | - | H | - | H | - | - | - | - | - | - | $OCF_2H$ | |
| $J_{23}$ | - | O | - | - | - | H | - | H | - | - | - | - | - | - | $CH_3$ | |
| $J_{24}$ | - | $CH_2$ | O | - | - | H | - | - | - | - | - | - | - | - | $OCH_3$ | |
| $J_{24}$ | - | O | O | - | - | H | - | - | - | - | - | - | - | - | $OC_2H_5$ | |
| $J_{24}$ | - | $CH_2$ | $CH_2$ | - | - | H | - | - | - | - | - | - | - | - | $CH_3$ | |
| $J_{25}$ | - | $CH_2$ | - | - | - | H | - | - | - | - | - | - | - | - | $OCH_3$ | |
| $J_{25}$ | - | $CH_2$ | - | - | - | H | - | - | - | - | - | - | - | - | $CH_3$ | |
| $J_{25}$ | - | O | - | - | - | H | - | - | - | - | - | - | - | - | $OCH_3$ | |
| $J_{26}$ | - | - | - | O | - | H | - | $CH_3$ | H | H | - | - | - | - | $OCH_3$ | |
| $J_{26}$ | - | - | - | O | - | H | - | $CH_3$ | H | H | - | - | - | - | $CH_3$ | |
| $J_{26}$ | - | - | - | O | - | H | - | $CH_3$ | H | H | - | - | - | - | $OCHF_2$ | |
| $J_{26}$ | - | - | - | S | - | H | - | $CH_3$ | H | H | - | - | - | - | $CH_3$ | |
| $J_{26}$ | - | - | - | S | - | H | - | $CH_3$ | H | H | - | - | - | - | $OCH_3$ | |
| $J_{26}$ | - | - | - | $SO_2$ | - | H | - | $CH_3$ | H | H | - | - | - | - | $CH_3$ | |
| $J_{26}$ | - | - | - | $SO_2$ | - | H | - | $CH_3$ | H | H | - | - | - | - | $OCH_3$ | |
| $J_{27}$ | - | $CH_2$ | - | O | - | H | - | $CH_3$ | H | - | - | - | - | - | $OCH_3$ | |
| $J_{27}$ | - | $CH_2$ | - | S | - | H | - | $CH_3$ | H | - | - | - | - | - | $OCH_3$ | |
| $J_{27}$ | - | $CH_2$ | - | $SO_2$ | - | H | - | $CH_3$ | H | - | - | - | - | - | $OCH_3$ | |
| $J_{27}$ | - | O | - | $SO_2$ | - | H | - | $CH_3$ | H | - | - | - | - | - | $OCH_3$ | |
| $J_{28}$ | - | - | - | - | O | H | - | H | H | - | - | - | - | - | $OCH_3$ | |
| $J_{28}$ | - | - | - | - | S | H | - | H | H | - | - | - | - | - | $OCH_3$ | |
| $J_{29}$ | - | $CH_2$ | - | - | - | H | - | H | H | - | $CH_3$ | H | - | - | $CH_3$ | |
| $J_{29}$ | - | $CH_2$ | - | - | - | H | - | H | H | - | H | H | - | - | $OCH_3$ | |
| $J_{30}$ | - | - | - | - | - | H | - | H | H | - | - | - | - | - | $OCH_3$ | |

Table XXVI (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_1$ | m.p. (°C) |
|---|---|---|---|---|---|---|---|---|---|---|---|---|---|---|---|---|
| $J_{30}$ | - | - | - | - | - | H | - | H | H | - | - | - | - | - | $OC_2H_5$ | |
| $J_{31}$ | - | $CH_2$ | - | - | - | H | - | - | - | - | - | - | - | - | $OCH_3$ | |
| $J_{31}$ | - | $CH_2$ | - | - | - | H | - | - | - | - | - | - | - | - | $OC_2H_5$ | |
| $J_{31}$ | - | O | - | - | - | H | - | - | - | - | - | - | - | - | $OCH_3$ | |
| $J_{31}$ | - | O | - | - | - | H | - | - | - | - | - | - | - | - | $OC_2H_5$ | |
| $J_{32}$ | - | $CH_2$ | - | - | - | - | - | - | - | - | - | - | Cl | - | $OCH_3$ | |
| $J_{32}$ | - | $CH_2$ | - | - | - | - | - | - | - | - | - | - | F | - | $OCH_3$ | |
| $J_{32}$ | - | $CH_2$ | - | - | - | - | - | - | - | - | - | - | Br | - | $OCH_3$ | |
| $J_{32}$ | - | $CH_2$ | - | - | - | - | - | - | - | - | - | - | $SO_2CH_3$ | - | $OCH_3$ | |
| $J_{32}$ | - | $CH_2$ | - | - | - | - | - | - | - | - | - | - | $OSO_2CH_3$ | - | $OCH_3$ | |
| $J_{32}$ | - | $CH_2$ | - | - | - | - | - | - | - | - | - | - | $SO_2N(CH_3)_2$ | - | $OCH_3$ | |
| $J_{32}$ | - | $CH_2$ | - | - | - | - | - | - | - | - | - | - | $CO_2CH_3$ | - | $OCH_3$ | |
| $J_{32}$ | - | O | - | - | - | - | - | - | - | - | - | - | Cl | - | $OCH_3$ | |
| $J_{32}$ | - | O | - | - | - | - | - | - | - | - | - | - | F | - | $OCH_3$ | |
| $J_{32}$ | - | O | - | - | - | - | - | - | - | - | - | - | Br | - | $OCH_3$ | |
| $J_{32}$ | - | O | - | - | - | - | - | - | - | - | - | - | $SO_2CH_3$ | - | $OCH_3$ | |
| $J_{32}$ | - | O | - | - | - | - | - | - | - | - | - | - | $OSO_2CH_3$ | - | $OCH_3$ | |
| $J_{32}$ | - | O | - | - | - | - | - | - | - | - | - | - | $SO_2N(CH_3)_2$ | - | $OCH_3$ | |
| $J_{32}$ | - | O | - | - | - | - | - | - | - | - | - | - | $CO_2CH_3$ | - | $OCH_3$ | |

Table XXVII

General Formula 6

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_1$ | $Y_3$ | m.p. (°C) |
|---|---|---|---|---|---|---|---|---|---|---|---|---|---|---|---|---|---|
| $J_{12}$ | 0 | $CH_2$ | - | - | - | H | - | - | - | - | - | - | - | $CH_3$ | $OCH_3$ | $CH_3$ | |
| $J_{12}$ | 0 | $CH_2$ | - | - | - | H | - | - | - | - | - | - | - | $CH_3$ | $OCH_3$ | H | |
| $J_{12}$ | 0 | $CH_2$ | - | - | - | H | - | - | - | - | - | - | - | $CH_3$ | $CH_3$ | $CH_3$ | |
| $J_{12}$ | 0 | O | - | - | - | H | - | - | - | - | - | - | - | $CH_3$ | $OCH_3$ | $CH_3$ | |
| $J_{12}$ | 0 | O | - | - | - | H | - | - | - | - | - | - | - | $CH_3$ | $OCH_3$ | H | |
| $J_{12}$ | 0 | O | - | - | - | H | - | - | - | - | - | - | - | $CH_3$ | $CH_3$ | $CH_3$ | |
| $J_{13}$ | 0 | $CH_2$ | - | - | - | H | $CH_3$ | - | - | - | - | - | - | - | $OCH_3$ | H | |
| $J_{13}$ | 0 | $CH_2$ | - | - | - | H | $CH_3$ | - | - | - | - | - | - | - | $OC_2H_5$ | H | |
| $J_{13}$ | 0 | $CH_2$ | - | - | - | H | $CH_3$ | - | - | - | - | - | - | - | $CH_3$ | $CH_3$ | |
| $J_{13}$ | 0 | $CH_2$ | - | - | - | H | $CH_3$ | - | - | - | - | - | - | - | $CH_3$ | H | |
| $J_{13}$ | 0 | O | - | - | - | H | $CH_3$ | - | - | - | - | - | - | - | $OCH_3$ | H | |
| $J_{13}$ | 1 | $CH_2$ | - | - | - | H | $CH_3$ | - | - | - | - | - | - | - | $OCH_3$ | H | |
| $J_{13}$ | 1 | $CH_2$ | - | - | - | H | $CH_3$ | - | - | - | - | - | - | - | $CH_3$ | $CH_3$ | |
| $J_{13}$ | 1 | O | - | - | - | H | $CH_3$ | - | - | - | - | - | - | - | $OCH_3$ | $CH_3$ | |
| $J_{14}$ | 0 | $CH_2$ | - | - | - | H | - | H | H | - | - | - | - | - | $OCH_3$ | H | |
| $J_{14}$ | 0 | $CH_2$ | - | - | - | H | - | H | H | - | - | - | - | - | $CH_3$ | H | |
| $J_{14}$ | 0 | $CH_2$ | - | - | - | H | - | H | H | - | - | - | - | - | $CH_3$ | $CH_3$ | |
| $J_{14}$ | 1 | $CH_2$ | - | - | - | H | - | H | H | - | - | - | - | - | $CH_3$ | H | |
| $J_{14}$ | 1 | $CH_2$ | - | - | - | H | - | H | H | - | - | - | - | - | $OCH_3$ | H | |
| $J_{14}$ | 1 | O | - | - | - | H | - | H | H | - | - | - | - | - | $CH_3$ | H | |
| $J_{15}$ | 0 | $CH_2$ | - | - | - | H | - | - | - | - | - | - | - | - | $OCH_3$ | H | |
| $J_{15}$ | 0 | O | - | - | - | H | - | - | - | - | - | - | - | - | $OCH_3$ | $CH_3$ | |
| $J_{15}$ | 1 | $CH_2$ | - | - | - | H | - | - | - | - | - | - | - | - | $OCH_3$ | H | |
| $J_{16}$ | 0 | - | - | - | - | H | $CH_3$ | - | - | - | - | - | - | - | $OCH_3$ | H | |
| $J_{16}$ | 0 | - | - | - | - | H | $CH_3$ | - | - | - | - | - | - | - | $OC_2H_5$ | $CH_3$ | |
| $J_{17}$ | 0 | - | - | - | - | H | $CH_3$ | - | - | - | - | - | - | - | $OCH_3$ | H | |
| $J_{18}$ | - | $CH_2$ | - | - | - | H | $CH_3$ | - | - | - | - | - | - | $CH_3$ | $OCH_3$ | $CH_3$ | |
| $J_{18}$ | - | $CH_2$ | - | - | - | H | $CH_3$ | - | - | - | - | - | - | $CH_3$ | $OCH_3$ | H | |
| $J_{19}$ | - | $CH_2$ | - | - | - | H | - | H | - | - | - | - | - | - | $OCH_3$ | H | |
| $J_{19}$ | - | O | - | - | - | H | - | H | - | - | - | - | - | - | $OCH_3$ | $CH_3$ | |

Table XXVII (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_1$ | $Y_3$ | m.p. (°C) |
|---|---|---|---|---|---|---|---|---|---|---|---|---|---|---|---|---|---|
| $J_{20}$ | - | $CH_2$ | - | - | - | H | - | - | - | - | - | - | - | - | $OCH_3$ | $CH_3$ | |
| $J_{21}$ | - | - | - | - | - | H | $CH_3$ | - | - | - | - | - | - | - | $OCH_3$ | H | |
| $J_{21}$ | - | - | - | - | - | H | $CH_3$ | - | - | - | - | - | - | - | $CH_3$ | H | |
| $J_{22}$ | - | - | - | - | - | H | $CH_3$ | - | - | - | - | - | - | - | $OCH_3$ | H | |
| $J_{23}$ | - | $CH_2$ | - | - | - | H | - | H | - | - | - | - | - | - | $OCH_3$ | H | |
| $J_{23}$ | - | $CH_2$ | - | - | - | H | - | H | - | - | - | - | - | - | $CH_3$ | H | |
| $J_{23}$ | - | O | - | - | - | H | - | H | - | - | - | - | - | - | $OCH_3$ | H | |
| $J_{23}$ | - | O | - | - | - | H | - | H | - | - | - | - | - | - | $CH_3$ | H | |
| $J_{24}$ | - | $CH_2$ | O | - | - | H | - | - | - | - | - | - | - | - | $OCH_3$ | H | |
| $J_{24}$ | - | O | O | - | - | H | - | - | - | - | - | - | - | - | $OCH_3$ | H | |
| $J_{24}$ | - | $CH_2$ | $CH_2$ | - | - | H | - | - | - | - | - | - | - | - | $OCH_3$ | H | |
| $J_{25}$ | - | $CH_2$ | - | - | - | H | - | - | - | - | - | - | - | - | $OCH_3$ | H | |
| $J_{25}$ | - | $CH_2$ | - | - | - | H | - | - | - | - | - | - | - | - | $CH_3$ | H | |
| $J_{25}$ | - | O | - | - | - | H | - | - | - | - | - | - | - | - | $CH_3$ | $CH_3$ | |
| $J_{26}$ | - | - | - | O | - | H | - | $CH_3$ | H | H | - | - | - | - | $OCH_3$ | $CH_3$ | |
| $J_{26}$ | - | - | - | O | - | H | - | $CH_3$ | H | H | - | - | - | - | $OCH_3$ | H | |
| $J_{26}$ | - | - | - | S | - | H | - | $CH_3$ | H | H | - | - | - | - | $OCH_3$ | H | |
| $J_{26}$ | - | - | - | S | - | H | - | $CH_3$ | H | H | - | - | - | - | $OCH_3$ | $CH_3$ | |
| $J_{26}$ | - | - | - | $SO_2$ | - | H | - | $CH_3$ | H | H | - | - | - | - | $OCH_3$ | H | |
| $J_{26}$ | - | - | - | $SO_2$ | - | H | - | $CH_3$ | H | H | - | - | - | - | $OCH_3$ | $CH_3$ | |
| $J_{27}$ | - | $CH_2$ | - | O | - | H | - | $CH_3$ | H | - | - | - | - | - | $OCH_3$ | H | |
| $J_{27}$ | - | $CH_2$ | - | S | - | H | - | $CH_3$ | H | - | - | - | - | - | $OCH_3$ | H | |
| $J_{27}$ | - | $CH_2$ | - | $SO_2$ | - | H | - | $CH_3$ | H | - | - | - | - | - | $OCH_3$ | H | |
| $J_{27}$ | - | O | - | $SO_2$ | - | H | - | $CH_3$ | H | - | - | - | - | - | $OCH_3$ | H | |
| $J_{28}$ | - | - | - | - | O | H | - | H | H | - | - | - | - | - | $OCH_3$ | $CH_3$ | |
| $J_{28}$ | - | - | - | - | S | H | - | H | H | - | - | - | - | - | $CH_3$ | H | |
| $J_{29}$ | - | $CH_2$ | - | - | - | H | - | H | H | - | H | H | - | - | $OCH_3$ | H | |
| $J_{29}$ | - | $CH_2$ | - | - | - | H | - | H | H | - | $CH_3$ | H | - | - | $OCH_3$ | H | |
| $J_{29}$ | - | - | - | - | - | H | - | H | H | - | H | H | - | - | $OCH_3$ | H | |
| $J_{29}$ | - | - | - | - | - | H | - | H | H | - | $CH_3$ | H | - | - | $OCH_3$ | H | |
| $J_{30}$ | - | $CH_2$ | - | - | - | H | - | - | - | - | - | - | - | - | $OCH_3$ | H | |

Table XXVII (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_1$ | $Y_3$ | m.p. (°C) |
|---|---|---|---|---|---|---|---|---|---|---|---|---|---|---|---|---|---|
| $J_{31}$ | - | $CH_2$ | - | - | - | H | - | - | - | - | - | - | - | - | $OCH_3$ | $CH_3$ | |
| $J_{31}$ | - | O | - | - | - | H | - | - | - | - | - | - | - | - | $OCH_3$ | H | |
| $J_{31}$ | - | O | - | - | - | H | - | - | - | - | - | - | - | - | $OCH_3$ | $CH_3$ | |
| $J_{32}$ | - | $CH_2$ | - | - | - | - | - | - | - | - | - | - | Cl | - | $CH_3$ | H | |
| $J_{32}$ | - | $CH_2$ | - | - | - | - | - | - | - | - | - | - | F | - | $OCH_3$ | H | |
| $J_{32}$ | - | $CH_2$ | - | - | - | - | - | - | - | - | - | - | Br | - | $OCH_3$ | H | |
| $J_{32}$ | - | $CH_2$ | - | - | - | - | - | - | - | - | - | - | $SO_2CH_3$ | - | $OCH_3$ | H | |
| $J_{32}$ | - | $CH_2$ | - | - | - | - | - | - | - | - | - | - | $OSO_2CH_3$ | - | $OCH_3$ | H | |
| $J_{32}$ | - | $CH_2$ | - | - | - | - | - | - | - | - | - | - | $SO_2N(CH_3)_2$ | - | $OCH_3$ | H | |
| $J_{32}$ | - | $CH_2$ | - | - | - | - | - | - | - | - | - | - | $CO_2CH_3$ | - | $OCH_3$ | H | |
| $J_{32}$ | - | O | - | - | - | - | - | - | - | - | - | - | Cl | - | $OCH_3$ | H | |
| $J_{32}$ | - | O | - | - | - | - | - | - | - | - | - | - | F | - | $OCH_3$ | H | |
| $J_{32}$ | - | O | - | - | - | - | - | - | - | - | - | - | Br | - | $OCH_3$ | H | |
| $J_{32}$ | - | O | - | - | - | - | - | - | - | - | - | - | $SO_2CH_3$ | - | $OCH_3$ | H | |
| $J_{32}$ | - | O | - | - | - | - | - | - | - | - | - | - | $OSO_2CH_3$ | - | $OCH_3$ | H | |
| $J_{32}$ | - | O | - | - | - | - | - | - | - | - | - | - | $SO_2N(CH_3)_2$ | - | $OCH_3$ | H | |
| $J_{32}$ | - | O | - | - | - | - | - | - | - | - | - | - | $CO_2CH_3$ | - | $OCH_3$ | H | |

Table XXVIII

General Formula 7

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_2$ | $Y_2$ | m.p. (°C) |
|---|---|---|---|---|---|---|---|---|---|---|---|---|---|---|---|---|---|
| $J_{12}$ | 0 | $CH_2$ | - | - | - | H | - | - | - | - | - | - | - | $CH_3$ | $CH_3$ | $OCH_3$ | |
| $J_{12}$ | 0 | $CH_2$ | - | - | - | H | - | - | - | - | - | | - | $CH_3$ | $C_2H_5$ | $OC_2H_5$ | |
| $J_{12}$ | 0 | $CH_2$ | - | - | - | H | - | - | - | - | - | - | - | $CH_3$ | $CH_2CF_3$ | $SCH_3$ | |
| $J_{12}$ | 0 | $CH_2$ | - | - | - | H | - | - | - | - | - | - | - | $CH_3$ | $CH_3$ | $SC_2H_5$ | |
| $J_{12}$ | 0 | $CH_2$ | - | - | - | H | - | - | - | - | - | - | - | $CH_3$ | $C_2H_5$ | $OCF_2H$ | |
| $J_{12}$ | 0 | $CH_2$ | - | - | - | H | - | - | - | - | - | - | - | $CH_3$ | $C_2H_5$ | $SCF_2H$ | |
| $J_{12}$ | 0 | O | - | - | - | H | - | - | - | - | - | - | - | $CH_3$ | $C_2H_5$ | $OCH_3$ | |
| $J_{12}$ | 0 | O | - | - | - | H | - | - | - | - | - | - | - | $CH_3$ | $C_2H_5$ | $SCH_3$ | |
| $J_{12}$ | 0 | O | - | - | - | H | - | - | - | - | - | - | - | $CH_3$ | $C_2H_5$ | $SCF_2H$ | |
| $J_{13}$ | 0 | $CH_2$ | - | - | - | H | $CH_3$ | - | - | - | - | - | - | - | $CH_2CF_3$ | $SCH_3$ | |
| $J_{13}$ | 0 | $CH_2$ | - | - | - | H | $CH_3$ | - | - | - | - | - | - | - | $CH_2CH_3$ | $SC_2H_5$ | |
| $J_{13}$ | 0 | O | - | - | - | H | $CH_3$ | - | - | - | - | - | - | - | $CH_2CH_3$ | $SC_2H_5$ | |
| $J_{13}$ | 1 | $CH_2$ | - | - | - | H | $CH_3$ | - | - | - | - | - | - | - | $CH_2CH_3$ | $SCH_3$ | |
| $J_{13}$ | 1 | $CH_2$ | - | - | - | H | $CH_3$ | - | - | - | - | - | - | - | $CH_2CF_3$ | $SC_2H_5$ | |
| $J_{13}$ | 1 | O | - | - | - | H | $CH_3$ | - | - | - | - | - | - | - | $C_2H_5$ | $SCH_3$ | |
| $J_{14}$ | 0 | $CH_2$ | - | - | - | H | - | H | H | - | - | - | - | - | $CH_3$ | $SC_2H_5$ | |
| $J_{14}$ | 0 | $CH_2$ | - | - | - | H | - | H | H | - | - | - | - | - | $CH_3$ | $SCH_3$ | |
| $J_{14}$ | 1 | $CH_2$ | - | - | - | H | - | H | H | - | - | - | - | - | $CH_3$ | $SCH_3$ | |
| $J_{14}$ | 1 | $CH_2$ | - | - | - | H | - | H | H | - | - | - | - | - | $CH_3$ | $SC_2H_5$ | |
| $J_{14}$ | 1 | O | - | - | - | H | - | H | H | - | - | - | - | - | $CH_3$ | $SC_2H_5$ | |
| $J_{15}$ | 0 | $CH_2$ | - | - | - | H | - | - | - | - | - | - | - | - | $CH_3$ | $SCH_3$ | |
| $J_{15}$ | 0 | O | - | - | - | H | - | - | - | - | - | - | - | - | $C_2H_5$ | $SCH_3$ | |
| $J_{15}$ | 1 | $CH_2$ | - | - | - | H | - | - | - | - | - | - | - | - | $CH_3$ | $SCH_3$ | |
| $J_{16}$ | 0 | - | - | - | - | H | $CH_3$ | - | - | - | - | - | - | - | $CH_3$ | $SCH_3$ | |
| $J_{16}$ | 0 | - | - | - | - | H | $CH_3$ | - | - | - | - | - | - | - | $C_2H_5$ | $SCH_3$ | |
| $J_{16}$ | 0 | - | - | - | - | H | $CH_3$ | - | - | - | - | - | - | - | $CH_2CF_3$ | $SCH_3$ | |
| $J_{17}$ | 0 | - | - | - | - | H | $CH_3$ | - | - | | - | - | - | - | $CH_3$ | $SCH_3$ | |
| $J_{18}$ | - | $CH_2$ | - | - | - | H | $CH_3$ | - | - | - | - | | - | $CH_3$ | $CH_2CH_3$ | $SC_2H_5$ | |
| $J_{18}$ | - | $CH_2$ | - | - | - | H | $CH_3$ | - | - | - | - | - | - | $CH_3$ | $CH_3$ | $SC_2H_5$ | |
| $J_{19}$ | - | $CH_2$ | - | - | - | H | - | H | - | - | - | - | - | - | $CH_3$ | $SCH_3$ | |

Table XXVIII (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_2$ | $Y_2$ | m.p. (°C) |
|---|---|---|---|---|---|---|---|---|---|---|---|---|---|---|---|---|---|
| $J_{19}$ | - | O | - | - | - | H | - | H | - | - |  | - | - | - | $CH_3$ | $SCH_3$ | |
| $J_{20}$ | - | $CH_2$ | - | - | - | H | - | - | - | - | - | - | - | - | $CH_3$ | $SCH_3$ | |
| $J_{21}$ | - | - | - | - | - | H | $CH_3$ | - | - | - | - |  | - | - | $CH_3$ | $SCH_3$ | |
| $J_{21}$ | - | - | - | - | - | H | $CH_3$ | - | - | - | - | - | - | - | $C_2H_5$ | $SC_2H_5$ | |
| $J_{22}$ | - | - | - | - | - | H | $CH_3$ | - | - | - | - | - | - | - | $CH_3$ | $SCH_3$ | |
| $J_{23}$ | - | $CH_2$ | - | - | - | H | - | H | - | - | - | - | - | - | $C_2H_5$ | $SCH_3$ | |
| $J_{23}$ | - | $CH_2$ | - | - | - | H | - | H | - | - | - | - | - | - | $CH_3$ | $SCH_3$ | |
| $J_{23}$ | - | O | - | - | - | H | - | H | - | - | - | - | - | - | $CH_3$ | $SC_2H_5$ | |
| $J_{23}$ | - | O | - | - | - | H | - | H | - | - |  | - | - | - | $C_2H_5$ | $SCH_3$ | |
| $J_{24}$ | - | $CH_2$ | O | - | - | H | - | - | - | - | - | - | - | - | $CH_3$ | $SCH_3$ | |
| $J_{24}$ | - | O | O | - | - | H | - | - | - | - | - | - | - | - | $CH_3$ | $SCH_3$ | |
| $J_{24}$ | - | $CH_2$ | $CH_2$ | - | - | H | - | - | - | - | - | - | - | - | $CH_3$ | $SC_2H_5$ | |
| $J_{25}$ | - | $CH_2$ | - | - | - | H | - | - | - | - | - | - | - | - | $CH_3$ | $SCH_3$ | |
| $J_{25}$ | - | $CH_2$ | - | - | - | H | - | - | - | - | - | - | - | - | $CH_3$ | $SC_2H_5$ | |
| $J_{25}$ | - | O | - | - | - | H | - | - | - | - | - | - | - | - | $CH_3$ | $SCH_3$ | |
| $J_{26}$ | - | - | - | O | - | H | - | $CH_3$ | H | H | - | - | - | - | $CH_3$ | $SCH_3$ | |
| $J_{26}$ | - | - | - | O | - | H | - | $CH_3$ | H | H | - | - | - | - | $CH_3$ | $SC_2H_5$ | |
| $J_{26}$ | - | - | - | O | - | H | - | $CH_3$ | H | H | - | - | - | - | $C_2H_5$ | $SCH_3$ | |
| $J_{26}$ | - | - | - | S | - | H | - | $CH_3$ | H | H | - | - | - | - | $CH_3$ | $SCH_3$ | |
| $J_{26}$ | - | - | - | S | - | H | - | $CH_3$ | H | H | - | - | - | - | $CH_3$ | $SC_2H_5$ | |
| $J_{26}$ | - | - | - | $SO_2$ | - | H | - | $CH_3$ | H | H | - | - | - | - | $CH_3$ | $SCH_3$ | |
| $J_{26}$ | - | - | - | $SO_2$ | - | H | - | $CH_3$ | H | H | - | - | - | - | $CH_3$ | $SC_2H_5$ | |
| $J_{27}$ | - | $CH_2$ | - | O | - | H | - | $CH_3$ | H | - | - | - | - | - | $C_2H_5$ | $SCH_3$ | |
| $J_{27}$ | - | $CH_2$ | - | S | - | H | - | $CH_3$ | H | - | - | - | - | - | $CH_3$ | $SCH_3$ | |
| $J_{27}$ | - | $CH_2$ | - | $SO_2$ | - | H | - | $CH_3$ | H | - | - | - | - | - | $CH_3$ | $SCH_3$ | |
| $J_{27}$ | - | O | - | $SO_2$ | - | H | - | $CH_3$ | H | - | - | - | - | - | $CH_3$ | $SCH_3$ | |
| $J_{28}$ | - | - | - | - | O | H | - | H | H | - | - | - | - | - | $CH_3$ | $SCH_3$ | |
| $J_{28}$ | - | - | - | - | S | H | - | H | H | - | - | - |  | - | $CH_3$ | $SCH_3$ | |
| $J_{29}$ | - | $CH_2$ | - | - | - | H | - | H | H | - | H | H | - | - | $CH_3$ | $SCH_3$ | |
| $J_{29}$ | - | $CH_2$ | - | - | - | H | - | H | H | - | $CH_3$ | H | - | - | $CH_3$ | $SCH_3$ | |
| $J_{30}$ | - | - | - | - | - | H | - | H | H | - | - | - | - | - | $CH_3$ | $SCH_3$ | |

Table XXVIII (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_2$ | $Y_2$ | m.p. (°C) |
|---|---|---|---|---|---|---|---|---|---|---|---|---|---|---|---|---|---|
| $J_{30}$ | - | - | - | - | - | H | - | H | H | - | - | - | - | - | $CH_3$ | $SC_2H_5$ | |
| $J_{31}$ | - | $CH_2$ | - | - | - | H | - | - | - | - | - | - | - | - | $CH_3$ | $SCH_3$ | |
| $J_{31}$ | - | $CH_2$ | - | | - | H | - | - | - | - | - | - | - | - | $CH_3$ | $SC_2H_5$ | |
| $J_{31}$ | - | O | - | - | - | H | - | - | - | - | - | - | - | - | $CH_3$ | $SCH_3$ | |
| $J_{31}$ | - | O | - | - | - | H | - | - | - | - | - | - | - | - | $C_2H_5$ | $SCH_3$ | |
| $J_{32}$ | - | $CH_2$ | - | - | - | - | - | - | - | - | - | - | Cl | - | $CH_3$ | $SCH_3$ | |
| $J_{32}$ | - | $CH_2$ | - | - | - | - | - | - | - | - | - | - | F | - | $CH_3$ | $SCH_3$ | |
| $J_{32}$ | - | $CH_2$ | - | - | - | - | - | - | - | - | - | - | Br | - | $CH_3$ | $SCH_3$ | |
| $J_{32}$ | - | $CH_2$ | - | - | - | - | - | - | - | - | - | - | $SO_2CH_3$ | - | $CH_3$ | $SCH_3$ | |
| $J_{32}$ | - | $CH_2$ | - | - | - | - | - | - | - | - | - | - | $OSO_2CH_3$ | - | $CH_3$ | $SCH_3$ | |
| $J_{32}$ | - | $CH_2$ | - | - | - | - | - | - | - | - | - | - | $SO_2N(CH_3)_2$ | - | $CH_3$ | $SCH_3$ | |
| $J_{32}$ | - | $CH_2$ | - | - | - | - | - | - | - | - | - | - | $CO_2CH_3$ | - | $CH_3$ | $SCH_3$ | |
| $J_{32}$ | - | O | - | - | - | - | - | - | - | - | - | - | Cl | - | $CH_3$ | $SCH_3$ | |
| $J_{32}$ | - | O | - | - | - | - | - | - | - | - | - | - | F | - | $CH_3$ | $SCH_3$ | |
| $J_{32}$ | - | O | - | - | - | - | - | - | - | - | - | - | Br | - | $CH_3$ | $SCH_3$ | |
| $J_{32}$ | - | O | - | - | - | - | - | - | - | - | - | - | $SO_2CH_3$ | - | $CH_3$ | $SCH_3$ | |
| $J_{32}$ | - | O | - | - | - | - | - | - | - | - | - | - | $OSO_2CH_3$ | - | $CH_3$ | $SCH_3$ | |
| $J_{32}$ | - | O | - | - | - | - | - | - | - | - | - | - | $SO_2N(CH_3)_2$ | - | $CH_3$ | $SCH_3$ | |
| $J_{32}$ | - | O | - | - | - | - | - | - | - | - | - | - | $CO_2CH_3$ | - | $CH_3$ | $SCH_3$ | |

Table XXIX

General Formula 8

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_3$ | m.p. (°C) |
|---|---|---|---|---|---|---|---|---|---|---|---|---|---|---|---|---|
| $J_{12}$ | 0 | $CH_2$ | - | - | - | H | - | - | - | - | - | - | - | $CH_3$ | $OCH_3$ | |
| $J_{12}$ | 0 | $CH_2$ | - | - | - | H | - | - | - | - | - | - | - | $CH_3$ | $CH_3$ | |
| $J_{12}$ | 0 | O | - | - | - | H | - | - | - | - | - | - | - | $CH_3$ | $CH_3$ | |
| $J_{12}$ | 0 | O | - | - | - | H | - | - | - | - | - | - | - | $CH_3$ | $OCH_3$ | |
| $J_{13}$ | 0 | $CH_2$ | - | - | - | H | $CH_3$ | - | - | - | - | - | - | - | $OCH_3$ | |
| $J_{13}$ | 0 | $CH_2$ | - | - | - | H | $CH_3$ | - | - | - | - | - | - | - | $CH_3$ | |
| $J_{13}$ | 0 | O | - | - | - | H | $CH_3$ | - | - | - | - | - | - | - | $CH_3$ | |
| $J_{13}$ | 1 | $CH_2$ | - | - | - | H | $CH_3$ | - | - | - | - | - | - | - | $OCH_3$ | |
| $J_{13}$ | 1 | $CH_2$ | - | - | - | H | $CH_3$ | - | - | - | - | - | - | - | $CH_3$ | |
| $J_{13}$ | 1 | O | - | - | - | H | $CH_3$ | - | - | - | - | - | - | - | $OCH_3$ | |
| $J_{14}$ | 0 | $CH_2$ | - | - | - | H | - | H | H | - | - | - | - | - | $CH_3$ | |
| $J_{14}$ | 0 | $CH_2$ | - | - | - | H | - | H | H | - | - | - | - | - | $OCH_3$ | |
| $J_{14}$ | 1 | $CH_2$ | - | - | - | H | - | H | H | - | - | - | - | - | $OCH_3$ | |
| $J_{14}$ | 1 | O | - | - | - | H | - | H | H | - | - | - | - | - | $OCH_3$ | |
| $J_{15}$ | 0 | $CH_2$ | - | - | - | H | - | - | - | - | - | - | - | - | $CH_3$ | |
| $J_{15}$ | 0 | O | - | - | - | H | - | - | - | - | - | - | - | - | $OCH_3$ | |
| $J_{15}$ | 1 | $CH_2$ | - | - | - | H | - | - | - | - | - | - | - | - | $OCH_3$ | |
| $J_{16}$ | 0 | - | - | - | - | H | $CH_3$ | - | - | - | - | - | - | - | $CH_3$ | |
| $J_{16}$ | 0 | - | - | - | - | H | $CH_3$ | - | - | - | - | - | - | - | $OCH_3$ | |
| $J_{17}$ | 0 | - | - | - | - | H | $CH_3$ | - | - | - | - | - | - | - | $OCH_3$ | |
| $J_{18}$ | - | $CH_2$ | - | - | - | H | $CH_3$ | - | - | - | - | - | - | $CH_3$ | $CH_3$ | |
| $J_{18}$ | - | $CH_2$ | - | - | - | H | $CH_3$ | - | - | - | - | - | - | $CH_3$ | $OCH_3$ | |
| $J_{19}$ | - | $CH_2$ | - | - | - | H | - | H | - | - | - | - | - | - | $CH_3$ | |
| $J_{19}$ | - | O | - | - | - | H | - | H | - | - | - | - | - | - | $OCH_3$ | |
| $J_{20}$ | - | $CH_2$ | - | - | - | H | - | - | - | - | - | - | - | - | $CH_3$ | |
| $J_{21}$ | - | - | - | - | - | H | $CH_3$ | - | - | - | - | - | - | - | $CH_3$ | |
| $J_{21}$ | - | - | - | - | - | H | $CH_3$ | - | - | - | - | - | - | - | $OCH_3$ | |
| $J_{22}$ | - | - | - | - | - | H | $CH_3$ | - | - | - | - | - | - | - | $CH_3$ | |
| $J_{23}$ | - | $CH_2$ | - | - | - | H | - | H | - | - | - | - | - | - | $OCH_3$ | |
| $J_{23}$ | - | $CH_2$ | - | - | - | H | - | H | - | - | - | - | - | - | $CH_3$ | |

Table XXIX (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_3$ | m.p. (°C) |
|---|---|---|---|---|---|---|---|---|---|---|---|---|---|---|---|---|
| $J_{23}$ | - | O | - | - | - | H | - | H | - | - | - | - | - | - | $OCH_3$ | |
| $J_{23}$ | - | O | - | - | - | H | - | H | - | - | - | - | - | - | $CH_3$ | |
| $J_{24}$ | - | $CH_2$ | O | - | - | H | - | - | - | - | - | - | - | - | $CH_3$ | |
| $J_{24}$ | - | O | O | - | - | H | - | - | - | - | - | - | - | - | $OCH_3$ | |
| $J_{24}$ | - | $CH_2$ | $CH_2$ | - | - | H | - | - | - | - | - | - | - | - | $OCH_3$ | |
| $J_{25}$ | - | $CH_2$ | - | - | - | H | - | - | - | - | - | - | - | - | $CH_3$ | |
| $J_{25}$ | - | $CH_2$ | - | - | - | H | - | - | - | - | - | - | - | - | $OCH_3$ | |
| $J_{25}$ | - | O | - | - | - | H | - | - | - | - | - | - | - | - | $OCH_3$ | |
| $J_{26}$ | - | - | - | O | - | H | - | $CH_3$ | H | H | - | - | - | - | $OCH_3$ | |
| $J_{26}$ | - | - | - | O | - | H | - | $CH_3$ | H | H | - | - | - | - | $CH_3$ | |
| $J_{26}$ | - | - | - | S | - | H | - | $CH_3$ | H | H | - | - | - | - | $CH_3$ | |
| $J_{26}$ | - | - | - | S | - | H | - | $CH_3$ | H | H | - | - | - | - | $OCH_3$ | |
| $J_{26}$ | - | - | - | $SO_2$ | - | H | - | $CH_3$ | H | H | - | - | - | - | $CH_3$ | |
| $J_{26}$ | - | - | - | $SO_2$ | - | H | - | $CH_3$ | H | H | - | - | - | - | $OCH_3$ | |
| $J_{27}$ | - | $CH_2$ | - | O | - | H | - | $CH_3$ | H | - | - | - | - | - | $CH_3$ | |
| $J_{27}$ | - | $CH_2$ | - | S | - | H | - | $CH_3$ | H | - | - | - | - | - | $OCH_3$ | |
| $J_{27}$ | - | $CH_2$ | - | $SO_2$ | - | H | - | $CH_3$ | H | - | - | - | - | - | $CH_3$ | |
| $J_{27}$ | - | O | - | $SO_2$ | - | H | - | $CH_3$ | H | - | - | - | - | - | $OCH_3$ | |
| $J_{28}$ | - | - | - | - | O | H | - | H | H | - | - | - | - | - | $CH_3$ | |
| $J_{28}$ | - | - | - | - | S | H | - | H | H | - | - | - | - | - | $CH_3$ | |
| $J_{29}$ | - | $CH_2$ | - | - | - | H | - | H | H | - | H | H | - | - | $OCH_3$ | |
| $J_{29}$ | - | $CH_2$ | - | - | - | H | - | H | H | - | $CH_3$ | H | - | - | $CH_3$ | |
| $J_{30}$ | - | - | - | - | - | H | - | H | H | - | - | - | - | - | $OCH_3$ | |
| $J_{30}$ | - | - | - | - | - | H | - | H | H | - | - | - | - | - | $CH_3$ | |
| $J_{31}$ | - | $CH_2$ | - | - | - | H | - | - | - | - | - | - | - | - | $OCH_3$ | |
| $J_{31}$ | - | $CH_2$ | - | - | - | H | - | - | - | - | - | - | - | - | $CH_3$ | |
| $J_{31}$ | - | O | - | - | - | H | - | - | - | - | - | - | - | - | $OCH_3$ | |
| $J_{31}$ | - | O | - | - | - | H | - | - | - | - | - | - | - | - | $CH_3$ | |
| $J_{32}$ | - | $CH_2$ | - | - | - | - | - | - | - | - | - | - | Cl | - | $OCH_3$ | |
| $J_{32}$ | - | $CH_2$ | - | - | - | - | - | - | - | - | - | - | F | - | $OCH_3$ | |
| $J_{32}$ | - | $CH_2$ | - | - | - | - | - | - | - | - | - | - | Br | - | $OCH_3$ | |

Table XXIX (continued)

| J | n | L | Q | $Q_1$ | $Q_2$ | $R_{20}$ | $R_{21}$ | $R_{23}$ | $R_{24}$ | $R_{25}$ | $R_{26}$ | $R_{27}$ | $R_{28}$ | $R_{22}$ | $X_3$ | m.p. (°C) |
|---|---|---|---|---|---|---|---|---|---|---|---|---|---|---|---|---|
| $J_{32}$ | - | $CH_2$ | - | - | - | - | - | - | - | - | - | - | $SO_2CH_3$ | - | $OCH_3$ | |
| $J_{32}$ | - | $CH_2$ | - | - | - | - | - | - | - | - | - | - | $OSO_2CH_3$ | - | $OCH_3$ | |
| $J_{32}$ | - | $CH_2$ | - | - | - | | - | - | - | - | - | - | $SO_2N(CH_3)_2$ | - | $OCH_3$ | |
| $J_{32}$ | - | $CH_2$ | - | - | - | | - | - | - | - | - | - | $CO_2CH_3$ | - | $OCH_3$ | |
| $J_{32}$ | - | O | - | - | - | - | - | - | - | - | - | - | Cl | - | $OCH_3$ | |
| $J_{32}$ | - | O | - | - | - | - | - | - | - | - | - | - | F | - | $OCH_3$ | |
| $J_{32}$ | - | O | - | - | - | - | - | - | - | - | - | - | Br | - | $OCH_3$ | |
| $J_{32}$ | - | O | - | - | - | - | - | - | - | - | - | - | $SO_2CH_3$ | - | $OCH_3$ | |
| $J_{32}$ | - | O | - | - | - | - | - | - | - | - | - | - | $OSO_2CH_3$ | - | $OCH_3$ | |
| $J_{32}$ | - | O | - | - | - | - | - | - | - | - | - | - | $SO_2N(CH_3)_2$ | - | $OCH_3$ | |
| $J_{32}$ | - | O | - | - | - | - | - | - | - | - | - | - | $CO_2CH_3$ | - | $OCH_3$ | |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

TABLE XXX

| Percent by Weight | | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 5-50 | 40-95 | 0-15 |
| Aqueous Suspension | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredients can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd. Edn., Dorland Books, Caldwell, N.J. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", Allured Publ. Corp., Ridgewood, New Jersey, as well as Sisely and Wood, "Encyclopedia of Surface Active Agents", Chemical Publ. Co., Inc., New York, 1964, list surfactants and recommended uses. All formulations can contain minor amounts of additives to reduce foam, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, Dec. 4, 1967, pp. 147ff. and "Perry's Chemical Engineer's Handbook", 4th Edn., McGraw-Hill, N.Y., 1963, pp. 8-59ff.

For further information regarding the art of formulation, see for example:

H.M. Loux, U.S. Patent 3,235,361, Feb. 15, 1966, Col. 6, Line 16 through Col. 7, Line 19 and Examples 10 through 41.

R. W. Luckenbaugh, U.S. Patent 3,309,192, March 14, 1967, Col. 5, Line 43 through Col. 7, Line 62 and Ex. 8, 12, 15, 39, 41, 52, 53, 58, 132, 138-140, 162-164, 166, 169-182.

H. Gysin and E. Knusli, U.S. Patent 2,891,855, June 23, 1959, Col. 3, Line 66 through Col. 5, Line 17 and Examples 1-4.

G. C. Klingman, "Weed Control as a Science", John Wiley & Sons, Inc., New York, 1961 pp. 81-96.

J. D. Fryer and S. A. Evans, "Weed Control Handbook", 5th Edn., Blackwell Scientific Publications, Oxford, 1968, pp. 101-103.

J. B. Buchanan, U.S. Patent 3,576,834, April 27, 1971, Col. 5, Line 36 through Col. 7, Line 70 and Ex. 1-4, 17, 106, 123-140.

R. R. Shaffer, U.S. Patent 3,560,616 Feb. 2, 1971, Col. 3, Line 48 through Col. 7, Line 26 and Examples 3-9, 11-18.

E. Somers, "Formulation", Chapter 6 in Torgeson, "Fungicides", Vol. I, Academic Press, New York, 1967.

In the following examples, all parts are by weight unless otherwise indicated.

## Example 13

Wettable Powder

| | |
|---|---|
| 5-[[(4,6-dimethoxyprimidin-2-yl)aminocarbonyl]aminosulfonylmethyl]-1,3-dimethyl-1H-pyrazole-4-carboxylic acid, ethyl ester | 80% |
| sodium alkylnaphthalenesulfonate | 2% |
| sodium ligninsulfonate | 2% |
| synthetic amorphous silica | 3% |
| kaolinite | 13% |

The ingredients are thoroughly blended, passed through a mill, such as an air mill or hammer mill, to produce an average particle size under 25 microns, reblended and sifted through a U.S.S. No. 50 sieve (0.3 mm opening) before packaging.

## Example 14

Wettable Powder

| | |
|---|---|
| N-[(4,6-dimethoxypyrimidin-2-yl)aminocarbonyl]-2,3-dihydro-2-methylbenzo[B]thiophene-2-methanesulfonamide-1,1-dioxide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

## Example 15

### Granule

| | |
|---|---|
| Wettable Powder of Example 10 | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules are dried and packaged.

## Example 16

### High Strength Concentrate

| | |
|---|---|
| 3-[[(4,6-dimethoxypyrimidin-2-yl)aminocarbonyl]aminosulfonylmethyl]-1,5-dimethyl-1H-pyrazole-4-carboxylic acid, ethyl ester | 98.5% |
| silica aerogel | 0.5% |
| synthetic amorphous fine silica | 1.0% |

The ingredients are blended and ground in a hammer mill to produce a high strength concentrate essentially all passing in U.S.S. No. 50 sieve (0.3 mm openings). This material may then be formulated in a variety of ways.

## Example 17

Dust

| | |
|---|---|
| wettable powder of Example 16 | 5% |
| pyrophyllite (powder) | 95% |

The wettable powder and the pyrophyllite diluent are thoroughly blended and then packaged. The product is suitable for use as a dust.

## Example 18

Low Strength Granule

| | |
|---|---|
| 1,3-dihydro-N-[(4,6-dimethoxypyrimidin-2-yl)aminocarbonyl]-1-oxo-benzo[c]furan-7-methanesulfonamide | 1% |
| N,N-dimethylformamide | 9% |
| attapulgite granules (U.S.S. 20-40 sieve; 0.84-0.42 mm) | 90% |

The active ingredient is dissolved in the solvent and the solution is sprayed upon dedusted granules in a double cone blender. After spraying of the solution has been completed, the blender is allowed to run for a short period and then the granules are packaged.

Example 19

Aqueous Suspension

| | |
|---|---|
| N-[(4-methoxy-6-methyl-1,3,5-triazin-2-yl)aminocarbonyl]-2-methylbenzo[B]thiophene-7-methanesulfonamide-1,1-dioxide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 20

Aqueous Suspension

| | |
|---|---|
| 5-[[(4,6-dimethoxypyrimidin-2-yl)aminocarbonyl]aminosulfonylmethyl]-1,3-dimethyl-1H-pyrazole-4-carboxylic acid, ethyl ester | 25% |
| hydrated attapulgite | 3% |
| crude calcium ligninsulfonate | 10% |
| water | 62% |

The ingredients are ground together in a ball, sand or roller mill until the solid particles have been reduced to diameters under 10 microns.

Example 21

Solution

| | |
|---|---|
| N-[(4,6-dimethoxypyrimidin-2-yl)aminocarbonyl]-2,3-dihydro-2-methylbenzo[B]thiophene-7-methanesulfonamide-1,1-dioxide, sodium salt | 5% |
| water | 95% |

The salt is added directly to the water with stirring to produce the solution, which may then be packaged for use.

## Example 22

High Strength Concentrate

| | |
|---|---|
| 1,3-dihydro-N-[(4,6-dimethoxypyrimidin-2-yl)aminocarbonyl]-1-oxo-benzo[c]furan-7-methanesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 23

Wettable Powder

| | |
|---|---|
| 1,3-dihydro-N-[(4-methoxy-6-methyl-1,3,5-triazin-2-yl)-aminocarbonyl]-1-oxo-benzo[c]furan-7-methanesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

## Example 24

Oil Suspension

| | |
|---|---|
| N-[(4-methoxy-6-methyl-1,3,5-triazin-2-yl)aminocarbonyl]-2-methylbenzo[B]thiophene-7-methanesulfonamide-1,1-dioxide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 25

Dust

| | |
|---|---|
| N-[(4-methoxy-6-methyl-1,3,5-triazin-2-yl)aminocarbonyl]-2-methylbenzo[B]thiophene-7-methanesulfonamide-1,1-dioxide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

## Example 26

### Extruded Pellet

| | |
|---|---|
| 3-[[(4,6-dimethoxypyrimidin-2-yl)aminocarbonyl]aminosulfonylmethyl]-1,5-dimethyl-1H-pyrazole-4-carboxylic acid, ethyl ester | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer milled and then moistened with about 12% water. The mixture is extruded as cylinders about 1-3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

## Example 27

### Solution

| | |
|---|---|
| 5-[[(4,6-dimethoxypyrimidin-2-yl)aminocarbonyl]aminosulfonylmethyl]-1,3-dimethyl-1H-pyrazole-4-carboxylic acid, ethyl ester | 5% |
| dimethylformamide | 95% |

Utility

The compounds of the present invention are active herbicides, having utility for broad-spectrum pre- and/or post-emergence weed contol in areas where complete control of all vegetation is desired, such as around fuel storage tanks, ammunition depots, industrial storage areas, parking lots, drive-in theaters, billboards, and highway and railroad structures.

The rates of application for the compounds of the invention are determined by a number of factors, including the types of weeds to be controlled, weather and climate, formulations selected, mode of application, amount of foliage present, etc. In general terms, the subject compounds should be applied at levels of around 0.05 to 5 kg/ha, the lower rates being suggested for use on lighter soils and/or those having a low organic matter content, or for situations where only short-term persistence is required.

The compounds of the invention may be used in combination with any other commercial herbicide; examples of which are those of the triazine, triazole, uracil, urea, amide, diphenylether, carbamate and bipyridylium types. The compounds may also be used in combination with mefluidide.

The herbicidal properties of the subject compounds were discovered in a number of greenhouse tests. The test procedure and results follow.

Test A

Seeds of crabgrass (Digitaria sp.), barnyard-grass (Echinochloa crusgalli), wild oats (Avena fatua), sicklepod (Cassia obtusifolia), morningglory (Ipomoea sp.), cocklebur (Xanthium pensylvanicum), sorghum, corn, soybean, sugar beet, rice, wheat, cotton and purple nutsedge (Cyperus rotundus) tubers were planted and treated pre-emergence with the test chemicals dissolved in a non-phytotoxic solvent. At the same time, these crop and weed species were treated post-emergence with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis or necrosis;
E = emergence inhibition;
G = growth retardation; and
H = formative effects.

The test compounds are referenced in the table of results as follows.

Compounds

Compound 1

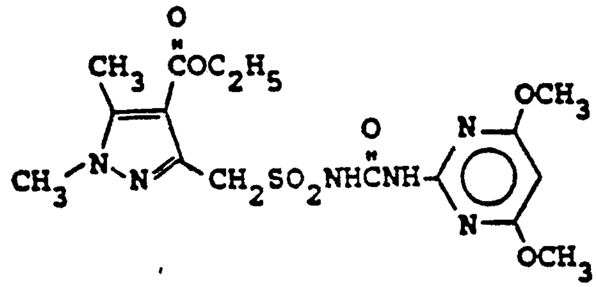

Compound 2

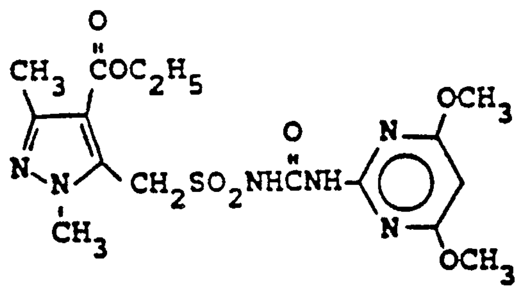

Compound 3

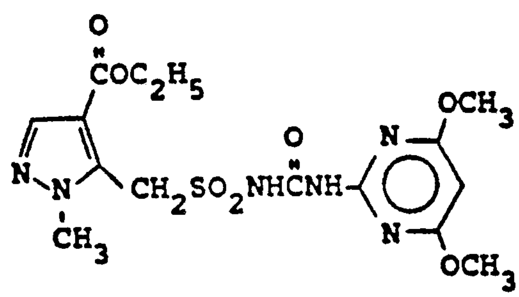

Compound 4

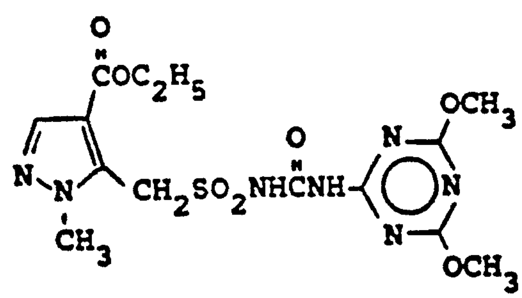

Compounds (Continued)

Compound 5

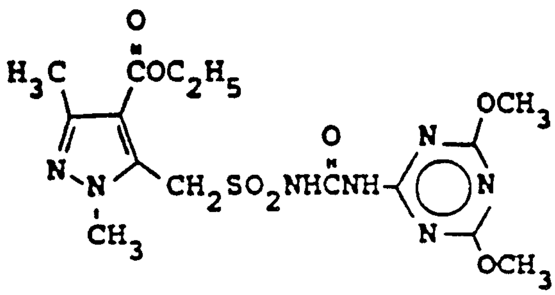

Compound 6

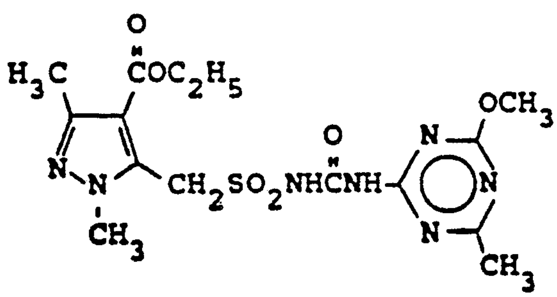

Compound 7

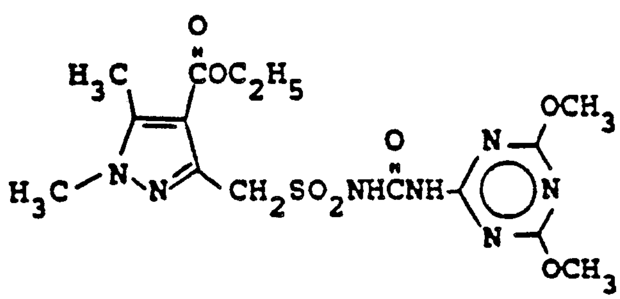

Compounds (Continued)

Compound 8

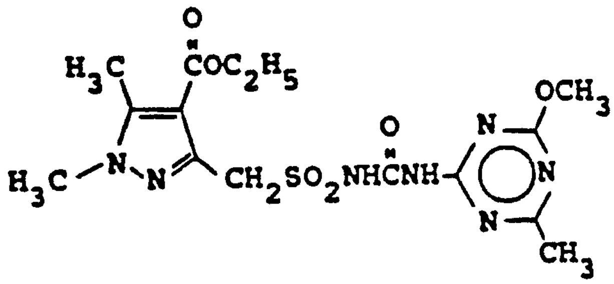

Compound 9.

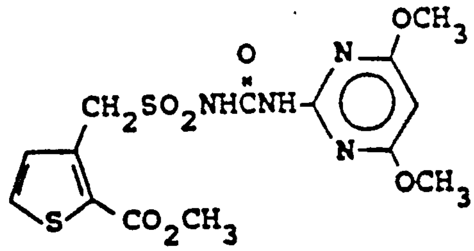

Compound 10

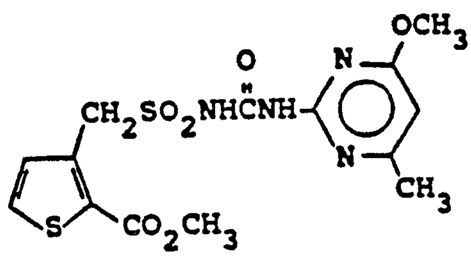

Compound 11

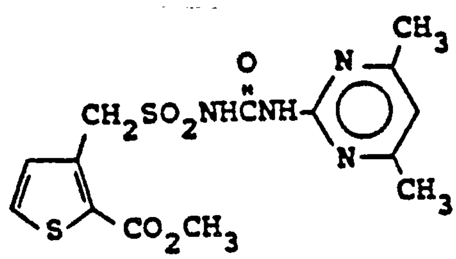

Compounds (Continued)

Compound 12

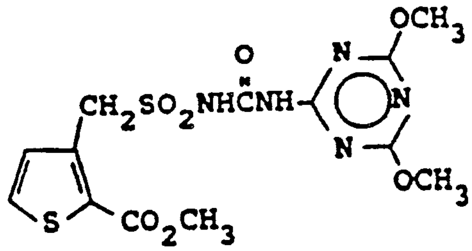

Compound 13

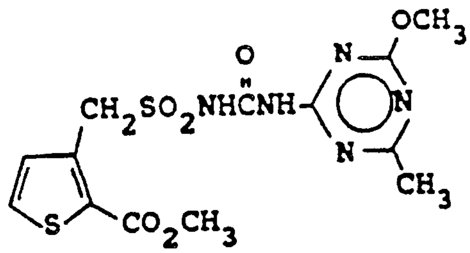

Compound 14

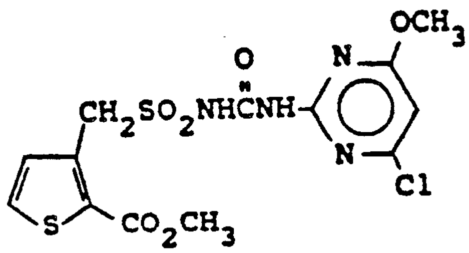

Compounds (Continued)

Compound 15

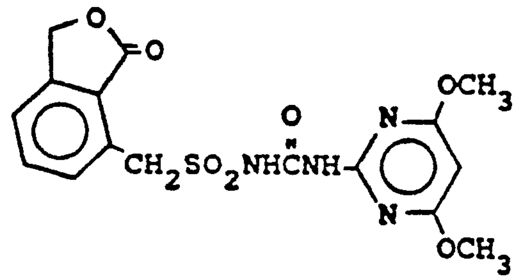

Compound 16

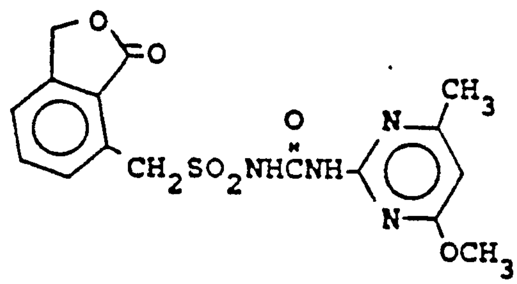

Compound 17

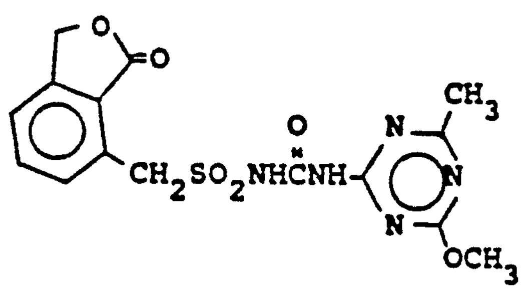

Compound 18

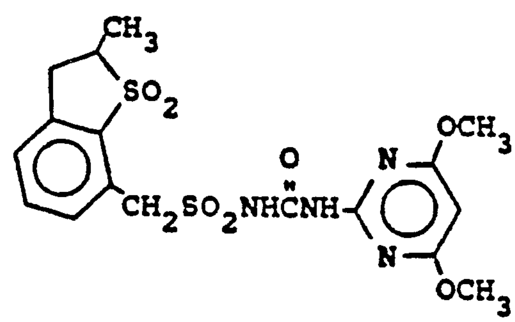

Compounds (continued)

Compound 19

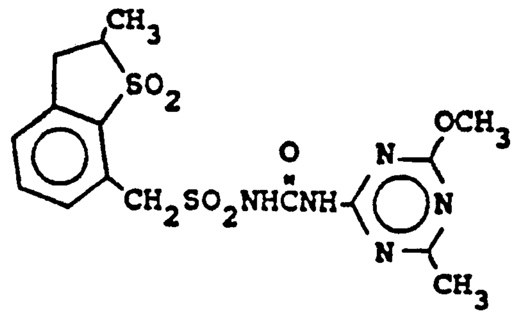

Compound 20

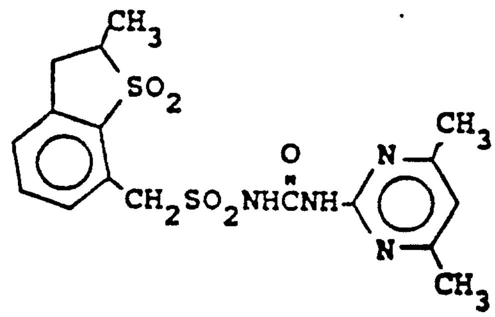

Compound 21

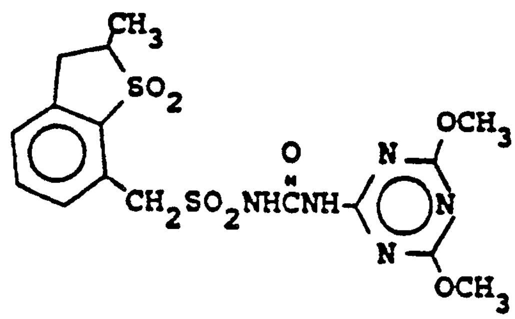

Compound 22

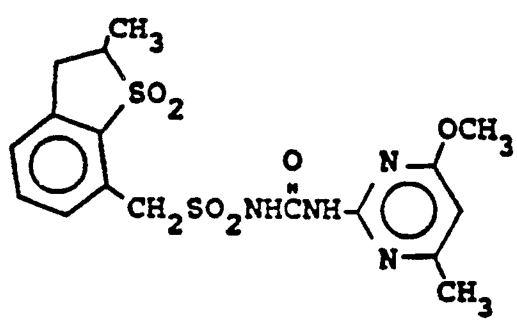

Compounds (continued)

Compound 23

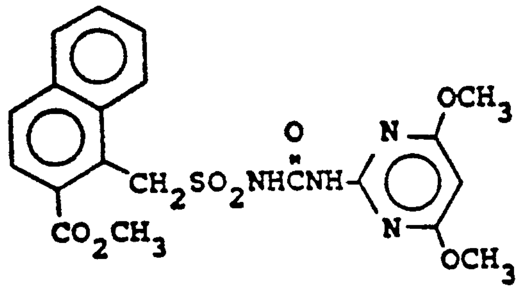

Compound 24

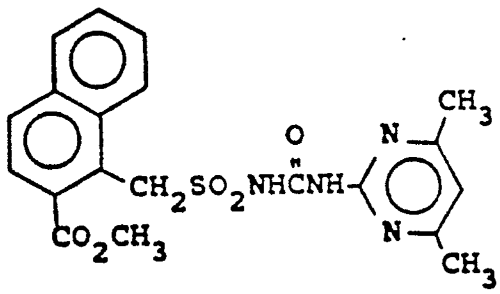

Compound 25

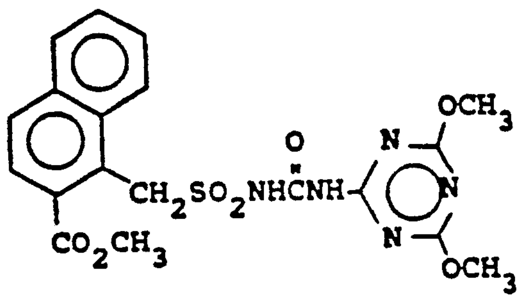

Compound 26

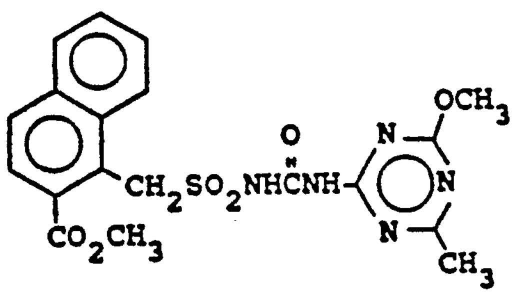

## Table A

| | Compound 1 | | |
|---|---|---|---|
| Rate kg/ha | 2.0 | 0.4 | 0.05 |
| POST-EMERGENCE | | | |
| Morningglory | 5C,9G | 2C,8G | 4G |
| Cocklebur | 2C,8G | 1C | 0 |
| Sicklepod | 2C,9H | 3C,7H | 3C,5G |
| Nutsedge | 8G | 5G | 7G |
| Crabgrass | 6G | 2G | 0 |
| Barnyardgrass | 3C,8H | 3C,8H | 2C,5H |
| Wild Oats | 2C,5G | 0 | 0 |
| Wheat | 4G | 0 | 0 |
| Corn | 2C,9G | 3C,8G | 3C,8H |
| Soybean | 3C,9H | 4C,8H | 3C,9G |
| Rice | 2G | 2G | 0 |
| Sorghum | 3C,8H | 3C,8H | 3C,8H |
| Sugar beet | 3C,7G | 3C,5G | 3G |
| Cotton | 3C,9G | 4C,7G | 3C,5H |
| PRE-EMERGENCE | | | |
| Morningglory | 5C,9G | 4C,7G | 3H |
| Cocklebur | - | 0 | 5H |
| Sicklepod | 4C,8G | 4C,6G | 3G |
| Nutsedge | 10E | 0 | 0 |
| Crabgrass | 6G | 2C,5G | 2C,3G |
| Barnyardgrass | 4C,9H | 2C,5G | 0 |
| Wild Oats | 4C,8G | 2C,3G | 0 |
| Wheat | 8G | 3G | 0 |
| Corn | 5C,9G | 4C,5G | 2C,2G |
| Soybean | 3C,5H | 2C,2H | 1C |
| Rice | 3C,8H | 2C,5G | 0 |
| Sorghum | 3C,9H | 3C,8G | 2C,3G |
| Sugar beet | 4C,8G | 3C,8G | 2C,4G |
| Cotton | 2C | 0 | 0 |

Table A (continued)

| | Compound 2 | | |
|---|---|---|---|
| Rate kg/ha | 2.0 | 0.4 | 0.05 |
| POST-EMERGENCE | | | |
| Morningglory | 9C | 9C | 9C |
| Cocklebur | 3H | 5G | 1H |
| Sicklepod | 5C,6G | 3C,4H | 4C,4H |
| Nutsedge | 8G | 8G | 8G |
| Crabgrass | 4G | 2G | 0 |
| Barnyardgrass | 5C,9H | 3C,9H | 2C,3H |
| Wild Oats | 2G | 0 | 0 |
| Wheat | 0 | 0 | 0 |
| Corn | 2C,8H | 3C,8H | 2C,5H |
| Soybean | 4C,9G | 5C,9G | 3C,7G |
| Rice | 2C,7G | 2C,7G | 4G |
| Sorghum | 3C,8H | 3C,7G | 5G |
| Sugar beet | 3C,8H | 1C,4G | 4G |
| Cotton | 4C,8G | 3C,8G | 2C,7G |
| PRE-EMERGENCE | | | |
| Morningglory | 9C | 9G | 8G |
| Cocklebur | 9H | 8H | 0 |
| Sicklepod | 9G | - | 8G |
| Nutsedge | 10E | 10E | 0 |
| Crabgrass | 4G | 3G | 0 |
| Barnyardgrass | 4C,9H | 3C,8H | 3G |
| Wild Oats | 2C,6G | 1C,3G | 2G |
| Wheat | 2C,8H | 4G | 0 |
| Corn | 2C,9H | 4C,9H | 2C |
| Soybean | 4C,7G | 3C,3H | 0 |
| Rice | 10E | 4C,9H | 2C |
| Sorghum | 6G,9H | 4C,9G | 2C,3H |
| Sugar beet | 5C,9G | 9G | 7G |
| Cotton | 9G | 2C,8G | 0 |

Table A (continued)

| | Compound 3 | | Compound 4 |
|---|---|---|---|
| Rate kg/ha | 0.4 | 0.05 | 0.4 |
| POST-EMERGENCE | | | |
| Morningglory | 4C,9G | 3C,6H | 2C,2H |
| Cocklebur | 2C,5H | 0 | 0 |
| Nutsedge | 3G | 4G | 0 |
| Crabgrass | 1C | 0 | 0 |
| Barnyardgrass | 0 | 0 | 0 |
| Wild Oats | 0 | 0 | 0 |
| Wheat | 0 | 0 | 0 |
| Corn | 0 | 0 | 0 |
| Soybean | 2C,8G | 2C,2H | 0 |
| Rice | 0 | 0 | 0 |
| Sorghum | 3C,5G | 2G | 0 |
| Sugar beets | 3C,6G | 2C,3G | 1C,1H |
| Cotton | 3C,8H | 0 | 0 |
| Sicklepod | 3C,8H | 3C,3H | 0 |
| PRE-EMERGENCE | | | |
| Morningglory | 2C,5H | 0 | 1C |
| Cocklebur | 0 | 0 | 0 |
| Nutsedge | 10E | 0 | 0 |
| Crabgrass | 0 | 0 | 0 |
| Barnyardgrass | 0 | 0 | 0 |
| Wild Oats | 0 | 0 | 0 |
| Wheat | 0 | 0 | 0 |
| Corn | 2C | 0 | 3G |
| Soybean | 2C,3H | 0 | 0 |
| Rice | 0 | 0 | 0 |
| Sorghum | 4C,5G | 0 | 0 |
| Sugar beet | 3C,8G | 2C | 2C,3G |
| Cotton | 4G | 0 | 0 |
| Sicklepod | 2C | 0 | 0 |

Table A (continued)

| | Cmpd. 5 | Cmpd. 6 | Cmpd. 7 |
|---|---|---|---|
| Rate kg/ha | 0.4 | 0.4 | 0.4 |
| POST-EMERGENCE | | | |
| Morningglory | 5C,8H | 2C,3H | 3C,5G |
| Cocklebur | 0 | 3G | 0 |
| Nutsedge | 0 | 0 | 0 |
| Crabgrass | 0 | 0 | 0 |
| Barnyardgrass | 2C,4H | 2C,5H | 0 |
| Wild Oats | 0 | 0 | 0 |
| Wheat | 3G | 0 | 0 |
| Corn | 3C,8H | 2C,5H | 0 |
| Soybean | 2C,3H | 2C,3H | 3C,6G |
| Rice | 4C,8G | 2C,6G | 4G |
| Sorghum | 5C,9H | 3C,5H | 3G |
| Sugar beets | 4C,8H | 3C,6H | 3C,6G |
| Cotton | 4G | 2H | 0 |
| Sicklepod | 0 | 0 | 1C |
| PRE-EMERGENCE | | | |
| Morningglory | 2C | 0 | 0 |
| Cocklebur | 0 | 0 | 0 |
| Nutsedge | 0 | 0 | 0 |
| Crabgrass | 0 | 0 | 0 |
| Barnyardgrass | 0 | 0 | 0 |
| Wild Oats | 0 | 0 | 0 |
| Wheat | 2G | 0 | 0 |
| Corn | 3G | 0 | 0 |
| Soybean | 1C | 0 | 0 |
| Rice | 2C | 0 | 0 |
| Sorghum | 2C,5G | 2C,6G | 0 |
| Sugar beet | 0 | 0 | 0 |
| Cotton | 0 | 0 | 0 |
| Sicklepod | 0 | 0 | 0 |

TABLE A (Continued)

| | Compound 8 | Compound 9 | |
|---|---|---|---|
| Rate (kg/ha) | 0.4 | 0.4 | 0.05 |
| POSTEMERGENCE | | | |
| Morningglory | 2C,3H | 4C,5G | 8G,2C |
| Cocklebur | 0 | 1H | 0 |
| Nutsedge | 0 | 2C,8G | 2C,5G |
| Crabgrass | 0 | 4H | 0 |
| Barnyardgrass | 0 | 1H | 0 |
| Wild Oats | 0 | 0 | 0 |
| Wheat | 0 | 0 | 0 |
| Corn | 0 | 0 | 0 |
| Soybean | 3C,7G | 3C,8G | 2C,9G |
| Rice | 3G | 2C,3G | 2G |
| Sorghum | 3C,5H | 2C,2H | 0 |
| Sugar Beets | 2C | 5C,8H | 1C,4G |
| Cotton | 0 | 3C,7G | 2C,5G |
| Cassia | 0 | 2C,5G | 0 |
| PREEMERGENCE | | | |
| Morningglory | 0 | 9G | 7G |
| Cocklebur | 0 | 2G | 0 |
| Nutsedge | 0 | 8G | 5G |
| Crabgrass | 0 | 2G | 0 |
| Barnyardgrass | 0 | 4G | 1C |
| Wild Oats | 0 | 2G | 0 |
| Wheat | 0 | 0 | 0 |
| Corn | 0 | 2C,8H | 2C,5G |
| Soybean | 0 | 2C | 0 |
| Rice | 0 | 4G | 2G |
| Sorghum | 0 | 2C,7G | 3G |
| Sugar Beets | 0 | 9G | 4G |
| Cotton | 0 | 5G | 0 |
| Cassia | 0 | 2C,7G | 0 |

Table A (continued)

| | Cmpd. 10 | Cmpd. 11 | | Cmpd. 12 | Cmpd. 13 |
|---|---|---|---|---|---|
| Rate kg/ha | 0.4 | 0.4 | 2.0 | 0.4 | 0.4 |
| POST-EMERGENCE | | | | | |
| Morningglory | 3C,7H | 1C | 0 | 2C,9G | 5C,9G |
| Cocklebur | 4C,9H | 2C,5G | 6H | 0 | 5C,9G |
| Nutsedge | 0 | 0 | 0 | 3G | 5G |
| Crabgrass | 0 | 0 | 0 | 2G | 4G |
| Barnyardgrass | 0 | 0 | 0 | 2H | 2G |
| Wild Oats | 0 | 0 | 0 | 0 | 0 |
| Wheat | 0 | 0 | 0 | 0 | 2G |
| Corn | 0 | 0 | 0 | 2H | 2C,8H |
| Soybean | 3C,8G | 0 | 2C,5G | 3C,9G | 4C,9G |
| Rice | 0 | 0 | 0 | 3C,9G | 2C,8H |
| Sorghum | 0 | 0 | 0 | 2C,9H | 3C,9G |
| Sugar beets | 2C,2G | 0 | 2H | 9C | 9C |
| Cotton | 3G | 0 | 0 | 4C,9G | 3C,8G |
| Sicklepod | 2C | 0 | - | 2C,3G | 4C,6G |
| PRE-EMERGENCE | | | | | |
| Morningglory | 8G | 0 | 4H | 2C,8H | 9G |
| Cocklebur | 3C,6G | 0 | 3H | 0 | 9H |
| Nutsedge | 0 | 0 | 0 | 2G | 2G |
| Crabgrass | 0 | 0 | 0 | 0 | 0 |
| Barnyardgrass | 0 | 0 | 0 | 6H | 5H |
| Wild Oats | 0 | 0 | 0 | 2C,4G | 2G |
| Wheat | 0 | 0 | 0 | 8G | 4G |
| Corn | 2C,4G | 0 | 0 | 2C,8G | 9G |
| Soybean | 0 | 0 | 0 | 2C,3H | 2C,4H |
| Rice | 0 | 0 | 0 | 3C,5G | 2C,2G |
| Sorghum | 2C,3G | 0 | 0 | 2C,9H | 9H |
| Sugar beet | 8G | 0 | 4H | 5C,9G | 5C,9G |
| Cotton | 0 | 0 | 0 | 2C,4G | 5G |
| Sicklepod | 0 | 0 | - | 1C | 3C,5G |

TABLE A (Continued)

| | Compound 14 | |
|---|---|---|
| Rate (kg/ha) | 0.4 | 2.0 |
| POSTEMERGENCE | | |
| Morningglory | 1C | 0 |
| Cocklebur | 3C,8H | 2C,7H |
| Nutsedge | 0 | 0 |
| Crabgrass | 0 | 0 |
| Barnyardgrass | 0 | 0 |
| Wild Oats | 0 | 0 |
| Wheat | 0 | 0 |
| Corn | 0 | 2H |
| Soybean | 0 | 0 |
| Rice | 0 | 0 |
| Sorghum | 0 | 0 |
| Sugar Beets | 0 | 0 |
| Cotton | 0 | 2H |
| Cassia | 0 | 0 |
| PREEMERGENCE | | |
| Morningglory | 1C | 7G |
| Cocklebur | 2C,5H | 5H |
| Nutsedge | 0 | 0 |
| Crabgrass | 0 | 0 |
| Barnyardgrass | 0 | 0 |
| Wild Oats | 0 | 0 |
| Wheat | 0 | 0 |
| Corn | 0 | 0 |
| Soybean | 0 | 0 |
| Rice | 0 | 0 |
| Sorghum | 0 | 0 |
| Sugar Beets | 0 | 0 |
| Cotton | 0 | 0 |
| Cassia | 0 | - |

Table A (Continued)

| | Cmpd. 15 | Cmpd. 16 | Cmpd. 17 | Cmpd. 18 |
|---|---|---|---|---|
| Rate kg/ha | 0.05 | 0.05 | 0.05 | 0.05 |
| POST-EMERGENCE | | | | |
| Morningglory | 6C,9G | 3C,9G | 3C,6H | 10C |
| Cocklebur | 5C,8G | 5C,9G | 3C,5G | 10C |
| Sicklepod | 4C,8G | 3C,3H | 2C,3G | 9C |
| Nutsedge | 3C,8G | 2G | 2C,3G | 5G |
| Crabgrass | 0 | 0 | 2C,5G | 3C,8G |
| Barnyardgrass | 0 | 0 | 3C,6H | 4C,9H |
| Wild Oats | 0 | 0 | 3C,6G | 3C,8G |
| Wheat | 0 | 0 | 3C,8G | 6G |
| Corn | 3C,9H | 1H | 3C,9G | 2U,9G |
| Soybean | 3C,9G | 2C,8G | 3H | 9C |
| Rice | 2C,6G | 2G | 5C,9G | 6C,9G |
| Sorghum | 3C,9H | 2C,9H | 4C,9H | 6C,9H |
| Sugar beet | 9C | 4C,9G | 3C,4H | 10C |
| Cotton | 3C,8H | 3C,5G | 0 | 9C |
| PRE-EMERGENCE | | | | |
| Morningglory | 8G | 2C,3H | 0 | 9G |
| Cocklebur | 2C,5H | 2C,2H | - | 8H |
| Sicklepod | 1C,2G | 0 | 0 | 2C,7G |
| Nutsedge | 2G | 0 | 0 | 4G |
| Crabgrass | 1C | 0 | 2G | 2C,5G |
| Barnyardgrass | 0 | 0 | 1H | 4C,9H |
| Wild Oats | 0 | 0 | 2C,7G | 2C,8H |
| Wheat | 4G | 3G | 2C,8G | 3C,9H |
| Corn | 2C,8H | 0 | 3C,7H | 2U,9G |
| Soybean | 2C,8H | 0 | 0 | 3C,8H |
| Rice | 2C,7G | 2G | 3C,8H | 5C,9H |
| Sorghum | 3C,8H | 3C,8H | 3C,9G | 5C,9H |
| Sugar beet | 9C | 3C,8G | 0 | 9C |
| Cotton | 8H | 1C | 0 | 9G |

Table A (continued)

| | Cmpd. 19 | Cmpd. 20 | Cmpd. 21 | Cmpd. 22 |
|---|---|---|---|---|
| Rate kg/ha | 0.05 | 0.05 | 0.05 | 0.05 |
| POST-EMERGENCE | | | | |
| Morningglory | 8G | 10C | 2C,6G | 10C |
| Cocklebur | 5G | 3C,9G | 3G | 10C |
| Nutsedge | 2C,3G | 3G | 0 | 3G |
| Crabgrass | 3C,7G | 3G | 0 | 5G |
| Barnyardgrass | 9C | 3C,6H | 3C,8H | 3C,8H |
| Wild Oats | 9C | 2C,4G | 4C,8G | 2C |
| Wheat | 10C | 3G | 9C | 9G |
| Corn | 9C | 4C,9H | 4C,9G | 3C,9H |
| Soybean | 5C,9H | 5C,9G | 3C,8H | 9C |
| Rice | 9C | 9C | 9C | 9C |
| Sorghum | 9C | 5C,9H | 5C,9G | 5C,9H |
| Sugar beet | 3C,7H | 9C | 3C,7G | 5C,9G |
| Cotton | 3C,7H | 5C,9G | 3C,5G | 5C,9G |
| Sicklepod | 3C,3H | 4C,7H | 2C,5G | 9C |
| PRE-EMERGENCE | | | | |
| Morningglory | 3C,5H | 9G | 8G | 9G |
| Cocklebur | 0 | 5H | 2H | 8H |
| Nutsedge | 0 | 0 | 2C | 3G |
| Crabgrass | 0 | 0 | 0 | 2C,5G |
| Barnyardgrass | 3C,5G | 0 | 1H | 2C,6G |
| Wild Oats | 8G | 2C,3G | 5G | 2C,5G |
| Wheat | 2C,8H | 2G | 2C,9H | 2C,7G |
| Corn | 3C,8H | 3C,3G | 2C,8H | 4C,9H |
| Soybean | 1C | 3C,5H | 2C,2H | 4C,6H |
| Rice | 5C,9H | 7H | 5C,9H | 5C,9H |
| Sorghum | 4C,9H | 3C,7H | 5C,9H | 4C,9G |
| Sugar beet | 8G | 9C | 5G | 9C |
| Cotton | 2G | 5G | 5G | 3C,7G |
| Sicklepod | 2C | 2C,2H | 1C | 2C,4G |

Table A (continued)

| | Cmpd. 23 | Cmpd. 24 | Cmpd. 25 | Cmpd. 26 |
|---|---|---|---|---|
| Rate kg/ha | 0.05 | 0.05 | 0.05 | 0.05 |
| POST-EMERGENCE | | | | |
| Morningglory | 3C,9G | 3G | 9C | 10C |
| Cocklebur | 2G | 3G | 2C | 5C,9G |
| Nutsedge | 5G | 0 | 2C,7G | 2C,6G |
| Crabgrass | 0 | 0 | 2G | 2C |
| Barnyardgrass | 0 | 0 | 0 | 0 |
| Wild Oats | 0 | 0 | 0 | 0 |
| Wheat | 0 | 0 | 0 | 0 |
| Corn | 0 | 0 | 1H | 3C,8H |
| Soybean | 5C,9G | 3C,8G | 5C,9G | 5C,9G |
| Rice | 0 | 0 | 0 | 0 |
| Sorghum | 0 | 0 | 3G | 3C,8H |
| Sugar beet | 3C,6G | 0 | 5C,9G | 5C,9G |
| Cotton | 9C | 2G | 4C,9G | 4C,9G |
| Sicklepod | 2C,3G | 1C | 3C,8G | 4C,6G |
| PRE-EMERGENCE | | | | |
| Morningglory | 8G | 3G | 9G | 9G |
| Cocklebur | 2H | 3G | - | 8H |
| Nutsedge | 3G | 0 | 5G | 0 |
| Crabgrass | 4G | 3G | 0 | 0 |
| Barnyardgrass | 0 | 0 | 3G | 3G |
| Wild Oats | 0 | 0 | 3G | 5G |
| Wheat | 0 | 0 | 4G | 8G |
| Corn | 0 | 3G | 2C,7G | 9G |
| Soybean | 1H | 0 | 2H | 3C,7G |
| Rice | 0 | 0 | 2G | 2G |
| Sorghum | 0 | 2G | 5G | 9G |
| Sugar beet | 5G | 2G | 5C,9G | 5C,9G |
| Cotton | 2G | 0 | 8G | 8G |
| Sicklepod | 5G | 4G | 2C,7G | 2C |

Test B

Postemergence

Two round pans (25 cm diameter by 12.5 cm deep) were filled with Woodstown sandy loam soil. One pan was planted with blackgrass (Alpecurus myosuroides), sugar beets, nutsedge (Cyperus rotundus) tubers, crabgrass (Digitaria sanguinalis), sicklepod (Cassia obtusifolia), teaweed (Sida spinosa), jimsonweed (Datura stramonium), velvetleaf (Abutilon theophrasti), oil seed rape (Brassica napus), and giant foxtail (Setaria faberii). The other pan was planted with wheat, cotton, rice, corn, soybean, wild oats (Avena fatua), cocklebur (Xantium pensylvanicum), morningglory (Ipomoea hederacea), johnsongrass (Sorghum halepense) and barnyardgrass (Echinochloa crusgalli). The plants were grown for approximately fourteen days, then sprayed post-emergence with the chemicals dissolved in a non-phytotoxic solvent.

Preemergence

Two round pans (25 cm diameter by 12.5 cm deep) were filled with Woodstown sandy loam soil. One pan was planted with blackgrass, sugar beets, nutsedge, crabgrass, sicklepod, teaweed, jimsonweed, velvetleaf, oil seed rape, and giant foxtail. The other pan was planted with wheat, cotton, rice, corn, soybeans, wild oats, cocklebur, morningglory, johnsongrass, and barnyardgrass. The two pans were sprayed pre-emergence with the chemicals dissolved in a non-phytotoxic solvent.

Treated plants and controls were maintained in the greenhouse for 28 days, then all treated plants were compared to controls and visually rated for plant response utilizing the rating system as described for Test A.

Response ratings are contained in Table B. The data show the potential utility of several of the compounds tested for selective weed control in crops such as wheat, soybeans and cotton.

Table B
PRE-EMERGENCE ON
WOODSTOWN SANDY LOAM

Compound 15

| POST-EMERGENCE Rate g/ha | 62 | 16 | 4 |
|---|---|---|---|
| Corn | 3G | 0 | 0 |
| Wheat | 0 | 0 | 0 |
| Rice | 5G | 3G | 0 |
| Soybean | 9G | 6G | 3G |
| Cotton | 7G | 3G | 0 |
| Sugar beet | 7G | 3G | 0 |
| Rape | - | - | - |
| Crabgrass | 0 | 0 | 0 |
| Johnsongrass | 9G | 4G | 0 |
| Blackgrass | 0 | 0 | 0 |
| Barnyardgrass | 3G | 0 | 0 |
| Nutsedge | 5G | 2G | 0 |
| Giant Foxtail | 6G | 0 | 0 |
| Wild Oats | 0 | 0 | 0 |
| Cocklebur | 8G | 3G | 0 |
| Morningglory | 9G | 8G | 3G |
| Teaweed | 0 | 0 | 0 |
| Sicklepod | 3G | 0 | 0 |
| Jimsonweed | 8G | 6G | 2G |
| Velvetleaf | 9G | 7G | 3G |

| PRE-EMERGENCE Rate g/ha | 250 | 62 |
|---|---|---|
| Corn | 2G | 0 |
| Wheat | 0 | 0 |
| Rice | 7G | 4G |
| Soybean | 3G | 0 |
| Cotton | 7G | 2G |
| Sugar beet | 9G | 3G |
| Rape | - | - |
| Crabgrass | 9G | 5G |
| Johnsongrass | 8G | 4G |
| Blackgrass | 8G | 3G |
| Barnyardgrass | 4G | 2G |
| Nutsedge | 8G | 5G |
| Giant Foxtail | 6G | 2G |
| Wild Oats | 0 | 0 |
| Cocklebur | 8G | 0 |
| Morningglory | 0 | 0 |
| Teaweed | 9G | 5G |
| Sicklepod | 10G | 6G |
| Jimsonweed | 8G | 6G |
| Velvetleaf | 8G | 4G |

Table B (Continued)

Compound 16

| POST-EMERGENCE Rate g/ha | 62 | 16 | 4 |
| --- | --- | --- | --- |
| Corn | 0 | 0 | 0 |
| Wheat | 0 | 0 | 0 |
| Rice | 6G | 4G | 0 |
| Soybean | 9G | 8G | 5G |
| Cotton | 5G | 3G | 0 |
| Sugar beet | 9G | 5G | 0 |
| Rape | - | - | - |
| Crabgrass | 3G | 0 | 0 |
| Johnsongrass | 5G | 2G | 0 |
| Blackgrass | 0 | 0 | 0 |
| Barnyardgrass | 3G | 0 | 0 |
| Nutsedge | 7G | 3G | 0 |
| Giant Foxtail | 7G | 4G | 0 |
| Wild Oats | 0 | 0 | 0 |
| Cocklebur | 9G | 5G | 2G |
| Morningglory | 9G | 6G | 3G |
| Teaweed | 4G | 0 | 0 |
| Sicklepod | 6G | 3G | 0 |
| Jimsonweed | 8G | 4G | 2G |
| Velvetleaf | 9G | 5G | 0 |

| PRE-EMERGENCE Rate g/ha | 250 | 62 | 16 |
| --- | --- | --- | --- |
| Corn | 0 | 0 | 0 |
| Wheat | 2G | 0 | 0 |
| Rice | 7G | 6G | 6G |
| Soybean | 2C | 0 | 0 |
| Cotton | 0 | 0 | 0 |
| Sugar beet | 10G | 8G | 0 |
| Rape | - | - | - |
| Crabgrass | 9G | 8G | 6G |
| Johnsongrass | 8G | 5G | 0 |
| Blackgrass | 8G | 4G | 0 |
| Barnyardgrass | 3G | 0 | 0 |
| Nutsedge | 8G | 5G | 2G |
| Giant Foxtail | 4G | 2G | 0 |
| Wild Oats | 0 | 0 | 0 |
| Cocklebur | 3G | 0 | 0 |
| Morningglory | 3G | 0 | 0 |
| Teaweed | 9G | 5G | 0 |
| Sicklepod | 8G | 8G | 5G |
| Jimsonweed | 9G | 6G | 0 |
| Velvetleaf | 8G | 3G | 0 |

Table B (Continued)

Compound 18[1]

| POST-EMERGENCE Rate g/ha | 62 | 16 | 4 | 1 |
|---|---|---|---|---|
| Corn | 100,70 | 100,60 | 90,30 | 80 |
| Wheat | 0,0 | 0,0 | 0,0 | 0,0 |
| Rice | 80,0 | 70,0 | 30,0 | 0,0 |
| Soybean | 100,90 | 100,90 | 100,80 | 70,20 |
| Cotton | 100,50 | 100,20 | 40,0 | 0,0 |
| Sugar beet | 100,90 | 40,90 | 0,40 | 0,0 |
| Rape | 100,100 | 100,90 | 100,90 | 50,40 |
| Crabgrass | 20,0 | 0,0 | 0,0 | 0,0 |
| Johnsongrass | 100,90 | 100,0 | 60,0 | 20 |
| Blackgrass | 100,70 | 70,0 | 30,0 | 0,0 |
| Barnyardgrass | 70,50 | 40,0 | 0,0 | 0,0 |
| Nutsedge | 40,- | 30,0 | 0,0 | 0,0 |
| Giant Foxtail | 50,0 | 0,0 | 0,0 | 0,0 |
| Wild Oats | 30,0 | 0,0 | 0,0 | 0,0 |
| Cocklebur | 100,90 | 60,70 | 30,0 | 0,0 |
| Morningglory | 100,90 | 40,90 | 0,90 | 0,50 |
| Teaweed | 80,80 | 50,80 | 30,0 | 0,0 |
| Sicklepod | 100,100 | 90,90 | 30,70 | 0,0 |
| Jimsonweed | 100,90 | 90,90 | 60,70 | 30,30 |
| Velvetleaf | 100,100 | 100,90 | 100,90 | 30,50 |

| PRE-EMERGENCE Rate g/ha | 250 | 62 | 16 |
|---|---|---|---|
| Corn | 90,100 | 40,30 | 0,0 |
| Wheat | 30,30 | 0,0 | 0,0 |
| Rice | 100 | 90,100 | 40,60 |
| Soybean | 80,100 | 60,50 | 20,0 |
| Cotton | 40,60 | 0,50 | 0,0 |
| Sugar beet | 90,100 | 30,100 | 0,50 |
| Rape | 100 | 80,50 | 30,0 |
| Crabgrass | 90,60 | 0,30 | 0,0 |
| Johnsongrass | 90,100 | 90,90 | 80,60 |
| Blackgrass | 90,100 | 70,80 | 0,40 |
| Barnyardgrass | 90,100 | 70,60 | 20,20 |
| Nutsedge | 0,0 | 0,0 | 0,0 |
| Giant Foxtail | 100 | 70,70 | 0,0 |
| Wild Oats | 60,40 | 30,0 | 0,0 |
| Cocklebur | 80,90 | 70,60 | 0,40 |
| Morningglory | 90,80 | 20,30 | 0,0 |
| Teaweed | 90,90 | 50,50 | 0,0 |
| Sicklepod | 70,50 | 30,20 | 0,0 |
| Jimsonweed | 90,100 | 70,90 | 20,30 |
| Velvetleaf | 90,100 | 70,60 | 20,30 |

[1] Note: there are two sets of data for each rate tested.

Table B (Continued)

Compound 19

| POST-EMERGENCE Rate g/ha | 62 | 16 | 4 |
|---|---|---|---|
| Corn | 80 | 50 | 0 |
| Wheat | 0 | 0 | 0 |
| Rice | 0 | 0 | 0 |
| Soybean | 90 | 80 | 60 |
| Cotton | 60 | 0 | 0 |
| Sugar beet | 70 | 60 | 30 |
| Rape | 100 | 90 | 60 |
| Crabgrass | 0 | 0 | 0 |
| Johnsongrass | 0 | 0 | 0 |
| Blackgrass | 80 | 0 | 0 |
| Barnyardgrass | 0 | 0 | 0 |
| Nutsedge | 0 | 0 | 0 |
| Giant Foxtail | 0 | 0 | 0 |
| Wild Oats | 0 | 0 | 0 |
| Cocklebur | 100 | 90 | 70 |
| Morningglory | 100 | 90 | 80 |
| Teaweed | 50 | 20 | 0 |
| Sicklepod | 0 | 0 | 0 |
| Jimsonweed | 90 | 80 | 0 |
| Velvetleaf | 80 | 0 | 0 |

| PRE-EMERGENCE Rate g/ha | 250 | 62 |
|---|---|---|
| Corn | 80 | 0 |
| Wheat | 0 | 0 |
| Rice | 80 | 30 |
| Soybean | 70 | 20 |
| Cotton | 20 | 0 |
| Sugar beet | 80 | 20 |
| Rape | 90 | 30 |
| Crabgrass | 0 | 0 |
| Johnsongrass | 90 | 80 |
| Blackgrass | 90 | 80 |
| Barnyardgrass | 70 | 0 |
| Nutsedge | 70 | 20 |
| Giant Foxtail | 70 | 0 |
| Wild Oats | 0 | 0 |
| Cocklebur | 90 | 0 |
| Morningglory | 90 | 0 |
| Teaweed | 60 | 20 |
| Sicklepod | 0 | 0 |
| Jimsonweed | 90 | 50 |
| Velvetleaf | 50 | 0 |

Table B (Continued)

Compound 20

| POST-EMERGENCE | | |
|---|---|---|
| Rate g/ha | 62 | 16 |
| Corn | 70 | 40 |
| Wheat | 50 | 0 |
| Rice | 70 | 30 |
| Soybean | 90 | 90 |
| Cotton | 0 | 0 |
| Sugar beet | 80 | 70 |
| Rape | 90 | 70 |
| Crabgrass | 20 | 0 |
| Johnsongrass | 20 | 0 |
| Blackgrass | 70 | 0 |
| Barnyardgrass | 0 | 0 |
| Nutsedge | 0 | 0 |
| Giant Foxtail | 0 | 0 |
| Wild Oats | 50 | 0 |
| Cocklebur | 0 | 0 |
| Morningglory | 90 | 60 |
| Teaweed | 30 | 0 |
| Sicklepod | 50 | 0 |
| Jimsonweed | 30 | 0 |
| Velvetleaf | 70 | 0 |

| PRE-EMERGENCE | | |
|---|---|---|
| Rate g/ha | 250 | 62 |
| Corn | 40 | 0 |
| Wheat | 0 | 0 |
| Rice | 90 | 70 |
| Soybean | 40 | 0 |
| Cotton | 20 | 0 |
| Sugar beet | 80 | 30 |
| Rape | 60 | 20 |
| Crabgrass | 20 | 0 |
| Johnsongrass | 70 | 30 |
| Blackgrass | 90 | 60 |
| Barnyardgrass | 0 | 0 |
| Nutsedge | 30 | 0 |
| Giant Foxtail | 20 | 0 |
| Wild Oats | 0 | 0 |
| Cocklebur | 0 | 0 |
| Morningglory | 0 | 0 |
| Teaweed | 60 | 0 |
| Sicklepod | 50 | 0 |
| Jimsonweed | 50 | 30 |
| Velvetleaf | 50 | 30 |

Table B (Continued)

Compound 21

| POST-EMERGENCE Rate g/ha | 62 | 16 | 4 |
|---|---|---|---|
| Corn | 100 | 90 | 60 |
| Wheat | 70 | 0 | 0 |
| Rice | 90 | 30 | 0 |
| Soybean | 100 | 90 | 90 |
| Cotton | 90 | 20 | 0 |
| Sugar beet | 70 | 20 | 0 |
| Rape | 100 | 90 | 70 |
| Crabgrass | 60 | 0 | 0 |
| Johnsongrass | 90 | 90 | 70 |
| Blackgrass | 90 | 80 | 20 |
| Barnyardgrass | 60 | 20 | 0 |
| Nutsedge | 0 | 0 | 0 |
| Giant Foxtail | 40 | 0 | 0 |
| Wild Oats | 40 | 0 | 0 |
| Cocklebur | 80 | 90 | 30 |
| Morningglory | 100 | 100 | 90 |
| Teaweed | 90 | 80 | 20 |
| Sicklepod | 90 | 60 | 20 |
| Jimsonweed | 80 | 50 | 0 |
| Velvetleaf | 100 | 90 | 40 |

| PRE-EMERGENCE Rate g/ha | 250 | 62 | 16 |
|---|---|---|---|
| Corn | 50 | 0 | 0 |
| Wheat | 40 | 0 | 0 |
| Rice | 100 | 90 | 50 |
| Soybean | 70 | 0 | 0 |
| Cotton | 30 | 0 | 0 |
| Sugar beet | 60 | 0 | 0 |
| Rape | 90 | 20 | 0 |
| Crabgrass | 60 | 30 | 0 |
| Johnsongrass | 90 | 90 | 30 |
| Blackgrass | 90 | 80 | 30 |
| Barnyardgrass | 90 | 50 | 0 |
| Nutsedge | 20 | 0 | 0 |
| Giant Foxtail | 90 | 50 | 0 |
| Wild Oats | 80 | 20 | 0 |
| Cocklebur | 60 | 0 | 0 |
| Morningglory | 0 | 0 | 0 |
| Teaweed | 90 | 50 | 20 |
| Sicklepod | 70 | 20 | 0 |
| Jimsonweed | 90 | 40 | 0 |
| Velvetleaf | 80 | 30 | 0 |

## Claims

1. A compound of the formula:

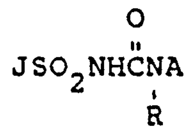

I

wherein J is

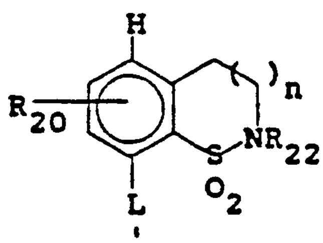, 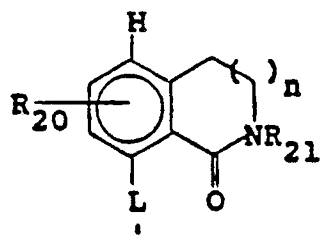, 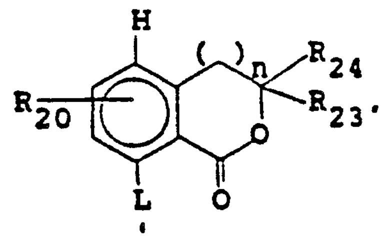,

$J_{12}$ $J_{13}$ $J_{14}$

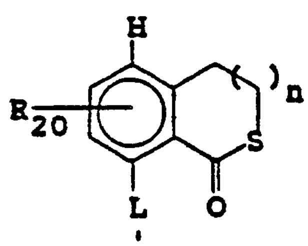, 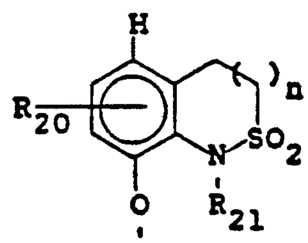, 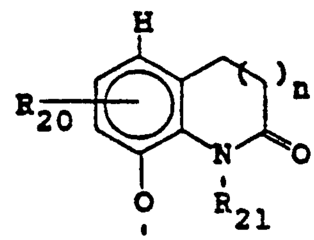,

$J_{15}$ $J_{16}$ $J_{17}$

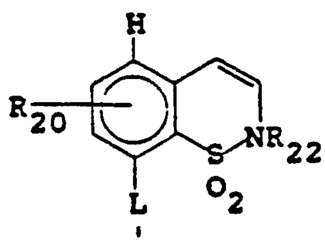, 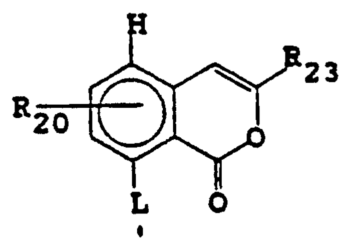, 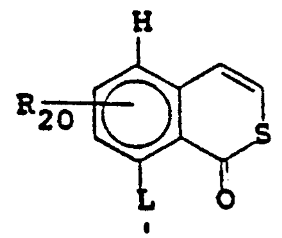,

$J_{18}$, $J_{19}$ $J_{20}$

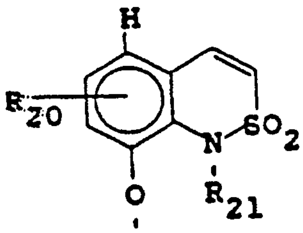,
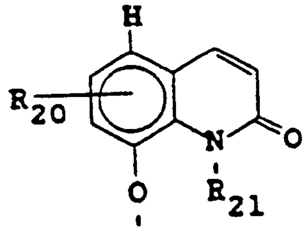,
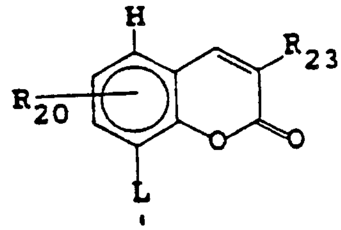,

J21 J22 J23

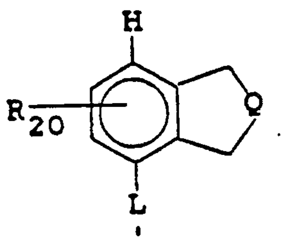,
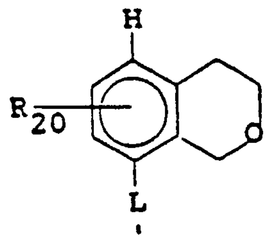,
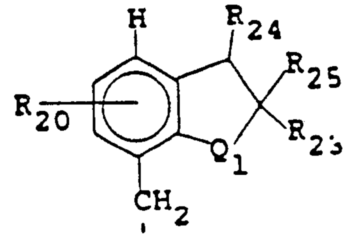,

J24 J25 J26

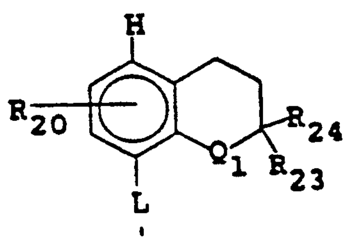,
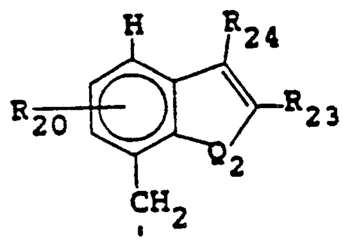,
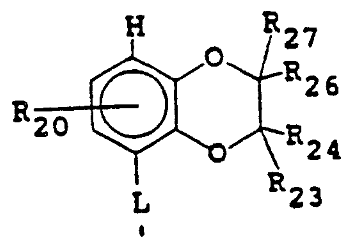,

J27 J28 J29

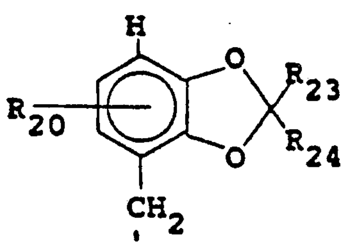,
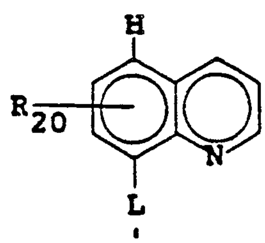

J30 J31

n is 0 or 1;
L is $CH_2$ or 0;
Q is $CH_2$ or 0;
$Q_1$ is 0, S or $SO_2$;
$Q_2$ is 0 or S;

R is H or $CH_3$;
$R_{20}$ is H, F, Cl, Br, $CH_3$ or $OCH_3$;
$R_{21}$ is H or $C_1$-$C_3$ alkyl;
$R_{22}$ is H, OH, $C_1$-$C_6$ alkyl, $C_1$-$C_6$ haloalkyl, $C_1$-$C_6$ alkoxylalkyl, $C_3$-$C_6$ alkenyl, $C_3$-$C_6$ alkynyl, $C_1$-$C_6$ alkoxy, phenyl, $C_3$-$C_6$ cycloalkyl, $C_4$-$C_7$ cycloalkylalkyl, $C_1$-$C_6$ alkylcarbonyl, $C_1$-$C_6$ alkoxycarbonyl, benzyl, $C_3$-$C_4$ haloalkenyl, $C_3$-$C_6$ haloalkynyl, $C_3$-$C_6$ alkylcarbonylalkyl, $C_3$-$C_6$ alkoxycarbonylalkyl or $C_1$-$C_4$ cyanoalkyl;
$R_{23}$ is H or $CH_3$;
$R_{24}$ is H or $CH_3$;
$R_{25}$ is H, $CH_3$ or $C_2H_5$;
$R_{26}$ is H or $CH_3$;
$R_{27}$ is H or $CH_3$;

A is 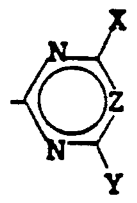 , 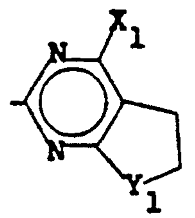 , 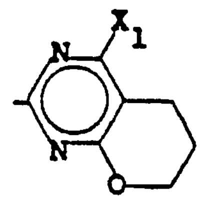 , 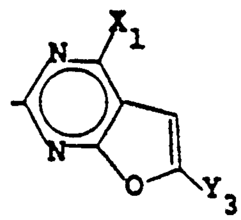 ,

A-1 A-2 A-3 A-4

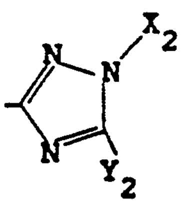 , 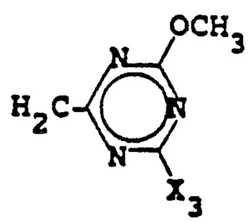 ;

A-5 A-6

X is $CH_3$, $OCH_3$, Cl, Br, $OCH_2CF_3$ or $OCHF_2$;
Y is $C_1$-$C_3$ alkyl, $CH_2F$, cyclopropyl, C≡CH, $OCH_3$, $OC_2H_5$, $NH_2$, $NHCH_3$, $N(CH_3)_2$, $OCH_2CH_2F$, $CF_3$, $SCH_3$, $OCH_2CH=CH_2$, $OCH_2C≡CH$, $OCH_2CH_2OCH_3$, or $OCF_2H$;
Z is CH or N;
$X_1$ is $CH_3$, $OCH_3$, $OC_2H_5$ or $OCF_2H$;
$Y_1$ is O or $CH_2$;
$X_2$ is $CH_3$, $C_2H_5$ or $CH_2CF_3$;
$Y_2$ is $OCH_3$, $OC_2H_5$, $SCH_3$, $SC_2H_5$, $OCF_2H$, $SCF_2H$, $CH_3$ or $CH_2CH_3$;
$X_3$ is $CH_3$ or $OCH_3$;
$Y_3$ is H or $CH_3$; and agriculturally suitable salts thereof; provided that

1) when X is Cl or Br, then Z is CH and Y is $OCH_3$, $OC_2H_5$, $NHCH_3$, $N(CH_3)_2$ or $OCF_2H$;
2) when X or Y is $OCF_2H$, then Z is CH.

2. The compound of Claim 1 where R is H and A is A-1.
3. The compound of Claim 2 where Y is $CH_3$, $C_2H_5$,'$OCH_3$, $OC_2H_5$, $CH_2F$ or $CF_3$; and L is $CH_2$.
4. The compound of Claim 3 where J is $J_{12}$, $J_{14}$, $J_{18}$, $J_{19}$, $J_{26}$ or $J_{27}$.
5. The compound of Claim 4 where $R_{20}$ is H and X is $CH_3$ or $OCH_3$.
6. The compound of Claim 1 which is 1,3-dihydro-N-[(4,6-dimethoxypyrimidin-2-yl)aminocarbonyl]-1-oxo-benzo[C]furan-7-methanesulfonamide, or an agriculturally suitable salt thereof.
7. The compound of Claim 1 which is 1,3-dihydro-N-[(4-methoxy-6-methyl-1,3,5-triazin-2-yl)aminocarbonyl]-1-oxo-benzo[C]furan-7-methanesulfonamide, or an agriculturally suitable salt thereof.
8. The compound of Claim 1 which is N-[(4,6-dimethoxypyrimidin-2-yl)aminocarbonyl]-2,3-dihydro-2-methylbenzo[B]thiophene-7-methanesulfonamide-1,1-dioxide, or an agriculturally suitable salt thereof.
9. The compound of Claim 1 which is N-[(4-methoxy-6-methyl-1,3,5-triazin-2-yl)aminocarbonyl]-2-methylbenzo[B]thiophene-7-methanesulfonamide-1,1-dioxide, or an agriculturally suitable salt thereof.

10. A composition suitable for controlling the growth of undesired vegetation which comprises an effective amount of a compound of any of Claims 1 to 9 and at least one of the following: surfactant, solid or liquid diluent.

11. A method for controlling the growth of undesired vegetation which comprises applying to the locus to be protected an effective amount of a compound of any of Claims 1 to 9.

12. A method for regulating the growth of plants which comprises applying to the locus of such plants an effective but substantially non-phytotoxic amount of a plant growth regulant selected from compounds of any of claims 1 to 9.

13. A process for the production of a compound of claim 1 which comprises:

(a) reacting a sulfonyl isocyanate of formula

$JSO_2NCO$ (II)

with an appropriate heterocyclic amine of formula

$HN(R)\text{-}A$ (III)

wherein J, R and A are as defined in claim 1, with the provisos that $R_{21}$ is $C_1$-$C_3$ alkyl and $R_{22}$ is not H or OH; or

(b) reacting a sulfonamide of formula

$JSO_2NH_2$ (IV)

wherein J is selected from $J_{12}$, $J_{13}$, $J_{18}$ or $J_{24}$-$J_{31}$ and L is $-CH_2-$; with an appropriate methyl carbamate of formula

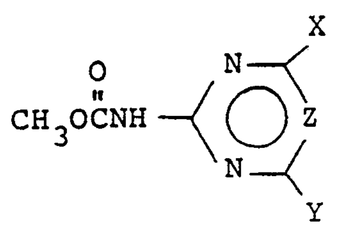

(V)

wherein X, Y and Z are as defined in claim 1; or

(c) reacting a sulfonyl carbamate of formula

$JSO_2NHC(O)OC_6H_5$ (VI)

where J is as defined in claim 1, other than $J_{16}$, $J_{17}$, $J_{21}$ or $J_{22}$ and where L is $-CH_2-$, with an appropriate heterocyclic amine of formula (III) as defined above; or

(d) reacting a sulfonamide of formula (III) wherein J is other than $J_{16}$, $J_{17}$, $J_{21}$ and $J_{22}$ and L is $-CH_2-$, with an appropriate heterocyclic phenyl carbamate of formula

$C_6H_5OC(O)NHA$ (VII)

wherein A is as defined in claim 1; or

(e) reacting a phenol of formula

J-H (IX)

wherein J is selected from $J_{12}$-$J_{25}$, $J_{27}$, $J_{29}$, or $J_{31}$ as defined in claim 1 and L is 0, with a chlorosulfonylurea of formula

$ClSO_2NHC(O)N(R)\text{-}A$ (VIII)

wherein A and R are as defined in claim 1.

Claims for the following Contracting State: AT

1. A process for the preparation of a compound of the formula:

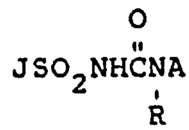

I

wherein J is

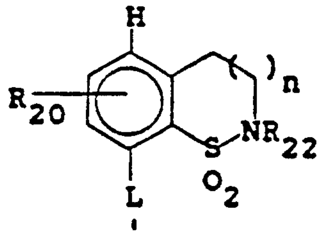 , 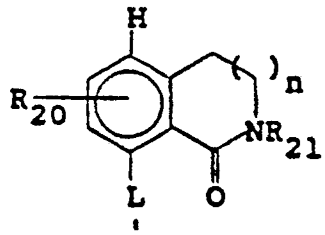 , 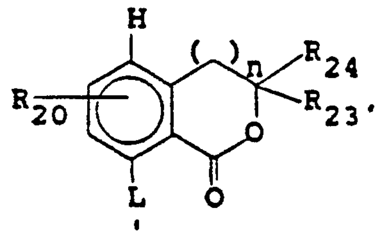

J12 J13 J14

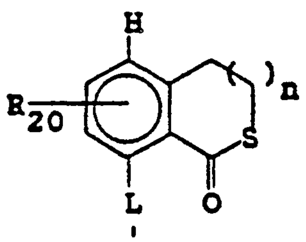 , 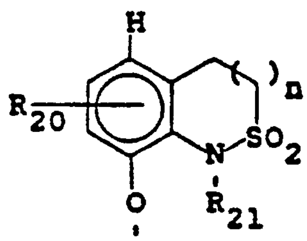 , 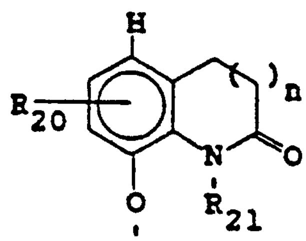 .

J15 J16 J17

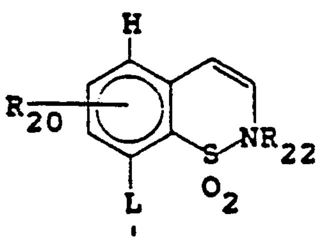 , 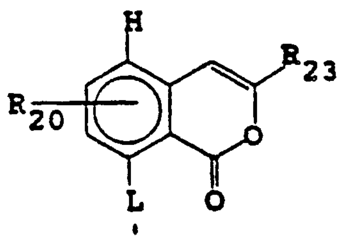 , 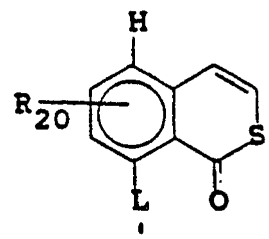 .

J18 J19 J20

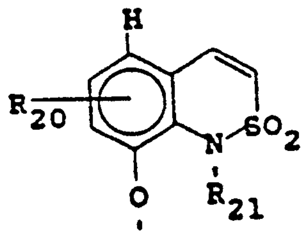 ,

J21

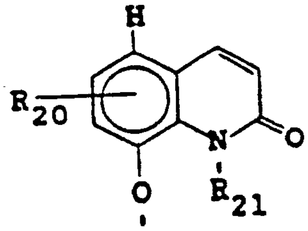 ,

J22

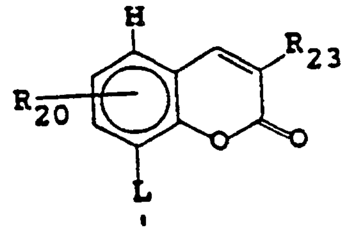 ,

J23

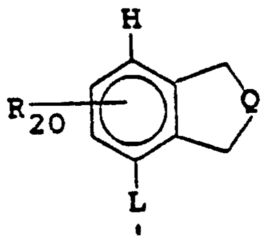 ,

J24

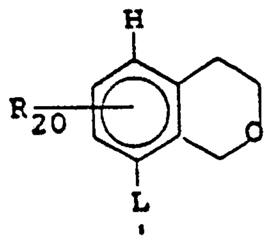 ,

J25

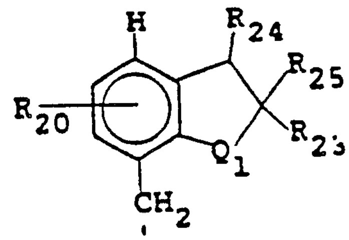 ,

J26

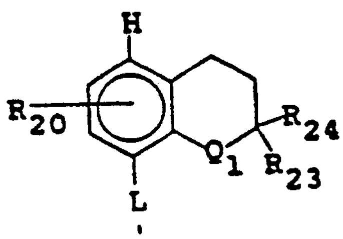 ,

J27

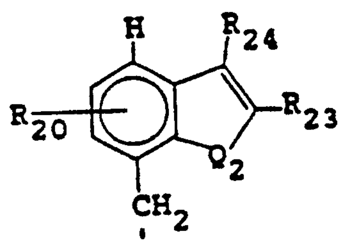 ,

J28

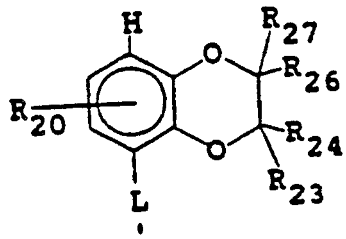 ,

J29

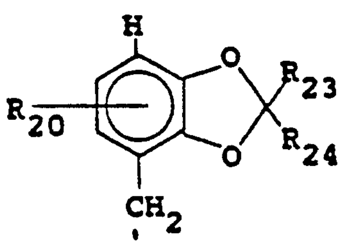 ,

J30

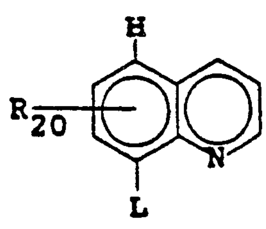

J31

n is O or 1;
L is $CH_2$ or O;
Q is $CH_2$ or O;
$Q_1$ is O, S or $SO_2$;
$Q_2$ is O or S;

R is H or $CH_3$;
$R_{20}$ is H, F, Cl, Br, $CH_3$ or $OCH_3$;
$R_{21}$ is H or $C_1$-$C_3$ alkyl;
$R_{22}$ is H, OH, $C_1$-$C_6$ alkyl, $C_1$-$C_6$ haloalkyl, $C_1$-$C_6$ alkoxylalkyl, $C_3$-$C_6$ alkenyl, $C_3$-$C_6$ alkynyl, $C_1$-$C_6$ alkoxy, phenyl, $C_3$-$C_6$ cycloalkyl, $C_4$-$C_7$ cycloalkylalkyl, $C_1$-$C_6$ alkylcarbonyl, $C_1$-$C_6$ alkoxycarbonyl, benzyl, $C_3$-$C_4$ haloalkenyl, $C_3$-$C_6$ haloalkynyl, $C_3$-$C_6$ alkylcarbonylalkyl, $C_3$-$C_6$ alkoxycarbonylalkyl or $C_1$-$C_4$ cyanoalkyl;
$R_{23}$ is H or $CH_3$;
$R_{24}$ is H or $CH_3$;
$R_{25}$ is H, $CH_3$ or $C_2H_5$;
$R_{26}$ is H or $CH_3$;
$R_{27}$ is H or $CH_3$;

A is 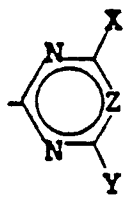 , 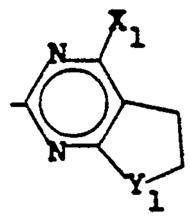 , 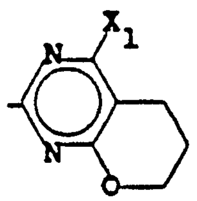 , 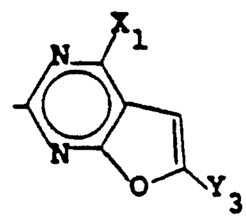 ,

A-1 A-2 A-3 A-4

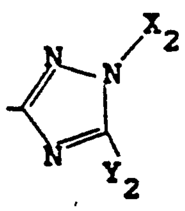 , 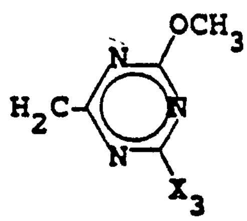 ;

A-5 A-6

X is $CH_3$, $OCH_3$, Cl, Br, $OCH_2CF_3$ or $OCHF_2$;
Y is $C_1$-$C_3$ alkyl, $CH_2F$, cyclopropyl, $C{\equiv}CH$, $OCH_3$, $OC_2H_5$, $NH_2$, $NHCH_3$, $N(CH_3)_2$, $OCH_2CH_2F$, $CF_3$, $SCH_3$, $OCH_2CH=CH_2$, $OCH_2C{\equiv}CH$, $OCH_2CH_2OCH_3$, or $OCF_2H$;
Z is CH or N;
$X_1$ is $CH_3$, $OCH_3$, $OC_2H_5$ or $OCF_2H$;
$Y_1$ is O or $CH_2$;
$X_2$ is $CH_3$, $C_2H_5$ or $CH_2CF_3$;
$Y_2$ is $OCH_3$, $OC_2H_5$, $SCH_3$, $SC_2H_5$, $OCF_2H$, $SCF_2H$, $CH_3$ or $CH_2CH_3$;
$X_3$ is $CH_3$ or $OCH_3$;
$Y_3$ is H or $CH_3$; or an agriculturally suitable salt thereof; provided that
1) when X is Cl or Br, then Z is CH and Y is $OCH_3$, $OC_2H_5$, $NHCH_3$, $N(CH_3)_2$ or $OCF_2H$;
2) when X or Y is $OCF_2H$, then Z is CH;
which comprises:
(a) reacting a sulfonyl isocyanate of formula
$JSO_2NCO$ (II)
with an appropriate heterocyclic amine of formula
H N-A (III)
R
wherein J, R and A are as defined above with the provisos that $R_{21}$ is $C_1$-$C_3$ alkyl and $R_{22}$ is not H or OH;
or
(b) reacting a sulfonamide of formula
$JSO_2NH_2$ (IV)
wherein J is selected from $J_{12}$, $J_{13}$, $J_{18}$ or $J_{24\text{-}31}$ and L is $-CH_2-$; with an appropriate methyl carbamate of formula

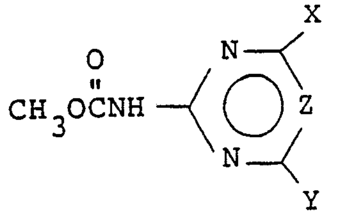

(V)

wherein X, Y and Z are as defined above; or

(c) reacting a sulfonyl carbamate of formula

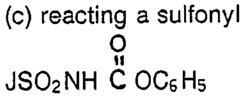

(VI)

where J is as defined above, other than $J_{16}$, $J_{17}$, $J_{21}$ or $J_{22}$ and where L is $-CH_2-$,

with an appropriate heterocyclic amine of formula (III) as defined above; or

(d) reacting a sulfonamide of formula (III) wherein J is other than $J_{16}$, $J_{17}$, $J_{21}$ and $J_{22}$ and L is $-CH_2-$, with an appropriate heterocyclic phenyl carbamate of formula

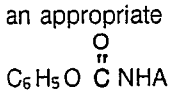

(VII)

wherein A is as defined above; or

(e) reacting a phenol of formula

J-H (IX)

wherein J is selected from $J_{12}$-$J_{25}$, $J_{27}$, $J_{29}$ or $J_{31}$ as defined in claim 1 and L is 0, with a chlorosulfonylurea of formula

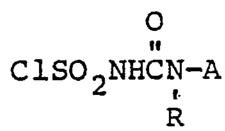

(VIII)

wherein A and R are as defined above.

2. The process of Claim 1 where R is H and A is A-1.

3. The process of Claim 2 where Y is $CH_3$, $C_2H_5$, $OCH_3$, $OC_2H_5$, $CH_2F$ or $CF_3$; and L is $CH_2$.

4. The compound of Claim 3 where J is $J_{12}$, $J_{14}$, $J_{18}$, $J_{19}$, $J_{26}$ or $J_{27}$.

5. The process of Claim 4 where $R_{20}$ is H and X is $CH_3$ or $OCH_3$.

6. The process of claim 1 wherein the product is 1,3-dihydro-N-[(4,6-dimethoxypyrimidin-2-yl)-aminocarbonyl]-1-oxo-benzo[C]furan-7-methanesulfonamide, or an agriculturally suitable salt thereof;
1,3-dihydro-N-[(4-methoxy-6-methyl-1,3,5-triazin-2-yl)aminocarbonyl]-1-oxo-benzo[C]furan-7-methanesulfonamide, or an agriculturally suitable salt thereof;
N-[(4,6-dimethoxypyrimidin-2-yl)aminocarbonyl]-2,3-dihydro-2-methylbenzo[B]thiophene-7-methanesulfonamide-1,1-dioxide, or an agriculturally suitable salt thereof; or
N-[(4-methoxy-6-methyl-1,3,5-triazin-2-yl)aminocarbonyl]-2-methylbenzo[B]thiophene-7-methanesulfonamide-1,1-dioxide, or an agriculturally suitable salt thereof.

7. A composition suitable for controlling the growth of undesired vegetation which comprises an effective amount of a compound of formula (I) as defined in any of Claims 1 to 6 and at least one of the following: surfactant, solid or liquid diluent.

8. A method for controlling the growth of undesired vegetation which comprises applying to the locus to be protected an effective amount of a compound of formula (I) as defined in any of Claims 1 to 6.

9. A method of regulating the growth of plants which comprises applying to the locus of such plants an effective but substantially non-phytotoxic amount of a plant growth regulant selected from compounds of formula (I) as defined in any of Claims 1 to 6.